# EUROPEAN PATENT SPECIFICATION

(11) **EP 3 277 680 B1**
(45) Date of publication and mention of the grant of the patent: **27.02.2019**
(21) Application number: 16711288.7
(22) Date of filing: 23.03.2016
(51) Int. Cl.: C07D 471/04, A01N 43/00, C07D 491/048, C07D 495/04, C07D 498/04, C07D 513/04, A01N 43/42, A01N 43/90, C07D 221/02, C07D 401/04, C07D 213/04

(54) **QUINOLINE COMPOUNDS**
CHINOLINVERBINDUNGEN
COMPOSÉS DE QUINOLÉINE

(30) Priority: 02.04.2015 EP 15162483
(43) Date of publication of application: 07.02.2018
(73) Proprietor: BASF SE, 67056 Ludwigshafen am Rhein (DE)
(72) Inventor: GRAMMENOS, Wassilios, 67071 Ludwigshafen (DE); BOUDET, Nadege, 69493 Hirschberg (DE); MUELLER, Bernd, 67227 Frankenthal (DE); WOLF, Antje, 67071 Ludwigshafen (DE); ESCRIBANO CUESTA, Ana, 68161 Mannheim (DE); CAMBEIS, Erica, 67258 Hessheim (DE); LOHMANN, Jan Klaas, 67245 Lambsheim (DE); GROTE, Thomas, 67157 Wachenheim (DE); KRETSCHMER, Manuel, New York, New York 10014 (US); HADEN, Egon, 67346 Speyer (DE); FEHR, Marcus, 67346 Speyer (DE)
(74) Representative: BASF IP Association
(86) International application number: PCT/EP2016/056317
(87) International publication number: WO 2016/156129

(56) References cited:
- EP-A1- 1 736 471
- EP-A1- 2 103 214

## Description

The present invention relates to quinoline compounds and the N-oxides and the salts thereof for combating phytopathogenic fungi, and to the use and methods for combating phytopathogenic fungi and to seeds coated with at least one such compound. The invention also relates to processes for preparing these compounds, intermediates, processes for preparing such intermediates, and to compositions comprising at least one compound I.

In many cases, in particular at low application rates, the fungicidal activity of the known fungicidal compounds is unsatisfactory. Based on this, it was an object of the present invention to provide compounds having improved activity and/or a broader activity spectrum against phytopathogenic harmful fungi. Closest compounds with fungicidal activity are present in EP 1 736 471 and EP 2 103 214.

Surprisingly, this object is achieved by the use of the inventive quinoline compounds of formula I having favorable fungicidal activity against phytopathogenic fungi.

Accordingly, the present invention relates to the compounds of the formula I 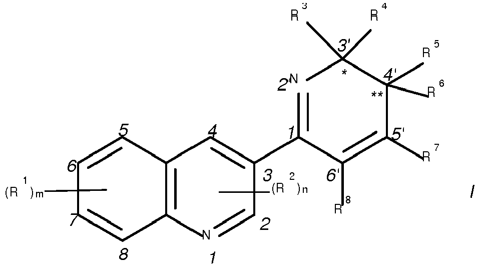 wherein
m is 0, 1, 2, 3 or 4;
R¹ is in each case independently selected from halogen, OH, CN, NO₂, SH, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl, C₁-C₆-alkoxy, C₃-C₆-cycloalkyl, five- or six-membered heteroaryl and aryl; wherein the heteroaryl contains one, two or three heteroatoms selected from N, O and S; and wherein
R^{x} is C₁-C₄-alkyl, C₁-C₄-halogenalkyl, unsubstituted aryl or aryl that is substituted by one, two, three, four or five substituents R^{x1} independently selected from C₁-C₄-alkyl; wherein the aliphatic moieties of R¹ are not further substituted or carry one, two, three or up to the maximum possible number of identical or different groups R^{1a} which independently of one another are selected from:
   R^{1a} halogen, OH, CN, C₁-C₆-alkoxy, C₃-C₆-cycloalkyl, C₃-C₆-halogencycloalkyl, C₁-C₄-halogenalkoxy, C₁-C₆-alkylthio and phenoxy, wherein the phenyl group is unsubstituted or carries one, two, three, four or five substituents R^{11a} selected from the group consisting of halogen, OH, C₁-C₄-alkyl, C₁-C₄-halogenalkyl, C₁-C₄-alkoxy and C₁-C₄-halogenalkoxy;
wherein the cycloalkyl, heteroaryl and aryl moieties of R¹ are not further substituted or carry one, two, three, four, five or up to the maximum number of identical or different groups R^{1b} which independently of one another are selected from:
   R^{1b} halogen, OH, CN, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy and C₁-C₆-alkylthio;
   n is 0, 1 or 2;
   R² is in each case independently selected from the substituents as defined for R¹, wherein the possible substituents for R² are R^{2a} and R^{2b}, respectively, which correspond to R^{1a} and R^{1b}, respectively;
   R³, R⁴ are independently selected from hydrogen, halogen, OH, CN, NO₂, SH, C₁-C₆-alkylthio, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl, C₁-C₆-alkoxy, C₁-C₆-halogenalkoxy, a saturated or partially unsaturated three-, four-, five-, six-, seven-, eight-, nine-, or ten-membered carbocycle or heterocycle, wherein in each case one or two CH₂ groups of the carbo- and heterocycle may be replaced by a group independently selected from C(=O) and C(=S), five- or six-membered heteroaryl and aryl; wherein the heterocycle and the heteroaryl contain independently one, two, three or four heteroatoms selected from N, O and S;
wherein the aliphatic moieties of R³ and R⁴ are independently not further substituted or carry one, two, three or up to the maximum possible number of identical or different groups R^{3a} or R^{4a}, respectively, which independently of one another are selected from:
   R^{3a}, R^{4a} halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, C₁-C₆-alkoxy, C₃-C₆-cycloalkyl, C₃-C₆-halogencycloalkyl, C₁-C₄-halogenalkoxy, C₁-C₆-alkylthio, aryl and phenoxy, wherein the aryl and phenyl groups are independently unsubstituted or carry one, two, three, four or five substituents selected from the group consisting of halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, NH-SO₂-R^{x}, C₁-C₆-alkylthio, C₁-C₄-alkyl, C₁-C₄-halogenalkyl, C₁-C₄-alkoxy and C₁-C₄-halogenalkoxy;
wherein the carbocyclic, heterocyclic, heteroaryl and aryl moieties of R³ and R⁴ are independently not further substituted or carry one, two, three, four, five or up to the maximum number of identical or different groups R^{3b} or R^{4b}, respectively, which independently of one another are selected from:
   R^{3b}, R^{4b} halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, NH-SO₂-R^{x}, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy, C₁-C₆-alkylthio, C₁-C₆-halogenalkylthio, C₁-C₄-alkoxy-C₁-C₄-alkyl, phenyl and phenoxy, wherein the phenyl groups are unsubstituted or carry one, two, three, four or five substituents selected from the group consisting of halogen, OH, C₁-C₄-alkyl, C₁-C₄-halogenalkyl, C₁-C₄-alkoxy and C₁-C₄-halogenalkoxy; and wherein R^{x} is as defined above;
   or R³, R⁴ together with the carbon atom to which they are bound (marked with * in formula I) form a saturated or partially unsaturated three-, four-, five-, six-, seven-, eight-, nine-, or ten-membered carbocycle or heterocycle; wherein the heterocycle contains one, two, three or four heteroatoms selected from N, O and S, wherein the heteroatom N may carry one substituent R^{N} selected from C₁-C₄-alkyl, C₁-C₄-halogenalkyl and SO₂Ph, wherein Ph is unsubstituted phenyl or phenyl that is substituted by one, two or three substituents selected from C₁-C₄-alkyl, and wherein the heteroatom S may be in the form of its oxide SO or SO₂, and wherein the carbocycle or heterocycle is unsubstituted or carries one, two, three or four substituents R³⁴ independently selected from halogen, OH, CN, NO₂, SH, NH₂, C₁-C₆-alkyl, C₁-C₆-halogenalkyl, C₁-C₆-alkoxy, C₁-C₆-halogenalkoxy, C₁-C₆-alkylthio, C₁-C₆-halogenalkylthio, C₁-C₄-alkoxy-C₁-C₄-alkyl, phenyl and phenoxy, wherein the phenyl groups are unsubstituted or carry one, two, three, four or five substituents R^{34a} selected from the group consisting of halogen, OH, C₁-C₄-alkyl, C₁-C₄-halogenalkyl, C₁-C₄-alkoxy and C₁-C₄-halogenalkoxy; and wherein in each case one or two CH₂ groups of the carbo- or heterocycle may be replaced by a group independently selected from C(=O) and C(=S);
   R⁵ is hydrogen, halogen, OH, CN, NO₂, SH, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl, C₁-C₆-alkoxy, C₃-C₆-cycloalkyl, saturated or partially unsaturated three-, four-, five-, six-, seven-, eight-, nine-, or ten-membered heterocycle, five- or six-membered heteroaryl or aryl; wherein the heterocycle or heteroaryl contains one, two or three heteroatoms selected from N, O and S; and wherein R^{x} is defined above; and
wherein the aliphatic moieties of R⁵ are not further substituted or carry one, two, three or up to the maximum possible number of identical or different groups R^{5a} which independently of one another are selected from:
   R^{5a} halogen, OH, CN, C₁-C₆-alkoxy, C₃-C₆-cycloalkyl, C₃-C₆-halogencycloalkyl, C₁-C₄-halogenalkoxy, C₁-C₆-alkylthio and phenoxy, wherein the phenyl group is unsubstituted or carries one, two, three, four or five substituents R^{55a} selected from the group consisting of halogen, OH, C₁-C₄-alkyl, C₁-C₄-halogenalkyl, C₁-C₄-alkoxy and C₁-C₄-halogenalkoxy;
wherein the cycloalkyl, heterocycle, heteroaryl and aryl moieties of R⁵ are not further substituted or carry one, two, three, four, five or up to the maximum number of identical or different groups R^{5b} which independently of one another are selected from:
   R^{5b} halogen, OH, CN, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy and C₁-C₆-alkylthio;
   R⁶ is independently selected from the substituents as defined for R⁵, wherein the possible substituents for R⁶ are R^{6a}, R^{66a} and R^{6b}, respectively, which correspond to R^{5a}, R^{55a} and R^{5b}, respectively;
   or R⁵ and R⁶ together with the carbon atom to which they are bound (marked with C** in formula I) form a saturated or partially unsaturated three-, four-, five-, six-, seven-, eight-, nine-, or ten-membered carbo- or heterocycle; wherein the heterocycle contains one, two, three or four heteroatoms selected from N, O and S, and wherein the carbocycle or heterocycle is unsubstituted or carries one, two, three or four substituents R⁵⁶ independently selected from halogen, OH, CN, NO₂, SH, NH₂, C₁-C₆-alkyl, C₁-C₆-halogenalkyl, C₁-C₆-alkoxy, C₁-C₆-halogenalkoxy, C₁-C₆-alkylthio, C₁-C₆-halogenalkylthio, C₁-C₄-alkoxy-C₁-C₄-alkyl, phenyl and phenoxy; wherein the phenyl groups are unsubstituted or carry one, two, three, four or five substituents R^{56a} selected from the group consisting of halogen, OH, C₁-C₄-alkyl, C₁-C₄-halogenalkyl, C₁-C₄-alkoxy and C₁-C₄-halogenalkoxy; and wherein in each case one or two CH₂ groups of the carbo- or heterocycle may be replaced by a group independently selected from C(=O) and C(=S); and
   R⁷ and R⁸ together with the carbon atoms to which they are bound form a five- or six-membered heteroaryl; wherein the heteroaryl contains one, two or three heteroatoms selected from N, O and S, and wherein the heteroaryl carries zero, one, two, three or four substituents (R⁷⁸)ₒ, wherein
      o is 0, 1, 2 or 3; and
   R⁷⁸ are independently selected from halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, NH-SO₂-R^{x}, CH(=O), C(=O)C₁-C₆-alkyl, C(=O)NH(C₁-C₆-alkyl), CR'=NOR", C₁-C₆-alkyl, C₂-C₆-alkenyl, C₂-C₆-alkynyl, C₁-C₆-alkoxy, C₁-C₆-halogenalkoxy, C₂-C₆-alkenyloxy, C₂-C₆-alkynyloxy, C₃-C₆-cycloalkyl, C₃-C₆-cycloalkenyl, three-, four-, five- or six-membered saturated or partially unsaturated heterocycle, five- or six-membered heteroaryl and phenyl; wherein the heterocycle or heteroaryl contains one, two or three heteroatoms selected from N, O and S; wherein R' and R" are independently selected from C₁-C₄-alkyl, C₂-C₆-alkenyl, C₂-C₆-alkynyl, C₃-C₆-cycloalkyl, saturated or partially unsaturated three-, four-, five-, six-, seven-, eight-, nine-, or ten-membered heterocycle, five- or six-membered heteroaryl or aryl; wherein the heterocycle or heteroaryl contains one, two or three heteroatoms selected from N, O and S, and wherein R' and/or R" are independently unsubstituted or carry one, two or three R'" independently selected from halogen, OH, CN, NO₂, SH, NH₂, NH(C₁-C₄-alkyl), N(C₁-C₄-alkyl)₂, NH-SO₂-R^{x}, C₁-C₆-alkyl, C₁-C₆-halogenalkyl, C₂-C₆-alkenyl, C₂-C₆-halogenalkenyl, C₂-C₆-alkynyl, C₂-C₆-halogenalkynyl, C₁-C₆-alkoxy, C₁-C₆-halogenalkoxy, C₃-C₆-cycloalkyl, C₃-C₆-halogencycloalkyl and phenyl; and wherein R^{x} is defined above;
   and
wherein the aliphatic moieties of R⁷⁸ are not further substituted or carry one, two, three or up to the maximum possible number of identical or different groups R^{78a} which independently of one another are selected from:
   R^{78a} halogen, OH, CN, C₁-C₆-alkoxy, C₃-C₆-cycloalkyl, C₃-C₆-cycloalkenyl, C₃-C₆-halogencycloalkyl, C₃-C₆-halogencycloalkenyl, C₁-C₄-halogenalkoxy, C₁-C₆-alkylthio, five- or six-membered heteroaryl, phenyl and phenoxy, wherein the heterorayl, phenyl and phenoxy group is unsubstituted or carries one, two, three, four or five substituents R^{78a'} selected from the group consisting of halogen, OH, C₁-C₄-alkyl, C₁-C₄-halogenalkyl, C₁-C₄-alkoxy and C₁-C₄-halogenalkoxy;
wherein the alicyclic, phenyl, heterocyclic and heteroaryl moieties of R⁷⁸ are not further substituted or carry one, two, three, four, five or up to the maximum number of identical or different groups R^{78b} which independently of one another are selected from:
   R^{78b} halogen, OH, CN, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy and C₁-C₆-alkylthio;
   and the N-oxides and the agriculturally acceptable salts thereof.

The numbering of the ring members in the compounds of the present invention is as given in formula I above:
Compounds of formula I can be accessed e.g. starting from alcohols of type II with nitriles of type III in the presence of an acid in an organic solvent (see for example US 2008/0275242 or WO2005/070917). Preferably, sulfuric acid or a sulfonic acid, in particular triflic acid, are used as acid. Most suitable solvents are hydrocarbons, preferably benzene or dichloromethane. In the following schemes, the optionally substituted heteroaryl fromed by R⁷ and R⁸ is sketched by a circle named "heteroaryl":

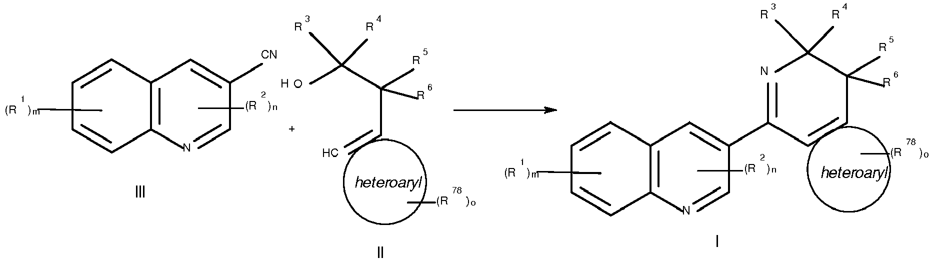

Depending on the nature of the starting materials, the reaction is performed at a temperature from -40°C to 200°C, in particular from -10°C to 120°C, more specifically from 0°C to 100°C, even more specifically from room or ambient temperature (about 23°C) to 80°C.

Nitriles of type III are either commercially available or can be prepared by a skilled person from the corresponding halides following literature precedures (see, for example Journal of Organic Chemistry, 76(2), 665-668; 2011; Angewandte Chemie, International Edition, 52(38), 10035-10039; 2013; WO2004/013094).

Alcohols of type II can be prepared as described below. A skilled person will realize that compounds of type III can be reacted with organometallic reagents, preferably alkyl Grignard or alkyl-Lithium reagents, in ethereal solvents, preferably THF at low temperatures and under inert conditions to furnish compounds of type II.

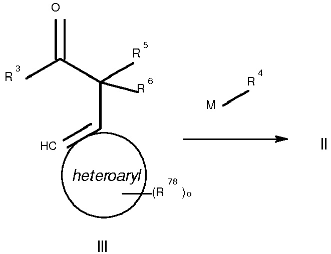

Alternatively, alcohols of type II can be prepared from epoxydes IIIa and compounds VI (see below):

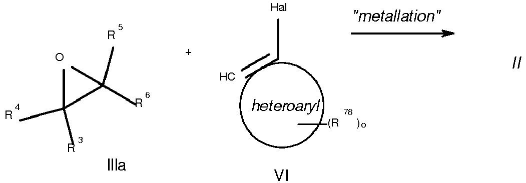

The metallation reaction may preferably be carried out using Lithium-organic compounds, such as for example n-butyl lithium, sec-butyl lithium or tert-butyl lithium to result in an exchange of halogen by lithium. Also suitable is the reaction with magnesium resulting in the formation of the respective Grignard reagents. A further possibility is the use of other Grignard reagents such as isopropyl-magnesium-bromide instead of Mg.

A typical preparation of compounds of type III can be achieved by reacting compounds of type IV with organometallic reagents, preferably alkyl Grignard or alkyl-Lithium reagents, in ethereal solvents, preferably THF at low temperatures and under inert conditions to furnish compounds of type III as previously reported (see for example WO2012051036; WO2011042918).

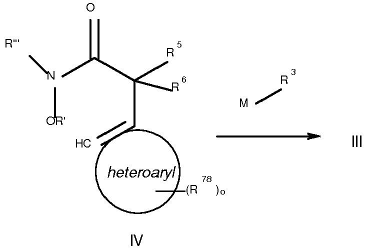

Compounds of type IV can be accessed by reacting a carbonyl compound of type V, preferably a carboxylic acid (X = OH) or an acid chloride (X = Cl), with NH(OR')R", wherein R' and R" are selected from (C₁-C₄)-alkyl, most preferably being methyl, in an organic solvent, preferably THF or dichloromethane. Typically the reaction is performed in a range between 0 °C and ambient temperature in the presence of an organic base, preferably NEt₃ or pyridine (see e.g. US 20130324506; Tetrahedron: Asymmetry, 17(4), 508-511; 2006). If X = OH, the addition of an activating reagent, preferably a carbodiimide, may be preferred (see for example ChemMedChem, 7(12), 2101-2112; 2012; 2011038204; Journal of Organic Chemistry, 76(1), 164-169; 2011).

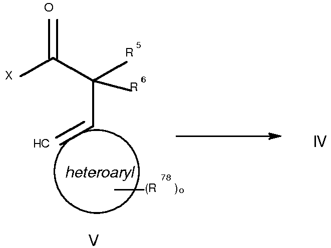

If required, compounds of type V can be prepared from the corresponding aryl halides of type VI (Hal is halogen, preferably Br or I). As described (Tetrahedron, 68(9), 2113-2120; 2012; Chemical Communications (Cambridge, United Kingdom), 49(60), 6767-6769; 2013), aryl halides will react with compounds of type VII in the presence of a transition metal catalyst, preferably a copper(I) salt, in an organic solvent, preferably DMF or DMSO, at elevated temperatures. Typically a base, preferably potassium phosphate, is added.

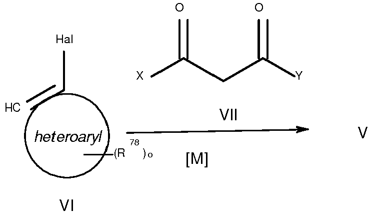

If appropriate, compounds of type II can be prepared as follows. A known or commercially available compound of type VIII can be reacted with an organometallic reagent of type IX, preferably a Grignard or an organolithium reagent, readily prepared by a skilled person. Preferably, the reaction is performed in a temperature range from -78 °C to room temperature under inert conditions in an ethereal solvent.

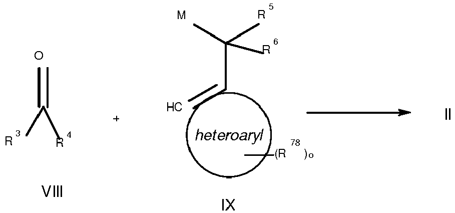

It may be preferred to access compounds I, where R⁵ and R⁶ are F (named compounds I-1) from the respective keto compound (named compounds IIA) as follows based on a literature precedent (US 2008/0275242). A skilled person will realize that compounds I-1 can be formed using a suitable halogenation agent, preferably diethyl aminosulfur trifluoride or phosphorus trihalides in an organic solvent, preferably a chlorinated hydrocarbon such as dichloromethane at, e.g., room temperature. If appropriate, the reaction can be performed at elevated temperatures.

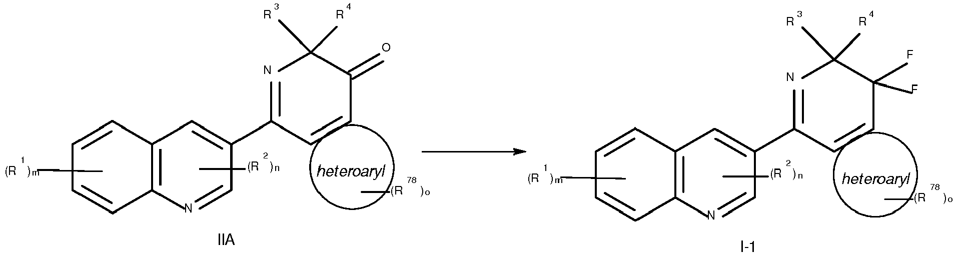

Compounds of type IIA can be accessed by reacting compounds of type I-2 (where R⁵ and R⁶ are halogen substituents (Hal'), in particular bromo) under aqueous or mildly acidic conditions in an organic solvent.

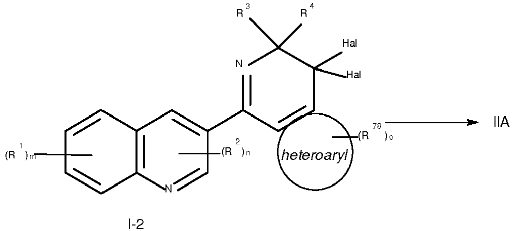

Said compounds I-2 can be prepared from compounds I-3 (where R⁵ and R⁶ are both hydrogen) by reaction with a halide source, preferably N-bromosuccinimide or 1,3-dibromo-5,5-dimethylhydantoin, in an organic solvent, preferably a hydrocarbon such as toluene or benzene, in the presence of an initiator, preferably azo-bis-isobutyronitrile, at elevated temperatures (see for example WO 2008/035379).

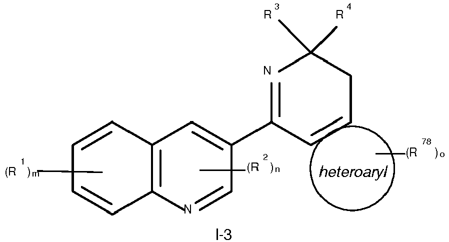

Alternatively, as described elsewhere (WO 2013/047749), compounds I-1 can be prepared directly from compounds I-2. To this end, compounds I-2 are reacted with hydrogen fluoride triethyl amine (HF NEt₃) in an organic solvent, preferably an aromatic hydrocarbon and at elevated temperatures.

Alternatively compounds I can be synthesized from heteroaryls X, which are commercially available or can be synthesized according to procedures known in literature, in which X¹ denotes for hydrogen or halogen (Cl, Br, I) and X² denotes for halogen (Cl, Br, I) or C₁-C₆-alkoxycarbonyl.

Compounds X can be metalated with Grignard-reagents (X³ denotes for Cl, Br or I)), for example methyl magnesium-X³, ethyl magnesiue-X³, isopropyl-magnesium-X³ and phenyl magnesium X³ among others, or lithium organic reagents like methyl-lithium, ethyl-lithium, butyl-lithium and phenyl-lithium among others, and reacted with compounds Xa to yield derivatives XI, whereas R³¹ and R⁴¹ independently from each other denote for C₁-C₆-alkyl, C₂-C₆-alkenyl, C₂-C₆-alkynyl, a saturated or partially unsaturated three-, four-, five-, six-, seven-, eight-, nine-, or ten-membered carbocycle or heterocycle, five- or six-membered heteroaryl and aryl.

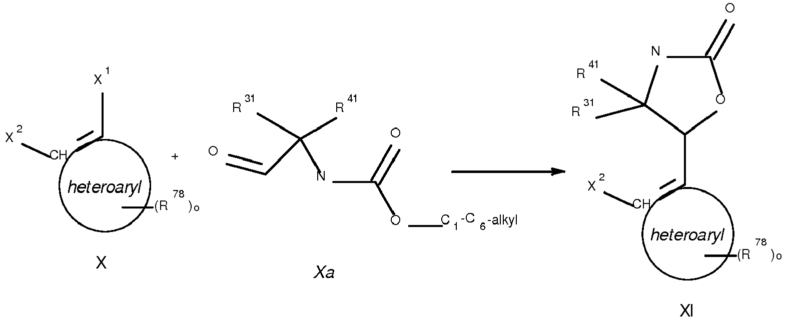

Subsequently compounds XI (X²=Cl, Br, I) can be reacted with carbon monoxide yielding esters XII following published literature (Science of Synthesis (2014), 2, 67-93; Comprehensive Inorganic Chemistry II (2013), 6, 1-24; RSC Catalysis Series (2015), 21 (New Trends in Cross-Coupling), 479-520; Metal-catalyzed Cross-Coupling Reactions and More (Editor: A. De Meijere) (2014), 1, 133-278; Domino Reactions (Editor L. Tietze) (2014), 7-30; Synthesis 2014, 46 (13), 1689-1708; RSC Advances (2014), 4 (20), 10367-10389), for example using Pd-catalyst (i.e. Pd(dppf)Cl₂ ([1,1'-bis(diphenylphosphino)ferrocene]dichloropalladium(II)) and sodium methanolat in methanol under elevated pressure (10-200 bar) of carbon monoxide.

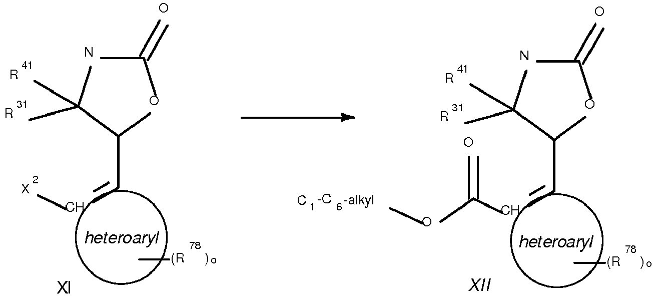

Compounds XII can be hydrolyzed using acidic or basic conditons, for example hydrochloric or sulfuric acid, or sodium or potassium carbonate, hydrogen carbonate or hydroxide in water or solvent mixtures with water and alcoholic solvents (preferably methanol, ethanol, isopropanol), or acetonitrile, acetone, dimethylformamide or N-methyl pyrrolidine, at temperatures from 0°C to 100°C yielding intermediates XIII.

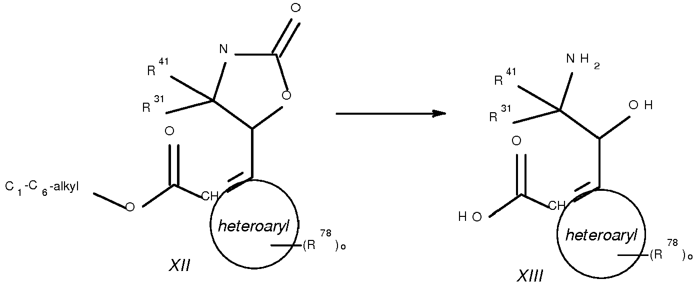

Intermediates XIII can be activitated with reagents like HATU (1-[Bis(dimethylamino)methylene]-1*H*-1,2,3-triazolo[4,5-*b*]pyridinium 3-oxid hexafluorophosphate), CDI (1,1'-Carbonyldiimidazole), DCC (*N*,*N*-Methanetetraylbis[cyclohexanamine]) and others known in literature (Eur. JOC 2013, 4325; Tetrahedron 2004, 60, 2447; Tetrahedron 2005, 61, 10827; Chem. Soc. Rev. 2009, 38, 606; Chem. Rev. 2011, 111, 6557) to further react and yield compounds XIV.

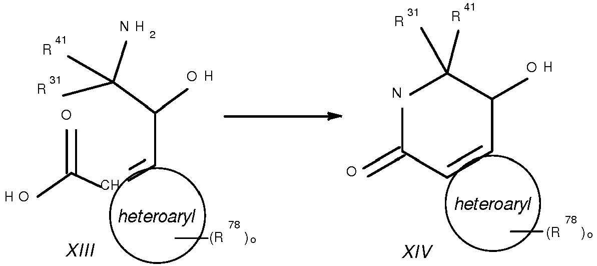

Furthermore compounds XIV are oxidized with MnO₂, hypochlorite, activated DMSO, Cr(VI)-containing reagents or employing other oxidizing conditions known in literature (Korean Chemical Society (2015), 36(12), 2799; Hudlicky, Oxidations in Organic Chemistry, American Chemical Society, Washington DC, 1990; Acc. Chem. Res. 2002, 35, 774; JACS 1984, 106, 3374; Tetrahedron Letters 56 (2015) 6878; Backvall, Modern Oxidation Methods, Wiley, Weinheim 2004; Tojo, Oxidation of Alcohols to Aldehydes and Ketones, Springer 2006) to provide carbonyl compounds XV.

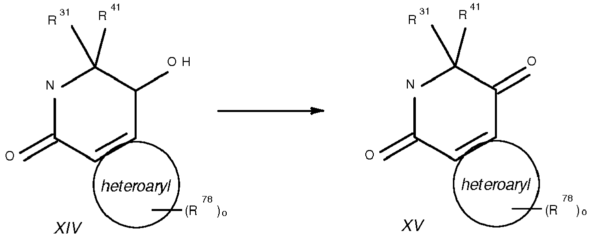

Subsequently the amides XV can be transferred into the triflate XVI by reaction of XV with trifluoromethyl sulfonic anhydride in an inert solvent, like dichloromethane, chloroform, carbon tetrachloride, benzene, toluene or chlorobenzene in the presence of a base, for example an organic base like pyridine, triethylamine or diisopropyl ethylamine or an aqueous base like solutions of sodium or potassium hydroxide, carbonate or hydrogen carbonate in water at temperatures preferably between 0°C and 100°C.

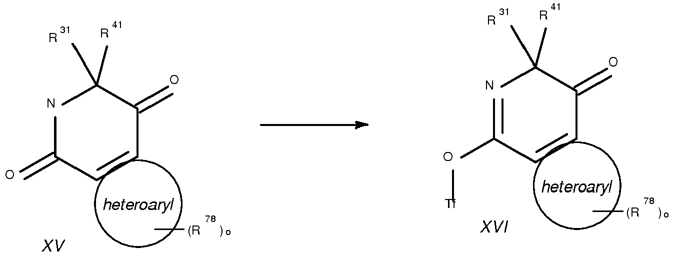

These compounds XVI are reacted with fluorination reagents (Kirsch, Modern Fluoroorganic Chemistry, Wiley 2013)) like deoxo-fluor (BAST, bis(2-methoxyethyl)aminosulfur trifluoride, Journal of Fluorine Chemistry (2016), 182, 41; Singh, et al. Synthesis 17, 2561, (2002)), DAST (Diethylaminoschwefeltrifluorid, Hudlicky Org. React. 35, 513, (1988)), Fluolead (4-*tert*-Butyl-2,6-dimethylphenylsulfur trifluoride, WO 2013118915; US 20080039660), Diethylaminodi-fluorosulfinium tetrafluoroborate (XtalFluor-E) or morpholinodifluorosulfinium tetrafluoroborate (XtalFluor-M) (Journal of organic chemistry (2010), 75(10), 3401) to yield difluoro compounds XVII.

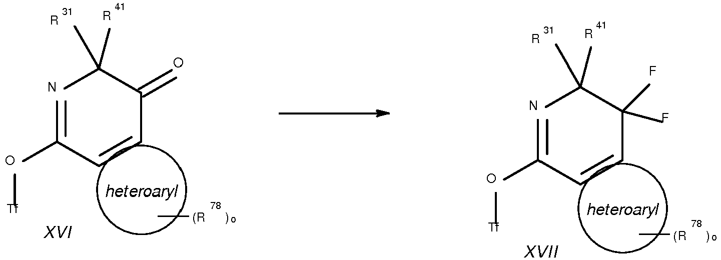

Subsequently these triflates XVII can be reacted under Suzuki conditions (European Journal of Organic Chemistry (2008),(12),2013) with boronic acids III*, in which R³¹¹ and R⁴¹¹ together with the groups they are attached to form a tetramethyl-1,3,2-dioxaborolane-ring or independently from one another mean hydrogen or C₁-C₆-alkyl to yield compounds I. 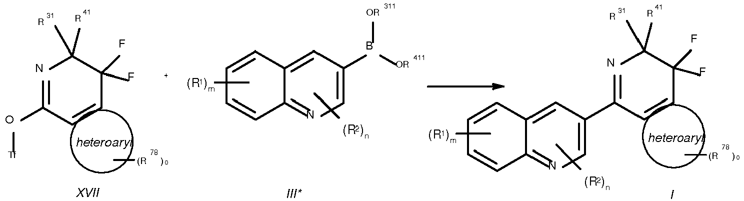 The N-oxides may be prepared from the inventive compounds according to conventional oxidation methods, e. g. by treating compounds I with an organic peracid such as metachloroperbenzoic acid (cf. WO 03/64572 or J. Med. Chem. 38(11), 1892-903, 1995); or with inorganic oxidizing agents such as hydrogen peroxide (cf. J. Heterocyc. Chem. 18(7), 1305-8, 1981) or oxone (cf. J. Am. Chem. Soc. 123(25), 5962-5973, 2001). The oxidation may lead to pure mono-N-oxides or to a mixture of different N-oxides, which can be separated by conventional methods such as chromatography.

If the synthesis yields mixtures of isomers, a separation is generally not necessarily required since in some cases the individual isomers can be interconverted during work-up for use or during application (e. g. under the action of light, acids or bases). Such conversions may also take place after use, e. g. in the treatment of plants in the treated plant, or in the harmful fungus to be controlled.

In the following, the intermediate compounds are further described. A skilled person will readily understand that the preferences for the substituents, also in particular the ones given in the tables below for the respective substituents, given herein in connection with compounds I apply for the intermediates accordingly. Thereby, the substituents in each case have independently of each other or more preferably in combination the meanings as defined herein.

The intermediate compounds of formula IIA are novel. Consequently, one aspect of the present invention relates to compounds of formula IIA: 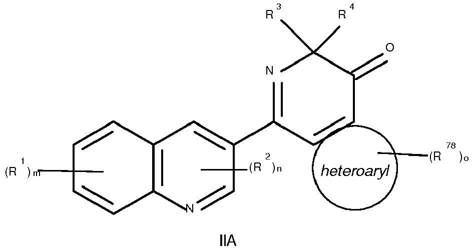 wherein the substituents R¹, R², R³, R⁴, m, n and o are as defined and preferably defined for formula I.

The compounds of formula IIA have fungicidal activity and the details below referring to the compounds I also apply to compounds IIA.

Particular embodiments of the compounds IIA are the following compounds IIA.A, IIA.B, IIA.C, IIA.D, IIA.E, IIA.F, IIA.G, IIA.H, IIA.I, IIA.J and IIA.K. In these formulae, the substituents R¹, m, R², n, R³, R⁴, R⁷⁸ and o are independently as defined or preferably defined herein:

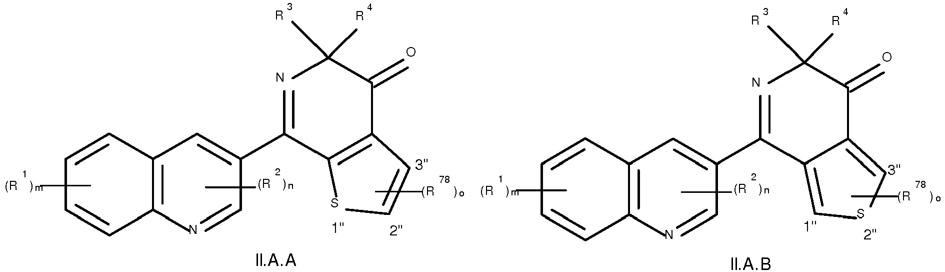

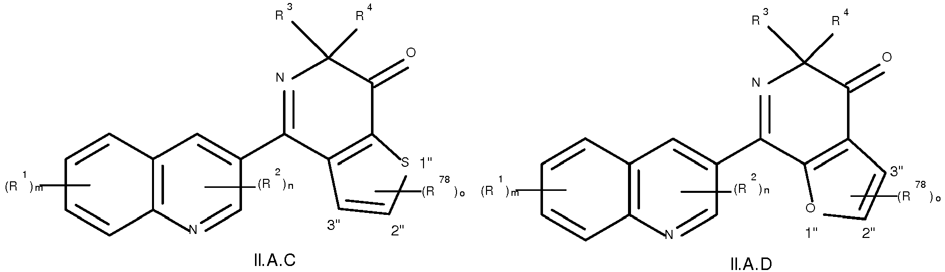

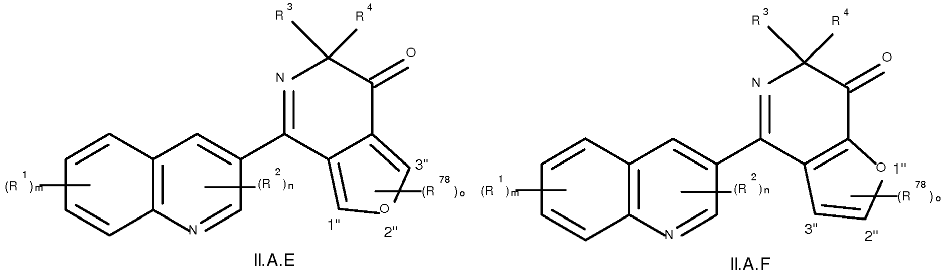

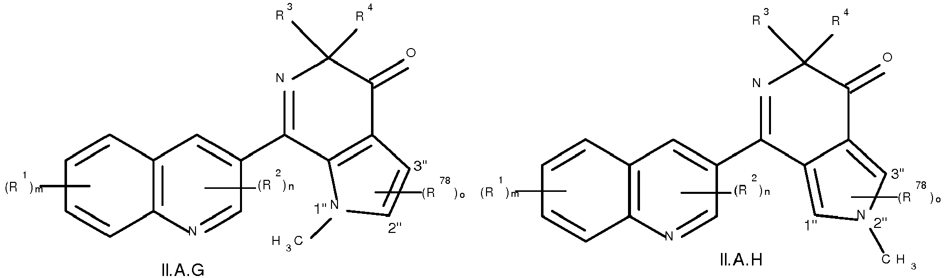

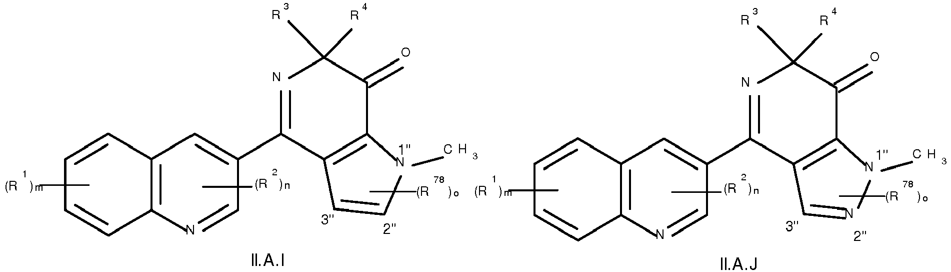

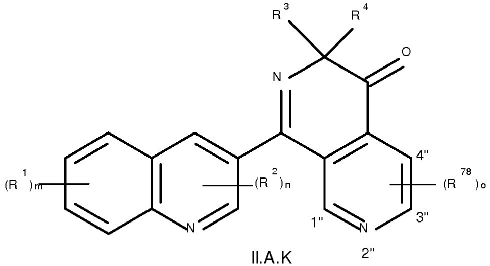

According to one embodiment, o in each of the formulae IIA.A, IIA.B, IIA.C, IIA.D, IIA.E, IIA.F, IIA.G, IIA.H, IIA.I, IIA.J and IIA.K, respectively, is 0, i.e. the heteroaryl is not substituted. These compounds are named IIA.A.1, IIA.B.1, IIA.C.1, IIA.D.1, IIA.E.1, IIA.F.1, IIA.G.1, IIA.H.1, IIA.1.1, IIA.J.1 and IIA.K.1, respectively.

Further preferred compounds I are the following compounds IIA.L, IIA.M, IIA.N, IIA.O, IIA.P, IIA.Q, IIA.R, IIA.S, IIA.T and IIA.U. In these formulae, the substituents R¹, m, R², n, R³, R⁴, R⁷⁸ and o are independently as defined or preferably defined herein:

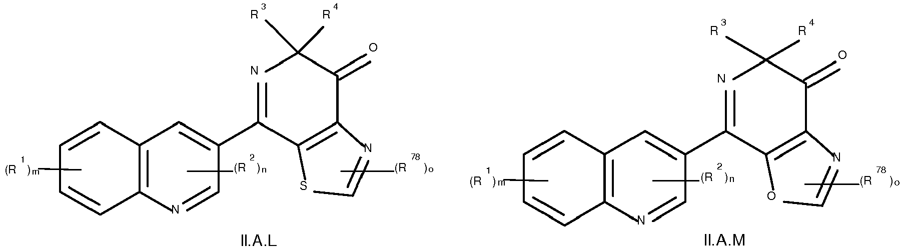

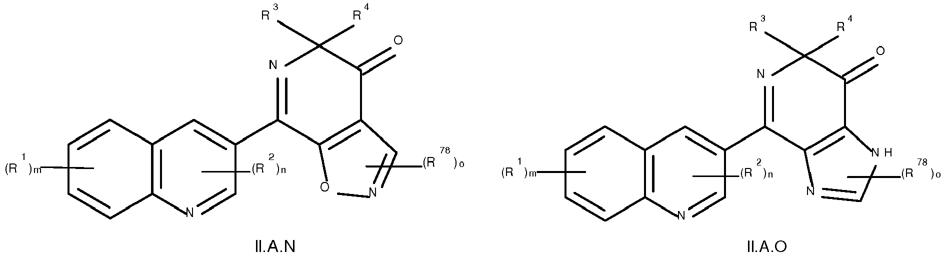

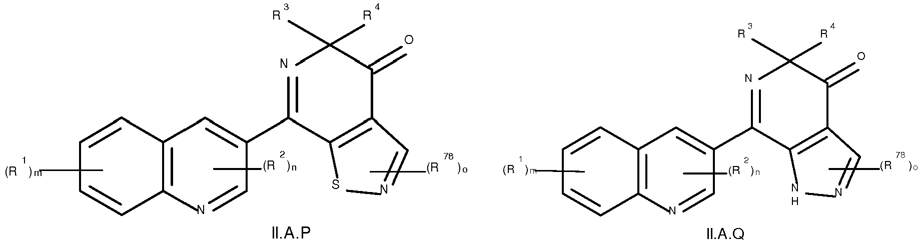

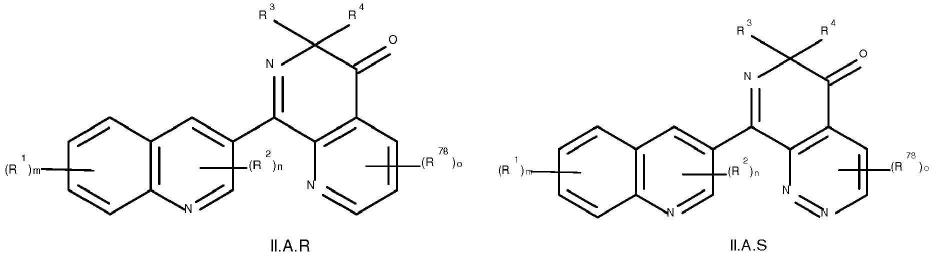

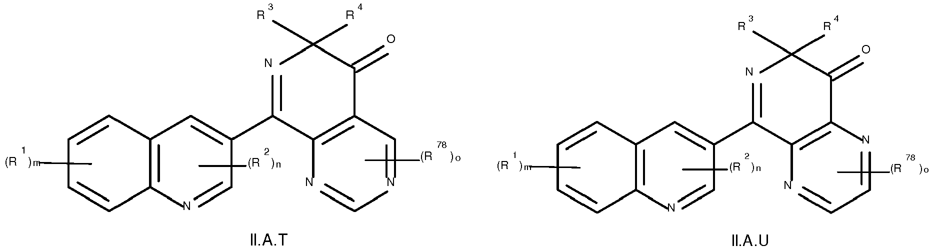

According to one embodiment, o in each of the formulae IIA.L, IIA.M, IIA.N, IIA.O, IIA.P, IIA.Q, IIA.R, IIA.S, IIA.T and IIA.U, respectively, is 0, i.e. the heteroaryl is not substituted. These compounds are named IIA.L.1, IIA.M.1, IIA.N.1, IIA.O.1, IIA.P.1, IIA.Q.1, IIA.R.1, IIA.S.1, IIA.T.1 and IIA.U.1, respectively.

Particular compounds II.A of the invention are the compounds of the formulae IIA.A, IIA.B, IIA.C, IIA.D, IIA.E, IIA.F, IIA.G, IIA.H, IIA.I, IIA.J and IIA.K that are compiled in the Tables 1-1 to 1-15, 2-1 to 2-15, 3-1 to 3-15, 4-1 to 4-15, 5-1 to 5-15, 6-1 to 6-15, 7-1 to 7-15, 8-1 to 8-15, 9-1 to 9-15, 10-1 to 10-8, 11-1 to 11-22. Each of the groups mentioned for a substituent in the tables is furthermore per se, independently of the combination in which it is mentioned, a particularly preferred aspect of the substituent in question.
Table 1-1 Compounds of the formula IIA.A in which o is 0 and the meaning for the combination of (R¹)ₘ, (R²)ₙ, R³ and R⁴ for each individual compound corresponds in each case to one line of Table A (compounds IIA.A.1-1.A-1 to IIA.A.1-1.A-1680).
Table 1-2 Compounds of the formula IIA.A in which o is 1, R⁷⁸ is 2"-F and the meaning for the combination of (R¹)ₘ, (R²)ₙ, R³ and R⁴ for each individual compound corresponds in each case to one line of Table A (compounds IIA.A.1-2.A-1 to IIA.A.1-2.A-1680).
Table 1-3 Compounds of the formula IIA.A in which o is 1, R⁷⁸ is 2"-CI and the meaning for the combination of (R¹)ₘ, (R²)ₙ, R³ and R⁴ for each individual compound corresponds in each case to one line of Table A (compounds IIA.A.1-3.A-1 to IIA.A.1-3.A-1680).
Table 1-4 Compounds of the formula IIA.A in which o is 1, R⁷⁸ is 2"-Br and the meaning for the combination of (R¹)ₘ, (R²)ₙ, R³ and R⁴ for each individual compound corresponds in each case to one line of Table A (compounds IIA.A.1-4.A-1 to IIA.A.1-4.A-1680).
Table 1-5 Compounds of the formula IIA.A in which o is 1, R⁷⁸ is 2"-CH₃ and the meaning for the combination of (R¹)ₘ, (R²)ₙ, R³ and R⁴ for each individual compound corresponds in each case to one line of Table A (compounds IIA.A.1-5.A-1 to IIA.A.1-5.A-1680).
Table 1-6 Compounds of the formula IIA.A in which o is 1, R⁷⁸ is 2"-OCH₃ and the meaning for the combination of (R¹)ₘ, (R²)ₙ, R³ and R⁴ for each individual compound corresponds in each case to one line of Table A (compounds IIA.A.1-6.A-1 to IIA.A.1-6.A-1680).
Table 1-7 Compounds of the formula IIA.A in which o is 1, R⁷⁸ is 2"-OCHF₂ and the meaning for the combination of (R¹)ₘ, (R²)ₙ, R³ and R⁴ for each individual compound corresponds in each case to one line of Table A (compounds IIA.A.1-7.A-1 to IIA.A.1-7.A-1680).
Table 1-8 Compounds of the formula IIA.A in which o is 1, R⁷⁸ is 2"-C₆H₅ and the meaning for the combination of (R¹)ₘ, (R²)ₙ, R³ and R⁴ for each individual compound corresponds in each case to one line of Table A (compounds IIA.A.1-8.A-1 to IIA.A.1-8.A-1680).
Table 1-9 Compounds of the formula IIA.A in which o is 1, R⁷⁸ is 3"-F and the meaning for the combination of (R¹)ₘ, (R²)ₙ, R³ and R⁴ for each individual compound corresponds in each case to one line of Table A (compounds IIA.A.1-9.A-1 to IIA.A.1-9.A-1680).
Table 1-10 Compounds of the formula IIA.A in which o is 1, R⁷⁸ is 3"-CI and the meaning for the combination of (R¹)ₘ, (R²)ₙ, R³ and R⁴ for each individual compound corresponds in each case to one line of Table A (compounds IIA.A.1-10.A-1 to IIA.A.1-10.A-1680).
Table 1-11 Compounds of the formula IIA.A in which o is 1, R⁷⁸ is 3"-Br and the meaning for the combination of (R¹)ₘ, (R²)ₙ, R³ and R⁴ for each individual compound corresponds in each case to one line of Table A (compounds IIA.A.1-11.A-1 to IIA.A.1-11.A-1680).
Table 1-12 Compounds of the formula IIA.A in which o is 1, R⁷⁸ is 3"-CH₃ and the meaning for the combination of (R¹)ₘ, (R²)ₙ, R³ and R⁴ for each individual compound corresponds in each case to one line of Table A (compounds IIA.A.1-12.A-1 to IIA.A.1-12.A-1680).
Table 1-13 Compounds of the formula IIA.A in which o is 1, R⁷⁸ is 3"-OCH₃ and the meaning for the combination of (R¹)ₘ, (R²)ₙ, R³ and R⁴ for each individual compound corresponds in each case to one line of Table A (compounds IIA.A.1-13.A-1 to IIA.A.1-13.A-1680).
Table 1-14 Compounds of the formula IIA.A in which o is 1, R⁷⁸ is 3"-OCHF₂ and the meaning for the combination of (R¹)ₘ, (R²)ₙ, R³ and R⁴ for each individual compound corresponds in each case to one line of Table A (compounds IIA.A.1-14.A-1 to IIA.A.1-14.A-1680).
Table 1-15 Compounds of the formula IIA.A in which o is 1, R⁷⁸ is 3"-C₆H₅ and the meaning for the combination of (R¹)ₘ, (R²)ₙ, R³ and R⁴ for each individual compound corresponds in each case to one line of Table A (compounds IIA.A.1-15.A-1 to IIA.A.1-15.A-1680).
Table 2-1 Compounds of the formula IIA.B in which o is 0 and the meaning for the combination of (R¹)ₘ, (R²)ₙ, R³ and R⁴ for each individual compound corresponds in each case to one line of Table A (compounds IIA.B.2-1.A-1 to IIA.B.2-1.A-1680).
Table 2-2 Compounds of the formula IIA.B in which o is 1, R⁷⁸ is 1"-F and the meaning for the combination of (R¹)ₘ, (R²)ₙ, R³ and R⁴ for each individual compound corresponds in each case to one line of Table A (compounds IIA.B.2-2.A-1 to IIA.B.2-2.A-1680).
Table 2-3 Compounds of the formula IIA.B in which o is 1, R⁷⁸ is 1"-Cl and the meaning for the combination of (R¹)ₘ, (R²)ₙ, R³ and R⁴ for each individual compound corresponds in each case to one line of Table A (compounds IIA.B.2-3.A-1 to IIA.B.2-3.A-1680).
Table 2-4 Compounds of the formula IIA.B in which o is 1, R⁷⁸ is 1"-Br and the meaning for the combination of (R¹)ₘ, (R²)ₙ, R³ and R⁴ for each individual compound corresponds in each case to one line of Table A (compounds IIA.B.2-4.A-1 to IIA.B.2-4.A-1680).
Table 2-5Compounds of the formula IIA.B in which o is 1, R⁷⁸ is 1"-CH₃ and the meaning for the combination of (R¹)ₘ, (R²)ₙ, R³ and R⁴ for each individual compound corresponds in each case to one line of Table A (compounds IIA.B.2-5.A-1 to IIA.B.2-5.A-1680).
Table 2-6 Compounds of the formula IIA.B in which o is 1, R⁷⁸ is 1"-OCH₃ and the meaning for the combination of (R¹)ₘ, (R²)ₙ, R³ and R⁴ for each individual compound corresponds in each case to one line of Table A (compounds IIA.B.2-6.A-1 to IIA.B.2-6.A-1680).
Table 2-7Compounds of the formula IIA.B in which o is 1, R⁷⁸ is 1"-OCHF₂ and the meaning for the combination of (R¹)ₘ, (R²)ₙ, R³ and R⁴ for each individual compound corresponds in each case to one line of Table A (compounds IIA.B.2-7.A-1 to IIA.B.2-7.A-1680).
Table 2-8 Compounds of the formula IIA.B in which o is 1, R⁷⁸ is 1"-C₆H₅ and the meaning for the combination of (R¹)ₘ, (R²)ₙ, R³ and R⁴ for each individual compound corresponds in each case to one line of Table A (compounds IIA.B.2-8.A-1 to IIA.B.2-8.A-1680).
Table 2-9 Compounds of the formula IIA.B in which o is 1, R⁷⁸ is 3"-F and the meaning for the combination of (R¹)ₘ, (R²)ₙ, R³ and R⁴ for each individual compound corresponds in each case to one line of Table A (compounds IIA.B.2-9.A-1 to IIA.B.2-9.A-1680).
Table 2-10 Compounds of the formula IIA.B in which o is 1, R⁷⁸ is 3"-CI and the meaning for the combination of (R¹)ₘ, (R²)ₙ, R³ and R⁴ for each individual compound corresponds in each case to one line of Table A (compounds IIA.B.2-10.A-1 to IIA.B.2-10.A-1680).
Table 2-11 Compounds of the formula IIA.B in which o is 1, R⁷⁸ is 3"-Br and the meaning for the combination of (R¹)ₘ, (R²)ₙ, R³ and R⁴ for each individual compound corresponds in each case to one line of Table A (compounds IIA.B.2-11.A-1 to IIA.B.2-11.A-1680).
Table 2-12 Compounds of the formula IIA.B in which o is 1, R⁷⁸ is 3"-CH₃ and the meaning for the combination of (R¹)ₘ, (R²)ₙ, R³ and R⁴ for each individual compound corresponds in each case to one line of Table A (compounds IIA.B.2-12.A-1 to IIA.B.2-12.A-1680).
Table 2-13 Compounds of the formula IIA.B in which o is 1, R⁷⁸ is 3"-OCH₃ and the meaning for the combination of (R¹)ₘ, (R²)ₙ, R³ and R⁴ for each individual compound corresponds in each case to one line of Table A (compounds IIA.B.2-13.A-1 to IIA.B.2-13.A-1680).
Table 2-14 Compounds of the formula IIA.B in which o is 1, R⁷⁸ is 3"-OCHF₂ and the meaning for the combination of (R¹)ₘ, (R²)ₙ, R³ and R⁴ for each individual compound corresponds in each case to one line of Table A (compounds IIA.B.2-14.A-1 to IIA.B.2-14.A-1680).
Table 2-15 Compounds of the formula IIA.B in which o is 1, R⁷⁸ is 3"-C₆H₅ and the meaning for the combination of (R¹)ₘ, (R²)ₙ, R³ and R⁴ for each individual compound corresponds in each case to one line of Table A (compounds IIA.B.2-15.A-1 to IIA.B.2-15.A-1680).
Table 3-1 Compounds of the formula IIA.C in which o is 0 and the meaning for the combination of (R¹)ₘ, (R²)ₙ, R³ and R⁴ for each individual compound corresponds in each case to one line of Table A (compounds IIA.C.3-1.A-1 to IIA.C.3-1.A-1680).
Table 3-2 Compounds of the formula IIA.C in which o is 1, R⁷⁸ is 2"-F and the meaning for the combination of (R¹)ₘ, (R²)ₙ, R³ and R⁴ for each individual compound corresponds in each case to one line of Table A (compounds IIA.C.3-2.A-1 to IIA.C.3-2.A-1680).
Table 3-3 Compounds of the formula IIA.C in which o is 1, R⁷⁸ is 2"-CI and the meaning for the combination of (R¹)ₘ, (R²)ₙ, R³ and R⁴ for each individual compound corresponds in each case to one line of Table A (compounds IIA.C.3-3.A-1 to IIA.C.3-3.A-1680).
Table 3-4 Compounds of the formula IIA.C in which o is 1, R⁷⁸ is 2"-Br and the meaning for the combination of (R¹)ₘ, (R²)ₙ, R³ and R⁴ for each individual compound corresponds in each case to one line of Table A (compounds IIA.C.3-4.A-1 to IIA.C.3-4.A-1680).
Table 3-5 Compounds of the formula IIA.C in which o is 1, R⁷⁸ is 2"-CH₃ and the meaning for the combination of (R¹)ₘ, (R²)ₙ, R³ and R⁴ for each individual compound corresponds in each case to one line of Table A (compounds IIA.C.3-5.A-1 to IIA.C.3-5.A-1680).
Table 3-6 Compounds of the formula IIA.C in which o is 1, R⁷⁸ is 2"-OCH₃ and the meaning for the combination of (R¹)ₘ, (R²)ₙ, R³ and R⁴ for each individual compound corresponds in each case to one line of Table A (compounds IIA.C.3-6.A-1 to IIA.C.3-6.A-1680).
Table 3-7Compounds of the formula IIA.C in which o is 1, R⁷⁸ is 2"-OCHF₂ and the meaning for the combination of (R¹)ₘ, (R²)ₙ, R³ and R⁴ for each individual compound corresponds in each case to one line of Table A (compounds IIA.C.3-7.A-1 to IIA.C.3-7.A-1680).
Table 3-8 Compounds of the formula IIA.C in which o is 1, R⁷⁸ is 2"-C₆H₅ and the meaning for the combination of (R¹)ₘ, (R²)ₙ, R³ and R⁴ for each individual compound corresponds in each case to one line of Table A (compounds IIA.C.3-8.A-1 to IIA.C.3-8.A-1680).
Table 3-9 Compounds of the formula IIA.C in which o is 1, R⁷⁸ is 3"-F and the meaning for the combination of (R¹)ₘ, (R²)ₙ, R³ and R⁴ for each individual compound corresponds in each case to one line of Table A (compounds IIA.C.3-9.A-1 to IIA.C.3-9.A-1680).
Table 3-10 Compounds of the formula IIA.C in which o is 1, R⁷⁸ is 3"-CI and the meaning for the combination of (R¹)ₘ, (R²)ₙ, R³ and R⁴ for each individual compound corresponds in each case to one line of Table A (compounds IIA.C.3-10.A-1 to IIA.C.3-10.A-1680).
Table 3-11 Compounds of the formula IIA.C in which o is 1, R⁷⁸ is 3"-Br and the meaning for the combination of (R¹)ₘ, (R²)ₙ, R³ and R⁴ for each individual compound corresponds in each case to one line of Table A (compounds IIA.C.3-11.A-1 to IIA.C.3-11.A-1680).
Table 3-12 Compounds of the formula IIA.C in which o is 1, R⁷⁸ is 3"-CH₃ and the meaning for the combination of (R¹)ₘ, (R²)ₙ, R³ and R⁴ for each individual compound corresponds in each case to one line of Table A (compounds IIA.C.3-12.A-1 to IIA.C.3-12.A-1680).
Table 3-13 Compounds of the formula IIA.C in which o is 1, R⁷⁸ is 3"-OCH₃ and the meaning for the combination of (R¹)ₘ, (R²)ₙ, R³ and R⁴ for each individual compound corresponds in each case to one line of Table A (compounds IIA.C.3-13.A-1 to IIA.C.3-13.A-1680).
Table 3-14 Compounds of the formula IIA.C in which o is 1, R⁷⁸ is 3"-OCHF₂ and the meaning for the combination of (R¹)ₘ, (R²)ₙ, R³ and R⁴ for each individual compound corresponds in each case to one line of Table A (compounds IIA.C.3-14.A-1 to IIA.C.3-14.A-1680).
Table 3-15 Compounds of the formula IIA.C in which o is 1, R⁷⁸ is 3"-C₆H₅ and the meaning for the combination of (R¹)ₘ, (R²)ₙ, R³ and R⁴ for each individual compound corresponds in each case to one line of Table A (compounds IIA.C.3-15.A-1 to IIA.C.3-15.A-1680).
Table 4-1 Compounds of the formula IIA.D in which o is 0 and the meaning for the combination of (R¹)ₘ, (R²)ₙ, R³ and R⁴ for each individual compound corresponds in each case to one line of Table A (compounds IIA.D.4-1.A-1 to IIA.D.4-1.A-1680).
Table 4-2 Compounds of the formula IIA.D in which o is 1, R⁷⁸ is 2"-F and the meaning for the combination of (R¹)ₘ, (R²)ₙ, R³ and R⁴ for each individual compound corresponds in each case to one line of Table A (compounds IIA.D.4-2.A-1 to IIA.D.4-2.A-1680).
Table 4-3 Compounds of the formula IIA.D in which o is 1, R⁷⁸ is 2"-CI and the meaning for the combination of (R¹)ₘ, (R²)ₙ, R³ and R⁴ for each individual compound corresponds in each case to one line of Table A (compounds IIA.D.4-3.A-1 to IIA.D.4-3.A-1680).
Table 4-4 Compounds of the formula IIA.D in which o is 1, R⁷⁸ is 2"-Br and the meaning for the combination of (R¹)ₘ, (R²)ₙ, R³ and R⁴ for each individual compound corresponds in each case to one line of Table A (compounds IIA.D.4-4.A-1 to IIA.D.4-4.A-1680).
Table 4-5 Compounds of the formula IIA.D in which o is 1, R⁷⁸ is 2"-CH₃ and the meaning for the combination of (R¹)ₘ, (R²)ₙ, R³ and R⁴ for each individual compound corresponds in each case to one line of Table A (compounds IIA.D.4-5.A-1 to IIA.D.4-5.A-1680).
Table 4-6Compounds of the formula IIA.D in which o is 1, R⁷⁸ is 2"-OCH₃ and the meaning for the combination of (R¹)ₘ, (R²)ₙ, R³ and R⁴ for each individual compound corresponds in each case to one line of Table A (compounds IIA.D.4-6.A-1 to IIA.D.4-6.A-1680).
Table 4-7Compounds of the formula IIA.D in which o is 1, R⁷⁸ is 2"-OCHF₂ and the meaning for the combination of (R¹)ₘ, (R²)ₙ, R³ and R⁴ for each individual compound corresponds in each case to one line of Table A (compounds IIA.D.4-7.A-1 to IIA.D.4-7.A-1680).
Table 4-8 Compounds of the formula IIA.D in which o is 1, R⁷⁸ is 2"-C₆H₅ and the meaning for the combination of (R¹)ₘ, (R²)ₙ, R³ and R⁴ for each individual compound corresponds in each case to one line of Table A (compounds IIA.D.4-8.A-1 to IIA.D.4-8.A-1680).
Table 4-9 Compounds of the formula IIA.D in which o is 1, R⁷⁸ is 3"-F and the meaning for the combination of (R¹)ₘ, (R²)ₙ, R³ and R⁴ for each individual compound corresponds in each case to one line of Table A (compounds IIA.D.4-9.A-1 to IIA.D.4-9.A-1680).
Table 4-10 Compounds of the formula IIA.D in which o is 1, R⁷⁸ is 3"-CI and the meaning for the combination of (R¹)ₘ, (R²)ₙ, R³ and R⁴ for each individual compound corresponds in each case to one line of Table A (compounds IIA.D.4-10.A-1 to IIA.D.4-10.A-1680).
Table 4-11 Compounds of the formula IIA.D in which o is 1, R⁷⁸ is 3"-Br and the meaning for the combination of (R¹)ₘ, (R²)ₙ, R³ and R⁴ for each individual compound corresponds in each case to one line of Table A (compounds IIA.D.4-11.A-1 to IIA.D.4-11.A-1680).
Table 4-12 Compounds of the formula IIA.D in which o is 1, R⁷⁸ is 3"-CH₃ and the meaning for the combination of (R¹)ₘ, (R²)ₙ, R³ and R⁴ for each individual compound corresponds in each case to one line of Table A (compounds IIA.D.4-12.A-1 to IIA.D.4-12.A-1680).
Table 4-13 Compounds of the formula IIA.D in which o is 1, R⁷⁸ is 3"-OCH₃ and the meaning for the combination of (R¹)ₘ, (R²)ₙ, R³ and R⁴ for each individual compound corresponds in each case to one line of Table A (compounds IIA.D.4-13.A-1 to IIA.D.4-13.A-1680).
Table 4-14 Compounds of the formula IIA.D in which o is 1, R⁷⁸ is 3"-OCHF₂ and the meaning for the combination of (R¹)ₘ, (R²)ₙ, R³ and R⁴ for each individual compound corresponds in each case to one line of Table A (compounds IIA.D.4-14.A-1 to IIA.D.4-14.A-1680).
Table 4-15 Compounds of the formula IIA.D in which o is 1, R⁷⁸ is 3"-C₆H₅ and the meaning for the combination of (R¹)ₘ, (R²)ₙ, R³ and R⁴ for each individual compound corresponds in each case to one line of Table A (compounds IIA.D.4-15.A-1 to IIA.D.4-15.A-1680).
Table 5-1 Compounds of the formula IIA.E in which o is 0 and the meaning for the combination of (R¹)ₘ, (R²)ₙ, R³ and R⁴ for each individual compound corresponds in each case to one line of Table A (compounds IIA.E.5-1.A-1 to IIA.E.5-1.A-1680).
Table 5-2 Compounds of the formula IIA.E in which o is 1, R⁷⁸ is 1"-F and the meaning for the combination of (R¹)ₘ, (R²)ₙ, R³ and R⁴ for each individual compound corresponds in each case to one line of Table A (compounds IIA.E.5-2.A-1 to IIA.E.5-2.A-1680).
Table 5-3 Compounds of the formula IIA.E in which o is 1, R⁷⁸ is 1"-Cl and the meaning for the combination of (R¹)ₘ, (R²)ₙ, R³ and R⁴ for each individual compound corresponds in each case to one line of Table A (compounds IIA.E.5-3.A-1 to IIA.E.5-3.A-1680).
Table 5-4 Compounds of the formula IIA.E in which o is 1, R⁷⁸ is 1"-Br and the meaning for the combination of (R¹)ₘ, (R²)ₙ, R³ and R⁴ for each individual compound corresponds in each case to one line of Table A (compounds IIA.E.5-4.A-1 to IIA.E.5-4.A-1680).
Table 5-5 Compounds of the formula IIA.E in which o is 1, R⁷⁸ is 1"-CH₃ and the meaning for the combination of (R¹)ₘ, (R²)ₙ, R³ and R⁴ for each individual compound corresponds in each case to one line of Table A (compounds IIA.E.5-5.A-1 to IIA.E.5-5.A-1680).
Table 5-6 Compounds of the formula IIA.E in which o is 1, R⁷⁸ is 1"-OCH₃ and the meaning for the combination of (R¹)ₘ, (R²)ₙ, R³ and R⁴ for each individual compound corresponds in each case to one line of Table A (compounds IIA.E.5-6.A-1 to IIA.E.5-6.A-1680).
Table 5-7 Compounds of the formula IIA.E in which o is 1, R⁷⁸ is 1"-OCHF₂ and the meaning for the combination of (R¹)ₘ, (R²)ₙ, R³ and R⁴ for each individual compound corresponds in each case to one line of Table A (compounds IIA.E.5-7.A-1 to IIA.E.5-7.A-1680).
Table 5-8 Compounds of the formula IIA.E in which o is 1, R⁷⁸ is 1"-C₆H₅ and the meaning for the combination of (R¹)ₘ, (R²)ₙ, R³ and R⁴ for each individual compound corresponds in each case to one line of Table A (compounds IIA.E.5-8.A-1 to IIA.E.5-8.A-1680).
Table 5-9 Compounds of the formula IIA.E in which o is 1, R⁷⁸ is 3"-F and the meaning for the combination of (R¹)ₘ, (R²)ₙ, R³ and R⁴ for each individual compound corresponds in each case to one line of Table A (compounds IIA.E.5-9.A-1 to IIA.E.5-9.A-1680).
Table 5-10 Compounds of the formula IIA.E in which o is 1, R⁷⁸ is 3"-CI and the meaning for the combination of (R¹)ₘ, (R²)ₙ, R³ and R⁴ for each individual compound corresponds in each case to one line of Table A (compounds IIA.E.5-10.A-1 to IIA.E.5-10.A-1680).
Table 5-11 Compounds of the formula IIA.E in which o is 1, R⁷⁸ is 3"-Br and the meaning for the combination of (R¹)ₘ, (R²)ₙ, R³ and R⁴ for each individual compound corresponds in each case to one line of Table A (compounds IIA.E.5-11.A-1 to IIA.E.5-11.A-1680).
Table 5-12 Compounds of the formula IIA.E in which o is 1, R⁷⁸ is 3"-CH₃ and the meaning for the combination of (R¹)ₘ, (R²)ₙ, R³ and R⁴ for each individual compound corresponds in each case to one line of Table A (compounds IIA.E.5-12.A-1 to IIA.E.5-12.A-1680).
Table 5-13 Compounds of the formula IIA.E in which o is 1, R⁷⁸ is 3"-OCH₃ and the meaning for the combination of (R¹)ₘ, (R²)ₙ, R³ and R⁴ for each individual compound corresponds in each case to one line of Table A (compounds IIA.E.5-13.A-1 to IIA.E.5-13.A-1680).
Table 5-14 Compounds of the formula IIA.E in which o is 1, R⁷⁸ is 3"-OCHF₂ and the meaning for the combination of (R¹)ₘ, (R²)ₙ, R³ and R⁴ for each individual compound corresponds in each case to one line of Table A (compounds IIA.E.5-14.A-1 to IIA.E.5-14.A-1680).
Table 5-15 Compounds of the formula IIA.E in which o is 1, R⁷⁸ is 3"-C₆H₅ and the meaning for the combination of (R¹)ₘ, (R²)ₙ, R³ and R⁴ for each individual compound corresponds in each case to one line of Table A (compounds IIA.E.5-15.A-1 to IIA.E.5-15.A-1680).
Table 6-1 Compounds of the formula IIA.F in which o is 0 and the meaning for the combination of (R¹)ₘ, (R²)ₙ, R³ and R⁴ for each individual compound corresponds in each case to one line of Table A (compounds IIA.F.6-1.A-1 to IIA.F.6-1.A-1680).
Table 6-2 Compounds of the formula IIA.F in which o is 1, R⁷⁸ is 2"-F and the meaning for the combination of (R¹)ₘ, (R²)ₙ, R³ and R⁴ for each individual compound corresponds in each case to one line of Table A (compounds IIA.F.6-2.A-1 to IIA.F.6-2.A-1680).
Table 6-3 Compounds of the formula IIA.F in which o is 1, R⁷⁸ is 2"-CI and the meaning for the combination of (R¹)ₘ, (R²)ₙ, R³ and R⁴ for each individual compound corresponds in each case to one line of Table A (compounds IIA.F.6-3.A-1 to IIA.F.6-3.A-1680).
Table 6-4 Compounds of the formula IIA.F in which o is 1, R⁷⁸ is 2"-Br and the meaning for the combination of (R¹)ₘ, (R²)ₙ, R³ and R⁴ for each individual compound corresponds in each case to one line of Table A (compounds IIA.F.6-4.A-1 to IIA.F.6-4.A-1680).
Table 6-5 Compounds of the formula IIA.F in which o is 1, R⁷⁸ is 2"-CH₃ and the meaning for the combination of (R¹)ₘ, (R²)ₙ, R³ and R⁴ for each individual compound corresponds in each case to one line of Table A (compounds IIA.F.6-5.A-1 to IIA.F.6-5.A-1680).
Table 6-6 Compounds of the formula IIA.F in which o is 1, R⁷⁸ is 2"-OCH₃ and the meaning for the combination of (R¹)ₘ, (R²)ₙ, R³ and R⁴ for each individual compound corresponds in each case to one line of Table A (compounds IIA.F.6-6.A-1 to IIA.F.6-6.A-1680).
Table 6-7 Compounds of the formula IIA.F in which o is 1, R⁷⁸ is 2"-OCHF₂ and the meaning for the combination of (R¹)ₘ, (R²)ₙ, R³ and R⁴ for each individual compound corresponds in each case to one line of Table A (compounds IIA.F.6-7.A-1 to IIA.F.6-7.A-1680).
Table 6-8 Compounds of the formula IIA.F in which o is 1, R⁷⁸ is 2"-C₆H₅ and the meaning for the combination of (R¹)ₘ, (R²)ₙ, R³ and R⁴ for each individual compound corresponds in each case to one line of Table A (compounds IIA.F.6-8.A-1 to IIA.F.6-8.A-1680).
Table 6-9 Compounds of the formula IIA.F in which o is 1, R⁷⁸ is 3"-F and the meaning for the combination of (R¹)ₘ, (R²)ₙ, R³ and R⁴ for each individual compound corresponds in each case to one line of Table A (compounds IIA.F.6-9.A-1 to IIA.F.6-9.A-1680).
Table 6-10 Compounds of the formula IIA.F in which o is 1, R⁷⁸ is 3"-CI and the meaning for the combination of (R¹)ₘ, (R²)ₙ, R³ and R⁴ for each individual compound corresponds in each case to one line of Table A (compounds IIA.F.6-10.A-1 to IIA.F.6-10.A-1680).
Table 6-11 Compounds of the formula IIA.F in which o is 1, R⁷⁸ is 3"-Br and the meaning for the combination of (R¹)ₘ, (R²)ₙ, R³ and R⁴ for each individual compound corresponds in each case to one line of Table A (compounds IIA.F.6-11.A-1 to IIA.F.6-11.A-1680).
Table 6-12 Compounds of the formula IIA.F in which o is 1, R⁷⁸ is 3"-CH₃ and the meaning for the combination of (R¹)ₘ, (R²)ₙ, R³ and R⁴ for each individual compound corresponds in each case to one line of Table A (compounds IIA.F.6-12.A-1 to IIA.F.6-12.A-1680).
Table 6-13 Compounds of the formula IIA.F in which o is 1, R⁷⁸ is 3"-OCH₃ and the meaning for the combination of (R¹)ₘ, (R²)ₙ, R³ and R⁴ for each individual compound corresponds in each case to one line of Table A (compounds IIA.F.6-13.A-1 to IIA.F.6-13.A-1680).
Table 6-14 Compounds of the formula IIA.F in which o is 1, R⁷⁸ is 3"-OCHF₂ and the meaning for the combination of (R¹)ₘ, (R²)ₙ, R³ and R⁴ for each individual compound corresponds in each case to one line of Table A (compounds IIA.F.6-14.A-1 to IIA.F.6-14.A-1680).
Table 6-15 Compounds of the formula IIA.F in which o is 1, R⁷⁸ is 3"-C₆H₅ and the meaning for the combination of (R¹)ₘ, (R²)ₙ, R³ and R⁴ for each individual compound corresponds in each case to one line of Table A (compounds IIA.F.6-15.A-1 to IIA.F.6-15.A-1680).
Table 7-1 Compounds of the formula IIA.G in which o is 0 and the meaning for the combination of (R¹)ₘ, (R²)ₙ, R³ and R⁴ for each individual compound corresponds in each case to one line of Table A (compounds IIA.G.7-1.A-1 to IIA.G.7-1.A-1680).
Table 7-2 Compounds of the formula IIA.G in which o is 1, R⁷⁸ is 2"-F and the meaning for the combination of (R¹)ₘ, (R²)ₙ, R³ and R⁴ for each individual compound corresponds in each case to one line of Table A (compounds IIA.G.7-2.A-1 to IIA.G.7-2.A-1680).
Table 7-3 Compounds of the formula IIA.G in which o is 1, R⁷⁸ is 2"-CI and the meaning for the combination of (R¹)ₘ, (R²)ₙ, R³ and R⁴ for each individual compound corresponds in each case to one line of Table A (compounds IIA.G.7-3.A-1 to IIA.G.7-3.A-1680).
Table 7-4 Compounds of the formula IIA.G in which o is 1, R⁷⁸ is 2"-Br and the meaning for the combination of (R¹)ₘ, (R²)ₙ, R³ and R⁴ for each individual compound corresponds in each case to one line of Table A (compounds IIA.G.7-4.A-1 to IIA.G.7-4.A-1680).
Table 7-5 Compounds of the formula IIA.G in which o is 1, R⁷⁸ is 2"-CH₃ and the meaning for the combination of (R¹)ₘ, (R²)ₙ, R³ and R⁴ for each individual compound corresponds in each case to one line of Table A (compounds IIA.G.7-5.A-1 to IIA.G.7-5.A-1680).
Table 7-6 Compounds of the formula IIA.G in which o is 1, R⁷⁸ is 2"-OCH₃ and the meaning for the combination of (R¹)ₘ, (R²)ₙ, R³ and R⁴ for each individual compound corresponds in each case to one line of Table A (compounds IIA.G.7-6.A-1 to IIA.G.7-6.A-1680).
Table 7-7 Compounds of the formula IIA.G in which o is 1, R⁷⁸ is 2"-OCHF₂ and the meaning for the combination of (R¹)ₘ, (R²)ₙ, R³ and R⁴ for each individual compound corresponds in each case to one line of Table A (compounds IIA.G.7-7.A-1 to IIA.G.7-7.A-1680).
Table 7-8 Compounds of the formula IIA.G in which o is 1, R⁷⁸ is 2"-C₆H₅ and the meaning for the combination of (R¹)ₘ, (R²)ₙ, R³ and R⁴ for each individual compound corresponds in each case to one line of Table A (compounds IIA.G.7-8.A-1 to IIA.G.7-8.A-1680).
Table 7-9 Compounds of the formula IIA.G in which o is 1, R⁷⁸ is 3"-F and the meaning for the combination of (R¹)ₘ, (R²)ₙ, R³ and R⁴ for each individual compound corresponds in each case to one line of Table A (compounds IIA.G.7-9.A-1 to IIA.G.7-9.A-1680).
Table 7-10 Compounds of the formula IIA.G in which o is 1, R⁷⁸ is 3"-CI and the meaning for the combination of (R¹)ₘ, (R²)ₙ, R³ and R⁴ for each individual compound corresponds in each case to one line of Table A (compounds IIA.G.7-10.A-1 to IIA.G.7-10.A-1680).
Table 7-11 Compounds of the formula IIA.G in which o is 1, R⁷⁸ is 3"-Br and the meaning for the combination of (R¹)ₘ, (R²)ₙ, R³ and R⁴ for each individual compound corresponds in each case to one line of Table A (compounds IIA.G.7-11.A-1 to IIA.G.7-11.A-1680).
Table 7-12 Compounds of the formula IIA.G in which o is 1, R⁷⁸ is 3"-CH₃ and the meaning for the combination of (R¹)ₘ, (R²)ₙ, R³ and R⁴ for each individual compound corresponds in each case to one line of Table A (compounds IIA.G.7-12.A-1 to IIA.G.7-12.A-1680).
Table 7-13 Compounds of the formula IIA.G in which o is 1, R⁷⁸ is 3"-OCH₃ and the meaning for the combination of (R¹)ₘ, (R²)ₙ, R³ and R⁴ for each individual compound corresponds in each case to one line of Table A (compounds IIA.G.7-13.A-1 to IIA.G.7-13.A-1680).
Table 7-14 Compounds of the formula IIA.G in which o is 1, R⁷⁸ is 3"-OCHF₂ and the meaning for the combination of (R¹)ₘ, (R²)ₙ, R³ and R⁴ for each individual compound corresponds in each case to one line of Table A (compounds IIA.G.7-14.A-1 to IIA.G.7-14.A-1680).
Table 7-15 Compounds of the formula IIA.G in which o is 1, R⁷⁸ is 3"-C₆H₅ and the meaning for the combination of (R¹)ₘ, (R²)ₙ, R³ and R⁴ for each individual compound corresponds in each case to one line of Table A (compounds IIA.G.7-15.A-1 to IIA.G.7-15.A-1680).
Table 8-1 Compounds of the formula IIA.H in which o is 0 and the meaning for the combination of (R¹)ₘ, (R²)ₙ, R³ and R⁴ for each individual compound corresponds in each case to one line of Table A (compounds IIA.H.8-1.A-1 to IIA.H.8-1.A-1680).
Table 8-2 Compounds of the formula IIA.H in which o is 1, R⁷⁸ is 1"-F and the meaning for the combination of (R¹)ₘ, (R²)ₙ, R³ and R⁴ for each individual compound corresponds in each case to one line of Table A (compounds IIA.H.8-2.A-1 to IIA.H.8-2.A-1680).
Table 8-3Compounds of the formula IIA.H in which o is 1, R⁷⁸ is 1"-Cl and the meaning for the combination of (R¹)ₘ, (R²)ₙ, R³ and R⁴ for each individual compound corresponds in each case to one line of Table A (compounds IIA.H.8-3.A-1 to IIA.H.8-3.A-1680).
Table 8-4Compounds of the formula IIA.H in which o is 1, R⁷⁸ is 1"-Br and the meaning for the combination of (R¹)ₘ, (R²)ₙ, R³ and R⁴ for each individual compound corresponds in each case to one line of Table A (compounds IIA.H.8-4.A-1 to IIA.H.8-4.A-1680).
Table 8-5Compounds of the formula IIA.H in which o is 1, R⁷⁸ is 1"-CH₃ and the meaning for the combination of (R¹)ₘ, (R²)ₙ, R³ and R⁴ for each individual compound corresponds in each case to one line of Table A (compounds IIA.H.8-5.A-1 to IIA.H.8-5.A-1680).
Table 8-6 Compounds of the formula IIA.H in which o is 1, R⁷⁸ is 1"-OCH₃ and the meaning for the combination of (R¹)ₘ, (R²)ₙ, R³ and R⁴ for each individual compound corresponds in each case to one line of Table A (compounds IIA.H.8-6.A-1 to IIA.H.8-6.A-1680).
Table 8-7Compounds of the formula IIA.H in which o is 1, R⁷⁸ is 1"-OCHF₂ and the meaning for the combination of (R¹)ₘ, (R²)ₙ, R³ and R⁴ for each individual compound corresponds in each case to one line of Table A (compounds IIA.H.8-7.A-1 to IIA.H.8-7.A-1680).
Table 8-8 Compounds of the formula IIA.H in which o is 1, R⁷⁸ is 1"-C₆H₅ and the meaning for the combination of (R¹)ₘ, (R²)ₙ, R³ and R⁴ for each individual compound corresponds in each case to one line of Table A (compounds IIA.H.8-8.A-1 to IIA.H.8-8.A-1680).
Table 8-9 Compounds of the formula IIA.H in which o is 1, R⁷⁸ is 3"-F and the meaning for the combination of (R¹)ₘ, (R²)ₙ, R³ and R⁴ for each individual compound corresponds in each case to one line of Table A (compounds IIA.H.8-9.A-1 to IIA.H.8-9.A-1680).
Table 8-10 Compounds of the formula IIA.H in which o is 1, R⁷⁸ is 3"-CI and the meaning for the combination of (R¹)ₘ, (R²)ₙ, R³ and R⁴ for each individual compound corresponds in each case to one line of Table A (compounds IIA.H.8-10.A-1 to IIA.H.8-10.A-1680).
Table 8-11 Compounds of the formula IIA.H in which o is 1, R⁷⁸ is 3"-Br and the meaning for the combination of (R¹)ₘ, (R²)ₙ, R³ and R⁴ for each individual compound corresponds in each case to one line of Table A (compounds IIAH.8-11.A-1 to IIA.H.8-11.A-1680).
Table 8-12 Compounds of the formula IIA.H in which o is 1, R⁷⁸ is 3"-CH₃ and the meaning for the combination of (R¹)ₘ, (R²)ₙ, R³ and R⁴ for each individual compound corresponds in each case to one line of Table A (compounds IIA.H.8-12.A-1 to IIA.H.8-12.A-1680).
Table 8-13 Compounds of the formula IIA.H in which o is 1, R⁷⁸ is 3"-OCH₃ and the meaning for the combination of (R¹)ₘ, (R²)ₙ, R³ and R⁴ for each individual compound corresponds in each case to one line of Table A (compounds IIA.H.8-13.A-1 to IIA.H.8-13.A-1680).
Table 8-14 Compounds of the formula IIA.H in which o is 1, R⁷⁸ is 3"-OCHF₂ and the meaning for the combination of (R¹)ₘ, (R²)ₙ, R³ and R⁴ for each individual compound corresponds in each case to one line of Table A (compounds IIA.H.8-14.A-1 to IIA.H.8-14.A-1680).
Table 8-15 Compounds of the formula IIA.H in which o is 1, R⁷⁸ is 3"-C₆H₅ and the meaning for the combination of (R¹)ₘ, (R²)ₙ, R³ and R⁴ for each individual compound corresponds in each case to one line of Table A (compounds IIA.H.8-15.A-1 to IIA.H.8-15.A-1680).
Table 9-1 Compounds of the formula IIA.I in which o is 0 and the meaning for the combination of (R¹)ₘ, (R²)ₙ, R³ and R⁴ for each individual compound corresponds in each case to one line of Table A (compounds IIA.I.9-1.A-1 to IIA.I.9-1.A-1680).
Table 9-2 Compounds of the formula IIA.I in which o is 1, R⁷⁸ is 2"-F and the meaning for the combination of (R¹)ₘ, (R²)ₙ, R³ and R⁴ for each individual compound corresponds in each case to one line of Table A (compounds IIA.I.9-2.A-1 to IIA.I.9-2.A-1680).
Table 9-3 Compounds of the formula IIA.I in which o is 1, R⁷⁸ is 2"-CI and the meaning for the combination of (R¹)ₘ, (R²)ₙ, R³ and R⁴ for each individual compound corresponds in each case to one line of Table A (compounds IIA.I.9-3.A-1 to IIA.I.9-3.A-1680).
Table 9-4 Compounds of the formula IIA.I in which o is 1, R⁷⁸ is 2"-Br and the meaning for the combination of (R¹)ₘ, (R²)ₙ, R³ and R⁴ for each individual compound corresponds in each case to one line of Table A (compounds IIA.I.9-4.A-1 to IIA.I.9-4.A-1680).
Table 9-5 Compounds of the formula IIA.I in which o is 1, R⁷⁸ is 2"-CH₃ and the meaning for the combination of (R¹)ₘ, (R²)ₙ, R³ and R⁴ for each individual compound corresponds in each case to one line of Table A (compounds IIA.1.9-5.A-1 to IIA.1.9-5.A-1680).
Table 9-6Compounds of the formula IIA.I in which o is 1, R⁷⁸ is 2"-OCH₃ and the meaning for the combination of (R¹)ₘ, (R²)ₙ, R³ and R⁴ for each individual compound corresponds in each case to one line of Table A (compounds IIA.1.9-6.A-1 to IIA.I.9-6.A-1680).
Table 9-7 Compounds of the formula IIA.I in which o is 1, R⁷⁸ is 2"-OCHF₂ and the meaning for the combination of (R¹)ₘ, (R²)ₙ, R³ and R⁴ for each individual compound corresponds in each case to one line of Table A (compounds IIA.I.9-7.A-1 to IIA.I.9-7.A-1680).
Table 9-8Compounds of the formula IIA.I in which o is 1, R⁷⁸ is 2"-C₆H₅ and the meaning for the combination of (R¹)ₘ, (R²)ₙ, R³ and R⁴ for each individual compound corresponds in each case to one line of Table A (compounds IIA.I.9-8.A-1 to IIA.I.9-8.A-1680).
Table 9-9Compounds of the formula IIA.I in which o is 1, R⁷⁸ is 3"-F and the meaning for the combination of (R¹)ₘ, (R²)ₙ, R³ and R⁴ for each individual compound corresponds in each case to one line of Table A (compounds IIA.I.9-9.A-1 to IIA.I.9-9.A-1680).
Table 9-10 Compounds of the formula IIA.I in which o is 1, R⁷⁸ is 3"-CI and the meaning for the combination of (R¹)ₘ, (R²)ₙ, R³ and R⁴ for each individual compound corresponds in each case to one line of Table A (compounds IIA.I.9-10.A-1 to IIA.I.9-10.A-1680).
Table 9-11 Compounds of the formula IIA.I in which o is 1, R⁷⁸ is 3"-Br and the meaning for the combination of (R¹)ₘ, (R²)ₙ, R³ and R⁴ for each individual compound corresponds in each case to one line of Table A (compounds IIA.I.9-11.A-1 to IIA.I.9-11.A-1680).
Table 9-12 Compounds of the formula IIA.I in which o is 1, R⁷⁸ is 3"-CH₃ and the meaning for the combination of (R¹)ₘ, (R²)ₙ, R³ and R⁴ for each individual compound corresponds in each case to one line of Table A (compounds IIA.I.9-12.A-1 to IIA.I.9-12.A-1680).
Table 9-13 Compounds of the formula IIA.I in which o is 1, R⁷⁸ is 3"-OCH₃ and the meaning for the combination of (R¹)ₘ, (R²)ₙ, R³ and R⁴ for each individual compound corresponds in each case to one line of Table A (compounds IIA.I.9-13.A-1 to IIA.I.9-13.A-1680).
Table 9-14 Compounds of the formula IIA.I in which o is 1, R⁷⁸ is 3"-OCHF₂ and the meaning for the combination of (R¹)ₘ, (R²)ₙ, R³ and R⁴ for each individual compound corresponds in each case to one line of Table A (compounds IIA.I.9-14.A-1 to IIA.I.9-14.A-1680).
Table 9-15 Compounds of the formula IIA.I in which o is 1, R⁷⁸ is 3"-C₆H₅ and the meaning for the combination of (R¹)ₘ, (R²)ₙ, R³ and R⁴ for each individual compound corresponds in each case to one line of Table A (compounds IIA.I.9-15.A-1 to IIA.I.9-15.A-1680).
Table 10-1 Compounds of the formula IIA.J in which o is 0 and the meaning for the combination of (R¹)ₘ, (R²)ₙ, R³ and R⁴ for each individual compound corresponds in each case to one line of Table A (compounds IIA.J.10-1.A-1 to IIA.J.10-1.A-1680).
Table 10-2 Compounds of the formula IIA.J in which o is 1, R⁷⁸ is 3"-F and the meaning for the combination of (R¹)ₘ, (R²)ₙ, R³ and R⁴ for each individual compound corresponds in each case to one line of Table A (compounds IIA.J.10-2.A-1 to IIA.J.10-2.A-1680).
Table 10-3 Compounds of the formula IIA.J in which o is 1, R⁷⁸ is 3"-CI and the meaning for the combination of (R¹)ₘ, (R²)ₙ, R³ and R⁴ for each individual compound corresponds in each case to one line of Table A (compounds IIAJ.10-3.A-1 to IIA.J.10-3.A-1680).
Table 10-4 Compounds of the formula IIA.J in which o is 1, R⁷⁸ is 3"-Br and the meaning for the combination of (R¹)ₘ, (R²)ₙ, R³ and R⁴ for each individual compound corresponds in each case to one line of Table A (compounds IIA.J.10-4.A-1 to IIA.J.10-4.A-1680).
Table 10-5 Compounds of the formula IIA.J in which o is 1, R⁷⁸ is 3"-CH₃ and the meaning for the combination of (R¹)ₘ, (R²)ₙ, R³ and R⁴ for each individual compound corresponds in each case to one line of Table A (compounds IIA.J.10-5.A-1 to IIA.J.10-5.A-1680).
Table 10-6 Compounds of the formula IIA.J in which o is 1, R⁷⁸ is 3"-OCH₃ and the meaning for the combination of (R¹)ₘ, (R²)ₙ, R³ and R⁴ for each individual compound corresponds in each case to one line of Table A (compounds IIA.J.10-6.A-1 to IIA.J.10-6.A-1680).
Table 10-7 Compounds of the formula IIA.J in which o is 1, R⁷⁸ is 3"-OCHF₂ and the meaning for the combination of (R¹)ₘ, (R²)ₙ, R³ and R⁴ for each individual compound corresponds in each case to one line of Table A (compounds IIA.J.10-7.A-1 to IIA.J.10-7.A-1680).
Table 10-8 Compounds of the formula IIA.J in which o is 1, R⁷⁸ is 3"-C₆H₅ and the meaning for the combination of (R¹)ₘ, (R²)ₙ, R³ and R⁴ for each individual compound corresponds in each case to one line of Table A (compounds IIA.J.10-8.A-1 to IIA.J.10-8.A-1680).
Table 11-1 Compounds of the formula IIA.K in which o is 0 and the meaning for the combination of (R¹)ₘ, (R²)ₙ, R³ and R⁴ for each individual compound corresponds in each case to one line of Table A (compounds IIA.K.11-1.A-1 to IIAK.11-1.A-1680).
Table 11-2 Compounds of the formula IIA.K in which o is 1, R⁷⁸ is 1"-F and the meaning for the combination of (R¹)ₘ, (R²)ₙ, R³ and R⁴ for each individual compound corresponds in each case to one line of Table A (compounds IIA.K.11-2.A-1 to IIA.K.11-2.A-1680).
Table 11-3 Compounds of the formula IIA.K in which o is 1, R⁷⁸ is 1"-Cl and the meaning for the combination of (R¹)ₘ, (R²)ₙ, R³ and R⁴ for each individual compound corresponds in each case to one line of Table A (compounds IIA.K.11-3.A-1 to IIA.K.11-3.A-1680).
Table 11-4 Compounds of the formula IIA.K in which o is 1, R⁷⁸ is 1"-Br and the meaning for the combination of (R¹)ₘ, (R²)ₙ, R³ and R⁴ for each individual compound corresponds in each case to one line of Table A (compounds IIA.K.11-4.A-1 to IIA.K.11-4.A-1680).
Table 11-5 Compounds of the formula IIA.K in which o is 1, R⁷⁸ is 1"-CH₃ and the meaning for the combination of (R¹)ₘ, (R²)ₙ, R³ and R⁴ for each individual compound corresponds in each case to one line of Table A (compounds IIA.K.11-5.A-1 to IIA.K.11-5.A-1680).
Table 11-6 Compounds of the formula IIA.K in which o is 1, R⁷⁸ is 1"-OCH₃ and the meaning for the combination of (R¹)ₘ, (R²)ₙ, R³ and R⁴ for each individual compound corresponds in each case to one line of Table A (compounds IIA.K.11-6.A-1 to IIA.K.11-6.A-1680).
Table 11-7 Compounds of the formula IIA.K in which o is 1, R⁷⁸ is 1"-OCHF₂ and the meaning for the combination of (R¹)ₘ, (R²)ₙ, R³ and R⁴ for each individual compound corresponds in each case to one line of Table A (compounds IIA.K.11-7.A-1 to IIA.K.11-7.A-1680).
Table 11-8 Compounds of the formula IIA.K in which o is 1, R⁷⁸ is 1"-C₆H₅ and the meaning for the combination of (R¹)ₘ, (R²)ₙ, R³ and R⁴ for each individual compound corresponds in each case to one line of Table A (compounds IIA.K.11-8.A-1 to IIA.K.11-8.A-1680).
Table 11-9 Compounds of the formula IIA.K in which o is 1, R⁷⁸ is 3"-F and the meaning for the combination of (R¹)ₘ, (R²)ₙ, R³ and R⁴ for each individual compound corresponds in each case to one line of Table A (compounds IIA.K.11-9.A-1 to IIA.K.11-9.A-1680).
Table 11-10 Compounds of the formula IIA.K in which o is 1, R⁷⁸ is 3"-CI and the meaning for the combination of (R¹)ₘ, (R²)ₙ, R³ and R⁴ for each individual compound corresponds in each case to one line of Table A (compounds IIA.K.11-10.A-1 to IIA.K.11-10.A-1680).
Table 11-11 Compounds of the formula IIA.K in which o is 1, R⁷⁸ is 3"-Br and the meaning for the combination of (R¹)ₘ, (R²)ₙ, R³ and R⁴ for each individual compound corresponds in each case to one line of Table A (compounds IIA.K.11-11.A-1 to IIAK.11-11.A-1680).
Table 11-12 Compounds of the formula IIA.K in which o is 1, R⁷⁸ is 3"-CH₃ and the meaning for the combination of (R¹)ₘ, (R²)ₙ, R³ and R⁴ for each individual compound corresponds in each case to one line of Table A (compounds IIA.K.11-12.A-1 to IIA.K.11-12.A-1680).
Table 11-13 Compounds of the formula IIA.K in which o is 1, R⁷⁸ is 3"-OCH₃ and the meaning for the combination of (R¹)ₘ, (R²)ₙ, R³ and R⁴ for each individual compound corresponds in each case to one line of Table A (compounds IIA.K.11-13.A-1 to IIA.K.11-13.A-1680).
Table 11-14 Compounds of the formula IIA.K in which o is 1, R⁷⁸ is 3"-OCHF₂ and the meaning for the combination of (R¹)ₘ, (R²)ₙ, R³ and R⁴ for each individual compound corresponds in each case to one line of Table A (compounds IIA.K.11-14.A-1 to IIA.K.11-14.A-1680).
Table 11-15 Compounds of the formula IIA.K in which o is 1, R⁷⁸ is 3"-C₆H₅ and the meaning for the combination of (R¹)ₘ, (R²)ₙ, R³ and R⁴ for each individual compound corresponds in each case to one line of Table A (compounds IIA.K.11-15.A-1 to IIA.K.11-15.A-1680).
Table 11-16 Compounds of the formula IIA.K in which o is 1, R⁷⁸ is 4"-F and the meaning for the combination of (R¹)ₘ, (R²)ₙ, R³ and R⁴ for each individual compound corresponds in each case to one line of Table A (compounds IIA.K.11-16.A-1 to IIA.K.11-16.A-1680).
Table 11-17 Compounds of the formula IIA.K in which o is 1, R⁷⁸ is 4"-CI and the meaning for the combination of (R¹)ₘ, (R²)ₙ, R³ and R⁴ for each individual compound corresponds in each case to one line of Table A (compounds IIA.K.11-17.A-1 to IIA.K.11-17.A-1680).
Table 11-18 Compounds of the formula IIA.K in which o is 1, R⁷⁸ is 4"-Br and the meaning for the combination of (R¹)ₘ, (R²)ₙ, R³ and R⁴ for each individual compound corresponds in each case to one line of Table A (compounds IIA.K.11-18.A-1 to IIA.K.11-18.A-1680).
Table 11-19 Compounds of the formula IIA.K in which o is 1, R⁷⁸ is 4"-CH₃ and the meaning for the combination of (R¹)ₘ, (R²)ₙ, R³ and R⁴ for each individual compound corresponds in each case to one line of Table A (compounds IIA.K.11-19.A-1 to IIA.K.11-19.A-1680).
Table 11-20 Compounds of the formula IIA.K in which o is 1, R⁷⁸ is 4"-OCH₃ and the meaning for the combination of (R¹)ₘ, (R²)ₙ, R³ and R⁴ for each individual compound corresponds in each case to one line of Table A (compounds IIA.K.11-20.A-1 to IIA.K.11-20.A-1680).
Table 11-21 Compounds of the formula IIA.K in which o is 1, R⁷⁸ is 4"-OCHF₂ and the meaning for the combination of (R¹)ₘ, (R²)ₙ, R³ and R⁴ for each individual compound corresponds in each case to one line of Table A (compounds IIA.K.11-21.A-1 to IIA.K.11-21.A-1680).

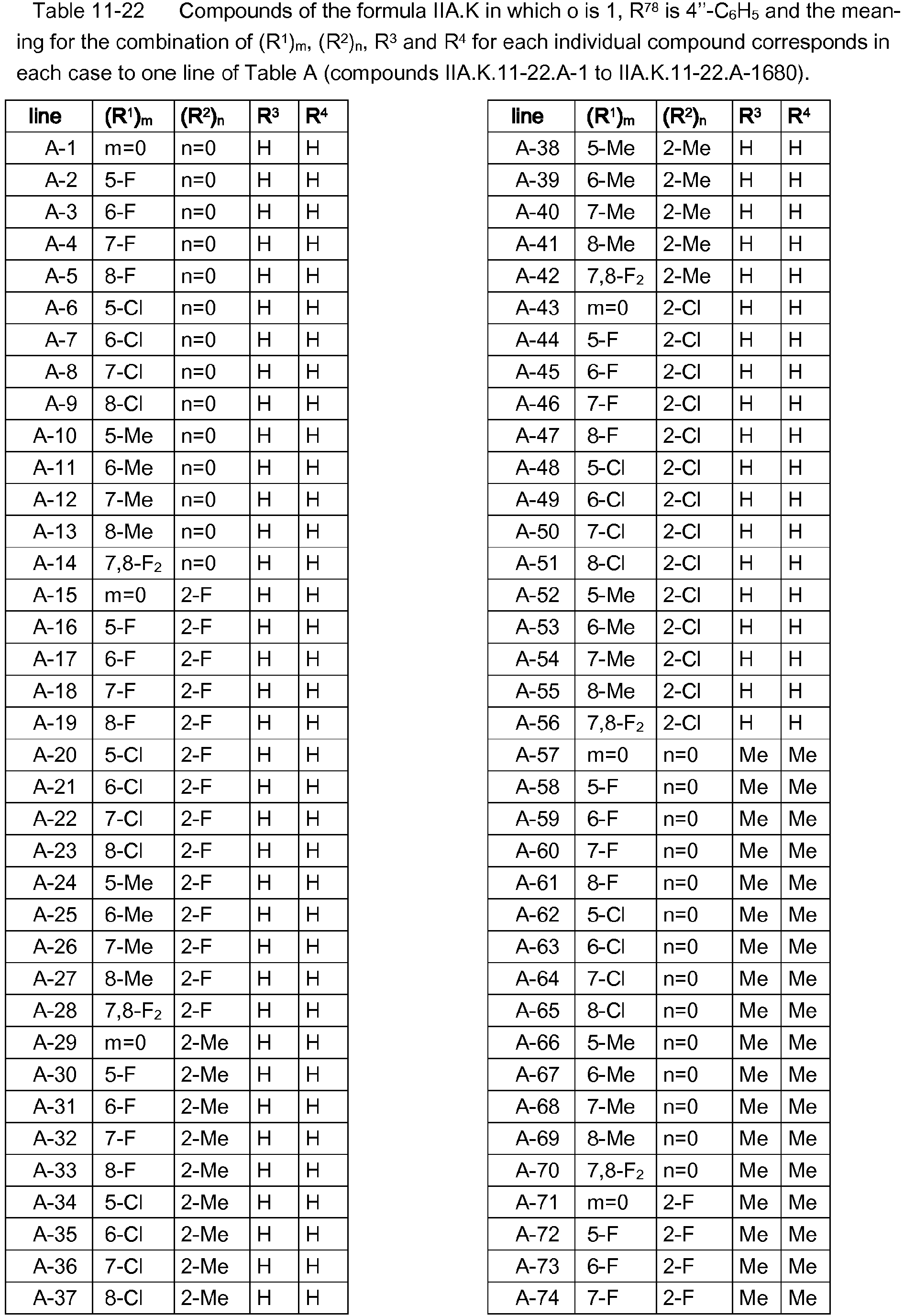

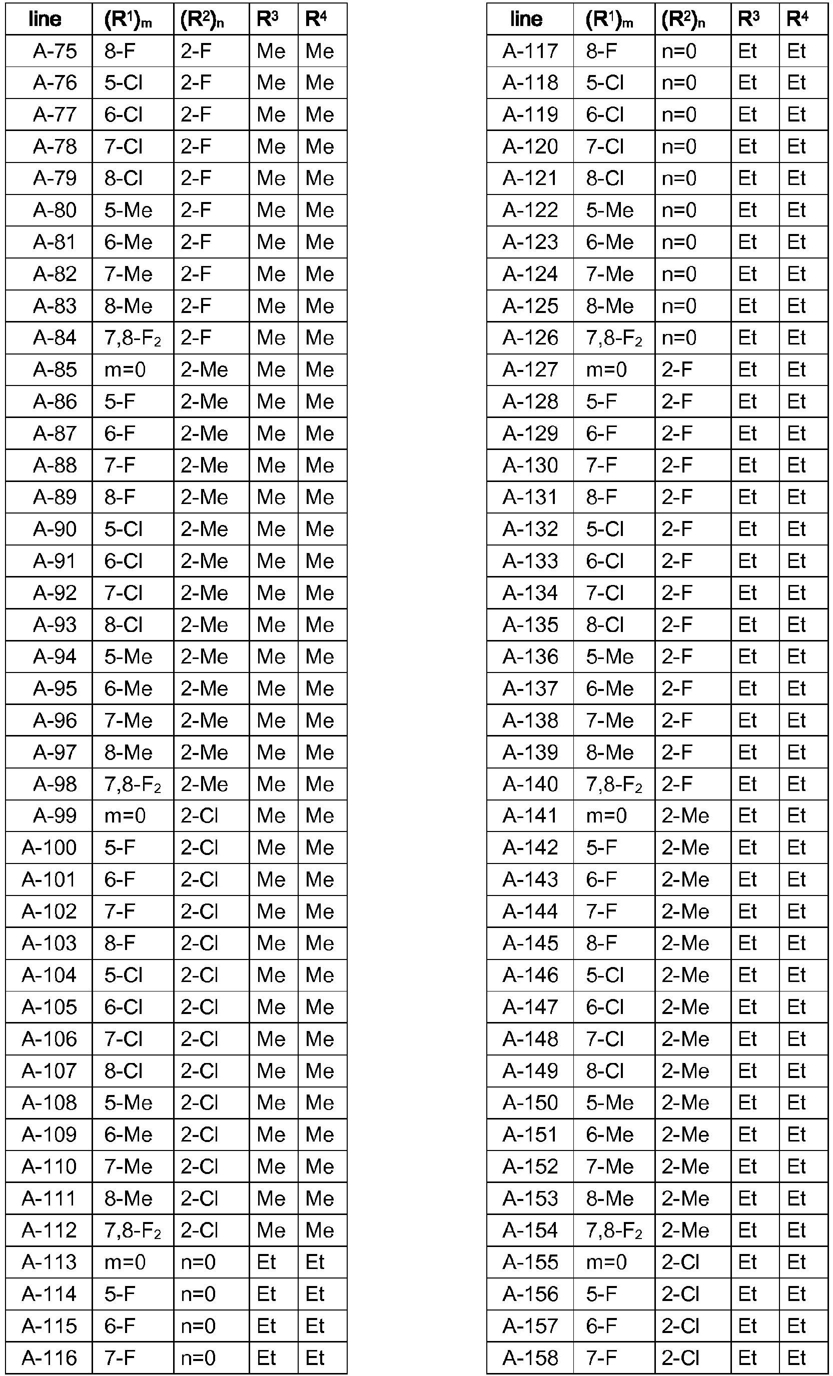

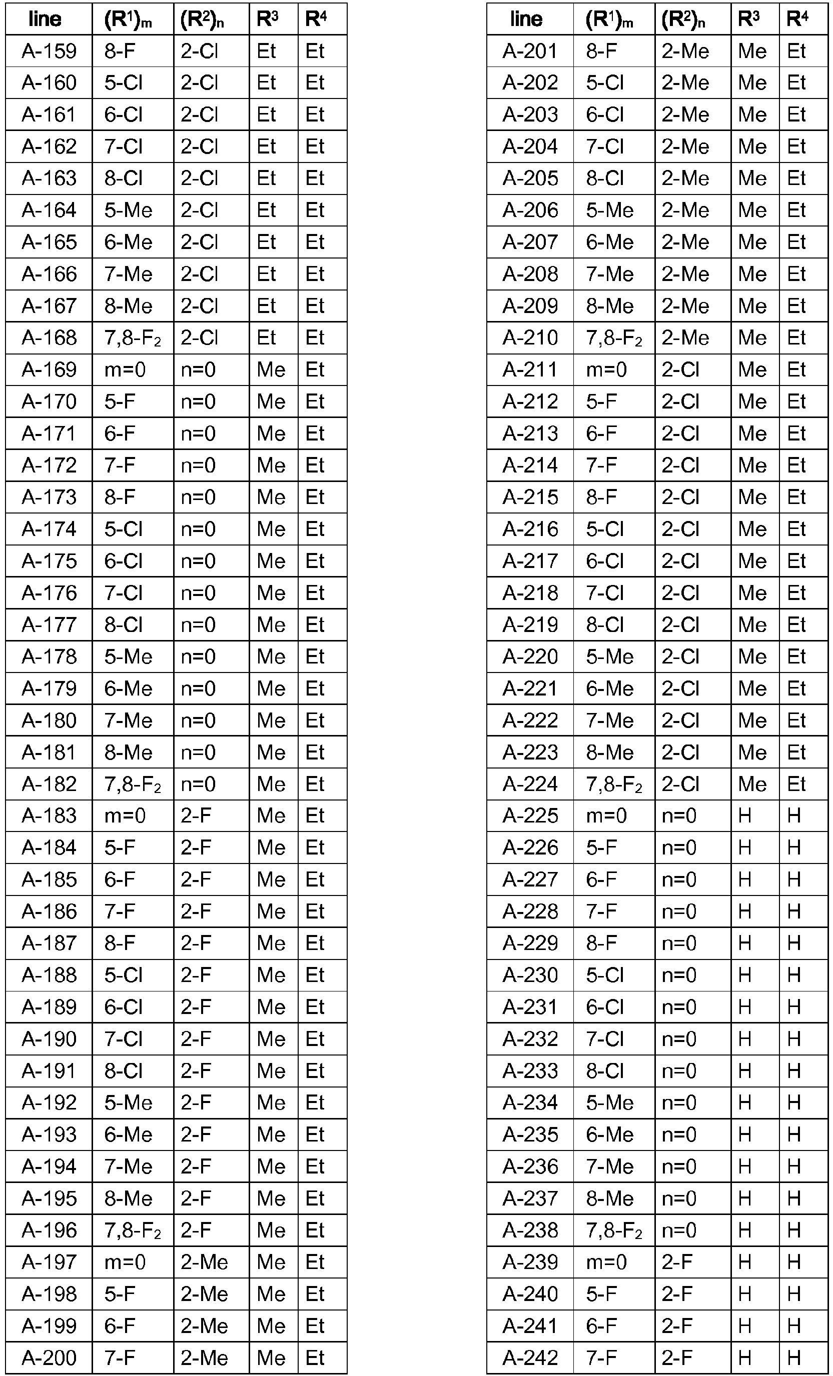

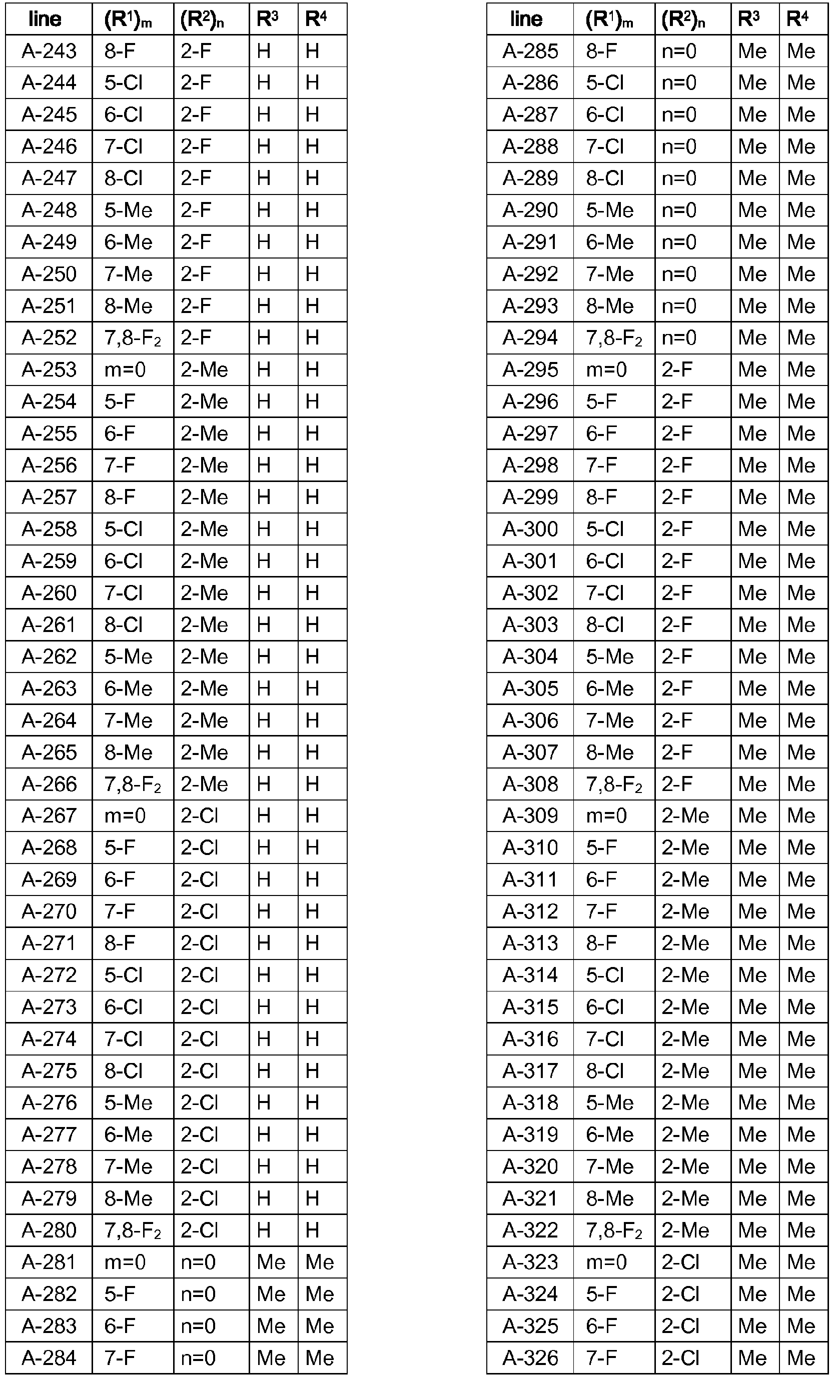

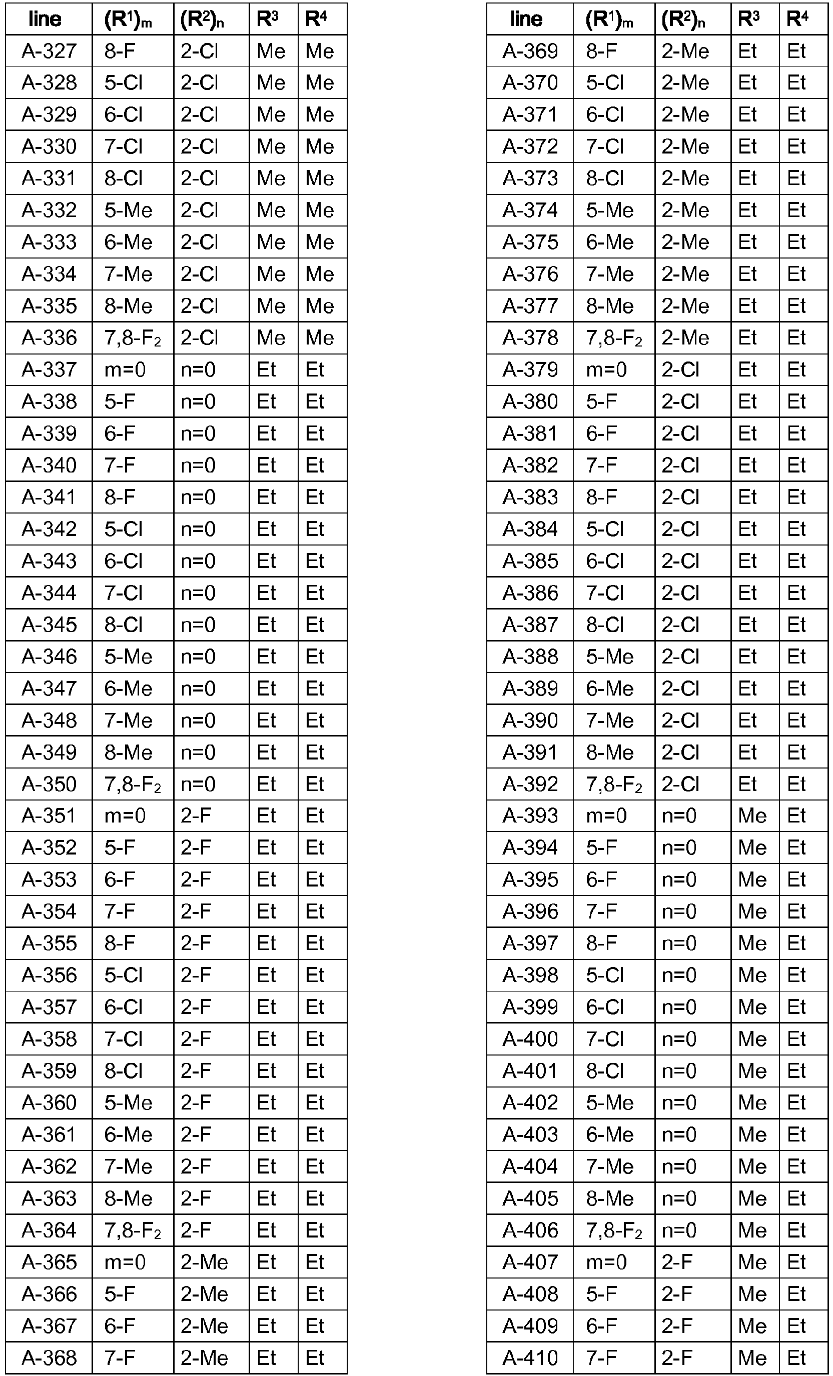

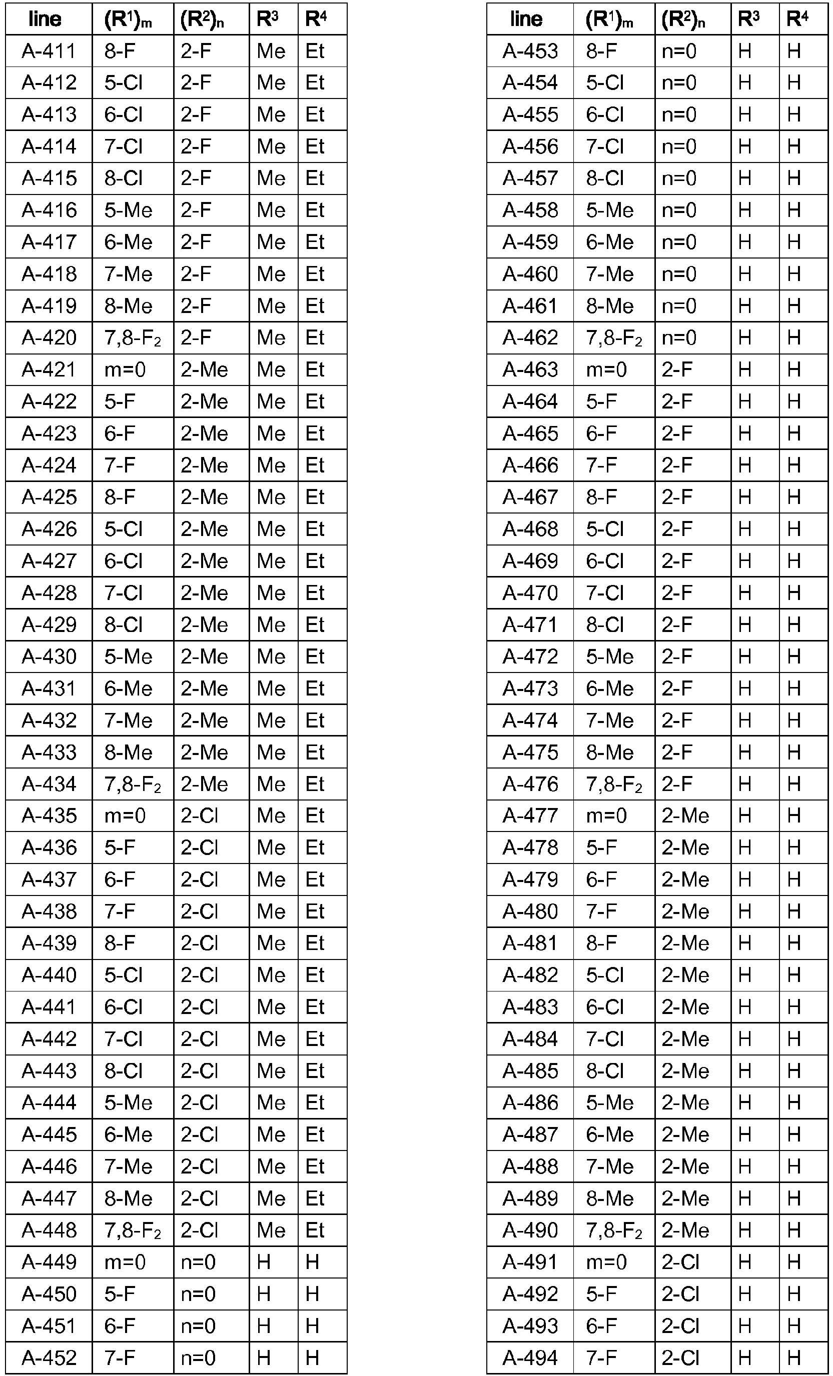

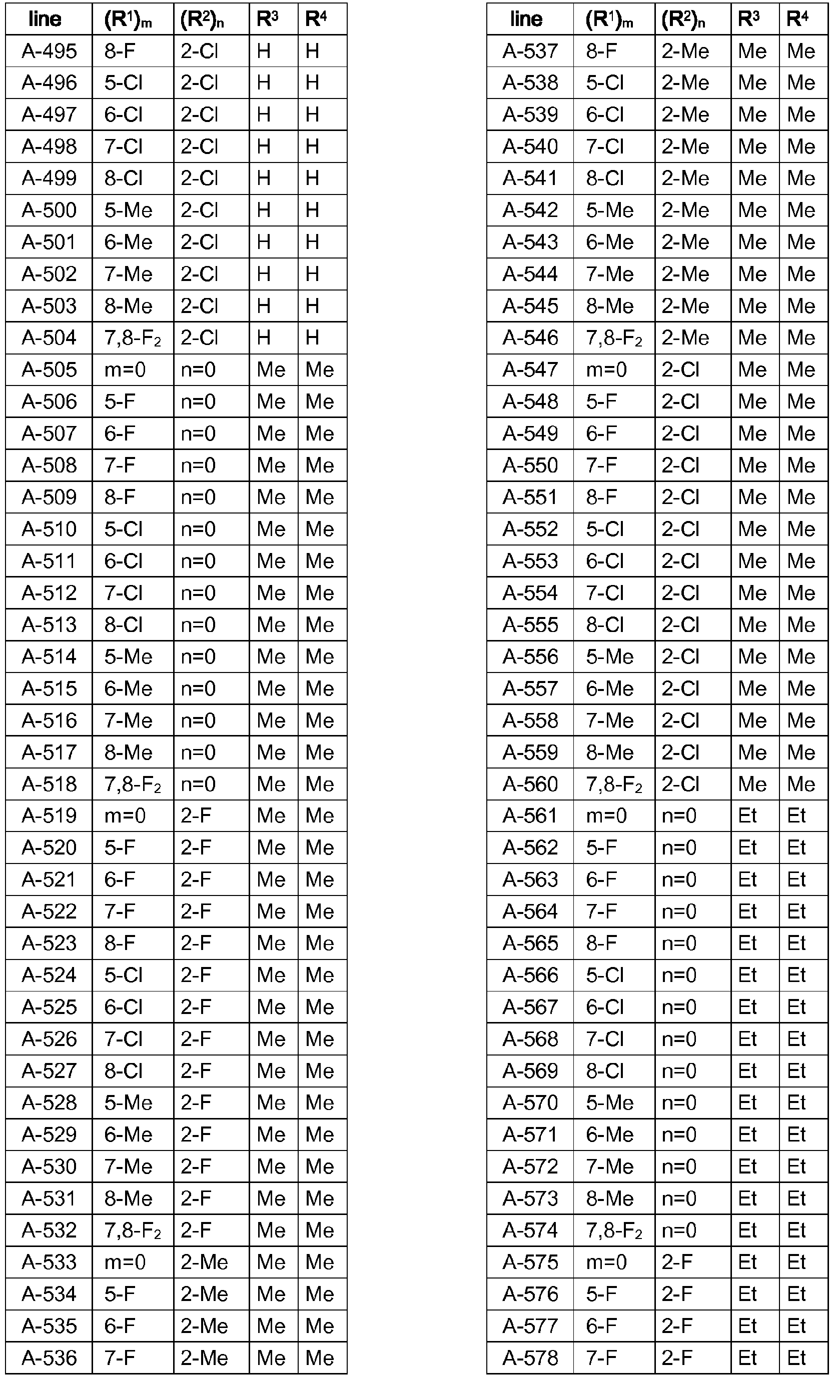

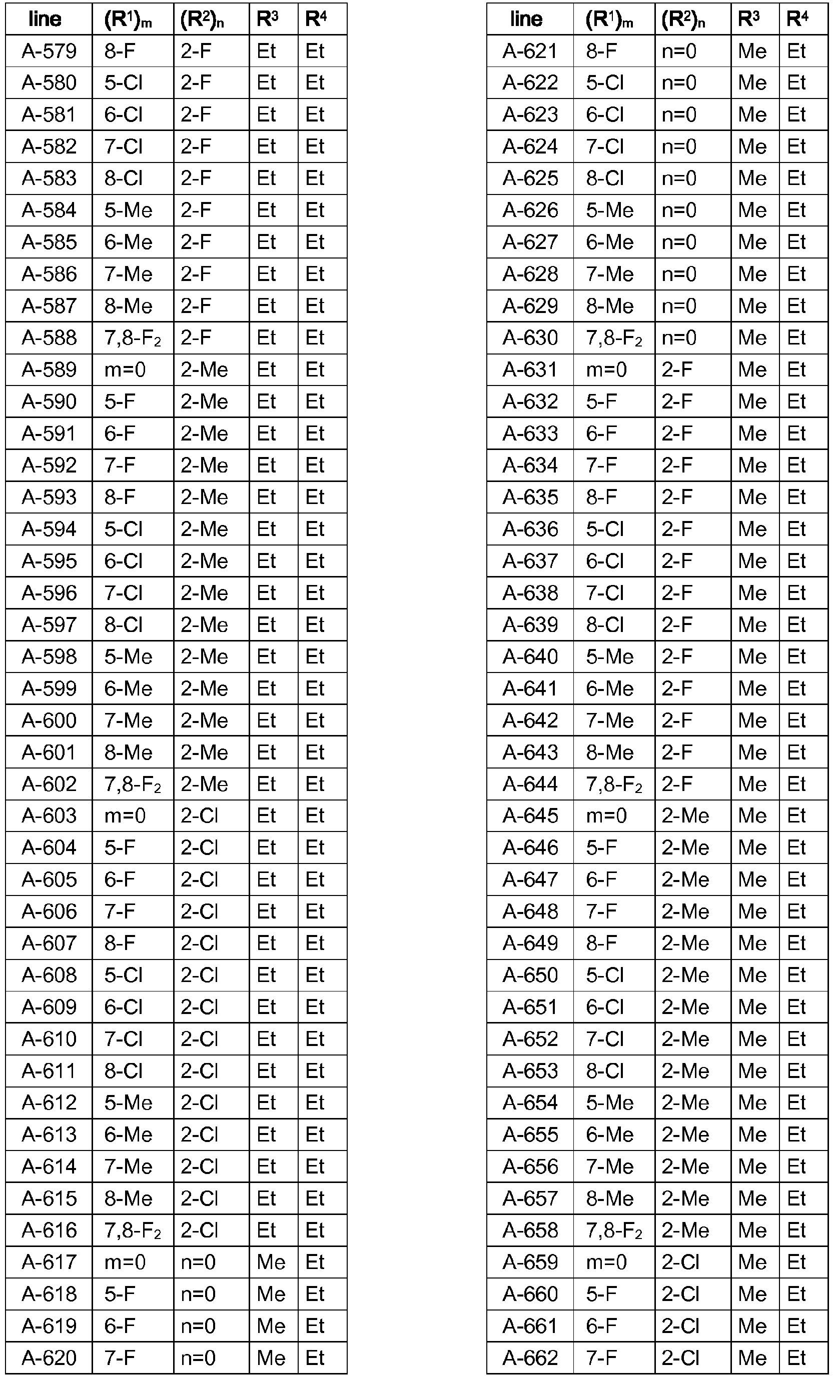

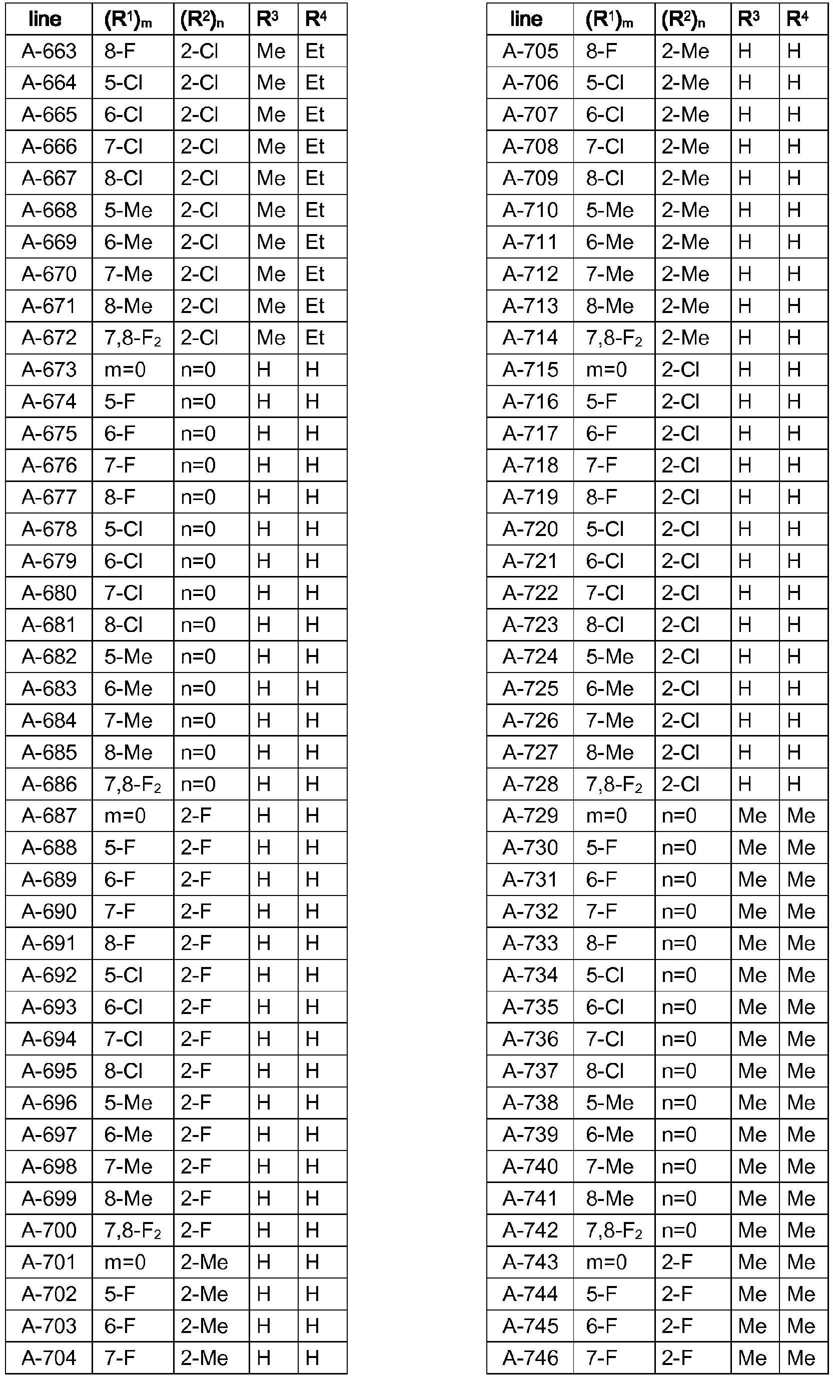

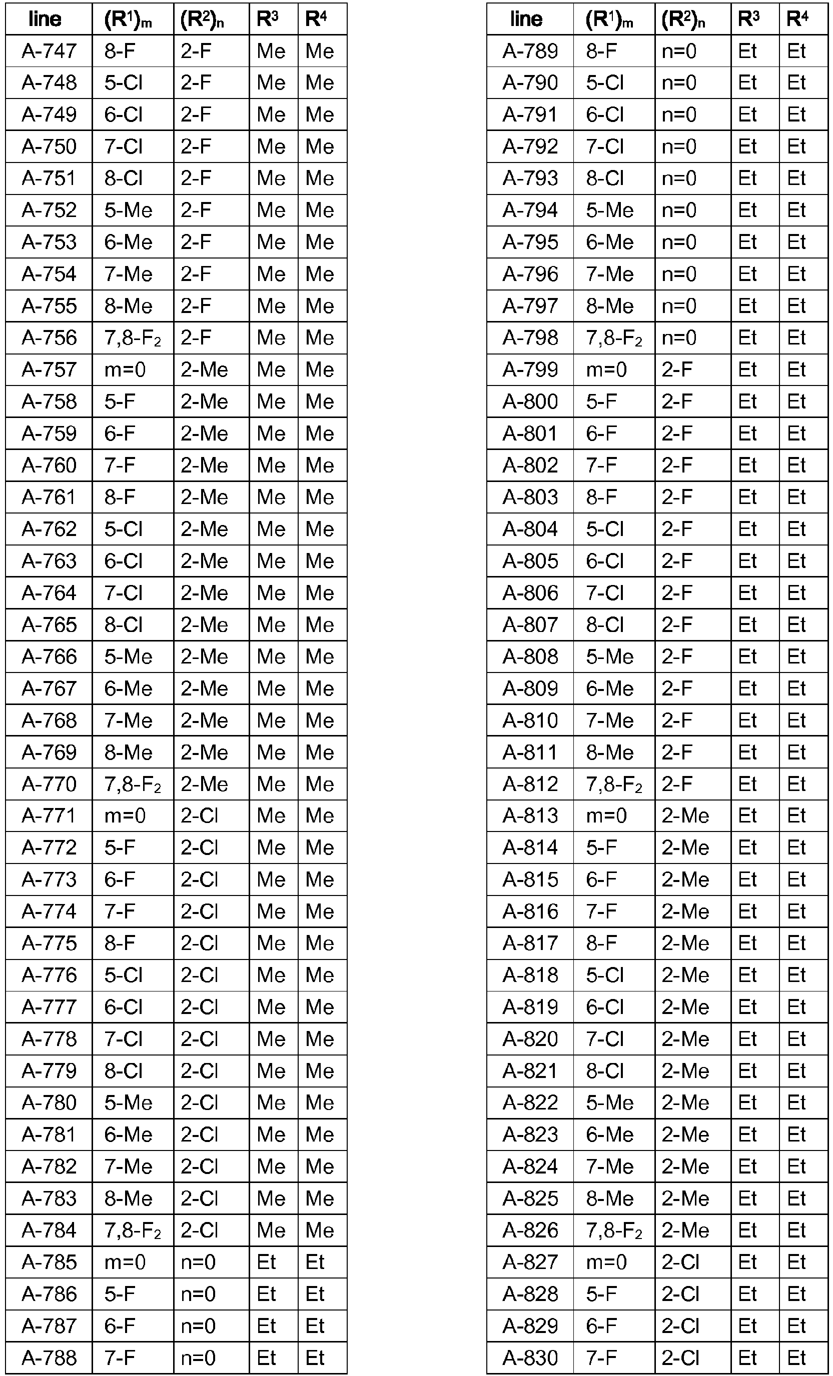

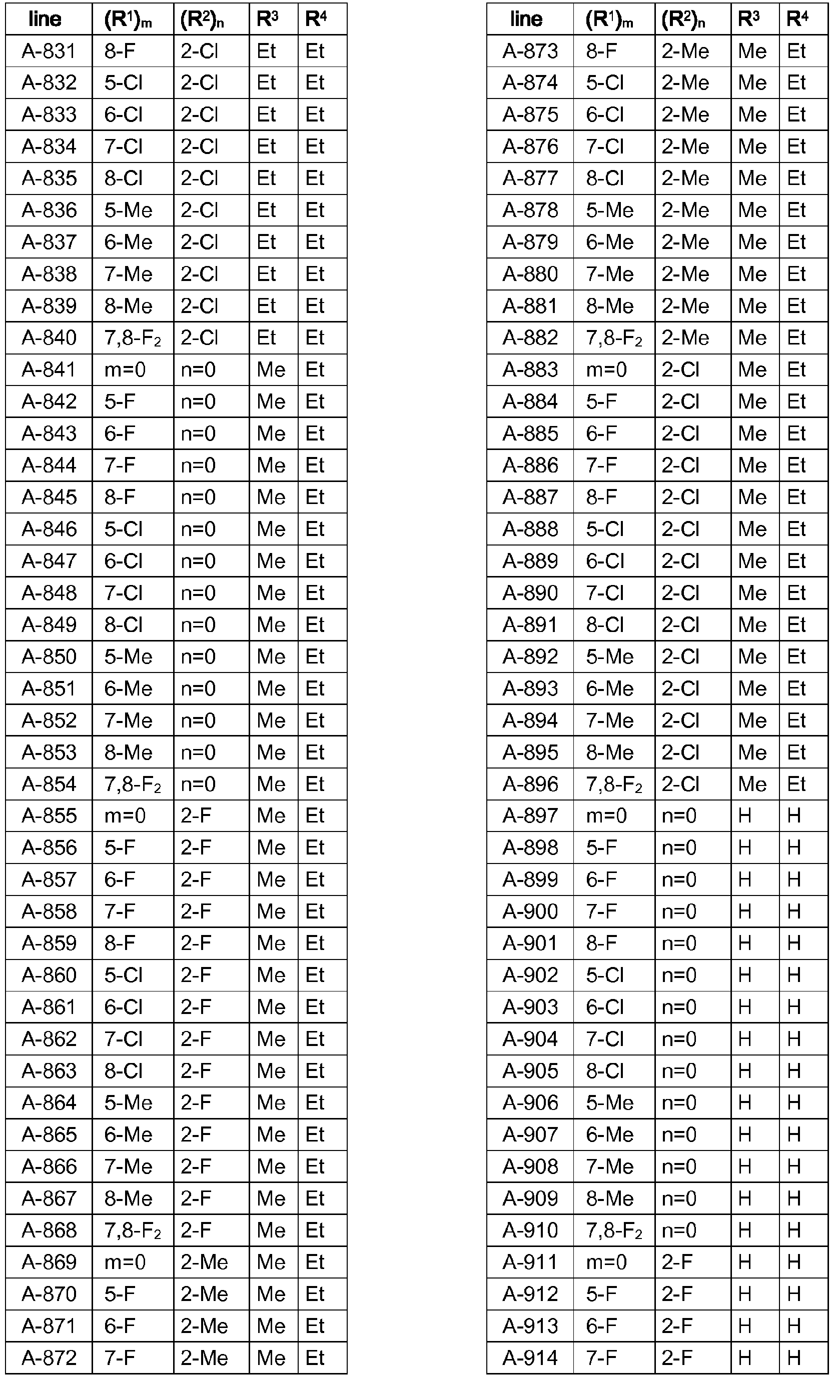

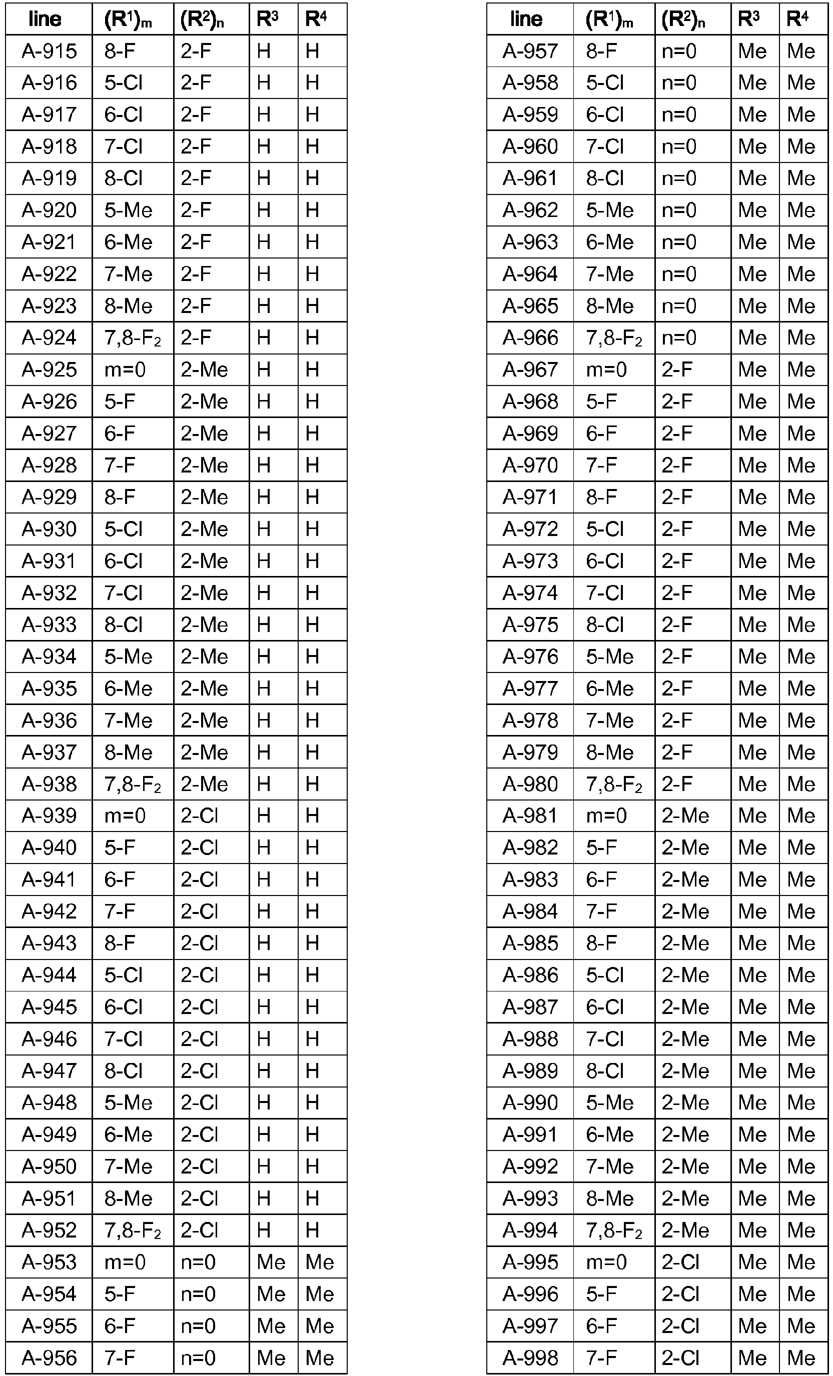

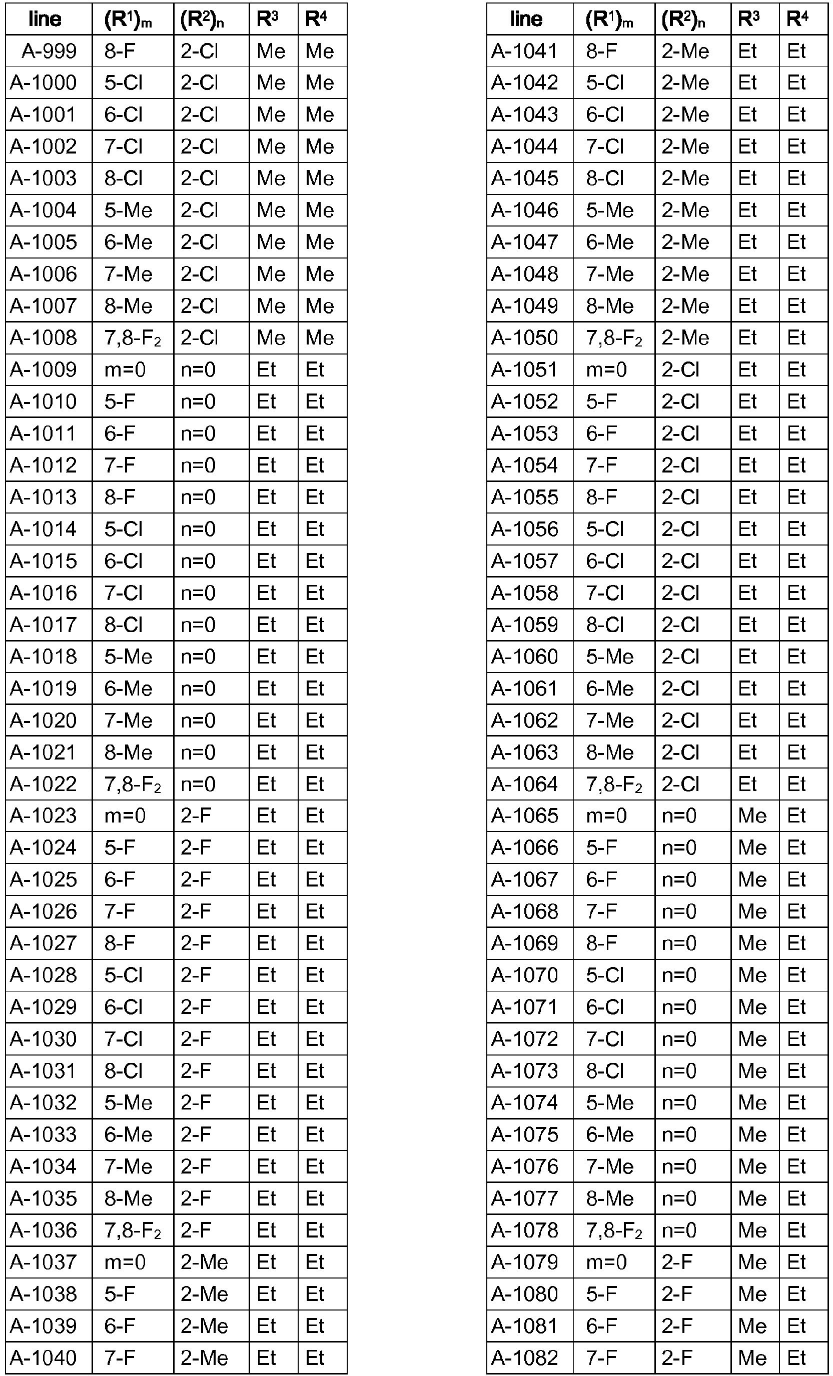

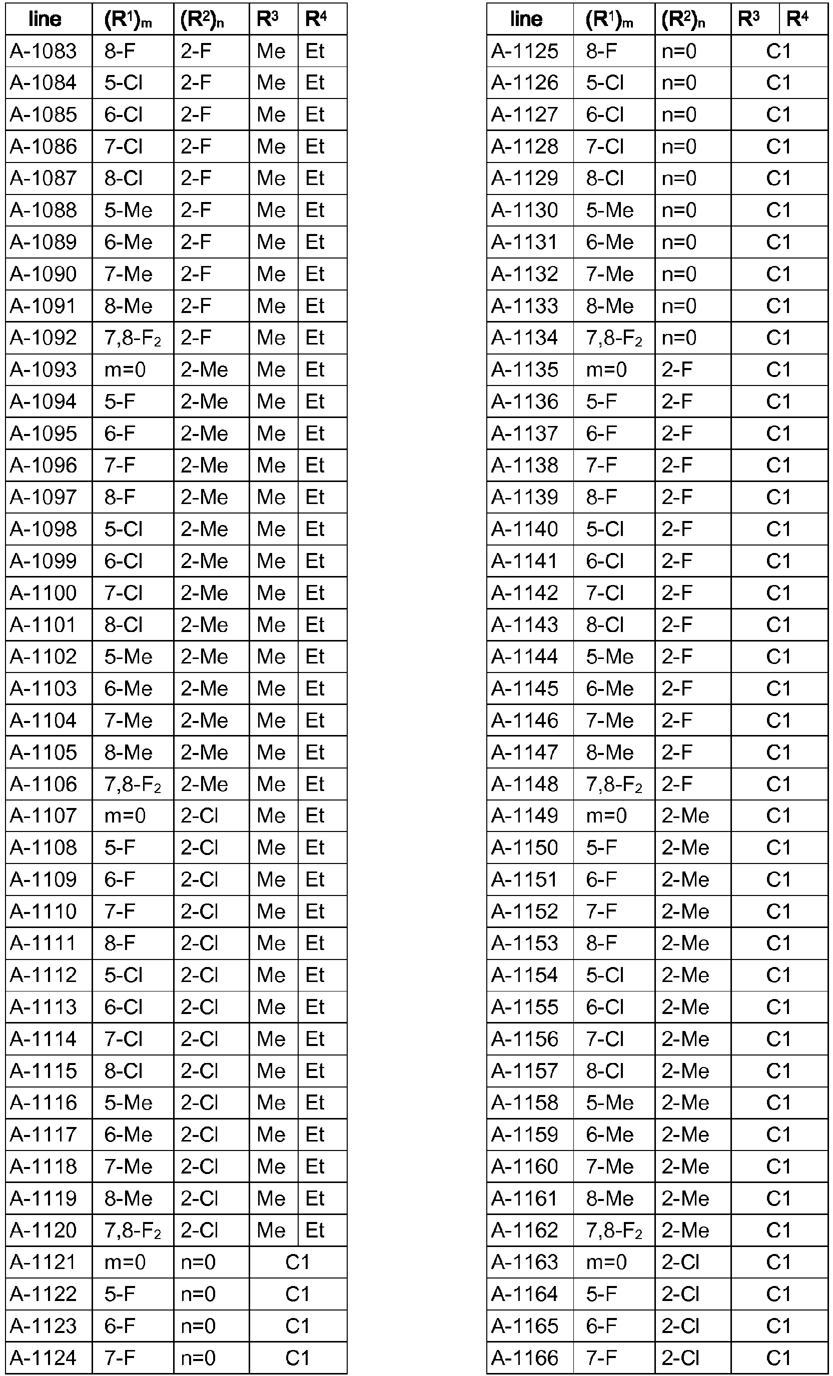

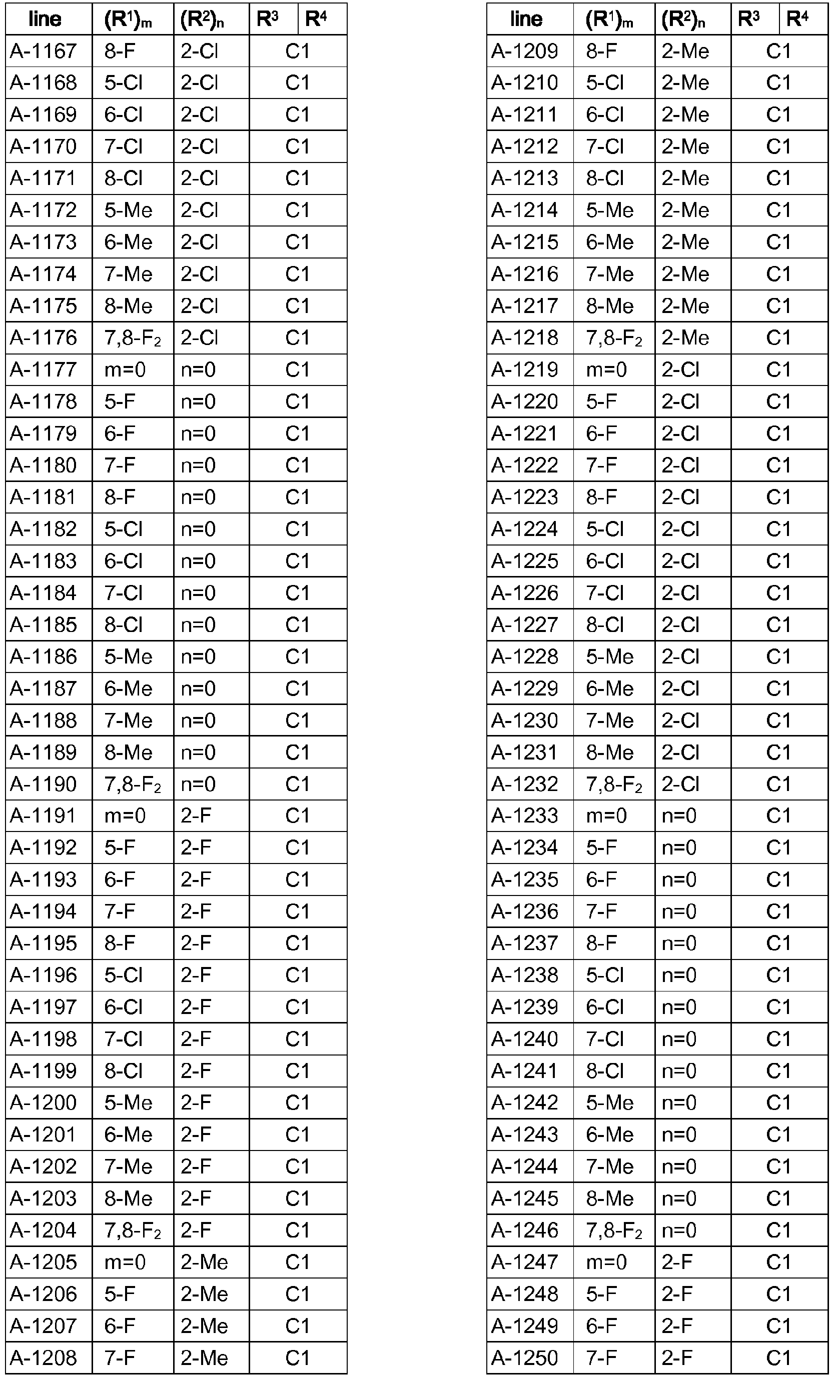

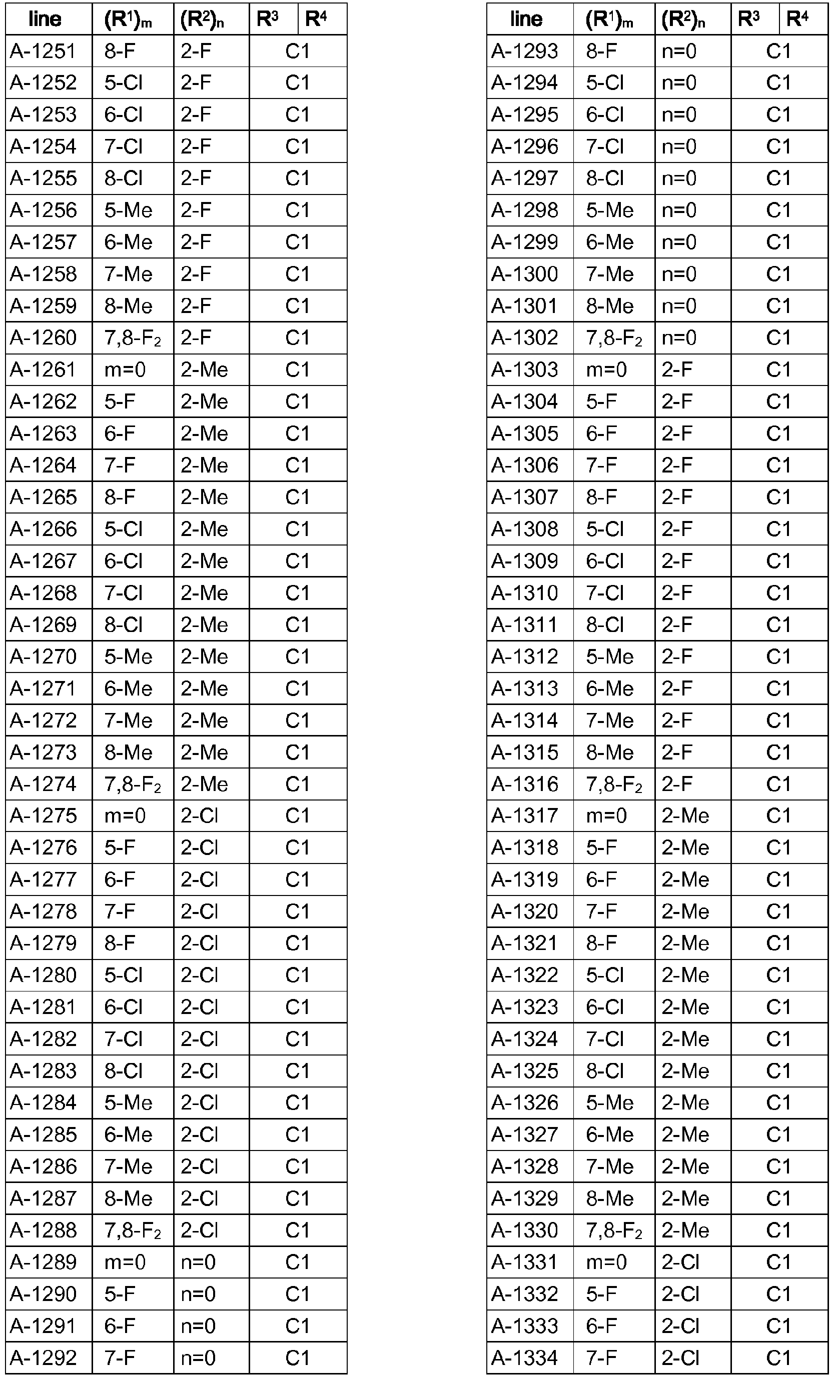

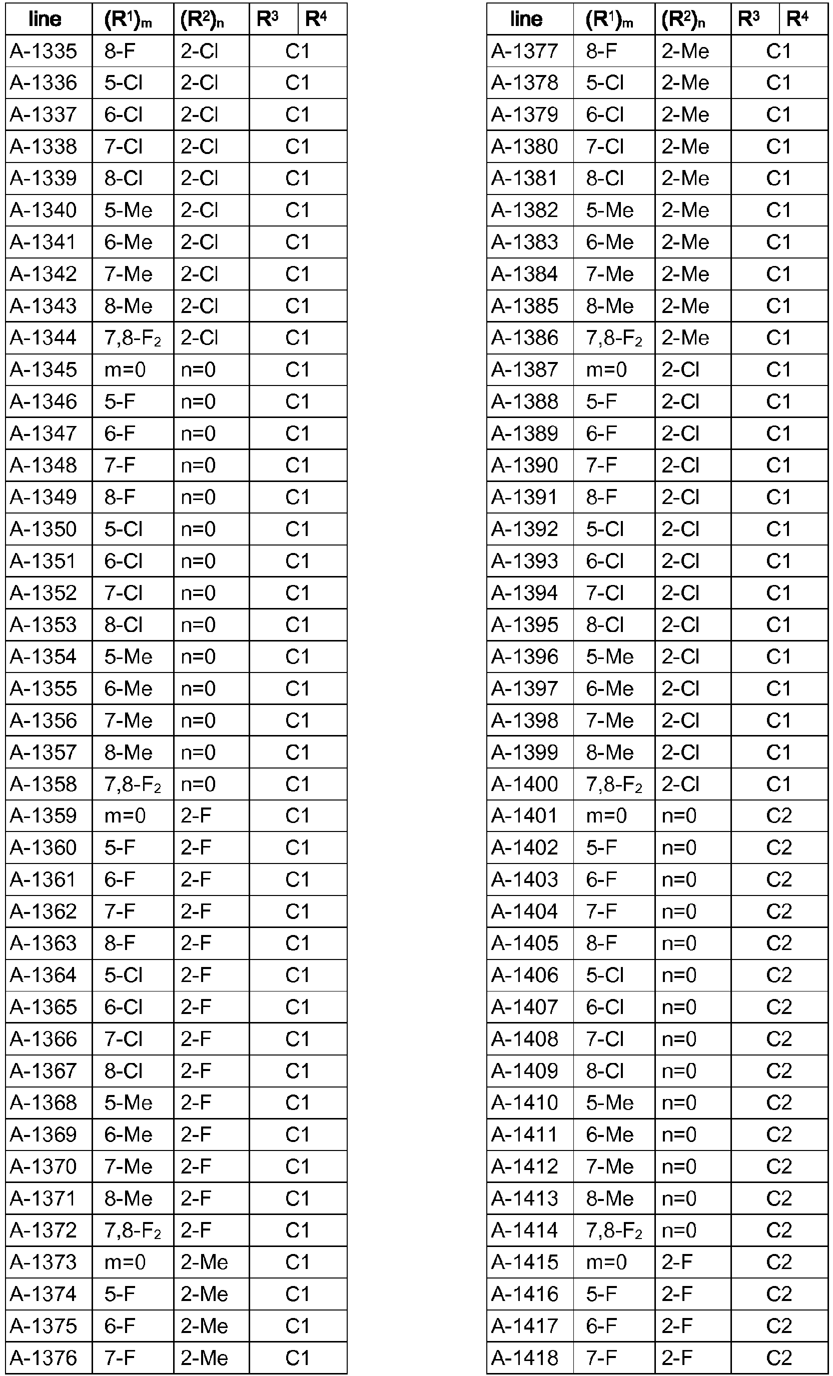

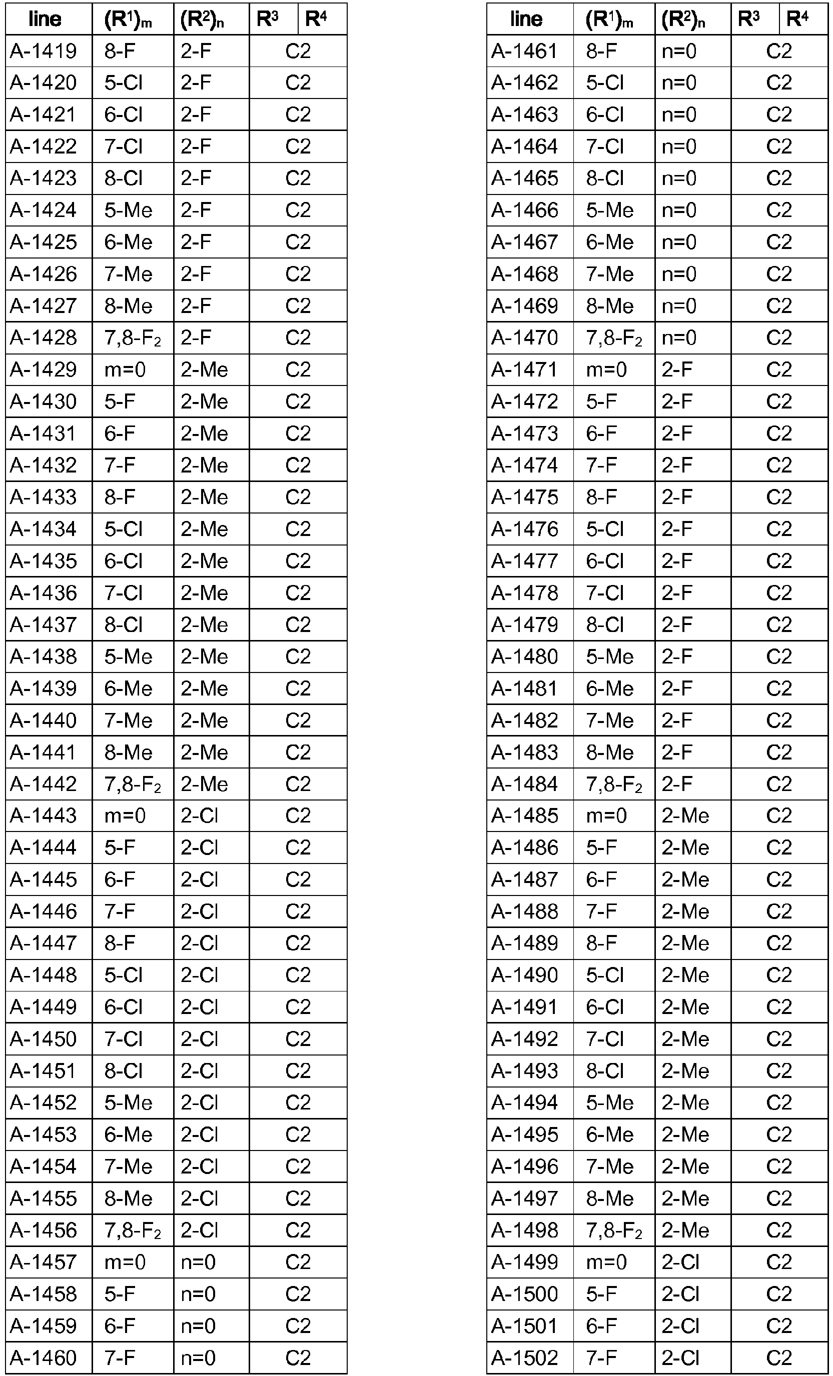

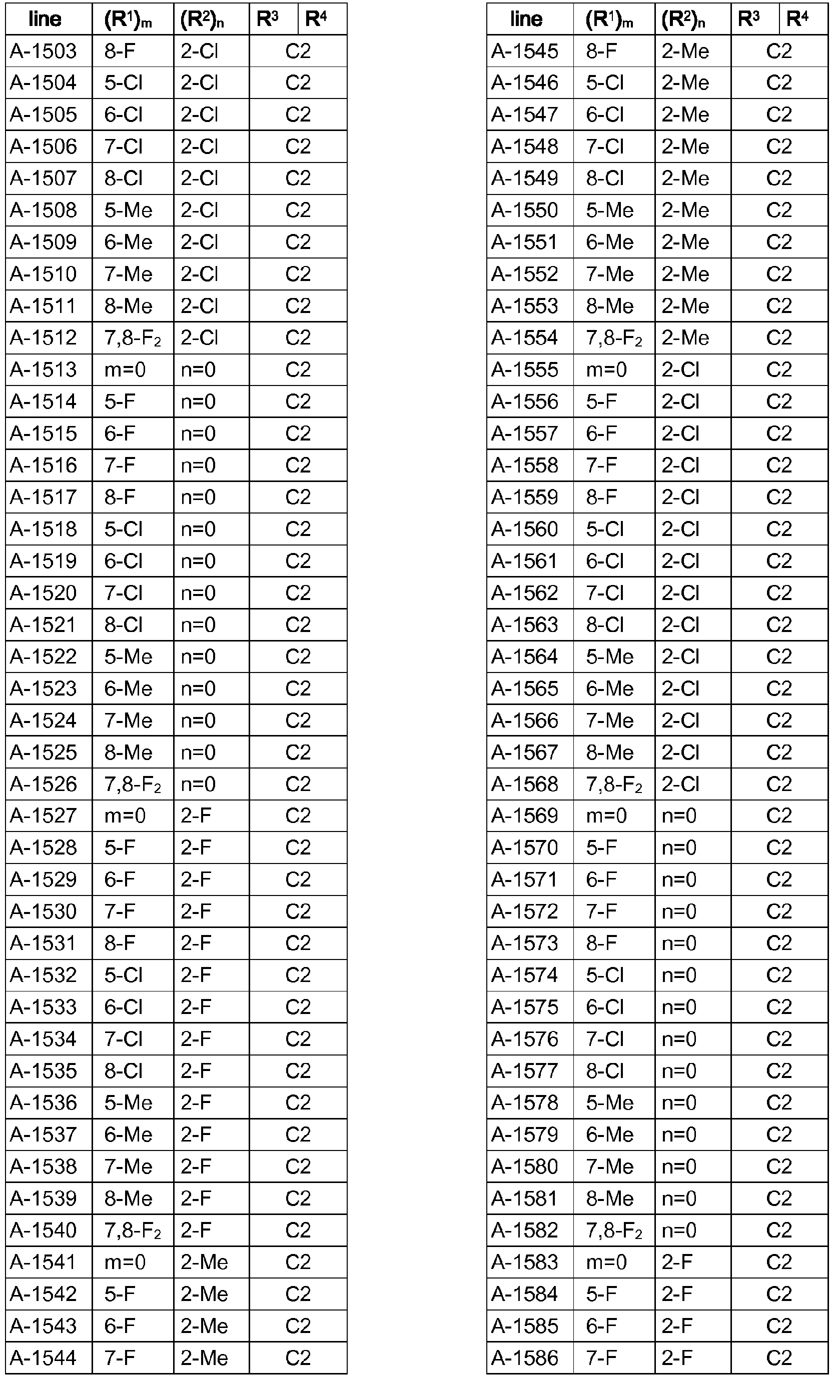

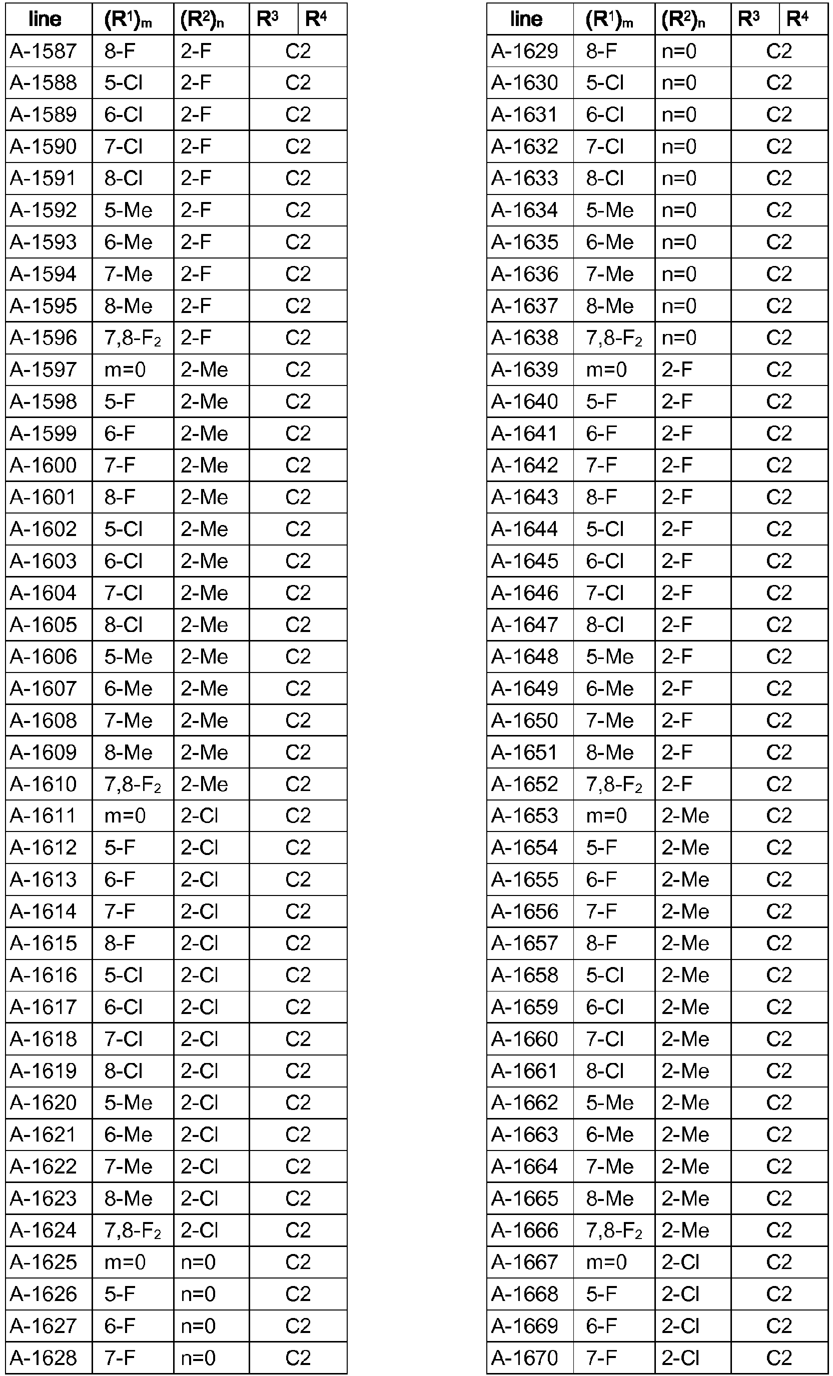

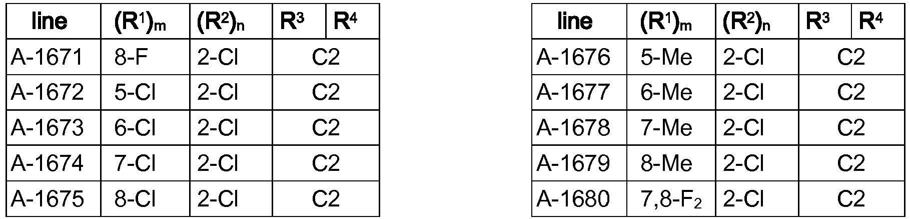

In the definitions of the variables given above, collective terms are used which are generally representative for the substituents in question. The term "Cₙ-Cₘ" indicates the number of carbon atoms possible in each case in the substituent or substituent moiety in question.

The term "halogen" refers to fluorine, chlorine, bromine and iodine.

The term "C₁-C₆-alkyl" refers to a straight-chained or branched saturated hydrocarbon group having 1 to 6 carbon atoms, e.g. methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. Likewise, the term "C₂-C₄-alkyl" refers to a straight-chained or branched alkyl group having 2 to 4 carbon atoms, such as ethyl, propyl (n-propyl), 1-methylethyl (iso-propoyl), butyl, 1-methylpropyl (sec.-butyl), 2-methylpropyl (iso-butyl), 1,1-dimethylethyl (tert.-butyl).

The term "C₁-C₆-halogenalkyl" refers to an alkyl group having 1 or 6 carbon atoms as defined above, wherein some or all of the hydrogen atoms in these groups may be replaced by halogen atoms as mentioned above. Examples are "C₁-C₂-halogenalkyl" groups such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl or pentafluoroethyl.

The term "C₁-C₆-hydroxyalkyl" refers to an alkyl group having 1 or 6 carbon atoms as defined above, wherein some or all of the hydrogen atoms in these groups may be replaced by OH groups.

The term "C₁-C₄-alkoxy-C₁-C₄-alkyl" refers to alkyl having 1 to 4 carbon atoms (as defined above), wherein one hydrogen atom of the alkyl radical is replaced by a C₁-C₄-alkoxy group (as defined above). Likewise, the term "C₁-C₆-alkoxy-C₁-C₄-alkyl" refers to alkyl having 1 to 4 carbon atoms (as defined above), wherein one hydrogen atom of the alkyl radical is replaced by a C₁-C₆-alkoxy group (as defined above).

The term "C₂-C₆-alkenyl" refers to a straight-chain or branched unsaturated hydrocarbon radical having 2 to 6 carbon atoms and a double bond in any position. Examples are "C₂-C₄-alkenyl" groups, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₆-alkynyl" refers to a straight-chain or branched unsaturated hydrocarbon radical having 2 to 6 carbon atoms and containing at least one triple bond. Examples are "C₂-C₄-alkynyl" groups, such as ethynyl, prop-1-ynyl, prop-2-ynyl (propargyl), but-1-ynyl, but-2-ynyl, but-3-ynyl, 1-methyl-prop-2-ynyl.

The term "C₁-C₆-alkoxy" refers to a straight-chain or branched alkyl group having 1 to 6 carbon atoms which is bonded via an oxygen, at any position in the alkyl group. Examples are "C₁-C₄-alkoxy" groups, such as methoxy, ethoxy, n-propoxy, 1-methylethoxy, butoxy, 1-methyl¬propoxy, 2-methylpropoxy or 1,1-dimethylethoxy.

The term "C₁-C₆-halogenalkoxy" refers to a C₁-C₆-alkoxy radical as defined above, wherein some or all of the hydrogen atoms in these groups may be replaced by halogen atoms as mentioned above. Examples are "C₁-C₄-halogenalkoxy" groups, such as OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-tri-fluoroethoxy, 2-chloro-2-fluoroethoxy, 2-chloro-2,2-difluoroethoxy, 2,2-dichloro-2-fluoroethoxy, 2,2,2-trichloro¬ethoxy, OC₂F₅, 2-fluoropropoxy, 3-fluoropropoxy, 2,2-difluoropropoxy, 2,3-difluoro¬propoxy, 2 chloropropoxy, 3-chloropropoxy, 2,3-dichloropropoxy, 2-bromo¬propoxy, 3 bromopropoxy, 3,3,3-trifluoropropoxy, 3,3,3-trichloropropoxy, OCH₂-C₂F₅, OCF₂-C₂F₅, 1-fluoromethyl-2-fluoroethoxy, 1-chloromethyl-2-chloroethoxy, 1-bromomethyl-2-bromo¬ethoxy, 4-fluorobutoxy, 4-chlorobutoxy, 4-bromobutoxy or nonafluorobutoxy.

The term "C₂-C₆-alkenyloxy" refers to a straight-chain or branched alkenyl group having 2 to 6 carbon atoms which is bonded via an oxygen, at any position in the alkenyl group. Examples are "C2-C4-alkenyloxy" groups.

The term "C₂-C₆-alkynyloxy" refers to a straight-chain or branched alkynyl group having 2 to 6 carbon atoms which is bonded via an oxygen, at any position in the alkynyl group. Examples are "C₂-C₄-alkynyloxy" groups.

The term "C₃-C₆-cycloalkyl" refers to monocyclic saturated hydrocarbon radicals having 3 to 6 carbon ring members, such as cyclopropyl, cyclobutyl, cyclopentyl, cyclohexyl. Accordingly, a saturated three-, four-, five-, six-, seven-, eight-, nine or ten-membered carbocyclyl or carbocycle is a "C₃-C₁₀-cycloalkyl".

The term "C₃-C₆-cycloalkenyl" refers to a monocyclic partially unsaturated 3-, 4- 5- or 6-membered carbocycle having 3 to 6 carbon ring members and at least one double bond, such as cyclopentenyl, cyclopentadienyl, cyclohexadienyl. Accordingly, a partially unsaturated three-, four-, five-, six-, seven-, eight-, nine or ten-membered carbocyclyl or carbocycle is a "C₃-C₁₀-cycloalkenyl".

The term "C₃-C₈-cycloalkyl-C₁-C₄-alkyl" refers to alkyl having 1 to 4 carbon atoms (as defined above), wherein one hydrogen atom of the alkyl radical is replaced by a cycloalkyl radical having 3 to 8 carbon atoms (as defined above).

The term "C₁-C₆-alkylthio" as used herein refers to straight-chain or branched alkyl groups having 1 to 6 carbon atoms (as defined above) bonded via a sulfur atom. Accordingly, the term "C₁-C₆-halogenalkylthio" as used herein refers to straight-chain or branched halogenalkyl group having 1 to 6 carbon atoms (as defined above) bonded through a sulfur atom, at any position in the halogenalkyl group.

The term "C(=O)-C₁-C₆-alkyl" refers to a radical which is attached through the carbon atom of the group C(=O) as indicated by the number valence of the carbon atom. The number of valence of carbon is 4, that of nitrogen is 3. Likewise the following terms are to be construed: NH(C₁-C₄-alkyl), N(C₁-C₄-alkyl)₂, NH(C3-C6-cycloalkyl), N(C₃-C₆-cycloalkyl)₂, C(=O)-NH(C₁-C₆-alkyl), C(=O)-N(C₁-C₆-alkyl)₂.

The term "saturated or partially unsaturated three-, four-, five-, six-, seven-, eight-, nine or ten-membered heterocyclyl or heterocycle, wherein the heterocyclyl or heterocycle contains 1, 2, 3 or 4 heteroatoms selected from N, O and S" is to be understood as meaning both saturated and partially unsaturated heterocycles, wherein the ring member atoms of the heterocycle include besides carbon atoms 1, 2, 3 or 4 heteroatoms independently selected from the group of O, N and S. For example:
a 3- or 4-membered saturated heterocycle which contains 1 or 2 heteroatoms from the group consisting of O, N and S as ring members such as oxirane, aziridine, thiirane, oxetane, azetidine, thiethane, [1,2]dioxetane, [1,2]dithietane, [1,2]diazetidine; and
a 5- or 6-membered saturated or partially unsaturated heterocycle which contains 1, 2 or 3 heteroatoms from the group consisting of O, N and S as ring members such as 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-piperidinyl, 3-piperidinyl, 4-piperidinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl and also the corresponding -ylidene radicals; and
a 7-membered saturated or partially unsaturated heterocycle such as tetra- and hexahydroazepinyl, such as 2,3,4,5-tetrahydro[1H]azepin-1-,-2-,-3-,-4-,-5-,-6- or-7-yl, 3,4,5,6-tetra-hydro[2H]azepin-2-,-3-,-4-,-5-,-6- or-7-yl, 2,3,4,7-tetrahydro[1H]azepin-1-,-2-,-3-,-4-,-5-,-6- or-7-yl, 2,3,6,7-tetrahydro[1H]azepin-1-,-2-,-3-,-4-,-5-,-6- or-7-yl, hexahydroazepin-1-,-2-,-3- or-4-yl, tetra- and hexahydrooxepinyl such as 2,3,4,5-tetrahydro[1H]oxepin-2-,-3-,-4-,-5-,-6- or-7-yl, 2,3,4,7-tetrahydro[1H]oxepin-2-,-3-,-4-,-5-,-6- or-7-yl, 2,3,6,7-tetrahydro[1H]oxepin-2-, -3-,-4-,-5-,-6- or-7-yl, hexahydroazepin-1-,-2-,-3- or-4-yl, tetra- and hexahydro-1,3-diazepinyl, tetra- and hexahydro-1,4-diazepinyl, tetra- and hexahydro-1,3-oxazepinyl, tetra- and hexahydro-1,4-oxazepinyl, tetra- and hexahydro-1,3-dioxepinyl, tetra- and hexahydro-1,4-dioxepinyl and the corresponding -ylidene radicals.

The term "5-or 6-membered heteroaryl" refers to aromatic ring systems incuding besides carbon atoms, 1, 2, 3 or 4 heteroatoms independently selected from the group consisting of N, O and S, for example,
a 5-membered heteroaryl such as pyrrol-1-yl, pyrrol-2-yl, pyrrol-3-yl, thien-2-yl, thien-3-yl, furan-2-yl, furan-3-yl, pyrazol-1-yl, pyrazol-3-yl, pyrazol-4-yl, pyrazol-5-yl, imidazol-1-yl, imidazol-2-yl, imidazol-4-yl, imidazol-5-yl, oxazol-2-yl, oxazol-4-yl, oxazol-5-yl, isoxazol-3-yl, isoxazol-4-yl, isoxazol-5-yl, thiazol-2-yl, thiazol-4-yl, thiazol-5-yl, isothiazol-3-yl, isothiazol-4-yl, isothiazol-5-yl, 1,2,4-triazolyl-1-yl, 1,2,4-triazol-3-yl 1,2,4-triazol-5-yl, 1,2,4-oxadiazol-3-yl, 1,2,4-oxadiazol-5-yl and 1,2,4-thiadiazol-3-yl, 1,2,4-thiadiazol-5-yl; or
a 6-membered heteroaryl, such as pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl and 1,3,5-triazin-2-yl and 1,2,4-triazin-3-yl.

Agriculturally acceptable salts of the inventive compounds encompass especially the salts of those cations or the acid addition salts of those acids whose cations and anions, respectively, have no adverse effect on the fungicidal action of said compounds. Suitable cations are thus in particular the ions of the alkali metals, preferably sodium and potassium, of the alkaline earth metals, preferably calcium, magnesium and barium, of the transition metals, preferably manganese, copper, zinc and iron, and also the ammonium ion which, if desired, may carry one to four C₁-C₄-alkyl substituents and/or one phenyl or benzyl substituent, preferably diisopropylammonium, tetramethylammonium, tetrabutylammonium, trimethylbenzylammonium, furthermore phosphonium ions, sulfonium ions, preferably tri(C₁-C₄-alkyl)sulfonium, and sulfoxonium ions, preferably tri(C₁-C₄-alkyl)sulfoxonium. Anions of useful acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate. They can be formed by reacting such inventive compound with an acid of the corresponding anion, preferably of hydrochloric acid, hydrobromic acid, sulfuric acid, phosphoric acid or nitric acid.

The inventive compounds can be present in atropisomers arising from restricted rotation about a single bond of asymmetric groups. They also form part of the subject matter of the present invention.

Depending on the substitution pattern, the compounds of formula I and their N-oxides may have one or more centers of chirality, in which case they are present as pure enantiomers or pure diastereomers or as enantiomer or diastereomer mixtures. Both, the pure enantiomers or diastereomers and their mixtures are subject matter of the present invention.

In the following, particular embodiments of the inventive compounds are described. Therein, specific meanings of the respective substituents are further detailled, wherein the meanings are in each case on their own but also in any combination with one another, particular embodiments of the present invention.

Furthermore, in respect of the variables, generally, the embodiments of the compounds I also apply to the intermediates.

R¹ according to the invention is in each case independently selected from halogen, OH, CN, NO₂, SH, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl, C₁-C₆-alkoxy, C₃-C₆-cycloalkyl, five- or six-membered heteroaryl and aryl; wherein the heteroaryl contains one, two or three heteroatoms selected from N, O and S; and wherein
R^{x} is C₁-C₄-alkyl, C₁-C₄-halogenalkyl, unsubstituted aryl or aryl that is substituted by one, two, three, four or five substituents R^{x1} independently selected from C₁-C₄-alkyl;
wherein the aliphatic moieties of R¹ are not further substituted or carry one, two, three or up to the maximum possible number of identical or different groups R^{1a} which independently of one another are selected from:
R^{1a} halogen, OH, CN, C₁-C₆-alkoxy, C₃-C₆-cycloalkyl, C₃-C₆-halogencycloalkyl, C₁-C₄-halogenalkoxy, C₁-C₆-alkylthio and phenoxy, wherein the phenyl group is unsubstituted or carries one, two, three, four or five substituents R^{11a} selected from the group consisting of halogen, OH, C₁-C₄-alkyl, C₁-C₄-halogenalkyl, C₁-C₄-alkoxy and C₁-C₄-halogenalkoxy;
wherein the cycloalkyl, heteroaryl and aryl moieties of R¹ are not further substituted or carry one, two, three, four, five or up to the maximum number of identical or different groups R^{1b} which independently of one another are selected from:
R^{1b} halogen, OH, CN, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy and C₁-C₆-alkylthio.

According to the invention, there can be zero, one, two, three or four R¹ present, namely for m is 0, 1, 2, 3 or 4.

According to one embodiment, m is 0.

According to a further embodiment, m is 1.

According to a further embodiment, m is 2 or 3. According to one specific embodiment thereof, m is 2, according to a further specific embodiment, m is 3.

According to one embodiment of the invention, one R¹ is attached to the 5-position as numbered in formula I above. According to one specific embodiment thereof, m is 1, according to a further specific embodiment, m is 2.

According to a further embodiment of the invention, one R¹ is attached to the 6-position as numbered in formula I above. According to one specific embodiment thereof, m is 1, according to a further specific embodiment, m is 2.

According to a further embodiment of the invention, one R¹ is attached to the 7-position as numbered in formula I above. According to one specific embodiment thereof, m is 1, according to a further specific embodiment, m is 2.

According to a further embodiment of the invention, one R¹ is attached to the 8-position as numbered in formula I above. According to one specific embodiment thereof, m is 1, according to a further specific embodiment, m is 2.

According to a further embodiment of the invention, two R¹ are attached in 7,8-position as numbered in formula I above. According to one specific embodiment thereof, m is 2, according to a further specific embodiment, m is 3.

For every R¹ that is present in the inventive compounds, the following embodiments and preferences apply independently of the meaning of any other R¹ that may be present in the ring. Furthermore, the particular embodiments and preferences given herein for R¹ apply independently for each of m=1, m=2, m=3 and m=4.

According to one specific embodiment, R¹ is halogen, in particular Br, F or Cl, more specifically F or Cl.

According to a further specific embodiment, R¹ is OH.

According to a further specific embodiment R¹ is NH₂, NH(C₁-C₄-alkyl), N(C₁-C₄-alkyl)₂ or NH-SO₂-R^{x}, wherein R^{x} is C₁-C₄-alkyl, C₁-C₄-halogenalkyl, unsubstituted aryl or aryl that is substituted by one, two, three, four or five substituents R^{x1} independently selected from C₁-C₄-alkyl.

According to a further specific embodiment, R¹ is C₁-C₆-alkyl, in particular C₁-C₄-alkyl, such as CH₃.

According to a further specific embodiment, R¹ is C₁-C₆-halogenalkyl, in particular C₁-C₄-halogenalkyl, such as CF₃, CHF₂, CH₂F, CCl₃, CHCl₂ or CH₂Cl.

According to still a further embodiment, R¹ is C₂-C₆-alkenyl or C₂-C₆-halogenalkenyl, in particular C₂-C₄-alkenyl or C₂-C₄-halogenalkenyl, such as CH=CH₂.

According to still a further embodiment, R¹ is C₂-C₆-alkynyl or C₂-C₆-halogenalkynyl, in particular C₂-C₄-alkynyl or C₂-C₄-halogenalkynyl, such as CΞCH.

According to a further specific embodiment, R¹ is C₁-C₆-alkoxy, in particular C₁-C₄-alkoxy, more specifically C₁-C₂-alkoxy such as OCH₃ or OCH₂CH₃.

According to a further specific embodiment, R¹ is C₁-C₆-halogenalkoxy, in particular C₁-C₄-halogenalkoxy, more specifically C₁-C₂-halogenalkoxy such as OCF₃, OCHF₂, OCH₂F, OCCl₃, OCHCl₂ or OCH₂Cl, in particular OCF₃, OCHF₂, OCCl₃ or OCHCl₂.

According to a further specific embodiment R¹ is C₃-C₆-cycloalkyl, in particular cyclopropyl.

In a further specific embodiment, R¹ is C₃-C₆-cycloalkyl, for example cyclopropyl, substituted by one, two, three or up to the maximum possible number of identical or different groups R^{1b} as defined and preferably herein.

According to a specific embodiment R¹ is C₃-C₆-halogencycloalkyl. In a special embodiment R¹ is fully or partially halogenated cyclopropyl.

According to still a further specific embodiment, R¹ is unsubstituted aryl or aryl that is substituted by one, two, three or four R^{1b}, as defined herein. In particular, R¹ is unsubstituted phenyl or phenyl that is substituted by one, two, three or four R^{1b}, as defined herein.

According to still a further specific embodiment, R¹ is unsubstituted 5- or 6-membered heteroaryl. According to still a further embodiment, R¹ is 5- or 6-membered heteroaryl that is substituted by one, two or three R^{1b}, as defined herein.

According to one further embodiment R¹ is in each case independently selected from halogen, OH, CN, NO₂, SH, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl, C₁-C₆-alkoxy and C₃-C₆-cycloalkyl; wherein the aliphatic moieties of R¹ are not further substituted or carry one, two, three, four or five identical or different groups R^{1a} as defined below and wherein the cycloalkyl moieties of R¹ are not further substituted or carry one, two, three, four or five identical or different groups R^{1b} as defined below.

According to a further embodiment, R¹ is independently selected from halogen, OH, C₁-C₆-alkyl, C₁-C₆-halogenalkyl, C₁-C₆-alkoxy and C₁-C₆-halogenalkoxy, in particular independently selected from F, Cl, Br, CN, OH, C₁-C₄-alkyl, C₁-C₄-halogenalkyl, C₁-C₄-alkoxy and C₁-C₄-halogenalkoxy.

R^{1a} are the possible substituents for the aliphatic moieties of R¹.

R^{1a} according to the invention is independently selected from halogen, OH, CN, C₁-C₆-alkoxy, C₃-C₆-cycloalkyl, C₃-C₆-halogencycloalkyl, C₁-C₄-halogenalkoxy, C₁-C₆-alkylthio and phenoxy, wherein the phenyl group is unsubstituted or carries one, two, three, four or five substituents R^{11a} selected from the group consisting of halogen, OH, C₁-C₄-alkyl, C₁-C₄-halogenalkyl, C₁-C₄-alkoxy and C₁-C₄-halogenalkoxy, in particular selected from halogen, C₁-C₂-alkyl, C₁-C₂-halogenalkyl, C₁-C₂-alkoxy and C₁-C₂-halogenalkoxy, more specifically selected from halogen, such as F, Cl and Br.

According to one embodiment R^{1a} is independently selected from halogen, OH, CN, C₁-C₂-alkoxy, C₃-C₆-cycloalkyl, C₃-C₆-halogencycloalkyl and C₁-C₂-halogenalkoxy. Specifically, R^{1a} is independently selected from F, Cl, OH, CN, C₁-C₂-alkoxy, cyclopropyl, 1-F-cyclopropyl, 1-Cl-cyclopropyl and C₁-C₂-halogenalkoxy.

According to one particular embodiment R^{1a} is independently selected from halogen, such as F, Cl, Br and I, more specifically F, Cl and Br.

According to a further embodiment, R^{1a} is independently selected from OH, C₃-C₆-cycloalkyl, C₃-C₆-halogencycloalkyl and C₁-C₂-halogenalkoxy. Specifically, R^{1a} is independently selected from OH, cyclopropyl and C₁-C₂-halogenalkoxy.

R^{1b}are the possible substituents for the cycloalkyl, heteroaryl and aryl moieties of R¹.

R^{1b} according to the invention is independently selected from halogen, OH, CN, C₁-C₄-alkyl, C₁-C₄-alkoxy, C₁-C₄-halogenalkyl, C₃-C₆-cycloalkyl, C₃-C₆-halogencycloalkyl and C₁-C₄-halogenalkoxy.

According to one embodiment thereof R^{1b} is independently selected from halogen, CN, C₁-C₂-alkyl, C₁-C₂-alkoxy, C₁-C₂-halogenalkyl, C₃-C₆-cycloalkyl, C₃-C₆-halogencycloalkyl and C₁-C₂-halogenalkoxy. Specifically, R^{1b} is independently selected from F, Cl, OH, CN, CH₃, OCH₃, cyclopropyl, 1-F-cyclopropyl, 1-Cl-cyclopropyl and halogenmethoxy.

According to a further embodiment thereof R^{1b} is independently selected from C₁-C₂-alkyl, C₁-C₂-alkoxy, C₁-C₂-halogenalkyl, C₃-C₆-cycloalkyl, C₃-C₆-halogencycloalkyl and C₁-C₂-halogenalkoxy. Specifically, R^{1b} is independently selected from OH, CH₃, OCH₃, cyclopropyl, 1-F-cyclopropyl, 1-Cl-cyclopropyl and halogenmethoxy, more specifically independently selected from OH, CH₃, OCH₃, cyclopropyl, 1-F-cyclopropyl, 1-Cl-cyclopropyl and OCHF₂.

Particularly preferred embodiments of (R¹)ₘ according to the invention are in Table P1 below, wherein each line of lines P1-1 to P1-62 corresponds to one particular embodiment of the invention, wherein P1-1 to P1-62 are also in any combination with one another a preferred embodiment of the present invention. Thereby, for every R¹ that is present in the inventive compounds, these specific embodiments and preferences apply independently of the meaning of any other R¹ that may be present in the ring:

**Table P1:**

| **No.** | **(R¹)ₘ** |
|---|---|
| P1-1 | m=0 |
| P1-2 | 5-Cl |
| P1-3 | 6-Cl |
| P1-4 | 7-Cl |
| P1-5 | 8-Cl |
| P1-6 | 5-F |
| P1-7 | 6-F |
| P1-8 | 7-F |
| P1-9 | 8-F |
| P1-10 | 5-Br |
| P1-11 | 6-Br |
| P1-12 | 7-Br |
| P1-13 | 8-Br |
| P1-14 | 5-OH |
| P1-15 | 6-OH |
| P1-16 | 7-OH |
| P1-17 | 8-OH |
| P1-18 | 5-CN |
| P1-19 | 6-CN |
| P1-20 | 7-CN |
| P1-21 | 8-CN |
| P1-22 | 5-NO₂ |
| P1-23 | 6-NO₂ |
| P1-24 | 7-NO₂ |
| P1-25 | 8-NO₂ |
| P1-26 | 5-CH₃ |
| P1-27 | 6-CH₃ |
| P1-28 | 7-CH₃ |
| P1-29 | 8-CH₃ |
| P1-30 | 5-CH₂CH₃ |
| P1-31 | 6-CH₂CH₃ |
| P1-32 | 7-CH₂CH₃ |
| P1-33 | 8-CH₂CH₃ |
| P1-34 | 5-CF₃ |
| P1-35 | 6-CF₃ |
| P1-36 | 7-CF₃ |
| P1-37 | 8-CF₃ |
| P1-38 | 5-CHF₂ |
| P1-39 | 6-CHF₂ |
| P1-40 | 7-CHF₂ |
| P1-41 | 8-CHF₂ |
| P1-42 | 5-OCH₃ |
| P1-43 | 6-OCH₃ |
| P1-44 | 7-OCH₃ |
| P1-45 | 8-OCH₃ |
| P1-46 | 5-OCH₂CH₃ |
| P1-47 | 6-OCH₂CH₃ |
| P1-48 | 7-OCH₂CH₃ |
| P1-49 | 8-OCH₂CH₃ |
| P1-50 | 5-OCF₃ |
| P1-51 | 6-OCF₃ |
| P1-52 | 7-OCF₃ |
| P1-53 | 8-OCF₃ |
| P1-54 | 5-OCHF₂ |
| P1-55 | 6-OCHF₂ |
| P1-56 | 7-OCHF₂ |
| P1-57 | 8-OCHF₂ |
| P1-58 | 5-NH-Ts |
| P1-59 | 6-NH-Ts |
| P1-60 | 7-NH-Ts |
| P1-61 | 8-NH-Ts |
| P1-62 | 7,8-F₂ |

R² according to the invention is is in each case independently selected from halogen, OH, CN, NO₂, SH, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl, C₁-C₆-alkoxy, C₃-C₆-cycloalkyl, five- or six-membered heteroaryl and aryl; wherein the heteroaryl contains one, two or three heteroatoms selected from N, O and S; and wherein R^{x} is C₁-C₄-alkyl, C₁-C₄-halogenalkyl, unsubstituted aryl or aryl that is substituted by one, two, three, four or five substituents R^{x2} independently selected from C₁-C₄-alkyl; wherein the aliphatic moieties of R² are not further substituted or carry one, two, three or up to the maximum possible number of identical or different groups R^{2a} which independently of one another are selected from:
R^{2a} halogen, OH, CN, C₁-C₆-alkoxy, C₃-C₆-cycloalkyl, C₃-C₆-halogencycloalkyl, C₁-C₄-halogenalkoxy, C₁-C₆-alkylthio and phenoxy, wherein the phenyl group is unsubstituted or carries one, two, three, four or five substituents R^{22a} selected from the group consisting of halogen, OH, C₁-C₄-alkyl, C₁-C₄-halogenalkyl, C₁-C₄-alkoxy and C₁-C₄-halogenalkoxy;
wherein the cycloalkyl, heteroaryl and aryl moieties of R² are not further substituted or carry one, two, three, four, five or up to the maximum number of identical or different groups R^{2b} which independently of one another are selected from:
R^{2b} halogen, OH, CN, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy and C₁-C₆-alkylthio.

According to the invention, there can be zero, one or two R² present, namely for n is 0, 1 or 2.

According to one embodiment, n is 0 or 1.

According to a further embodiment, n is 0.

According to still a further embodiment, n is 1.

According to still a further embodiment, n is 2.

According to one embodiment of the invention, one R² is attached to the 2-position as numbered in formula I above. According to one specific embodiment thereof, n is 1, according to a further specific embodiment, n is 2.

According to a further embodiment of the invention, one R² is attached to the 4-position as numbered in formula I above. According to one specific embodiment thereof, n is 1, according to a further specific embodiment, n is 2.

According to a further embodiment of the invention, two R² are attached in 2,4-position as numbered in formula I above.

For every R² that is present in the inventive compounds, the following embodiments and preferences apply independently of the meaning of the other R² that may be present in the ring. Furthermore, the particular embodiments and preferences given herein for R² apply independently for each of n=1 and n=2.

According to one specific embodiment, R² is halogen, in particular Br, F or Cl, more specifically F or Cl.

According to a further specific embodiment, R² is OH.

According to a further specific embodiment R² is NH₂, NH(C₁-C₄-alkyl), N(C₁-C₄-alkyl)₂ or NH-SO₂-R^{x}, wherein R^{x} is C₁-C₄-alkyl, C₁-C₄-halogenalkyl, unsubstituted aryl or aryl that is substituted by one, two, three, four or five substituents R^{x2} independently selected from C₁-C₄-alkyl.

According to a further specific embodiment, R² is C₁-C₆-alkyl, in particular C₁-C₄-alkyl, such as CH₃.

According to a further specific embodiment, R² is C₁-C₆-halogenalkyl, in particular C₁-C₄-halogenalkyl, such as CF₃, CHF₂, CH₂F, CCl₃, CHCl₂ or CH₂Cl.

According to still a further embodiment, R² is C₂-C₆-alkenyl or C₂-C₆-halogenalkenyl, in particular C₂-C₄-alkenyl or C₂-C₄-halogenalkenyl, such as CH=CH₂.

According to still a further embodiment, R² is C₂-C₆-alkynyl or C₂-C₆-halogenalkynyl, in particular C₂-C₄-alkynyl or C₂-C₄-halogenalkynyl, such as CΞCH.

According to a further specific embodiment, R² is C₁-C₆-alkoxy, in particular C₁-C₄-alkoxy, more specifically C₁-C₂-alkoxy such as OCH₃ or OCH₂CH₃.

According to a further specific embodiment, R² is C₁-C₆-halogenalkoxy, in particular C₁-C₄-halogenalkoxy, more specifically C₁-C₂-halogenalkoxy such as OCF₃, OCHF₂, OCH₂F, OCCl₃, OCHCl₂ or OCH₂Cl, in particular OCF₃, OCHF₂, OCCl₃ or OCHCl₂.

According to a further specific embodiment R² is C₃-C₆-cycloalkyl, in particular cyclopropyl.

In a further specific embodiment, R² is C₃-C₆-cycloalkyl, for example cyclopropyl, substituted by one, two, three or up to the maximum possible number of identical or different groups R^{2b} as defined and preferably herein.

According to a specific embodiment R² is C₃-C₆-halogencycloalkyl. In a special embodiment R² is fully or partially halogenated cyclopropyl.

According to still a further specific embodiment, R² is unsubstituted aryl or aryl that is substituted by one, two, three or four R^{2b}, as defined herein. In particular, R² is unsubstituted phenyl or phenyl that is substituted by one, two, three or four R^{2b}, as defined herein.

According to still a further specific embodiment, R² is unsubstituted 5- or 6-membered heteroaryl. According to still a further embodiment, R² is 5- or 6-membered heteroaryl that is substituted by one, two or three R^{2b}, as defined herein.

According to one further embodiment R² is in each case independently selected from halogen, OH, CN, NO₂, SH, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl, C₁-C₆-alkoxy and C₃-C₆-cycloalkyl; wherein the aliphatic moieties of R² are not further substituted or carry one, two, three, four or five identical or different groups R^{2a} as defined below and wherein the cycloalkyl moieties of R² are not further substituted or carry one, two, three, four or five identical or different groups R^{2b} as defined below.

According to a further embodiment, R² is independently selected from halogen, OH, C₁-C₆-alkyl, C₁-C₆-halogenalkyl, C₁-C₆-alkoxy and C₁-C₆-halogenalkoxy, in particular independently selected from F, Cl, Br, CN, OH, C₁-C₄-alkyl, C₁-C₄-halogenalkyl, C₁-C₄-alkoxy and C₁-C₄-halogenalkoxy.

R^{2a} are the possible substituents for the aliphatic moieties of R².

R^{2a} according to the invention is independently selected from halogen, OH, CN, C₁-C₆-alkoxy, C₃-C₆-cycloalkyl, C₃-C₆-halogencycloalkyl, C₁-C₄-halogenalkoxy, C₁-C₆-alkylthio and phenoxy, wherein the phenyl group is unsubstituted or carries one, two, three, four or five substituents R^{22a} selected from the group consisting of halogen, OH, C₁-C₄-alkyl, C₁-C₄-halogenalkyl, C₁-C₄-alkoxy and C₁-C₄-halogenalkoxy, in particular selected from halogen, C₁-C₂-alkyl, C₁-C₂-halogenalkyl, C₁-C₂-alkoxy and C₁-C₂-halogenalkoxy, more specifically selected from halogen, such as F, Cl and Br.

According to one embodiment R^{2a} is independently selected from halogen, OH, CN, C₁-C₂-alkoxy, C₃-C₆-cycloalkyl, C₃-C₆-halogencycloalkyl and C₁-C₂-halogenalkoxy. Specifically, R^{2a} is independently selected from F, Cl, OH, CN, C₁-C₂-alkoxy, cyclopropyl, 1-F-cyclopropyl, 1-Cl-cyclopropyl and C₁-C₂-halogenalkoxy.

According to one particular embodiment R^{2a} is independently selected from halogen, such as F, Cl, Br and I, more specifically F, Cl and Br.

According to a further embodiment, R^{2a} is independently selected from OH, C₃-C₆-cycloalkyl, C₃-C₆-halogencycloalkyl and C₁-C₂-halogenalkoxy. Specifically, R^{2a} is independently selected from
OH, cyclopropyl and C₁-C₂-halogenalkoxy.

R^{2b} are the possible substituents for the cycloalkyl, heteroaryl and aryl moieties of R².

R^{2b} according to the invention is independently selected from halogen, OH, CN, C₁-C₄-alkyl, C₁-C₄-alkoxy, C₁-C₄-halogenalkyl, C₃-C₆-cycloalkyl, C₃-C₆-halogencycloalkyl and C₁-C₄-halogenalkoxy.

According to one embodiment thereof R^{2b} is independently selected from halogen, CN, C₁-C₂-alkyl, C₁-C₂-alkoxy, C₁-C₂-halogenalkyl, C₃-C₆-cycloalkyl, C₃-C₆-halogencycloalkyl and C₁-C₂-halogenalkoxy. Specifically, R^{2b} is independently selected from F, Cl, OH, CN, CH₃, OCH₃, cyclopropyl, 1-F-cyclopropyl, 1-Cl-cyclopropyl and halogenmethoxy.

According to a further embodiment thereof R^{2b} is independently selected from C₁-C₂-alkyl, C₁-C₂-alkoxy, C₁-C₂-halogenalkyl, C₃-C₆-cycloalkyl, C₃-C₆-halogencycloalkyl and C₁-C₂-halogenalkoxy. Specifically, R^{2b} is independently selected from OH, CH₃, OCH₃, cyclopropyl, 1-F-cyclopropyl, 1-Cl-cyclopropyl and halogenmethoxy, more specifically independently selected from OH, CH₃, OCH₃, cyclopropyl, 1-F-cyclopropyl, 1-Cl-cyclopropyl and OCHF₂.

Particularly preferred embodiments of (R²)ₘ according to the invention are in Table P2 below, wherein each line of lines P2-1 to P2-32 corresponds to one particular embodiment of the invention, wherein P2-1 to P2-32 are also in any combination with one another a preferred embodiment of the present invention. Thereby, for every R² that is present in the inventive compounds, these specific embodiments and preferences apply independently of the meaning of any other R² that may be present in the ring:

**Table P2:**

| **No.** | **(R²)ₙ** |
|---|---|
| P2-1 | n=0 |
| P2-2 | 2-Cl |
| P2-3 | 4-Cl |
| P2-4 | 2-F |
| P2-5 | 4-F |
| P2-6 | 2-Br |
| P2-7 | 4-Br |
| P2-8 | 2-OH |
| P2-9 | 4-OH |
| P2-10 | 2-CN |
| P2-11 | 4-CN |
| P2-12 | 2-NO₂ |
| P2-13 | 4-NO₂ |
| P2-14 | 2-CH₃ |
| P2-15 | 4-CH₃ |
| P2-16 | 2-CH₂CH₃ |
| P2-17 | 4-CH₂CH₃ |
| P2-18 | 2-CF₃ |
| P2-19 | 4-CF₃ |
| P2-20 | 2-CHF₂ |
| P2-21 | 4-CHF₂ |
| P2-22 | 2-OCH₃ |
| P2-23 | 4-OCH₃ |
| P2-24 | 2-OCH₂CH₃ |
| P2-25 | 4-OCH₂CH₃ |
| P2-26 | 2-OCF₃ |
| P2-27 | 4-OCF₃ |
| P2-28 | 2-OCHF₂ |
| P2-29 | 4-OCHF₂ |
| P2-30 | 2-NH-Ts |
| P2-31 | 4-NH-Ts |
| P2-32 | 2,4-F₂ |

R³, R⁴ are independently selected from hydrogen, halogen, OH, CN, NO₂, SH, C₁-C₆-alkylthio, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl, C₁-C₆-alkoxy, C₁-C₆-halogenalkoxy, a saturated or partially unsaturated three-, four-, five-, six-, seven-, eight-, nine-, or ten-membered carbocycle or heterocycle, wherein in each case one or two CH₂ groups of the carbo- and heterocycle may be replaced by a group independently selected from C(=O) and C(=S), five- or six-membered heteroaryl and aryl; wherein the heterocycle and the heteroaryl contain independently one, two, three or four heteroatoms selected from N, O and S; wherein the aliphatic moieties of R³ and R⁴ are independently not further substituted or carry one, two, three or up to the maximum possible number of identical or different groups R^{3a} or R^{4a}, respectively, which independently of one another are selected from:
R^{3a}, R⁴ halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, C₁-C₆-alkoxy,C₃-C₆-cycloalkyl, C₃-C₆-halogencycloalkyl, C₁-C₄-halogenalkoxy, C₁-C₆-alkylthio, aryl and phenoxy, wherein the aryl and phenyl groups are independently unsubstituted or carry one, two, three, four or five substituents selected from the group consisting of halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, NH-SO₂-R^{x}, C₁-C₆-alkylthio, C₁-C₄-alkyl, C₁-C₄-halogenalkyl, C₁-C₄-alkoxy and C₁-C₄-halogenalkoxy;
wherein the carbocyclic, heterocyclic, heteroaryl and aryl moieties of R³ and R⁴ are independently not further substituted or carry one, two, three, four, five or up to the maximum number of identical or different groups R^{3b} or R^{4b}, respectively, which independently of one another are selected from:
R^{3b}, R^{4b} halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, NH-SO₂-R^{x}, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy, C₁-C₆-alkylthio, C₁-C₆-halogenalkylthio, C₁-C₄-alkoxy-C₁-C₄-alkyl, phenyl and phenoxy, wherein the phenyl groups are unsubstituted or carry one, two, three, four or five substituents selected from the group consisting of halogen, OH, C₁-C₄-alkyl, C₁-C₄-halogenalkyl, C₁-C₄-alkoxy and C₁-C₄-halogenalkoxy; and wherein R^{x} is as defined above;
or R³, R⁴ together with the carbon atom to which they are bound (marked with * in formula I) form a saturated or partially unsaturated three-, four-, five-, six-, seven-, eight-, nine-, or ten-membered carbocycle or heterocycle; wherein the heterocycle contains one, two, three or four heteroatoms selected from N, O and S, wherein the heteroatom N may carry one substituent R^{N} selected from C₁-C₄-alkyl, C₁-C₄-halogenalkyl and SO₂Ph, wherein Ph is unsubstituted phenyl or phenyl that is substituted by one, two or three substituents selected from C₁-C₄-alkyl, and wherein the heteroatom S may be in the form of its oxide SO or SO₂, and wherein the carbocycle or heterocycle is unsubstituted or carries one, two, three or four substituents R³⁴ independently selected from halogen, OH, CN, NO₂, SH, NH₂, C₁-C₆-alkyl, C₁-C₆-halogenalkyl, C₁-C₆-alkoxy, C₁-C₆-halogenalkoxy, C₁-C₆-alkylthio, C₁-C₆-halogenalkylthio, C₁-C₄-alkoxy-C₁-C₄-alkyl, phenyl and phenoxy, wherein the phenyl groups are unsubstituted or carry one, two, three, four or five substituents R^{34a} selected from the group consisting of halogen, OH, C₁-C₄-alkyl, C₁-C₄-halogenalkyl, C₁-C₄-alkoxy and C₁-C₄-halogenalkoxy; and wherein in each case one or two CH₂ groups of the heterocycle may be replaced by a group independently selected from C(=O) and C(=S).

According to one embodiment, R³ is selected from hydrogen, halogen, OH, CN, SH, C₁-C₆-alkylthio, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl, C₁-C₆-alkoxy and C₁-C₆-halogenalkoxy, in particular hydrogen, halogen, OH, CN, C₁-C₄-alkylthio, C₁-C₆-alkyl, C₂-C₆-alkenyl, C₂-C₆-alkynyl, C₁-C₆-alkoxy and C₁-C₆-halogenalkoxy, wherein R^{x} is defined below; and wherein the aliphatic moieties of R⁴ are not further substituted or carry one, two, three or up to the maximum possible number of identical or different groups R^{3a} as defined below.

According to a further embodiment, R³ is selected from halogen, OH, CN, SH, C₁-C₆-alkylthio, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl, C₁-C₆-alkoxy and C₁-C₆-halogenalkoxy, in particular halogen, OH, CN, C₁-C₄-alkylthio, C₁-C₆-alkyl, C₂-C₆-alkenyl, C₂-C₆-alkynyl, C₁-C₆-alkoxy and C₁-C₆-halogenalkoxy, wherein R^{x} is defined below; and wherein the aliphatic moieties of R⁴ are not further substituted or carry one, two, three or up to the maximum possible number of identical or different groups R^{3a} as defined below.

According to one particular embodiment, R³ is hydrogen.

According to a further particular embodiment, R³ is hydrogen or C₁-C₆-alkyl, such as hydrogen, methyl, ethyl, n-propyl, i-propyl, n-butyl, i-butyl, tert-butyl, n-pentyl or i-pentyl.

According to a further particular embodiment, R³ is C₁-C₆-alkyl, such as methyl, ethyl, n-propyl, i-propyl, n-butyl, i-butyl, tert-butyl, n-pentyl or i-pentyl.

According to a further particular embodiment, R³ is hydrogen or C₁-C₆-halogenalkyl, in particular hydrogen or C₁-C₄-halogenalkyl, more specifically hydrogen or C₁-C₂-halogenalkyl, such as H, CF₃, CCl₃, FCH₂, ClCH₂, F₂CH, Cl₂CH, CF₃CH₂, CCl₃CH₂ or CF₂CHF₂.

According to a further particular embodiment, R³ is C₁-C₆-halogenalkyl, in particular C₁-C₄-halogenalkyl, more specifically C₁-C₂-halogenalkyl, such as CF₃, CCl₃, FCH₂, ClCH₂, F₂CH, Cl₂CH, CF₃CH₂, CCl₃CH₂ or CF₂CHF₂.

According to a further particular embodiment, R³ is hydrogen or phenyl-C₁-C₆-alkyl, such as hydrogen or phenyl-CH₂, wherein the phenyl moiety in each case is unsubstituted or substituted by one, two or three identical or different groups R^{3b} which independently of one another are selected from halogen, C₁-C₂-alkyl, C₁-C₂-alkoxy, C₁-C₂-halogenalkyl and C₁-C₂-halogenalkoxy, in particular selected from F, Cl, Br, methyl, OCH₃, CF₃ and OCF₃.

According to a further particular embodiment, R³ is phenyl-C₁-C₆-alkyl, such as phenyl-CH₂, wherein the phenyl moiety in each case is unsubstituted or substituted by one, two or three identical or different groups R^{3b} which independently of one another are selected from halogen, C₁-C₂-alkyl, C₁-C₂-alkoxy, C₁-C₂-halogenalkyl and C₁-C₂-halogenalkoxy, in particular F, Cl, Br, methyl, OCH₃, CF₃ and OCF₃.

According to a further particular embodiment, R³ is hydrogen or aryl, in particular H or phenyl, wherein the aryl or phenyl moiety in each case is unsubstituted or carries one, two or three identical or different groups R^{3b} which independently of one another are selected from halogen, C₁-C₂-alkyl, C₁-C₂-alkoxy, C₁-C₂-halogenalkyl and C₁-C₂-halogenalkoxy, in particular F, Cl, Br, methyl, OCH₃, CF₃ and OCF₃. In one embodiment, R³ is H or unsubstituted phenyl. In another embodiment, R³ is H or phenyl, that is substituted by one, two or three, in particular one, halogen, in particular selected from F, Cl and Br, more specifically selected from F and Cl.

According to a further particular embodiment, R⁴ is aryl, in particular phenyl, wherein the aryl or phenyl moiety in each case is unsubstituted or carries one, two or three identical or different groups R^{3b} which independently of one another are selected from halogen, C₁-C₂-alkyl, C₁-C₂-alkoxy, C₁-C₂-halogenalkyl and C₁-C₂-halogenalkoxy, in particular F, Cl, Br, methyl, OCH₃, CF₃ and OCF₃. In one embodiment, R³ is unsubstituted phenyl. In another embodiment, R³ is phenyl, that is substituted by one, two or three, in particular one, halogen, in particular selected from F, Cl and Br, more specifically selected from F and Cl.

According to a further embodiment, R³ is hydrogen or a partially unsaturated three-, four-, five-, six-, seven-, eight-, nine-, or ten-membered carbocycle or heterocycle, in particular three-, four-, five- or six-membered, wherein the heterocycle contains one, two, three or four heteroatoms selected from N, O and S, and wherein the carbocycle and heterocycle are unsubstituted or carry one, two, three or four substituents R^{3b} as defined below. According to one embodiment thereof, the carbocycle or heterocycle is unsubstituted.

According to a further embodiment, R³ is a partially unsaturated three-, four-, five-, six-, seven-, eight-, nine-, or ten-membered carbocycle or heterocycle, in particular three-, four-, five- or six-membered, wherein the heterocycle contains one, two, three or four heteroatoms selected from N, O and S, and wherein the carbocycle and heterocycle are unsubstituted or carry one, two, three or four substituents R^{3b} as defined below. According to one embodiment thereof, the carbocycle or heterocycle is unsubstituted.

According to still a further embodiment, R³ is hydrogen or a saturated three-, four-, five-, six-, seven-, eight-, nine-, or ten-membered carbocycle or heterocycle, in particular three-, four-, five- or six-membered, wherein the heterocycle contains one, two, three or four heteroatoms selected from N, O and S, and wherein the carbocycle and heterocycle are unsubstituted or carry one, two, three or four substituents R^{3b} as defined below. According to one embodiment thereof, the carbocycle or heterocycle is unsubstituted.

According to still a further embodiment, R³ is a saturated three-, four-, five-, six-, seven-, eight-, nine-, or ten-membered carbocycle or heterocycle, in particular three-, four-, five- or six-membered, wherein the heterocycle contains one, two, three or four heteroatoms selected from N, O and S, and wherein the carbocycle and heterocycle are unsubstituted or carry one, two, three or four substituents R^{3b} as defined below. According to one embodiment thereof, the carbocycle or heterocycle is unsubstituted.

According to a further embodiment, R³ is hydrogen or a partially unsaturated three-, four-, five-, six-, seven-, eight-, nine-, or ten-membered heterocycle, in particular three-, four-, five- or six-membered, wherein the heterocycle contains one, two, three or four heteroatoms selected from N, O and S, and wherein the heterocycle is unsubstituted or carries one, two, three or four substituents R^{3b} as defined below. According to one embodiment thereof, the heterocycle is unsubstituted.

According to a further embodiment, R³ is a partially unsaturated three-, four-, five-, six-, seven-, eight-, nine-, or ten-membered heterocycle, in particular three-, four-, five- or six-membered, wherein the heterocycle contains one, two, three or four heteroatoms selected from N, O and S, and wherein the heterocycle is unsubstituted or carries one, two, three or four substituents R^{3b} as defined below. According to one embodiment thereof, the heterocycle is unsubstituted.

According to a further embodiment, R⁴ is hydrogen or a saturated three-, four-, five-, six-, seven-, eight-, nine-, or ten-membered heterocycle, in particular three-, four-, five- or six-membered, wherein the heterocycle contains one, two, three or four heteroatoms selected from N, O and S, and wherein the heterocycle is unsubstituted or carries one, two, three or four substituents R^{4a} as defined below. According to one embodiment thereof, the heterocycle is unsubstituted.

According to a further embodiment, R⁴ is a saturated three-, four-, five-, six-, seven-, eight-, nine-, or ten-membered heterocycle, in particular three-, four-, five- or six-membered, wherein the heterocycle contains one, two, three or four heteroatoms selected from N, O and S, and wherein the heterocycle is unsubstituted or carries one, two, three or four substituents R^{3b} as defined below. According to one embodiment thereof, the heterocycle is unsubstituted.

According to a further embodiment, in the embodiments of R³ described above, the heterocycle contains preferably one, two or three, more specifically one or two heteroatoms selected from N, O and S. More specifically, the hetereocycle contains one heteroatom selected from N, O and S. In particular, the heterocycle contains one or two, in particular one O.

According to one particular embodiment, R³ is hydrogen or a 4-membered saturated heterocycle which contains 1 or 2 heteroatoms, in particular 1 heteroatom, from the group consisting of N, O and S, as ring members. In one embodiment, the heterocycle contains one O as heteroatom. For example, the formed heterocycle is oxetane. According to one embodiment thereof, the heterocycle is unsubstituted, i.e. it does not carry any substituent R^{3b}. According to a further embodiment, it carries one, two, three or four R^{3b}.

According to one particular embodiment, R³ is a 4-membered saturated heterocycle which contains 1 or 2 heteroatoms, in particular 1 heteroatom, from the group consisting of N, O and S, as ring members. In one embodiment, the heterocycle contains one O as heteroatom. For example, the formed heterocycle is oxetane. According to one embodiment thereof, the heterocycle is unsubstituted, i.e. it does not carry any substituent R^{3b}. According to a further embodiment, it carries one, two, three or four R^{3b}.

According to a further particular embodiment, R³ is hydrogen or a 5-membered saturated heterocycle which contains 1, 2 or 3, in particular 1 or 2, heteroatoms from the group consisting of N, O and S, as ring members. In one embodiment, the heterocycle contains one O as heteroatom. According to one embodiment thereof, the heterocycle is unsubstituted, i.e. it does not carry any substituent R^{3b}. According to a further embodiment, it carries one, two, three or four R^{3b}.

According to a further particular embodiment, R³ is a 5-membered saturated heterocycle which contains 1, 2 or 3, in particular 1 or 2, heteroatoms from the group consisting of N, O and S, as ring members. In one embodiment, the heterocycle contains one O as heteroatom. According to one embodiment thereof, the heterocycle is unsubstituted, i.e. it does not carry any substituent R^{3b}. According to a further embodiment, it carries one, two, three or four R^{3b}.

According to a further particular embodiment, R³ is hydrogen or a 6-membered saturated heterocycle which contains 1, 2 or 3, in particular 1 or 2, heteroatoms from the group consisting of N, O and S as ring members. According to one embodiment thereof, the heterocycle is unsubstituted, i.e. it does not carry any substituent R^{3b}. According to a further embodiment, it carries one, two, three or four R^{3b}. According to one specific embodiment thereof, said 6-membered saturated heterocycle contains 1 or 2, in particular 1, heteroatom(s) O. According to one embodiment thereof, the respective 6-membered heterocycle is unsubstituted, i.e. it does not carry any substituent R^{3b}. According to a further embodiment, it carries one, two, three or four R^{3b}.

According to a further particular embodiment, R³ is a 6-membered saturated heterocycle which contains 1, 2 or 3, in particular 1 or 2, heteroatoms from the group consisting of N, O and S as ring members. According to one embodiment thereof, the heterocycle is unsubstituted, i.e. it does not carry any substituent R^{3b}. According to a further embodiment, it carries one, two, three or four R^{3b}. According to one specific embodiment thereof, said 6-membered saturated heterocycle contains 1 or 2, in particular 1, heteroatom(s) O. According to one embodiment thereof, the respective 6-membered heterocycle is unsubstituted, i.e. it does not carry any substituent R^{3b}. According to a further embodiment, it carries one, two, three or four R^{3b}.

According to a further embodiment, R³ is hydrogen or a partially unsaturated three-, four-, five-, six-, seven-, eight-, nine-, or ten-membered carbocycle, in particular three-, four-, five- or six-membered, wherein the carbocycle is unsubstituted or carries one, two, three or four substituents R^{3b} as defined below. According to one embodiment thereof, the carbocycle is unsubstituted.

According to a further embodiment, R³ is a partially unsaturated three-, four-, five-, six-, seven-, eight-, nine-, or ten-membered carbocycle, in particular three-, four-, five- or six-membered, wherein the carbocycle is unsubstituted or carries one, two, three or four substituents R^{3b} as defined below. According to one embodiment thereof, the carbocycle is unsubstituted.

According to a further embodiment, R³ is hydrogen or a saturated three-, four-, five-, six-, seven-, eight-, nine-, or ten-membered carbocycle, in particular three-, four-, five- or six-membered, wherein the carbocycle is unsubstituted or carries one, two, three or four substituents R^{3b} as defined below. According to one embodiment thereof, the carbocycle is unsubstituted.

According to a further embodiment, R³ is a saturated three-, four-, five-, six-, seven-, eight-, nine-, or ten-membered carbocycle, in particular three-, four-, five- or six-membered, wherein the carbocycle is unsubstituted or carries one, two, three or four substituents R^{3b} as defined below. According to one embodiment thereof, the carbocycle is unsubstituted.

According to one particular embodiment, R³ is hydrogen or a 3-membered saturated carbocycle. According to one embodiment thereof, the carbocycle is unsubstituted, i.e. it does not carry any substituent R^{4a}. According to a further embodiment, it carries one, two, three or four R^{3b}.

According to one particular embodiment, R³ is a 3-membered saturated carbocycle. According to one embodiment thereof, the carbocycle is unsubstituted, i.e. it does not carry any substituent R^{3b}. According to a further embodiment, it carries one, two, three or four R^{3b}.

According to one particular embodiment, R³ is hydrogen or a 4-membered saturated carbocycle. According to one embodiment thereof, the carbocycle is unsubstituted, i.e. it does not carry any substituent R^{3b}. According to a further embodiment, it carries one, two, three or four R^{3b}.

According to one particular embodiment, R³ is a 4-membered saturated carbocycle. According to one embodiment thereof, the carbocycle is unsubstituted, i.e. it does not carry any substituent R^{3b}. According to a further embodiment, it carries one, two, three or four R^{3b}.

According to one particular embodiment, R³ is hydrogen or a 5-membered saturated carbocycle. According to one embodiment thereof, the carbocycle is unsubstituted, i.e. it does not carry any substituent R^{3b}. According to a further embodiment, it carries one, two, three or four R^{3b}.

According to one particular embodiment, R³ is a 5-membered saturated carbocycle. According to one embodiment thereof, the carbocycle is unsubstituted, i.e. it does not carry any substituent R^{3b}. According to a further embodiment, it carries one, two, three or four R^{3b}.

According to one particular embodiment, R³ is hydrogen or a 6-membered saturated carbocycle. According to one embodiment thereof, the carbocycle is unsubstituted, i.e. it does not carry any substituent R^{3b}. According to a further embodiment, it carries one, two, three or four R^{3b}.

According to one particular embodiment, R³ is a 6-membered saturated carbocycle. According to one embodiment thereof, the carbocycle is unsubstituted, i.e. it does not carry any substituent R^{3b}. According to a further embodiment, it carries one, two, three or four R^{3b}.

According to a further particular embodiment, R³ is selected from hydrogen, C₁-C₆-alkyl, C₁-C₆-halogenalkyl, phenyl-C₁-C₆-alkyl, halogenphenyl-C₁-C₆-alkyl, phenyl, halogenphenyl and three-, four-, five- or six-membered carbocycle, wherein the carbocycle is unsubstituted or carries one, two, three or four substituents R^{3b} as defined below. According to one embodiment thereof, the carbocycle is unsubstituted. In a particular embodiment, R³ is selected from hydrogen, C₁-C₆-alkyl, C₁-C₆-halogenalkyl, phenyl-CH₂, halogenphenyl-CH₂, phenyl, halogenphenyl and three-, four-, five- or six-membered carbocycle, wherein the carbocycle is unsubstituted or carries one, two, three or four substituents R^{3b} as defined below.

According to a further particular embodiment, R³ is selected from C₁-C₆-alkyl, C₁-C₆-halogenalkyl, phenyl-C₁-C₆-alkyl, halogenphenyl-C₁-C₆-alkyl, phenyl, halogenphenyl and three-, four-, five- or six-membered carbocycle, wherein the carbocycle is unsubstituted or carries one, two, three or four substituents R^{3b} as defined below. According to one embodiment thereof, the carbocycle is unsubstituted. In a particular embodiment, R³ is selected from C₁-C₆-alkyl, C₁-C₆-halogenalkyl, phenyl-CH₂, halogenphenyl-CH₂, phenyl, halogenphenyl and three-, four-, five- or six-membered carbocycle, wherein the carbocycle is unsubstituted or carries one, two, three or four substituents R^{3b} as defined below.

Particularly preferred embodiments of R³ according to the invention are in Table P3 below, wherein each line of lines P3-1 to P3-33 corresponds to one particular embodiment of the invention, wherein P3-1 to P3-33 are also in any combination with one another a preferred embodiment of the present invention. The connection point to the carbon atom, to which R³ is bound is marked with "#" in the drawings.

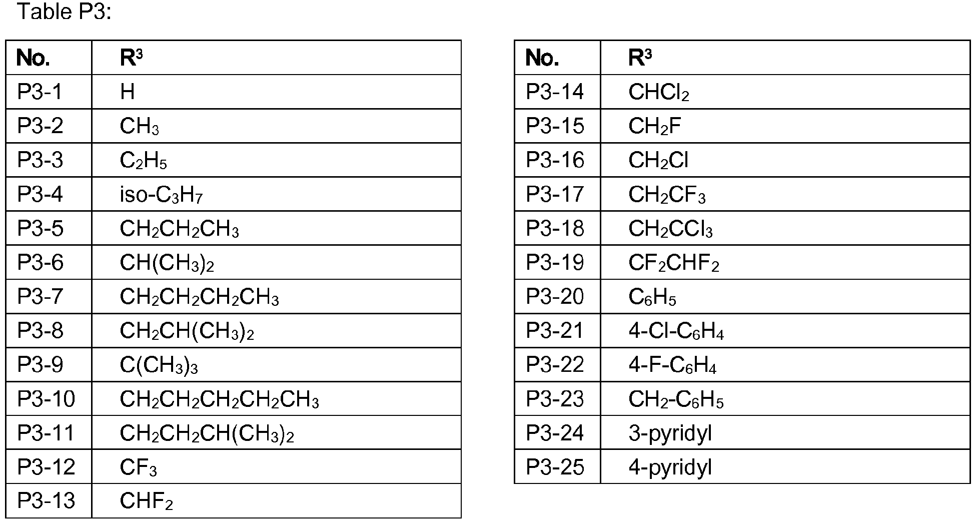

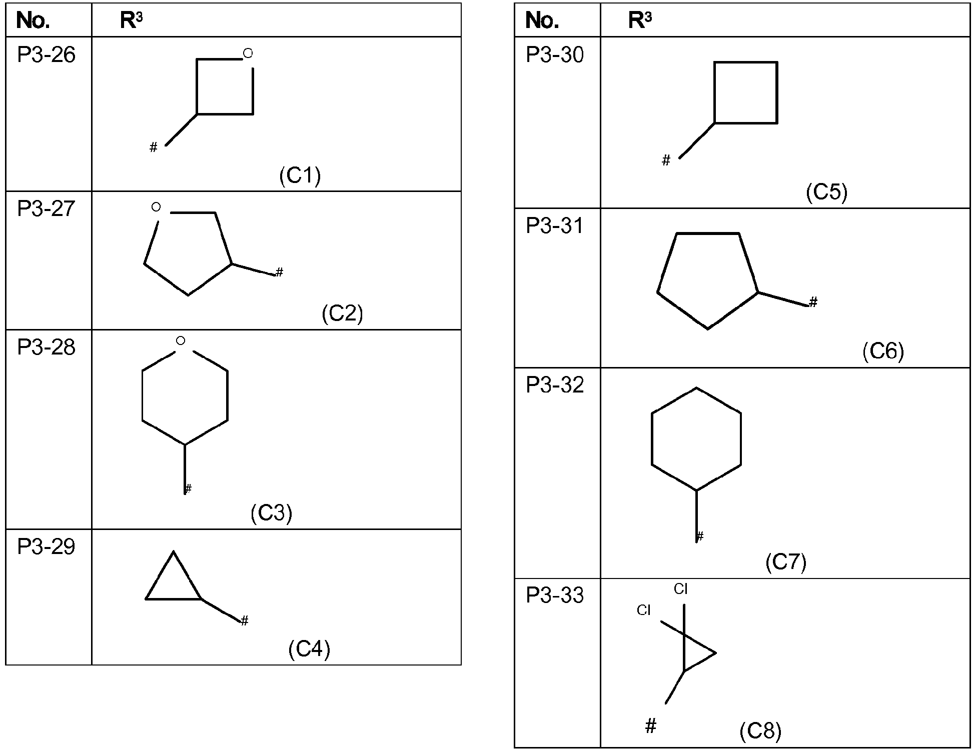

According to one embodiment, R⁴ is selected from hydrogen, halogen, OH, CN, SH, C₁-C₆-alkylthio, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl, C₁-C₆-alkoxy and C₁-C₆-halogenalkoxy, in particular hydrogen, halogen, OH, CN, C₁-C₄-alkylthio, C₁-C₆-alkyl, C₂-C₆-alkenyl, C₂-C₆-alkynyl, C₁-C₆-alkoxy and C₁-C₆-halogenalkoxy, wherein R^{x} is defined below; and wherein the aliphatic moieties of R⁴ are not further substituted or carry one, two, three or up to the maximum possible number of identical or different groups R^{4a} as defined below.

According to a further embodiment, R⁴ is selected from halogen, OH, CN, SH, C₁-C₆-alkylthio, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl, C₁-C₆-alkoxy and C₁-C₆-halogenalkoxy, in particular halogen, OH, CN, C₁-C₄-alkylthio, C₁-C₆-alkyl, C₂-C₆-alkenyl, C₂-C₆-alkynyl, C₁-C₆-alkoxy and C₁-C₆-halogenalkoxy, wherein R^{x} is defined below; and wherein the aliphatic moieties of R⁴ are not further substituted or carry one, two, three or up to the maximum possible number of identical or different groups R^{4a} as defined below.

According to one particular embodiment, R⁴ is hydrogen.

According to a further particular embodiment, R⁴ is hydrogen or C₁-C₆-alkyl, such as hydrogen, methyl, ethyl, n-propyl, i-propyl, n-butyl, i-butyl, tert-butyl, n-pentyl or i-pentyl.

According to a further particular embodiment, R⁴ is C₁-C₆-alkyl, such as methyl, ethyl, n-propyl, i-propyl, n-butyl, i-butyl, tert-butyl, n-pentyl or i-pentyl.

According to a further particular embodiment, R⁴ is hydrogen or C₁-C₆-halogenalkyl, in particular hydrogen or C₁-C₄-halogenalkyl, more specifically hydrogen or C₁-C₂-halogenalkyl, such as H, CF₃, CCl₃, FCH₂, ClCH₂, F₂CH, Cl₂CH, CF₃CH₂, CCl₃CH₂ or CF₂CHF₂.

According to a further particular embodiment, R⁴ is C₁-C₆-halogenalkyl, in particular C₁-C₄-halogenalkyl, more specifically C₁-C₂-halogenalkyl, such as CF₃, CCl₃, FCH₂, ClCH₂, F₂CH, Cl₂CH, CF₃CH₂, CCl₃CH₂ or CF₂CHF₂.

According to a further particular embodiment, R⁴ is hydrogen or phenyl-C₁-C₆-alkyl, such as hydrogen or phenyl-CH₂, wherein the phenyl moiety in each case is unsubstituted or substituted by one, two or three identical or different groups R^{4b} which independently of one another are selected from halogen, C₁-C₂-alkyl, C₁-C₂-alkoxy, C₁-C₂-halogenalkyl and C₁-C₂-halogenalkoxy, in particular selected from F, Cl, Br, methyl, OCH₃, CF₃ and OCF₃.

According to a further particular embodiment, R⁴ is phenyl-C₁-C₆-alkyl, such as phenyl-CH₂, wherein the phenyl moiety in each case is unsubstituted or substituted by one, two or three identical or different groups R^{4b} which independently of one another are selected from halogen, C₁-C₂-alkyl, C₁-C₂-alkoxy, C₁-C₂-halogenalkyl and C₁-C₂-halogenalkoxy, in particular F, Cl, Br, methyl, OCH₃, CF₃ and OCF₃.

According to a further particular embodiment, R⁴ is hydrogen or aryl, in particular H or phenyl, wherein the aryl or phenyl moiety in each case is unsubstituted or carries one, two or three identical or different groups R^{4b} which independently of one another are selected from halogen, C₁-C₂-alkyl, C₁-C₂-alkoxy, C₁-C₂-halogenalkyl and C₁-C₂-halogenalkoxy, in particular F, Cl, Br, methyl, OCH₃, CF₃ and OCF₃. In one embodiment, R⁴ is H or unsubstituted phenyl. In another embodiment, R⁴ is H or phenyl, that is substituted by one, two or three, in particular one, halogen, in particular selected from F, Cl and Br, more specifically selected from F and Cl.

According to a further particular embodiment, R⁴ is aryl, in particular phenyl, wherein the aryl or phenyl moiety in each case is unsubstituted or carries one, two or three identical or different groups R^{4b} which independently of one another are selected from halogen, C₁-C₂-alkyl, C₁-C₂-alkoxy, C₁-C₂-halogenalkyl and C₁-C₂-halogenalkoxy, in particular F, Cl, Br, methyl, OCH₃, CF₃ and OCF₃. In one embodiment, R⁴ is unsubstituted phenyl. In another embodiment, R⁴ is phenyl, that is substituted by one, two or three, in particular one, halogen, in particular selected from F, Cl and Br, more specifically selected from F and Cl.

According to a further embodiment, R⁴ is hydrogen or a partially unsaturated three-, four-, five-, six-, seven-, eight-, nine-, or ten-membered carbocycle or heterocycle, in particular three-, four-, five- or six-membered, wherein the heterocycle contains one, two, three or four heteroatoms selected from N, O and S, and wherein the carbocycle and heterocycle are unsubstituted or carry one, two, three or four substituents R^{4b} as defined below. According to one embodiment thereof, the carbocycle or heterocycle is unsubstituted.

According to a further embodiment, R⁴ is a partially unsaturated three-, four-, five-, six-, seven-, eight-, nine-, or ten-membered carbocycle or heterocycle, in particular three-, four-, five- or six-membered, wherein the heterocycle contains one, two, three or four heteroatoms selected from N, O and S, and wherein the carbocycle and heterocycle are unsubstituted or carry one, two, three or four substituents R^{4b} as defined below. According to one embodiment thereof, the carbocycle or heterocycle is unsubstituted.

According to still a further embodiment, R⁴ is hydrogen or a saturated three-, four-, five-, six-, seven-, eight-, nine-, or ten-membered carbocycle or heterocycle, in particular three-, four-, five- or six-membered, wherein the heterocycle contains one, two, three or four heteroatoms selected from N, O and S, and wherein the carbocycle and heterocycle are unsubstituted or carry one, two, three or four substituents R^{4b} as defined below. According to one embodiment thereof, the carbocycle or heterocycle is unsubstituted.

According to still a further embodiment, R⁴ is a saturated three-, four-, five-, six-, seven-, eight-, nine-, or ten-membered carbocycle or heterocycle, in particular three-, four-, five- or six-membered, wherein the heterocycle contains one, two, three or four heteroatoms selected from N, O and S, and wherein the carbocycle and heterocycle are unsubstituted or carry one, two, three or four substituents R^{4b} as defined below. According to one embodiment thereof, the carbocycle or heterocycle is unsubstituted.

According to a further embodiment, R⁴ is hydrogen or a partially unsaturated three-, four-, five-, six-, seven-, eight-, nine-, or ten-membered heterocycle, in particular three-, four-, five- or six-membered, wherein the heterocycle contains one, two, three or four heteroatoms selected from N, O and S, and wherein the heterocycle is unsubstituted or carries one, two, three or four substituents R^{4b} as defined below. According to one embodiment thereof, the heterocycle is unsubstituted.

According to a further embodiment, R⁴ is a partially unsaturated three-, four-, five-, six-, seven-, eight-, nine-, or ten-membered heterocycle, in particular three-, four-, five- or six-membered, wherein the heterocycle contains one, two, three or four heteroatoms selected from N, O and S, and wherein the heterocycle is unsubstituted or carries one, two, three or four substituents R^{4b} as defined below. According to one embodiment thereof, the heterocycle is unsubstituted.

According to a further embodiment, R⁴ is hydrogen or a saturated three-, four-, five-, six-, seven-, eight-, nine-, or ten-membered heterocycle, in particular three-, four-, five- or six-membered, wherein the heterocycle contains one, two, three or four heteroatoms selected from N, O and S, and wherein the heterocycle is unsubstituted or carries one, two, three or four substituents R^{4a} as defined below. According to one embodiment thereof, the heterocycle is unsubstituted.

According to a further embodiment, R⁴ is a saturated three-, four-, five-, six-, seven-, eight-, nine-, or ten-membered heterocycle, in particular three-, four-, five- or six-membered, wherein the heterocycle contains one, two, three or four heteroatoms selected from N, O and S, and wherein the heterocycle is unsubstituted or carries one, two, three or four substituents R^{4b} as defined below. According to one embodiment thereof, the heterocycle is unsubstituted.

According to a further embodiment, in the embodiments of R⁴ described above, the heterocycle contains preferably one, two or three, more specifically one or two heteroatoms selected from N, O and S. More specifically, the hetereocycle contains one heteroatom selected from N, O and S. In particular, the heterocycle contains one or two, in particular one O.

According to one particular embodiment, R⁴ is hydrogen or a 4-membered saturated heterocycle which contains 1 or 2 heteroatoms, in particular 1 heteroatom, from the group consisting of N, O and S, as ring members. In one embodiment, the heterocycle contains one O as heteroatom. For example, the formed heterocycle is oxetane. According to one embodiment thereof, the heterocycle is unsubstituted, i.e. it does not carry any substituent R^{4b}. According to a further embodiment, it carries one, two, three or four R^{4b}.

According to one particular embodiment, R⁴ is a 4-membered saturated heterocycle which contains 1 or 2 heteroatoms, in particular 1 heteroatom, from the group consisting of N, O and S, as ring members. In one embodiment, the heterocycle contains one O as heteroatom. For example, the formed heterocycle is oxetane. According to one embodiment thereof, the heterocycle is unsubstituted, i.e. it does not carry any substituent R^{4b}. According to a further embodiment, it carries one, two, three or four R^{4b}.

According to a further particular embodiment, R⁴ is hydrogen or a 5-membered saturated heterocycle which contains 1, 2 or 3, in particular 1 or 2, heteroatoms from the group consisting of N, O and S, as ring members. In one embodiment, the heterocycle contains one O as heteroatom. According to one embodiment thereof, the heterocycle is unsubstituted, i.e. it does not carry any substituent R^{4b}. According to a further embodiment, it carries one, two, three or four R^{4b}.

According to a further particular embodiment, R⁴ is a 5-membered saturated heterocycle which contains 1, 2 or 3, in particular 1 or 2, heteroatoms from the group consisting of N, O and S, as ring members. In one embodiment, the heterocycle contains one O as heteroatom. According to one embodiment thereof, the heterocycle is unsubstituted, i.e. it does not carry any substituent R^{4b}. According to a further embodiment, it carries one, two, three or four R^{4b}.

According to a further particular embodiment, R⁴ is hydrogen or a 6-membered saturated heterocycle which contains 1, 2 or 3, in particular 1 or 2, heteroatoms from the group consisting of N, O and S as ring members. According to one embodiment thereof, the heterocycle is unsubstituted, i.e. it does not carry any substituent R^{4b}. According to a further embodiment, it carries one, two, three or four R^{4b}. According to one specific embodiment thereof, said 6-membered saturated heterocycle contains 1 or 2, in particular 1, heteroatom(s) O. According to one embodiment thereof, the respective 6-membered heterocycle is unsubstituted, i.e. it does not carry any substituent R^{4b}. According to a further embodiment, it carries one, two, three or four R^{4b}.

According to a further particular embodiment, R⁴ is a 6-membered saturated heterocycle which contains 1, 2 or 3, in particular 1 or 2, heteroatoms from the group consisting of N, O and S as ring members. According to one embodiment thereof, the heterocycle is unsubstituted, i.e. it does not carry any substituent R^{4b}. According to a further embodiment, it carries one, two, three or four R^{4b}. According to one specific embodiment thereof, said 6-membered saturated heterocycle contains 1 or 2, in particular 1, heteroatom(s) O. According to one embodiment thereof, the respective 6-membered heterocycle is unsubstituted, i.e. it does not carry any substituent R^{4b}. According to a further embodiment, it carries one, two, three or four R^{4b}.

According to a further embodiment, R⁴ is hydrogen or a partially unsaturated three-, four-, five-, six-, seven-, eight-, nine-, or ten-membered carbocycle, in particular three-, four-, five- or six-membered, wherein the carbocycle is unsubstituted or carries one, two, three or four substituents R^{4b} as defined below. According to one embodiment thereof, the carbocycle is unsubstituted.

According to a further embodiment, R⁴ is a partially unsaturated three-, four-, five-, six-, seven-, eight-, nine-, or ten-membered carbocycle, in particular three-, four-, five- or six-membered, wherein the carbocycle is unsubstituted or carries one, two, three or four substituents R^{4b} as defined below. According to one embodiment thereof, the carbocycle is unsubstituted.

According to a further embodiment, R⁴ is hydrogen or a saturated three-, four-, five-, six-, seven-, eight-, nine-, or ten-membered carbocycle, in particular three-, four-, five- or six-membered, wherein the carbocycle is unsubstituted or carries one, two, three or four substituents R^{4b} as defined below. According to one embodiment thereof, the carbocycle is unsubstituted.

According to a further embodiment, R⁴ is a saturated three-, four-, five-, six-, seven-, eight-, nine-, or ten-membered carbocycle, in particular three-, four-, five- or six-membered, wherein the carbocycle is unsubstituted or carries one, two, three or four substituents R^{4b} as defined below. According to one embodiment thereof, the carbocycle is unsubstituted.

According to one particular embodiment, R⁴ is hydrogen or a 3-membered saturated carbocycle. According to one embodiment thereof, the carbocycle is unsubstituted, i.e. it does not carry any substituent R^{4a}. According to a further embodiment, it carries one, two, three or four R^{4b}.

According to one particular embodiment, R⁴ is a 3-membered saturated carbocycle. According to one embodiment thereof, the carbocycle is unsubstituted, i.e. it does not carry any substituent R^{4b}. According to a further embodiment, it carries one, two, three or four R^{4b}.

According to one particular embodiment, R⁴ is hydrogen or a 4-membered saturated carbocycle. According to one embodiment thereof, the carbocycle is unsubstituted, i.e. it does not carry any substituent R^{4b}. According to a further embodiment, it carries one, two, three or four R^{4b}.

According to one particular embodiment, R⁴ is a 4-membered saturated carbocycle. According to one embodiment thereof, the carbocycle is unsubstituted, i.e. it does not carry any substituent R^{4b}. According to a further embodiment, it carries one, two, three or four R^{4b}.

According to one particular embodiment, R⁴ is hydrogen or a 5-membered saturated carbocycle. According to one embodiment thereof, the carbocycle is unsubstituted, i.e. it does not carry any substituent R^{4b}. According to a further embodiment, it carries one, two, three or four R^{4b}.

According to one particular embodiment, R⁴ is a 5-membered saturated carbocycle. According to one embodiment thereof, the carbocycle is unsubstituted, i.e. it does not carry any substituent R^{4b}. According to a further embodiment, it carries one, two, three or four R^{4b}.

According to one particular embodiment, R⁴ is hydrogen or a 6-membered saturated carbocycle. According to one embodiment thereof, the carbocycle is unsubstituted, i.e. it does not carry any substituent R^{4b}. According to a further embodiment, it carries one, two, three or four R^{4b}.

According to one particular embodiment, R⁴ is a 6-membered saturated carbocycle. According to one embodiment thereof, the carbocycle is unsubstituted, i.e. it does not carry any substituent R^{4b}. According to a further embodiment, it carries one, two, three or four R^{4b}.

According to a further particular embodiment, R⁴ is selected from hydrogen, C₁-C₆-alkyl, C₁-C₆-halogenalkyl, phenyl-C₁-C₆-alkyl, halogenphenyl-C₁-C₆-alkyl, phenyl, halogenphenyl and three-, four-, five- or six-membered carbocycle, wherein the carbocycle is unsubstituted or carries one, two, three or four substituents R^{4b} as defined below. According to one embodiment thereof, the carbocycle is unsubstituted. In a particular embodiment, R⁴ is selected from hydrogen, C₁-C₆-alkyl, C₁-C₆-halogenalkyl, phenyl-CH₂, halogenphenyl-CH₂, phenyl, halogenphenyl and three-, four-, five- or six-membered carbocycle, wherein the carbocycle is unsubstituted or carries one, two, three or four substituents R^{4b} as defined below.

According to a further particular embodiment, R⁴ is selected from C₁-C₆-alkyl, C₁-C₆-halogenalkyl, phenyl-C₁-C₆-alkyl, halogenphenyl-C₁-C₆-alkyl, phenyl, halogenphenyl and three-, four-, five- or six-membered carbocycle, wherein the carbocycle is unsubstituted or carries one, two, three or four substituents R^{4b} as defined below. According to one embodiment thereof, the carbocycle is unsubstituted. In a particular embodiment, R⁴ is selected from C₁-C₆-alkyl, C₁-C₆-halogenalkyl, phenyl-CH₂, halogenphenyl-CH₂, phenyl, halogenphenyl and three-, four-, five- or six-membered carbocycle, wherein the carbocycle is unsubstituted or carries one, two, three or four substituents R^{4b} as defined below.

Particularly preferred embodiments of R⁴ according to the invention are in Table P4 below, wherein each line of lines P4-1 to P4-33 corresponds to one particular embodiment of the invention, wherein P4-1 to P4-33 are also in any combination with one another a preferred embodiment of the present invention. The connection point to the carbon atom, to which R⁴ is bound is marked with "#" in the drawings.

**Table P4:**

| **No.** | **R⁴** |
|---|---|
| P4-1 | H |
| P4-2 | CH₃ |
| P4-3 | C₂H₅ |
| P4-4 | iso-C₃H₇ |
| P4-5 | CH₂CH₂CH₃ |
| P4-6 | CH(CH₃)₂ |
| P4-7 | CH₂CH₂CH₂CH₃ |
| P4-8 | CH₂CH(CH₃)₂ |
| P4-9 | C(CH₃)₃ |
| P4-10 | CH₂CH₂CH₂CH₂CH₃ |
| P4-11 | CH₂CH₂CH(CH₃)₂ |
| P4-12 | CF₃ |
| P4-13 | CHF₂ |
| P4-14 | CHCl₂ |
| P4-15 | CH₂F |
| P4-16 | CH₂Cl |
| P4-17 | CH₂CF₃ |
| P4-18 | CH₂CCl₃ |
| P4-19 | CF₂CHF₂ |
| P4-20 | C₆H₅ |
| P4-21 | 4-Cl-C₆H₄ |
| P4-22 | 4-F-C₆H₄ |
| P4-23 | CH₂-C₆H₅ |
| P4-24 | 3-pyridyl |
| P4-25 | 4-pyridyl |
| P4-26 | C1 |
| P4-27 | C2 |
| P4-28 | C3 |
| P4-29 | C4 |
| P4-30 | C5 |
| P4-31 | C6 |
| P4-32 | C7 |
| P4-33 | C8 |

According to a further embodiment, R³ and R⁴ together with the carbon atom to which they are bound (marked with * in formula I) form a saturated or partially unsaturated three-, four-, five-, six-, seven-, eight-, nine-, or ten-membered carbocycle or heterocycle; wherein the heterocycle contains one, two, three or four heteroatoms selected from N, O and S, wherein the heteroatom N may carry one substituent R^{N} selected from C₁-C₄-alkyl, C₁-C₄-halogenalkyl and SO₂Ph, wherein Ph is unsubstituted phenyl or phenyl that is substituted by one, two or three substituents selected from C₁-C₄-alkyl, and wherein the heteroatom S may be in the form of its oxide SO or SO₂, and wherein the carbocycle or heterocycle is unsubstituted or carries one, two, three or four substituents R³⁴ independently selected from halogen, OH, CN, NO₂, SH, NH₂, C₁-C₆-alkyl, C₁-C₆-halogenalkyl, C₁-C₆-alkoxy,C₁-C₆-halogenalkoxy, C₁-C₆-alkylthio,C₁-C₆-halogenalkylthio, C₁-C₄-alkoxy-C₁-C₄-alkyl, phenyl and phenoxy, wherein the phenyl groups are unsubstituted or carry one, two, three, four or five substituents R^{34a} selected from the group consisting of halogen, OH, C₁-C₄-alkyl, C₁-C₄-halogenalkyl, C₁-C₄-alkoxy and C₁-C₄-halogenalkoxy; and wherein in each case one or two CH₂ groups of the carbo- or heterocycle may be replaced by a group independently selected from C(=O) and C(=S).

According to one embodiment, R³ and R⁴ together with the carbon atom to which they are bound (marked with * in formula I) form a saturated or partially unsaturated three-, four-, five-, six-, seven-, eight-, nine-, or ten-membered heterocycle that is unsubstituted or substituted.

According to a further embodiment, the heterocycle formed by R³ and R⁴ is saturated.

According to a further embodiment, the heterocycle formed by R³ and R⁴ is a saturated unsubstituted or substituted heterocycle, wherein the heterocycle contains one, two or three, more particularly one or two, specifically one, heteroatom(s) selected from NH, NR^{N}, O, S, S(=O) and S(=O)₂, wherein R^{N} is defined and preferably defined above. According to one embodiment, this saturated heterocycle is unsubstituted. According to a further embodiment, the saturated heterocycle carries one, two, three or four substituents R³⁴. In one further particular embodiment, said heterocycle is four- or six-membered.

According to a further embodiment, the unsubstituted or substituted and saturated or partially unsaturated heterocycle is three-, four-, five- or six-membered and contains one, two or three, more particularly one or two, heteroatoms selected from NH, NR^{N}, O, S, S(=O) and S(=O)₂, wherein R^{N} is as defined above or preferably selected from C₁-C₂-alkyl, C₁-C₂-halogenalkyl and SO₂Ph, wherein Ph is unsubstituted phenyl or phenyl that is substituted by one C₁-C₂-alkyl. In one further particular embodiment, said heterocycle is four- or six-membered.

According to a further embodiment, the heterocycle formed by R³ and R⁴ contains one, two or three, more specifically one or two, heteroatoms selected from NH and NR^{N}, wherein R^{N} is as defined and preferably defined below, more particularly selected from C₁-C₂-alkyl, C₁-C₂-halogenalkyl and SO₂Ph, wherein Ph is unsubstituted phenyl or phenyl that is substituted by one methyl. In one embodiment thereof, it contains one or two heteroatoms NH, in particular one NH. In another embodiment, it contains one or two heteroatoms NR^{N}, in particular one NR^{N}, wherein R^{N} in each case is as defined and preferably defined above.

According to a further embodiment, the heterocycle formed by R³ and R⁴ contains one, two or three, more specifically one or two, in particular one, heteroatom(s) selected from S, S(=O) and S(=O)₂. In one embodiment thereof, it contains one or two heteroatoms S, in particular one S. In another embodiment, it contains one or two heteroatoms S(=O), in particular one S(=O). In still another embodiment, it contains one or two heteroatoms S(=O)₂, in particular one S(=O)₂.

According to a further embodiment, the heterocycle formed by R³ and R⁴ contains one or two heteroatoms O. In one embodiment thereof, it contains one heteroatom O. In another embodiment, it contains two heteroatoms O.

According to a further embodiment, the heterocycle formed by R³ and R⁴ is unsubstituted, i.e. it does not carry any substituent R³⁴. According to a further embodiment, it carries one, two, three or four R³⁴.

According to one particular embodiment, R³ and R⁴ together form a 4-membered saturated heterocycle which contains 1 or 2 heteroatoms, in particular 1 heteroatom, from the group consisting of NH, NR^{N}, O, S, S(=O) and S(=O)₂, as ring members, wherein R^{N} is defined and preferably defined above. In one embodiment, the heterocycle contains one O as heteroatom. For example, the formed heterocycle is oxetane. According to one embodiment thereof, the heterocycle is unsubstituted, i.e. it does not carry any substituent R³⁴. According to a further embodiment, it carries one, two, three or four R³⁴.

According to a further particular embodiment, R³ and R⁴ together form a 5-membered saturated heterocycle which contains 1, 2 or 3, in particular 1 or 2, heteroatoms from the group consisting of NH, NR^{N}, O, S, S(=O) and S(=O)₂, as ring members, wherein R^{N} is as defined and preferably defined above. According to one embodiment thereof, the heterocycle is unsubstituted, i.e. it does not carry any substituent R³⁴. According to a further embodiment, it carries one, two, three or four R³⁴.

According to a further particular embodiment, R³ and R⁴ together form a 6-membered saturated heterocycle which contains 1, 2 or 3, in particular 1 or 2, heteroatoms from the group consisting of NH, NR^{N}, O, S, S(=O) and S(=O)₂, as ring members, wherein R^{N} is as defined and preferably defined below. According to one embodiment thereof, the heterocycle is unsubstituted, i.e. it does not carry any substituent R³⁴. According to a further embodiment, it carries one, two, three or four R³⁴. According to one specific embodiment thereof, said 6-membered saturated heterocycle contains 1 or 2 heteroatoms selected from NH and NR^{N}. According to a further specific embodiment thereof, said 6-membered saturated heterocycle contains 1 or 2 heteroatoms O. According to a further specific embodiment thereof, said 6-membered saturated heterocycle contains 1 or 2 heteroatoms selected from S, S(=O) and S(=O)₂. According to one embodiment thereof, the respective 6-membered heterocycle is unsubstituted, i.e. it does not carry any substituent R³⁴. According to a further embodiment, it carries one, two, three or four R³⁴.

According to one further embodiment R³ together with R⁴ and with the carbon atom to which they are bound form a saturated three-, four-, five-, six-, seven-, eight-, nine-, or ten-membered, in particular three-, four-, five- or six-membered carbocycle, more specifically five- or six-membered carbocycle, that is unsubstituted or carries one, two, three or four substituents R³⁴ as defined below. According to one embodiment thereof, R³ and R⁴ form a cyclopropyl, that is unsubstituted or carries one, two, three or four substituents R³⁴ as defined below. According to a further embodiment thereof, R³ and R⁴ form a cyclobutyl, that is unsubstituted or carries one, two, three or four substituents R³⁴ as defined below. According to still a further embodiment thereof, R³ and R⁴ form a cyclopentyl, that is unsubstituted or carries one, two, three or four substituents R³⁴ as defined below. According to still a further embodiment thereof, R³ and R⁴ form a cyclohexyl, that is unsubstituted or carries one, two, three or four substituents R³⁴ as defined below. According to still a further embodiment thereof, R³ and R⁴ form a cycloheptyl, that is unsubstituted or carries one, two, three or four substituents R³⁴ as defined below.

R³⁴ are the possible substituents for the carbo- or heterocycle formed by R³ and R⁴ and are independently selected from halogen, OH, CN, NO₂, SH, NH₂, C₁-C₆-alkyl, C₁-C₆-halogenalkyl, C₁-C₆-alkoxy,C₁-C₆-halogenalkoxy, C₁-C₆-alkylthio,C₁-C₆-halogenalkylthio, C₁-C₄-alkoxy-C₁-C₄-alkyl, phenyl and phenoxy, wherein the phenyl groups are unsubstituted or carry one, two, three, four or five substituents R^{34a} selected from the group consisting of halogen, OH, C₁-C₄-alkyl, C₁-C₄-halogenalkyl, C₁-C₄-alkoxy and C₁-C₄-halogenalkoxy; and wherein in each case one or two CH₂ groups of the carbo- or heterocycle may be replaced by a group independently selected from C(=O) and C(=S).

In one preferred embodiment, R³⁴ is in each case independently selected from halogen, OH, CN, SH, C₁-C₆-alkyl, C₁-C₆-halogenalkyl, C₁-C₆-alkoxy,C₁-C₆-halogenalkoxy and C₁-C₆-alkylthio. In one further preferred embodiment, R³⁴ is in each case independently selected from halogen, C₁-C₆-alkyl and C₁-C₆-halogenalkyl. In one further particular embodiment, R³⁴ is in each case independently selected from C₁-C₆-alkyl, such as methyl and ethyl.

R^{N} is the substituent of the heteroatom NR^{N} that is contained in the heterocycle formed by R³ and R⁴ in some of the inventive compounds. R^{N} is selected from C₁-C₄-alkyl, C₁-C₄-halogenalk and SO₂Ph, wherein Ph is unsubstituted phenyl or phenyl that is substituted by one, two or three substituents selected from C₁-C₄-alkyl. In one preferred embodiment, R^{N} is in each case independently selected from C₁-C₂-alkyl, C₁-C₂-halogenalkyl and SO₂Ph, wherein Ph is unsubstituted phenyl or phenyl that is substituted by one methyl substituents. In one particular embodiment, R^{N} is in each case independently selected from C₁-C₂-alkyl, more particularly methyl. In one particular embodiment, R^{N} is in each case independently selected from SO₂Ph, wherein Ph is unsubstituted phenyl or phenyl that is substituted by one methyl.

Particularly preferred embodiments of combinations of R³ and R⁴ according to the invention are in Table P34 below, wherein each line of lines P34-1 to P34-256 corresponds to one particular embodiment of the invention, wherein P34-1 to P34-256 are also in any combination with one another a preferred embodiment of the present invention. The carbon atom, to which R³ and R⁴ are bound is marked with * in the drawings. "Ts" in the drawings stands for the tosylgroup SO₂-(p-CH₃)phenyl. The abbreviations of the cycles (C1 to C8) are explained in Table P3 above

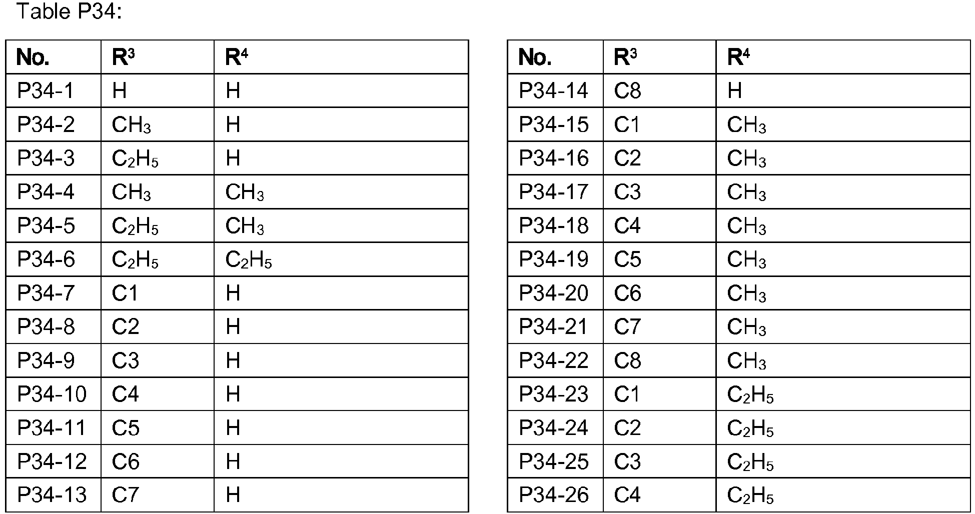

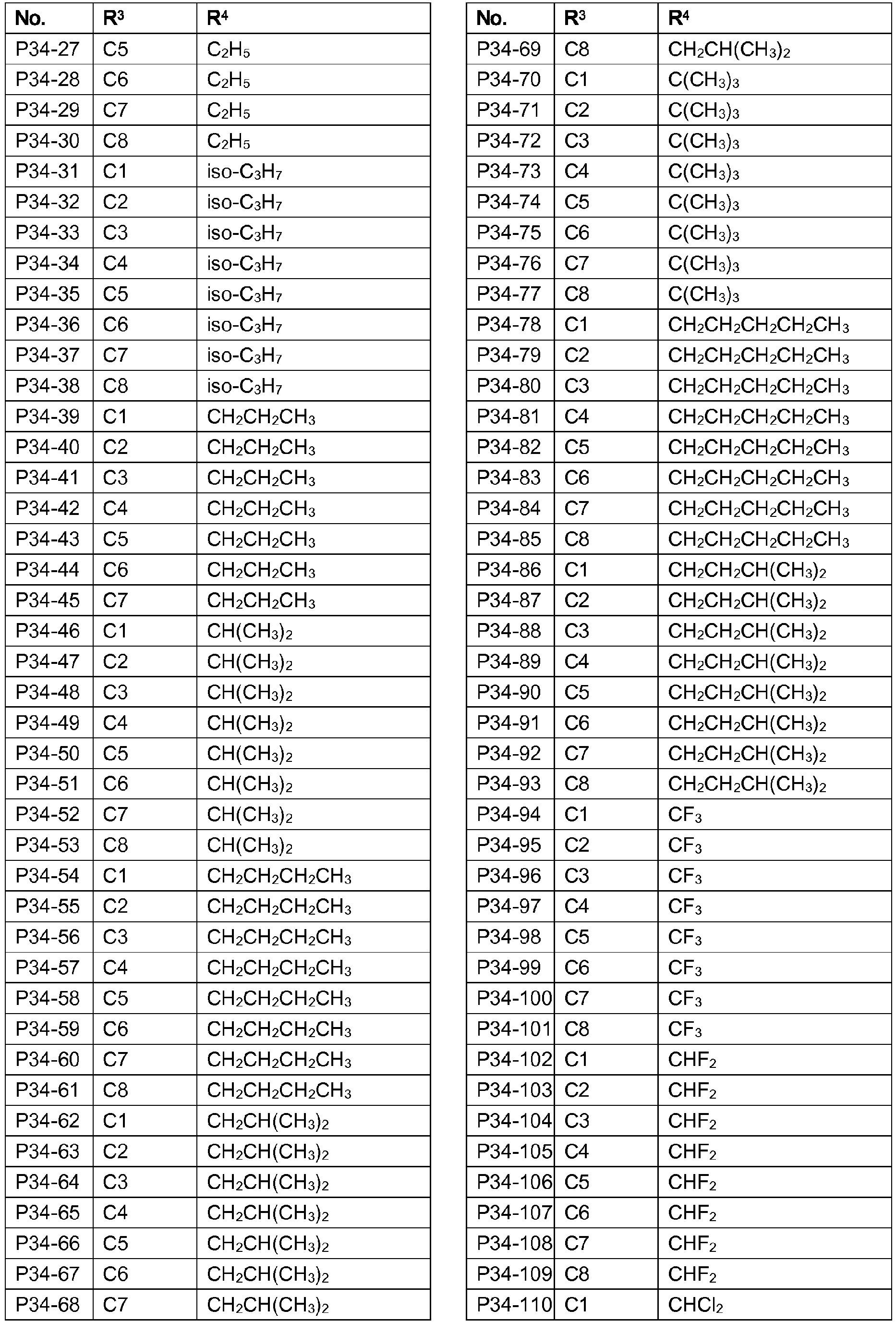

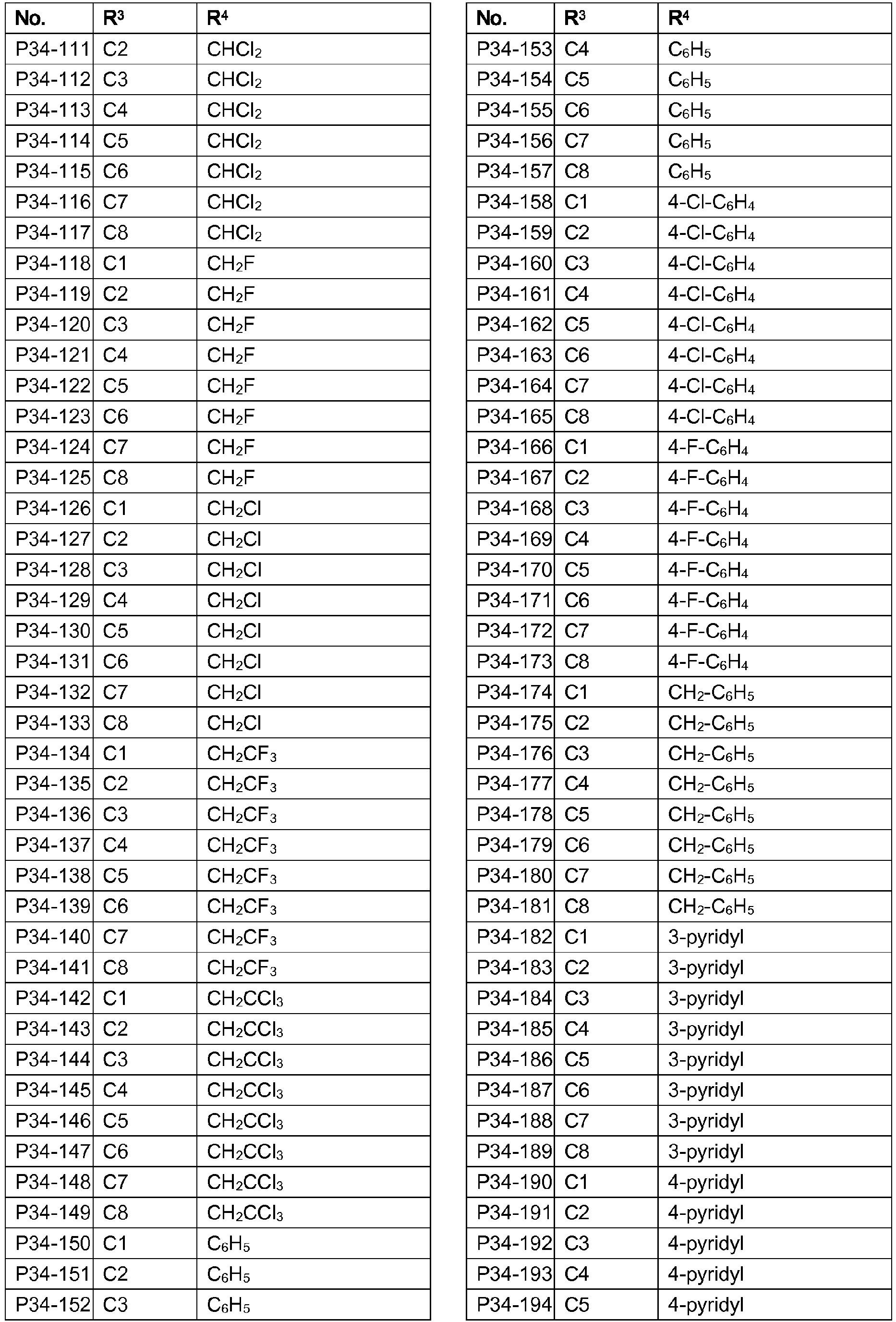

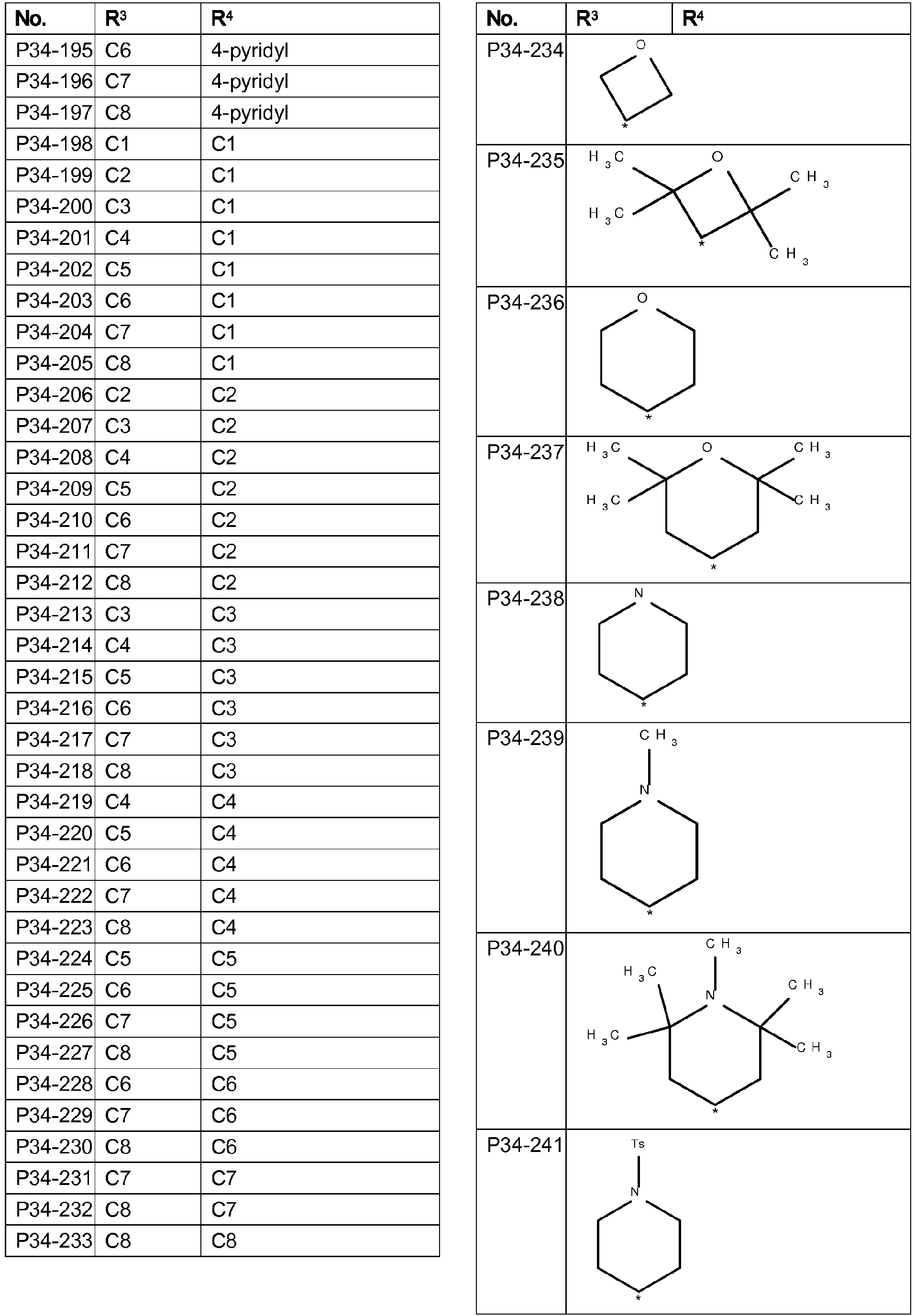

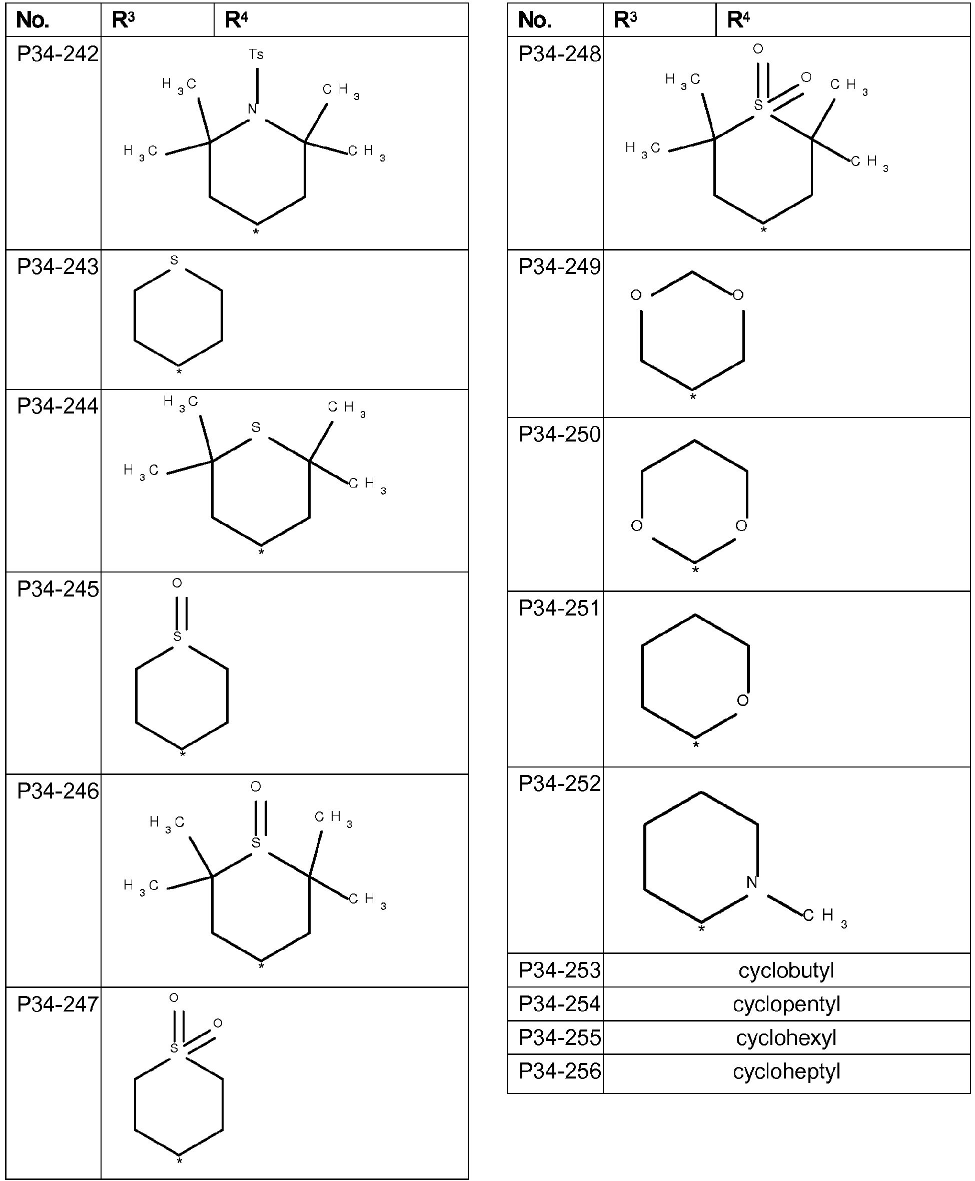

R^{x} in the substituent NH-SO₂-R^{x} is in each case independently selected from C₁-C₄-alkyl, C₁-C₄-halogenalkyl, unsubstituted aryl and aryl that is substituted by one, two, three, four or five substituents R^{x1} independently selected from C₁-C₄-alkyl. In particular, R^{x} is in each case independently selected from C₁-C₄-alkyl and phenyl that is substituted by one, two or three R^{x1} independently selected from C₁-C₂-alkyl, more specifically R^{x} is in each case independently selected from C₁-C₄-alkyl and phenyl that is substituted by one CH₃., more specifically SO₂-R^{x} is the tosyl group ("Ts").

R^{3a} are the possible substituents for the the aliphatic moieties of R³ and the R^{3a} are in each case independently selected from halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, C₁-C₆-alkoxy, C₃-C₆-cycloalkyl, C₃-C₆-halogencycloalkyl, C₁-C₄-halogenalkoxy, C₁-C₆-alkylthio,aryl and phenoxy, wherein the aryl and phenyl groups are independently unsubstituted or carry one, two, three, four or five substituents selected from the group consisting of halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, NH-SO₂-R^{x}, C₁-C₆-alkylthio, C₁-C₄-alkyl, C₁-C₄-halogenalkyl, C₁-C₄-alkoxy and C₁-C₄-halogenalkoxy.

In one preferred embodiment, R^{3a} is in each case independently selected from halogen, OH, CN, C₁-C₆-alkoxy, C₁-C₆-halogenalkoxy, phenyl and halogenphenyl, wherein the halogenphenyl carries one, two or three halogen selected from the group consisting of F, Cl and Br. In one further preferred embodiment, R^{3a} is in each case independently selected from halogen, phenyl and halogenphenyl, wherein the halogenphenyl carries one, two or three halogen selected from the group consisting of F, Cl and Br, in particular selected from F and Cl.

R^{3b} are the possible substituents for the carbocycle, heterocycle, heteroaryl and aryl moieties and are independently selected from halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, NH-SO₂-R^{x}, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy, C₁-C₆-alkylthio, C₁-C₆-halogenalkylthio, C₁-C₄-alkoxy-C₁-C₄-alkyl, phenyl and phenoxy, wherein the phenyl groups are unsubstituted or carry one, two, three, four or five substituents selected from the group consisting of halogen, OH, C₁-C₄-alkyl, C₁-C₄-halogenalkyl, C₁-C₄-alkoxy and C₁-C₄-halogenalkoxy.

In one preferred embodiment, R^{3b} is in each case independently selected from halogen, OH, CN, SH, C₁-C₆-alkyl, C₁-C₆-halogenalkyl, C₁-C₆-alkoxy, C₁-C₆-halogenalkoxy and C₁-C₆-alkylthio. In one further preferred embodiment, R^{3b} is in each case independently selected from halogen, C₁-C₆-alkyl and C₁-C₆-halogenalkyl. In one further particular embodiment, R^{3b} is in each case independently selected from C₁-C₆-alkyl, such as methyl and ethyl. In one further particular embodiment, R^{3b} is in each case independently selected from halogen, such as F, Cl and Br.

R^{4a} are the possible substituents for the the aliphatic moieties of R⁴ and the R^{4a} are in each case independently selected from halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, C₁-C₆-alkoxy, C₃-C₆-cycloalkyl, C₃-C₆-halogencycloalkyl, C₁-C₄-halogenalkoxy, C₁-C₆-alkylthio,aryl and phenoxy, wherein the aryl and phenyl groups are independently unsubstituted or carry one, two, three, four or five substituents selected from the group consisting of halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, NH-SO₂-R^{x}, C₁-C₆-alkylthio, C₁-C₄-alkyl, C₁-C₄-halogenalkyl, C₁-C₄-alkoxy and C₁-C₄-halogenalkoxy.

In one preferred embodiment, R^{4a} is in each case independently selected from halogen, OH, CN, C₁-C₆-alkoxy, C₁-C₆-halogenalkoxy, phenyl and halogenphenyl, wherein the halogenphenyl carries one, two or three halogen selected from the group consisting of F, Cl and Br. In one further preferred embodiment, R^{4a} is in each case independently selected from halogen, phenyl and halogenphenyl, wherein the halogenphenyl carries one, two or three halogen selected from the group consisting of F, Cl and Br, in particular selected from F and Cl.

R^{4b} are the possible substituents for the carbocycle, heterocycle, heteroaryl and aryl moieties and are independently selected from halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, NH-SO₂-RX, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy, C₁-C₆-alkylthio, C₁-C₆-halogenalkylthio, C₁-C₄-alkoxy-C₁-C₄-alkyl, phenyl and phenoxy, wherein the phenyl groups are unsubstituted or carry one, two, three, four or five substituents selected from the group consisting of halogen, OH, C₁-C₄-alkyl, C₁-C₄-halogenalkyl, C₁-C₄-alkoxy and C₁-C₄-halogenalkoxy.

In one preferred embodiment, R^{4b} is in each case independently selected from halogen, OH, CN, SH, C₁-C₆-alkyl, C₁-C₆-halogenalkyl, C₁-C₆-alkoxy, C₁-C₆-halogenalkoxy and C₁-C₆-alkylthio. In one further preferred embodiment, R^{4b} is in each case independently selected from halogen, C₁-C₆-alkyl and C₁-C₆-halogenalkyl. In one further particular embodiment, R^{4b} is in each case independently selected from C₁-C₆-alkyl, such as methyl and ethyl. In one further particular embodiment, R^{4b} is in each case independently selected from halogen, such as F, Cl and Br.

R⁵ according to the invention is hydrogen, halogen, OH, CN, NO₂, SH, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl, C₁-C₆-alkoxy, C₃-C₆-cycloalkyl, saturated or partially unsaturated three-, four-, five-, six-, seven-, eight-, nine-, or ten-membered heterocycle, five- or six-membered heteroaryl or aryl; wherein the heterocyle or heteroaryl contains one, two or three heteroatoms selected from N, O and S; and wherein R^{x} is defined above; and wherein the aliphatic moieties of R⁵ are not further substituted or carry one, two, three or up to the maximum possible number of identical or different groups R^{5a} which independently of one another are selected from:
R^{5a} halogen, OH, CN, C₁-C₆-alkoxy, C₃-C₆-cycloalkyl, C₃-C₆-halogencycloalkyl, C₁-C₄-halogenalkoxy, C₁-C₆-alkylthio and phenoxy, wherein the phenyl group is unsubstituted or carries one, two, three, four or five substituents R^{55a} selected from the group consisting of halogen, OH, C₁-C₄-alkyl, C₁-C₄-halogenalkyl, C₁-C₄-alkoxy and C₁-C₄-halogenalkoxy;
wherein the cycloalkyl, heterocyle, heteroaryl and aryl moieties of R⁵ are not further substituted or carry one, two, three, four, five or up to the maximum number of identical or different groups R^{5b} which independently of one another are selected from:
R^{5b} halogen, OH, CN, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy and C₁-C₆-alkylthio;
or R⁵ together with R⁶ and with the carbon atom to which they are bound with the carbon atom to which they are bound (marked with C** in formula I) form a saturated or partially unsaturated three-, four-, five-, six-, seven-, eight-, nine-, or ten-membered carbo- or heterocycle; wherein the heterocycle contains one, two, three or four heteroatoms selected from N, O and S, and wherein the carbocycle or heterocycle is unsubstituted or carries one, two, three or four substituents R⁵⁶ independently selected from halogen, OH, CN, NO₂, SH, NH₂, C₁-C₆-alkyl, C₁-C₆-halogenalkyl, C₁-C₆-alkoxy, C₁-C₆-halogenalkoxy, C₁-C₆-alkylthio, C₁-C₆-halogenalkylthio, C₁-C₄-alkoxy-C₁-C₄-alkyl, phenyl and phenoxy; wherein the phenyl groups are unsubstituted or carry one, two, three, four or five substituents R^{56a} selected from the group consisting of halogen, OH, C₁-C₄-alkyl, C₁-C₄-halogenalkyl, C₁-C₄-alkoxy and C₁-C₄-halogenalkoxy; and
wherein in each case one or two CH₂ groups of the carbo- or heterocycle may be replaced by a group independently selected from C(=O) and C(=S).

According to one embodiment, R⁵ is selected from hydrogen, halogen, OH, CN, NO₂, SH, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl and C₁-C₆-alkoxy; or R⁵ together with R⁶ and with the carbon atom to which they are bound form a saturated three-, four-, five- or six-membered carbocycle; wherein the carbocycle is unsubstituted or substituted by R⁵⁶ as defined below; in particular selected from hydrogen, F, Cl, Br, OH, CN, NH₂, NH(C₁-C₄-alkyl), N(C₁-C₄-alkyl)₂, NH-SO₂-R^{x}, C₁-C₄-alkyl and C₁-C₄-alkoxy; or R⁵ together with R⁶ and with the carbon atom to which they are bound form a saturated three-, four-, five- or six-membered carbocycle; wherein the carbocycle is unsubstituted or substituted by R⁵⁶ as defined below; and wherein R^{x} is as defined and preferably defined below; and wherein the aliphatic moieties of R⁵ are not further substituted or carry one, two, three or up to the maximum possible number of identical or different groups R^{5a} as defined and preferably defined below.

According to one embodiment, R⁵ is selected from halogen, OH, CN, NO₂, SH, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl and C₁-C₆-alkoxy; or R⁵ together with R⁶ and with the carbon atom to which they are bound form a saturated three-, four-, five- or six-membered carbocycle; wherein the carbocycle is unsubstituted or substituted by R⁵⁶ as defined below; in particular selected from F, Cl, Br, OH, CN, NH₂, NH(C₁-C₄-alkyl), N(C₁-C₄-alkyl)₂, NH-SO₂-RX, C₁-C₄-alkyl and C₁-C₄-alkoxy; or R⁵ together with R⁶ and with the carbon atom to which they are bound form a saturated three-, four-, five- or six-membered carbocycle; wherein the carbocycle is unsubstituted or substituted by R⁵⁶ as defined below; and wherein R^{x} is as defined and preferably defined below; and wherein the aliphatic moieties of R⁵ are not further substituted or carry one, two, three or up to the maximum possible number of identical or different groups R^{5a} as defined and preferably defined below.

According to still a further embodiment, R⁵ is selected from hydrogen, halogen, OH, CN, C₁-C₆-alkyl and C₁-C₆-alkoxy; or R⁵ together with R⁶ and with the carbon atom to which they are bound form a saturated three-, four-, five- or six-membered carbocycle; wherein the carbocycle is unsubstituted or substituted by R⁵⁶ as defined below; in particular selected from hydrogen, F, Cl, Br, OH, CN, C₁-C₄-alkyl and C₁-C₄-alkoxy; or R⁵ together with R⁶ and with the carbon atom to which they are bound form a saturated three-, four-, five- or six-membered carbocycle; wherein the carbocycle is unsubstituted or substituted by R⁵⁶ as defined below; and wherein the aliphatic moieties of R⁵ are not further substituted or carry one, two, three or up to the maximum possible number of identical or different groups R^{5a} as defined and preferably defined below.

According to still a further embodiment, R⁵ is selected from halogen, OH, CN, C₁-C₆-alkyl and C₁-C₆-alkoxy; or R⁵ together with R⁶ and with the carbon atom to which they are bound form a saturated three-, four-, five- or six-membered carbocycle; wherein the carbocycle is unsubstituted or substituted by R⁵⁶ as defined below; in particular selected from F, Cl, Br, OH, CN, C₁-C₄-alkyl and C₁-C₄-alkoxy; or R⁵ together with R⁶ and with the carbon atom to which they are bound form a saturated three-, four-, five- or six-membered carbocycle; wherein the carbocycle is unsubstituted or substituted by R⁵⁶ as defined below; and wherein the aliphatic moieties of R⁵ are not further substituted or carry one, two, three or up to the maximum possible number of identical or different groups R^{5a} as defined and preferably defined below.

According to a further embodiment, R⁵ is selected from hydrogen, F, Cl, Br, OH, CN, C₁-C₄-alkyl, C₁-C₄-halogenalkyl, C₁-C₄-alkoxy and C₁-C₆-halogenalkoxy; or R⁵ together with R⁶ and with the carbon atom to which they are bound form a saturated three-, four-, five- or six-membered, in particular 5- or 6-membered, carbocycle; in particular selected from hydrogen, F, Cl, OH, C₁-C₄-alkyl, C₁-C₄-halogenalkyl, C₁-C₄-alkoxy and C₁-C₆-halogenalkoxy; or R⁵ together with R⁶ and with the carbon atom to which they are bound form a saturated three-, four-, five- or six-membered carbocycle, in particular 5- or 6-membered.

According to still a further embodiment, R⁵ is selected from F, Cl, Br, OH, CN, C₁-C₄-alkyl, C₁-C₄-halogenalkyl, C₁-C₄-alkoxy and C₁-C₆-halogenalkoxy; or R⁵ together with R⁶ and with the carbon atom to which they are bound form a saturated three-, four-, five- or six-membered, in particular 5- or 6-membered, carbocycle; in particular selected from F, Cl, OH, C₁-C₄-alkyl, C₁-C₄-halogenalkyl, C₁-C₄-alkoxy and C₁-C₆-halogenalkoxy; or R⁵ together with R⁶ and with the carbon atom to which they are bound form a saturated three-, four-, five- or six-membered, in particular 5- or 6-membered, carbocycle.

According to a further embodiment, R⁵ is selected from hydrogen, NH₂, NH(C₁-C₄-alkyl), N(C₁-C₄-alkyl)₂ and NH-SO₂-R^{x}, wherein R^{x} is as defined and preferably defined below, in particular selected from hydrogen, NH₂, NH(C₁-C₂-alkyl), N(C₁-C₂-alkyl)₂ and NH-SO₂-RX, wherein R^{x} is para-methyl-phenyl.

According to still a further embodiment, R⁵ is selected from NH₂, NH(C₁-C₄-alkyl), N(C₁-C₄-alkyl)₂ and NH-SO₂-R^{x}, wherein R^{x} is as defined and preferably defined below, in particular selected from NH₂, NH(C₁-C₂-alkyl), N(C₁-C₂-alkyl)₂ and NH-SO₂-R^{x}, wherein R^{x} is para-methyl-phenyl.

According to still a further embodiment, R⁵ is selected from hydrogen, C₃-C₆-cycloalkyl, five- or six-membered heteroaryl or aryl, in particular C₃-C₆-cycloalkyl, five- or six-membered heteroaryl or phenyl; wherein the heteroaryl contains one, two or three heteroatoms selected from N, O and S; and wherein the cycloalkyl, heteroaryl and aryl moieties are not further substituted or carry one, two, three, four, five or up to the maximum number of identical or different groups R^{5b} as defined and preferably defined below.

According to still a further embodiment, R⁵ is selected from C₃-C₆-cycloalkyl, five- or six-membered heteroaryl or aryl, in particular C₃-C₆-cycloalkyl, five- or six-membered heteroaryl or phenyl; wherein the heteroaryl contains one, two or three heteroatoms selected from N, O and S; and wherein the cycloalkyl, heteroaryl and aryl moieties are not further substituted or carry one, two, three, four, five or up to the maximum number of identical or different groups R^{5b} as defined and preferably defined below.

According to one specific embodiment, R⁵ is hydrogen.

According to one further specific embodiment, R⁵ is hydrogen or halogen, in particular H, Br, F or Cl, according to one embodiment it is H or F, according to a further embodiment, it is H or Cl.

According to one specific embodiment, R⁵ is halogen, in particular Br, F or Cl, according to one embodiment it is F, according to a further embodiment, it is Cl.

According to a further specific embodiment, R⁵ is H or OH. According to still a further specific embodiment, R⁵ is OH.

According to a further specific embodiment, R⁵ is hydrogen or C₁-C₆-alkyl, in particular hydrogen or C₁-C₄-alkyl, such as hydrogen, CH₃ or C₂H₅, more specifically hydrogen or CH₃.

According to still a further specific embodiment, R⁵ is C₁-C₆-alkyl, in particular C₁-C₄-alkyl, such as CH₃ or C₂H₅, more specifically CH₃.

According to a further specific embodiment, R⁵ is hydrogen or C₁-C₆-halogenalkyl, in particular hydrogen or C₁-C₄-halogenalkyl, such as H, CF₃, CHF₂, CH₂F, CCl₃, CHCl₂ or CH₂Cl.

According to still a further specific embodiment, R⁵ is C₁-C₆-halogenalkyl, in particular C₁-C₄-halogenalkyl, such as CF₃, CHF₂, CH₂F, CCl₃, CHCl₂ or CH₂Cl.

According to a further embodiment, R⁵ is hydrogen, C₂-C₆-alkenyl or C₂-C₆-halogenalkenyl, in particular hydrogen, C₂-C₄-alkenyl or C₂-C₄-halogenalkenyl, such as hydrogen or CH=CH₂.

According to still a further embodiment, R⁵ is C₂-C₆-alkenyl or C₂-C₆-halogenalkenyl, in particular C₂-C₄-alkenyl or C₂-C₄-halogenalkenyl, such as CH=CH₂.

According to a further embodiment, R⁵ is hydrogen, C₂-C₆-alkynyl or C₂-C₆-halogenalkynyl, in particular hydrogen, C₂-C₄-alkynyl or C₂-C₄-halogenalkynyl, such as hydrogen or C≡CH.

According to still a further embodiment, R⁵ is C₂-C₆-alkynyl or C₂-C₆-halogenalkynyl, in particular C₂-C₄-alkynyl or C₂-C₄-halogenalkynyl, such as C≡CH.

According to a further specific embodiment, R⁵ is hydrogen or C₁-C₆-alkoxy, in particular hydrogen or C₁-C₄-alkoxy, more specifically hydrogen or C₁-C₂-alkoxy such as H, OCH₃ or OCH₂CH₃.

According to still a further specific embodiment, R⁵ is C₁-C₆-alkoxy, in particular C₁-C₄-alkoxy, more specifically C₁-C₂-alkoxy such as OCH₃ or OCH₂CH₃.

According to a further specific embodiment, R⁵ is hydrogen or C₁-C₆-halogenalkoxy, in particular hydrogen or C₁-C₄-halogenalkoxy, more specifically hydrogen or C₁-C₂-halogenalkoxy such as H, OCF₃, OCHF₂, OCH₂F, OCCl₃, OCHCl₂ or OCH₂Cl, in particular H, OCF₃, OCHF₂, OCCl₃ or OCHCl₂.

According to still a further specific embodiment, R⁵ is C₁-C₆-halogenalkoxy, in particular C₁-C₄-halogenalkoxy, more specifically C₁-C₂-halogenalkoxy such as OCF₃, OCHF₂, OCH₂F, OCCl₃, OCHCl₂ or OCH₂Cl, in particular OCF₃, OCHF₂, OCCl₃ or OCHCl₂.

According to a further specific embodiment R⁵ is hydrogen or C₃-C₆-cycloalkyl, in particular hydrogen or cyclopropyl. According to still a further specific embodiment R⁵ is C₃-C₆-cycloalkyl, in particular cyclopropyl.

In still a further specific embodiment, R⁵ is hydrogen or C₃-C₆-cycloalkyl, for example cyclopropyl, substituted by one, two, three or up to the maximum possible number of identical or different groups R^{5b} as defined and preferably defined below.

In still a further specific embodiment, R⁵ is C₃-C₆-cycloalkyl, for example cyclopropyl, substituted by one, two, three or up to the maximum possible number of identical or different groups R^{5b} as defined and preferably defined below.

According to a specific embodiment R⁵ is hydrogen or C₃-C₆-halogencycloalkyl. In a special embodiment R⁵ is hydrogen or fully or partially halogenated cyclopropyl. According to a further specific embodiment R⁵ is C₃-C₆-halogencycloalkyl, in particular fully or partially halogenated cyclopropyl.

According to a further specific embodiment R⁵ is NH₂, NH(C₁-C₄-alkyl), N(C₁-C₄-alkyl)₂ or NH-SO₂-R^{x}, wherein R^{x} is C₁-C₄-alkyl, C₁-C₄-halogenalkyl, unsubstituted aryl or aryl that is substituted by one, two, three, four or five substituents R^{x1} independently selected from C₁-C₄-alkyl.

R⁶ according to the invention is hydrogen, halogen, OH, CN, NO₂, SH, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl, C₁-C₆-alkoxy, C₃-C₆-cycloalkyl, saturated or partially unsaturated three-, four-, five-, six-, seven-, eight-, nine-, or ten-membered heterocycle, five- or six-membered heteroaryl or aryl; wherein the heterocycle, heteroaryl contains one, two or three heteroatoms selected from N, O and S; and wherein R^{x} is defined above; and wherein the aliphatic moieties of R⁶ are not further substituted or carry one, two, three or up to the maximum possible number of identical or different groups R^{6a} which independently of one another are selected from:
R^{6a} halogen, OH, CN, C₁-C₆-alkoxy, C₃-C₆-cycloalkyl, C₃-C₆-halogencycloalkyl, C₁-C₄-halogenalkoxy, C₁-C₆-alkylthio and phenoxy, wherein the phenyl group is unsubstituted or carries one, two, three, four or five substituents R^{66a} selected from the group consisting of halogen, OH, C₁-C₄-alkyl, C₁-C₄-halogenalkyl, C₁-C₄-alkoxy and C₁-C₄-halogenalkoxy;
wherein the cycloalkyl, heterocyle, heteroaryl and aryl moieties of R⁶ are not further substituted or carry one, two, three, four, five or up to the maximum number of identical or different groups R^{6b} which independently of one another are selected from:
R^{6b} halogen, OH, CN, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy and C₁-C₆-alkylthio;
or R⁶ together with R⁵ and with the carbon atom to which they are bound with the carbon atom to which they are bound (marked with C** in formula I) form a saturated or partially unsaturated three-, four-, five-, six-, seven-, eight-, nine-, or ten-membered carbo- or heterocycle; wherein the heterocycle contains one, two, three or four heteroatoms selected from N, O and S, and wherein the carbocycle or heterocycle is unsubstituted or carries one, two, three or four substituents R⁵⁶ independently selected from halogen, OH, CN, NO₂, SH, NH₂, C₁-C₆-alkyl, C₁-C₆-halogenalkyl, C₁-C₆-alkoxy, C₁-C₆-halogenalkoxy, C₁-C₆-alkylthio, C₁-C₆-halogenalkylthio, C₁-C₄-alkoxy-C₁-C₄-alkyl, phenyl and phenoxy; wherein the phenyl groups are unsubstituted or carry one, two, three, four or five substituents R^{56a} selected from the group consisting of halogen, OH, C₁-C₄-alkyl, C₁-C₄-halogenalkyl, C₁-C₄-alkoxy and C₁-C₄-halogenalkoxy; and wherein in each case one or two CH₂ groups of the carbo- or heterocycle may be replaced by a group independently selected from C(=O) and C(=S).

According to one embodiment, R⁶ is selected from hydrogen, halogen, OH, CN, NO₂, SH, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl and C₁-C₆-alkoxy; or R⁶ together with R⁵ and with the carbon atom to which they are bound form a saturated three-, four-, five- or six-membered carbocycle; wherein the carbocycle is unsubstituted or substituted by R⁵⁶ as defined below; in particular selected from hydrogen, F, Cl, Br, OH, CN, NH₂, NH(C₁-C₄-alkyl), N(C₁-C₄-alkyl)₂, NH-SO₂-R^{x}, C₁-C₄-alkyl and C₁-C₄-alkoxy; or R⁶ together with R⁵ and with the carbon atom to which they are bound form a saturated three-, four-, five- or six-membered carbocycle; wherein the carbocycle is unsubstituted or substituted by R⁵⁶ as defined below; and wherein R^{x} is as defined and preferably defined below; and wherein the aliphatic moieties of R⁶ are not further substituted or carry one, two, three or up to the maximum possible number of identical or different groups R^{6a} as defined and preferably defined below.

According to one embodiment, R⁶ is selected from halogen, OH, CN, NO₂, SH, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl and C₁-C₆-alkoxy; or R⁶ together with R⁵ and with the carbon atom to which they are bound form a saturated three-, four-, five- or six-membered carbocycle; wherein the carbocycle is unsubstituted or substituted by R⁵⁶ as defined below; in particular selected from F, Cl, Br, OH, CN, NH₂, NH(C₁-C₄-alkyl), N(C₁-C₄-alkyl)₂, NH-SO₂-R^{x}, C₁-C₄-alkyl and C₁-C₄-alkoxy; or R⁶ together with R⁵ and with the carbon atom to which they are bound form a saturated three-, four-, five- or six-membered carbocycle; wherein the carbocycle is unsubstituted or substituted by R⁵⁶ as defined below; and wherein R^{x} is as defined and preferably defined below; and wherein the aliphatic moieties of R⁶ are not further substituted or carry one, two, three or up to the maximum possible number of identical or different groups R^{6a} as defined and preferably defined below.

According to still a further embodiment, R⁶ is selected from hydrogen, halogen, OH, CN, C₁-C₆-alkyl and C₁-C₆-alkoxy; or R⁶ together with R⁵ and with the carbon atom to which they are bound form a saturated three-, four-, five- or six-membered carbocycle; wherein the carbocycle is unsubstituted or substituted by R⁵⁶ as defined below; in particular selected from hydrogen, F, Cl, Br, OH, CN, C₁-C₄-alkyl and C₁-C₄-alkoxy; or R⁶ together with R⁵ and with the carbon atom to which they are bound form a saturated three-, four-, five- or six-membered carbocycle; wherein the carbocycle is unsubstituted or substituted by R⁵⁶ as defined below; and wherein the aliphatic moieties of R⁶ are not further substituted or carry one, two, three or up to the maximum possible number of identical or different groups R^{6a} as defined and preferably defined below.

According to still a further embodiment, R⁶ is selected from halogen, OH, CN, C₁-C₆-alkyl and C₁-C₆-alkoxy; or R⁶ together with R⁵ and with the carbon atom to which they are bound form a saturated three-, four-, five- or six-membered carbocycle; wherein the carbocycle is unsubstituted or substituted by R⁵⁶ as defined below; in particular selected from F, Cl, Br, OH, CN, C₁-C₄-alkyl and C₁-C₄-alkoxy; or R⁶ together with R⁵ and with the carbon atom to which they are bound form a saturated three-, four-, five- or six-membered carbocycle; wherein the carbocycle is unsubstituted or substituted by R⁵⁶ as defined below; and wherein the aliphatic moieties of R⁶ are not further substituted or carry one, two, three or up to the maximum possible number of identical or different groups R^{6a} as defined and preferably defined below.

According to a further embodiment, R⁶ is selected from hydrogen, F, Cl, Br, OH, CN, C₁-C₄-alkyl, C₁-C₄-halogenalkyl, C₁-C₄-alkoxy and C₁-C₆-halogenalkoxy; or R⁶ together with R⁵ and with the carbon atom to which they are bound form a saturated three-, four-, five- or six-membered, in particular 5- or 6-membered, carbocycle; in particular selected from hydrogen, F, Cl, OH, C₁-C₄-alkyl, C₁-C₄-halogenalkyl, C₁-C₄-alkoxy and C₁-C₆-halogenalkoxy; or R⁶ together with R⁵ and with the carbon atom to which they are bound form a saturated three-, four-, five- or six-membered carbocycle, in particular 5- or 6-membered.

According to still a further embodiment, R⁶ is selected from F, Cl, Br, OH, CN, C₁-C₄-alkyl, C₁-C₄-halogenalkyl, C₁-C₄-alkoxy and C₁-C₆-halogenalkoxy; or R⁶ together with R⁵ and with the carbon atom to which they are bound form a saturated three-, four-, five- or six-membered, in particular 5- or 6-membered, carbocycle; in particular selected from F, Cl, OH, C₁-C₄-alkyl, C₁-C₄-halogenalkyl, C₁-C₄-alkoxy and C₁-C₆-halogenalkoxy; or R⁶ together with R⁵ and with the carbon atom to which they are bound form a saturated three-, four-, five- or six-membered, in particular 5- or 6-membered, carbocycle.

According to a further embodiment, R⁶ is selected from hydrogen, NH₂, NH(C₁-C₄-alkyl), N(C₁-C₄-alkyl)₂ and NH-SO₂-R^{x}, wherein R^{x} is as defined and preferably defined below, in particular selected from hydrogen, NH₂, NH(C₁-C₂-alkyl), N(C₁-C₂-alkyl)₂ and NH-SO₂-R^{x}, wherein R^{x} is para-methyl-phenyl.

According to still a further embodiment, R⁶ is selected from NH₂, NH(C₁-C₄-alkyl), N(C₁-C₄-alkyl)₂ and NH-SO₂-R^{x}, wherein R^{x} is as defined and preferably defined below, in particular selected from NH₂, NH(C₁-C₂-alkyl), N(C₁-C₂-alkyl)₂ and NH-SO₂-R^{x}, wherein R^{x} is para-methyl-phenyl.

According to still a further embodiment, R⁶ is selected from hydrogen, C₃-C₆-cycloalkyl, five- or six-membered heteroaryl or aryl, in particular C₃-C₆-cycloalkyl, five- or six-membered heteroaryl or phenyl; wherein the heteroaryl contains one, two or three heteroatoms selected from N, O and S; and wherein the cycloalkyl, heteroaryl and aryl moieties are not further substituted or carry one, two, three, four, five or up to the maximum number of identical or different groups R^{6b} as defined and preferably defined below.

According to still a further embodiment, R⁶ is selected from C₃-C₆-cycloalkyl, five- or six-membered heteroaryl or aryl, in particular C₃-C₆-cycloalkyl, five- or six-membered heteroaryl or phenyl; wherein the heteroaryl contains one, two or three heteroatoms selected from N, O and S; and wherein the cycloalkyl, heteroaryl and aryl moieties are not further substituted or carry one, two, three, four, five or up to the maximum number of identical or different groups R^{6b} as defined and preferably defined below.

According to one specific embodiment, R⁶ is hydrogen.

According to one further specific embodiment, R⁶ is hydrogen or halogen, in particular H, Br, F or Cl, according to one embodiment it is H or F, according to a further embodiment, it is H or Cl.

According to one specific embodiment, R⁶ is halogen, in particular Br, F or Cl, according to one embodiment it is F, according to a further embodiment, it is Cl.

According to a further specific embodiment, R⁶ is H or OH. According to still a further specific embodiment, R⁶ is OH.

According to a further specific embodiment, R⁶ is hydrogen or C₁-C₆-alkyl, in particular hydrogen or C₁-C₄-alkyl, such as hydrogen, CH₃ or C₂H₆, more specifically hydrogen or CH₃.

According to still a further specific embodiment, R⁶ is C₁-C₆-alkyl, in particular C₁-C₄-alkyl, such as CH₃ or C₂H₆, more specifically CH₃.

According to a further specific embodiment, R⁶ is hydrogen or C₁-C₆-halogenalkyl, in particular hydrogen or C₁-C₄-halogenalkyl, such as H, CF₃, CHF₂, CH₂F, CCl₃, CHCl₂ or CH₂Cl.

According to still a further specific embodiment, R⁶ is C₁-C₆-halogenalkyl, in particular C₁-C₄-halogenalkyl, such as CF₃, CHF₂, CH₂F, CCl₃, CHCl₂ or CH₂Cl.

According to a further embodiment, R⁶ is hydrogen, C₂-C₆-alkenyl or C₂-C₆-halogenalkenyl, in particular hydrogen, C₂-C₄-alkenyl or C₂-C₄-halogenalkenyl, such as hydrogen or CH=CH₂. According to still a further embodiment, R⁶ is C₂-C₆-alkenyl or C₂-C₆-halogenalkenyl, in particular C₂-C₄-alkenyl or C₂-C₄-halogenalkenyl, such as CH=CH₂.

According to a further embodiment, R⁶ is hydrogen, C₂-C₆-alkynyl or C₂-C₆-halogenalkynyl, in particular hydrogen, C₂-C₄-alkynyl or C₂-C₄-halogenalkynyl, such as hydrogen or C≡CH.

According to still a further embodiment, R⁶ is C₂-C₆-alkynyl or C₂-C₆-halogenalkynyl, in particular C₂-C₄-alkynyl or C₂-C₄-halogenalkynyl, such as C≡CH.

According to a further specific embodiment, R⁶ is hydrogen or C₁-C₆-alkoxy, in particular hydrogen or C₁-C₄-alkoxy, more specifically hydrogen or C₁-C₂-alkoxy such as H, OCH₃ or OCH₂CH₃.

According to still a further specific embodiment, R⁶ is C₁-C₆-alkoxy, in particular C₁-C₄-alkoxy, more specifically C₁-C₂-alkoxy such as OCH₃ or OCH₂CH₃.

According to a further specific embodiment, R⁶ is hydrogen or C₁-C₆-halogenalkoxy, in particular hydrogen or C₁-C₄-halogenalkoxy, more specifically hydrogen or C₁-C₂-halogenalkoxy such as H, OCF₃, OCHF₂, OCH₂F, OCCl₃, OCHCl₂ or OCH₂Cl, in particular H, OCF₃, OCHF₂, OCCl₃ or OCHCl₂.

According to still a further specific embodiment, R⁶ is C₁-C₆-halogenalkoxy, in particular C₁-C₄-halogenalkoxy, more specifically C₁-C₂-halogenalkoxy such as OCF₃, OCHF₂, OCH₂F, OCCl₃, OCHCl₂ or OCH₂Cl, in particular OCF₃, OCHF₂, OCCl₃ or OCHCl₂.

According to a further specific embodiment R⁶ is hydrogen or C₃-C₆-cycloalkyl, in particular hydrogen or cyclopropyl. According to still a further specific embodiment R⁶ is C₃-C₆-cycloalkyl, in particular cyclopropyl.

In still a further specific embodiment, R⁶ is hydrogen or C₃-C₆-cycloalkyl, for example cyclopropyl, substituted by one, two, three or up to the maximum possible number of identical or different groups R^{6b} as defined and preferably defined below.

In still a further specific embodiment, R⁶ is C₃-C₆-cycloalkyl, for example cyclopropyl, substituted by one, two, three or up to the maximum possible number of identical or different groups R^{6b} as defined and preferably defined below.

According to a specific embodiment R⁶ is hydrogen or C₃-C₆-halogencycloalkyl. In a special embodiment R⁶ is hydrogen or fully or partially halogenated cyclopropyl. According to a further specific embodiment R⁶ is C₃-C₆-halogencycloalkyl, in particular fully or partially halogenated cyclopropyl.

According to a further specific embodiment R⁶ is NH₂, NH(C₁-C₄-alkyl), N(C₁-C₄-alkyl)₂ or NH-SO₂-R^{x}, wherein R^{x} is C₁-C₄-alkyl, C₁-C₄-halogenalkyl, unsubstituted aryl or aryl that is substituted by one, two, three, four or five substituents R^{x1} independently selected from C₁-C₄-alkyl.

According to a further specific embodiment R⁵ together with R⁶ and with the carbon atom to which they are bound form a saturated or partially unsaturated three-, four-, five-, six-, seven-, eight-, nine-, or ten-membered carbo- or heterocycle wherein the heterocycle contains one, two, three or four heteroatoms selected from N, O and S, and wherein the carbocycle or heterocycle is unsubstituted or carries one, two, three or four substituents R⁵⁶ as defined below; and wherein in each case one or two CH₂ groups of the carbo- or heterocycle may be replaced by a group independently selected from C(=O) and C(=S).

According to one further embodiment R⁵ together with R⁶ and with the carbon atom to which they are bound form a saturated three-, four-, five-, six-, seven-, eight-, nine-, or ten-membered, in particular five- or six-membered carbocycle, that is unsubstituted or carries one, two, three or four substituents R⁵⁶ as defined below. According to one embodiment thereof, R⁵ and R⁶ form a cyclopentyl, that is unsubstituted or carries one, two, three or four substituents R⁵⁶ as defined below. According to one embodiment thereof, R⁵ and R⁶ form a cyclohexyl, that is unsubstituted or carries one, two, three or four substituents R⁵⁶ as defined below.

According to one further embodiment R⁵ together with R⁶ and with the carbon atom to which they are bound form a saturated three-, four-, five-, six-, seven-, eight-, nine-, or ten-membered, in particular three-, four-, five- or six-membered carbocycle, more specifically five- or six-membered carbocycle, that is unsubstituted or carries one, two, three or four substituents R⁵⁶ as defined below. According to one embodiment thereof, R⁵ and R⁶ form a cyclopropyl, that is unsubstituted or carries one, two, three or four substituents R⁵⁶ as defined below. According to a further embodiment thereof, R⁵ and R⁶ form a cyclobutyl, that is unsubstituted or carries one, two, three or four substituents R⁵⁶ as defined below. According to still a further embodiment thereof, R⁵ and R⁶ form a cyclopentyl, that is unsubstituted or carries one, two, three or four substituents R⁵⁶ as defined below. According to still a further embodiment thereof, R⁵ and R⁶ form a cyclohexyl, that is unsubstituted or carries one, two, three or four substituents R⁵⁶ as defined below.

According to still one further embodiment R⁵ together with R⁶ and with the carbon atom to which they are bound form a saturated or partially unsaturated three-, four-, five-, six-, seven-, eight-, nine-, or ten-membered, in particular five- or six-membered, heterocycle, wherein the heterocycle contains one, two, three or four, in particular one or two, heteroatoms selected from N, O and S, and wherein the heterocycle is unsubstituted or carries one, two, three or four substituents R⁵⁶ as defined below.

According to still one further embodiment R⁵ together with R⁶ and with the carbon atom to which they are bound form a saturated or partially unsaturated three-, four-, five-, six-, seven-, eight-, nine-, or ten-membered, in particular five- or six-membered, heterocycle, wherein the heterocycle contains one, two, three or four, in particular one or two, heteroatoms selected from N, O and S, and wherein the heterocycle is unsubstituted or carries one, two, three or four substituents R⁵⁶ as defined below.

In one particular embodiment, R⁵ and R⁶ are independently selected from hydrogen, halogen and C₁-C₆-alkyl, in particular selected from H, F, Cl, Br and CH₃, more specifically selected from H, F and CH₃ In a further embodiment, R⁵ and R⁶ are independently selected from halogen and C₁-C₆-alkyl, in particular selected from H, F, Cl, Br and CH₃, more specifically selected from F and CH₃.

In still a further embodiment, R⁵ and R⁶ are both hydrogen.

In still a further embodiment, R⁵ is hydrogen and R⁶ is not hydrogen and selected from the substituents as defined above, in particular selected from halogen, OH, C₁-C₆-alkyl and C₁-C₆-alkoxy, in particular selected from F, Cl, Br, OH, CH₃, C₂H₅, OCH₃ and OC₂H₅, more specifically selected from F, Cl, Br and CH₃.

In still a further embodiment, R⁵ and R⁶ are both different from hydrogen and independently selected from the substituents as defined above, in particular selected from halogen, OH, C₁-C₆-alkyl and C₁-C₆-alkoxy, in particular selected from F, Cl, Br, OH, CH₃, C₂H₅, OCH₃ and OC₂H₅, more specifically selected from F, Cl, Br and CH₃.

In a further particular embodiment, R⁵ and R⁶ are independently selected from hydrogen, halogen and C₁-C₆-alkyl, or R⁵ and R⁶ together with the carbon atom to which they are bound form a saturated three-, four-, five-, six-, seven-, eight-, nine-, or ten-membered, in particular three, four-, five- or six-membered carbocycle, that is unsubstituted or carries one, two, three or four substituents R⁵⁶ as defined below. In particular R⁵ and R⁶ are independently selected from H, F, Cl, Br and CH₃, or together with the carbon atom to which they are bound form a saturated three-, four-, five- or six-membered carbocycle, that is unsubstituted or carries one, two, three or four substituents R⁵⁶ as defined below.

In a further particular embodiment, R⁵ and R⁶ are independently selected from halogen and C₁-C₆-alkyl, or R⁵ and R⁶ together with the carbon atom to which they are bound form a saturated three-, four-, five-, six-, seven-, eight-, nine-, or ten-membered, in particular three, four-, five- or six-membered carbocycle, that is unsubstituted or carries one, two, three or four substituents R⁵⁶ as defined below. In particular R⁵ and R⁶ are independently selected from F, Cl, Br and CH₃, or together with the carbon atom to which they are bound form a saturated three-, four-, five- or six-membered carbocycle, that is unsubstituted or carries one, two, three or four substituents R⁵⁶ as defined below.

R^{x} in the substituent NH-SO₂-R^{x} is in each case independently selected from C₁-C₄-alkyl, C₁-C₄-halogenalkyl, unsubstituted aryl and aryl that is substituted by one, two, three, four or five substituents R^{x1} independently selected from C₁-C₄-alkyl. In particular, R^{x} is in each case independently selected from C₁-C₄-alkyl and phenyl that is substituted by one, two or three R^{x1} independently selected from C₁-C₂-alkyl, more specifically R^{x} is in each case independently selected from C₁-C₄-alkyl and phenyl that is substituted by one CH₃., more specifically SO₂-R^{x} is the tosyl group ("Ts").

R^{5a} are the possible substituents of the aliphatic moieties of R⁵. There may be one, two, three or up to the maximum possible number of identical or different groups R^{5a} present, specifically, there are one, two, three or four, if at all. The R^{5a} are independently of one another selected from halogen, OH, CN, C₁-C₆-alkoxy, C₃-C₆-cycloalkyl, C₃-C₆-halogencycloalkyl, C₁-C₄-halogenalkoxy, C₁-C₆-alkylthio and phenoxy, wherein the phenyl group is unsubstituted or carries one, two, three, four or five, more specifically one, two or three, substituents R^{55a} selected from the group consisting of halogen, OH, C₁-C₄-alkyl, C₁-C₄-halogenalkyl, C₁-C₄-alkoxy and C₁-C₄-halogenalkoxy, in particular selected from F, Cl, OH, CH₃, halomethyl, cyclopropyl, halogencyclopropyl, OCH₃ and halogenmethoxy. In one embodiment, the R^{55a} are independently selected from halogen, in particular F, Cl and Br, more specifically selected from F and Cl.

According to one embodiment, R^{5a} are independently selected from halogen, OH, C₁-C₂-alkoxy, C₁-C₂-halogenalkoxy, C₃-C₆-cycloalkyl, C₃-C₆-halogencycloalkyl, in particular selected from F, Cl, OH, cyclopropyl, halogencyclopropyl, OCH₃ and halogenmethoxy.

According to one particular embodiment, R^{5a} are independently selected from halogen, in particular F, Cl and Br, more specifically F and Cl.

R^{5b} are the possible substituents of the cycloalkyl, heteroaryl and aryl moieties of R⁵. There may be one, two, three, four, five or up to the maximum number of identical or different groups R^{5b}, more specifically one, two or three, if at all. The R^{5b} are independently of one another selected from halogen, OH, CN, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy and C₁-C₆-alkylthio.

According to one embodiment, R^{5b} are independently selected from halogen, OH, CN, C₁-C₂-alkyl, C₁-C₂-alkoxy, C₃-C₆-cycloalkyl, C₃-C₆-halogencycloalkyl, C₁-C₂-halogenalkoxy and C₁-C₂-alkylthio, in particular selected from F, Cl, CH₃, halogenmethyl, cyclopropyl, halogencyclopropyl, OCH₃ and halogenmethoxy.

According to one particular embodiment, R^{5b} are independently selected from halogen and C₁-C₂-alkyl, in particular from F, Cl and CH₃. Specifically, R^{5b} are selected from halogen.

R^{6a} are the possible substituents of the aliphatic moieties of R⁶. There may be one, two, three or up to the maximum possible number of identical or different groups R^{6a} present, specifically, there are one, two, three or four, if at all. The R^{6a} are independently of one another selected from halogen, OH, CN, C₁-C₆-alkoxy, C₃-C₆-cycloalkyl, C₃-C₆-halogencycloalkyl, C₁-C₄-halogenalkoxy, C₁-C₆-alkylthio and phenoxy, wherein the phenyl group is unsubstituted or carries one, two, three, four or five, more specifically one, two or three, substituents R^{66a} selected from the group consisting of halogen, OH, C₁-C₄-alkyl, C₁-C₄-halogenalkyl, C₁-C₄-alkoxy and C₁-C₄-halogenalkoxy, in particular selected from F, Cl, OH, CH₃, halomethyl, cyclopropyl, halogencyclopropyl, OCH₃ and halogenmethoxy. In one embodiment, the R^{66a} are independently selected from halogen, in particular F, Cl and Br, more specifically selected from F and Cl.

According to one embodiment, R^{6a} are independently selected from halogen, OH, C₁-C₂-alkoxy, C₁-C₂-halogenalkoxy, C₃-C₆-cycloalkyl, C₃-C₆-halogencycloalkyl, in particular selected from F, Cl, OH, cyclopropyl, halogencyclopropyl, OCH₃ and halogenmethoxy.

According to one particular embodiment, R^{6a} are independently selected from halogen, in particular F, Cl and Br, more specifically F and Cl.

R^{6b} are the possible substituents of the cycloalkyl, heteroaryl and aryl moieties of R⁶. There may be one, two, three, four, five or up to the maximum number of identical or different groups R^{6b}, more specifically one, two or three, if at all. The R^{6b} are independently of one another selected from halogen, OH, CN, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy and C₁-C₆-alkylthio.

According to one embodiment, R^{6b} are independently selected from halogen, OH, CN, C₁-C₂-alkyl, C₁-C₂-alkoxy, C₃-C₆-cycloalkyl, C₃-C₆-halogencycloalkyl, C₁-C₂-halogenalkoxy and C₁-C₂-alkylthio, in particular selected from F, Cl, CH₃, halogenmethyl, cyclopropyl, halogencyclopropyl, OCH₃ and halogenmethoxy.

According to one particular embodiment, R^{6b} are independently selected from halogen and C₁-C₂-alkyl, in particular from F, Cl and CH₃. Specifically, R^{6b} are selected from halogen.

R⁵⁶ are the possible substituents of the carbo- or heterocycle fromed by R⁵ and R⁶. There may be one, two, three or four substituents R⁵⁶ present, if at all. R⁵⁶ are independently selected from halogen, OH, CN, NO₂, SH, NH₂, C₁-C₆-alkyl, C₁-C₆-halogenalkyl, C₁-C₆-alkoxy, C₁-C₆-halogenalkoxy, C₁-C₆-alkylthio, C₁-C₆-halogenalkylthio, C₁-C₄-alkoxy-C₁-C₄-alkyl, phenyl and phenoxy; wherein the phenyl groups are unsubstituted or carry one, two, three, four or five, in particular one, two or three, substituents R^{56a} selected from the group consisting of halogen, OH, C₁-C₄-alkyl, C₁-C₄-halogenalkyl, C₁-C₄-alkoxy and C₁-C₄-halogenalkoxy.

In one embodiment, R⁵⁶ are independently selected from halogen, OH, CN, C₁-C₄-alkyl, C₁-C₄-halogenalkyl, C₁-C₄-alkoxy, C₁-C₄-halogenalkoxy, C₁-C₄-alkylthio, phenyl and phenoxy; wherein the phenyl groups are unsubstituted or carry one, two or three substituents R^{56a} selected from the group consisting of halogen, OH, CH₃, halogenmethyl, OCH₃ and halogenmethoxy.

In a further embodiment, R⁵⁶ are independently selected from halogen, OH, CN, C₁-C₂-alkyl, C₁-C₂-halogenalkyl, C₁-C₂-alkoxy and C₁-C₂-halogenalkoxy, in particular selected from F, Cl, OH, CH₃, halogenmethyl, OCH₃ and halogenmethoxy.

In one particular embodiment, R⁵⁶ are independently selected from halogen and C₁-C₂-alkyl, in particular from F, Cl and CH₃. Specifically, R⁵⁶ are selected from halogen, such as F and Cl.

Particularly preferred embodiments of the combination of R⁵ and R⁶ according to the invention are in Table P56 below, wherein each line of lines P56-1 to P56-125 corresponds to one particular embodiment of the invention, wherein P56-1 to P56-125 are also in any combination with one another a preferred embodiment of the present invention.

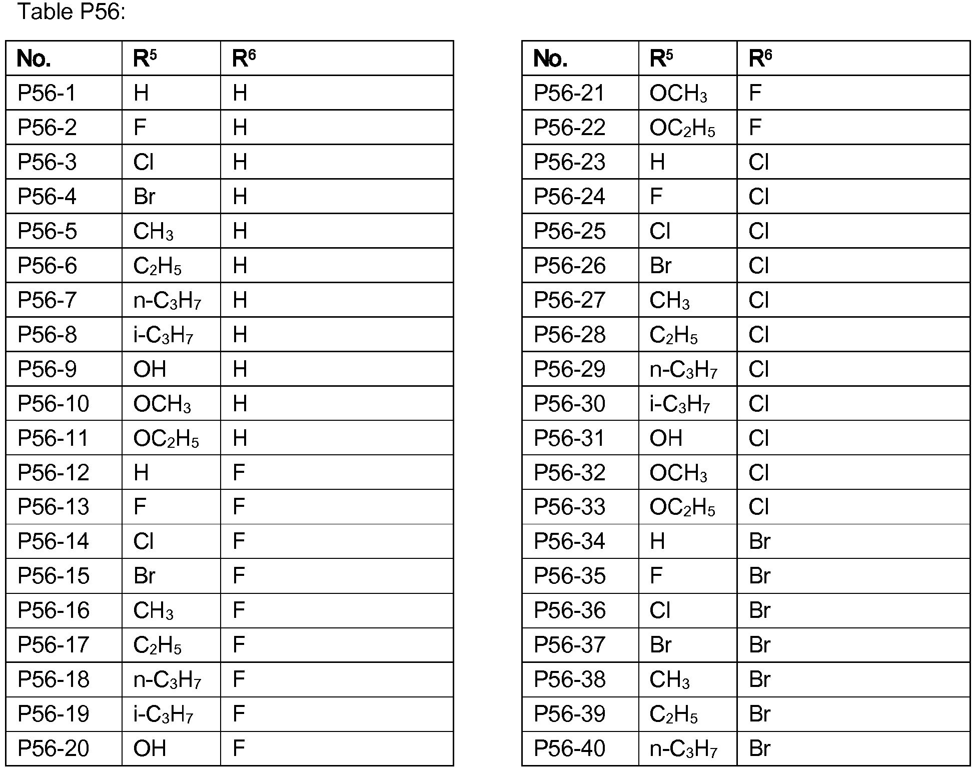

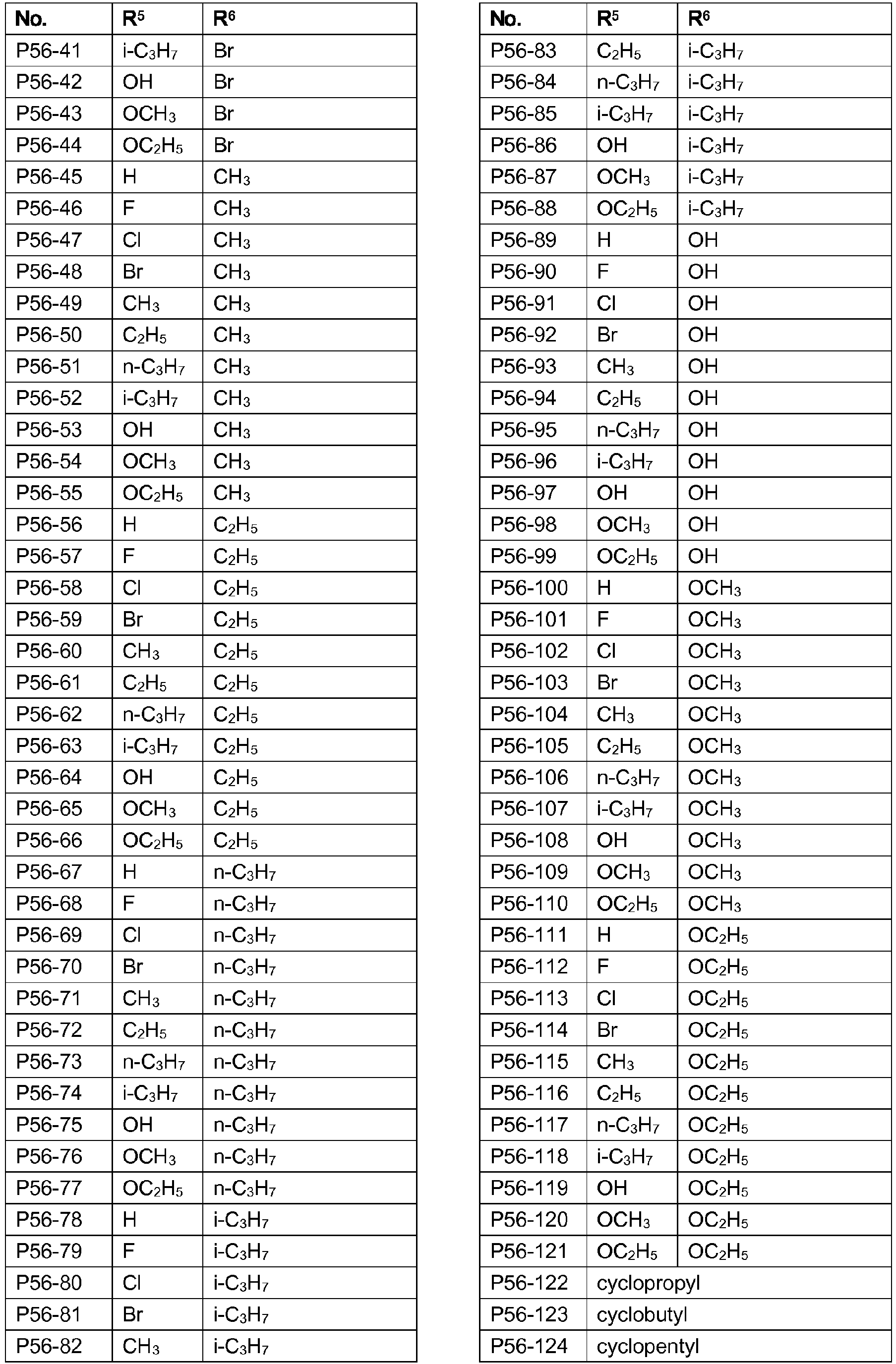

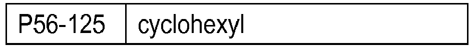

R⁷ and R⁸ together with the carbon atoms to which they are bound together with the carbon atoms to which they are bound form a five- or six-membered heteroaryl; wherein the heteroaryl contains one, two or three heteroatoms selected from N, O and S, and wherein the heteroaryl carries zero, one, two, three or four substituents (R⁷⁸)ₒ, wherein o is 0, 1, 2 or 3; and

R⁷⁸ are independently selected from are independently selected from halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, NH-SO₂-R^{x}, CH(=O), C(=O)C₁-C₆-alkyl, C(=O)NH(C₁-C₆-alkyl), CR'=NOR", C₁-C₆-alkyl, C₂-C₆-alkenyl, C₂-C₆-alkynyl, C₁-C₆-alkoxy, C₁-C₆-halogenalkoxy, C₂-C₆-alkenyloxy, C₂-C₆-alkynyloxy, C₃-C₆-cycloalkyl, C₃-C₆-cycloalkenyl, three-, four-, five- or six-membered saturated or partially unsaturated heterocycle, five- or six-membered heteroaryl and phenyl; wherein the heterocycle or heteroaryl contains one, two or three heteroatoms selected from N, O and S; wherein R' and R" are independently selected from C₁-C₄-alkyl, C₂-C₆-alkenyl, C₂-C₆-alkynyl, C₃-C₆-cycloalkyl, saturated or partially unsaturated three-, four-, five-, six-, seven-, eight-, nine-, or ten-membered heterocycle, five- or six-membered heteroaryl or aryl; wherein the heterocycle or heteroaryl contains one, two or three heteroatoms selected from N, O and S, and wherein R' and/or R" are independently unsubstituted or carry one, two or three R'" independently selected from halogen, OH, CN, NO₂, SH, NH₂, NH(C₁-C₄-alkyl), N(C₁-C₄-alkyl)₂, NH-SO₂-R^{x}, C₁-C₆-alkyl, C₁-C₆-halogenalkyl, C₂-C₆-alkenyl, C₂-C₆-halogenalkenyl, C₂-C₆-alkynyl, C₂-C₆-halogenalkynyl, C₁-C₆-alkoxy, C₁-C₆-halogenalkoxy, C₃-C₆-cycloalkyl, C₃-C₆-halogencycloalkyl and phenyl; and wherein R^{x} is defined above; and wherein the aliphatic moieties of R⁷⁸ are not further substituted or carry one, two, three or up to the maximum possible number of identical or different groups R^{78a} which independently of one another are selected from:
R^{78a} halogen, OH, CN, C₁-C₆-alkoxy, C₃-C₆-cycloalkyl, C₃-C₆-cycloalkenyl, C₃-C₆-halogencycloalkyl, C₃-C₆-halogencycloalkenyl, C₁-C₄-halogenalkoxy, C₁-C₆-alkylthio, five- or six-membered heteroaryl, phenyl and phenoxy, wherein the heterorayl, phenyl and phenoxy group is unsubstituted or carries one, two, three, four or five substituents R^{78a'} selected from the group consisting of halogen, OH, C₁-C₄-alkyl, C₁-C₄-halogenalkyl, C₁-C₄-alkoxy and C₁-C₄-halogenalkoxy;
wherein the alicyclic, phenyl, heterocyclic and heteroaryl moieties of R⁷⁸ are not further substituted or carry one, two, three, four, five or up to the maximum number of identical or different groups R^{78b} which independently of one another are selected from:
   R^{78b} halogen, OH, CN, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy and C₁-C₆-alkylthio.

According to one embodiment, R⁷ and R⁸ together with the carbon atoms to which they are bound form a five- or six-membered heteroaryl; wherein the heteroaryl contains one or two heteroatoms selected from N, O and S, and wherein the heteroaryl carries zero, one or two substituents (R⁷⁸)ₒ, as defined and preferably defined herein, wherein o is 0, 1 or 2. According to one specific embodiment, o is 0. According to a further embodiment, o is 1 or 2. Particular embodiments thereof are listed in Table P78.

According to a further embodiment, R⁷ and R⁸ together with the carbon atoms to which they are bound form a five- or six-membered heteroaryl; wherein the heteroaryl contains one or two heteroatoms N, and wherein the heteroaryl carries zero, one or two substituents (R⁷⁸)ₒ, as defined and preferably defined herein, wherein o is 0, 1 or 2. According to one specific embodiment, o is 0. According to a further embodiment, o is 1 or 2. Particular embodiments thereof are listed in Table P78.

According to a further embodiment, R⁷ and R⁸ together with the carbon atoms to which they are bound form a five- or six-membered heteroaryl; wherein the heteroaryl contains one or two heteroatoms selected from S and O, and wherein the heteroaryl carries zero, one or two substituents (R⁷⁸)ₒ, as defined and preferably defined herein, wherein o is 0, 1 or 2. According to one specific embodiment, o is 0. According to a further embodiment, o is 1 or 2. Particular embodiments thereof are listed in Table P78.

According to a further embodiment, R⁷ and R⁸ together with the carbon atoms to which they are bound form a five- or six-membered heteroaryl; wherein the heteroaryl contains one heteroatom S, and wherein the heteroaryl carries zero, one or two substituents (R⁷⁸)ₒ, as defined and preferably defined herein, wherein o is 0, 1 or 2. According to one specific embodiment, o is 0. According to a further embodiment, o is 1 or 2. Particular embodiments thereof are listed in Table P78.

According to a further embodiment, R⁷ and R⁸ together with the carbon atoms to which they are bound form a five- or six-membered heteroaryl; wherein the heteroaryl contains one heteroatom O, and wherein the heteroaryl carries zero, one or two substituents (R⁷⁸)ₒ, as defined and preferably defined herein, wherein o is 0, 1 or 2. According to one specific embodiment, o is 0. According to a further embodiment, o is 1 or 2. Particular embodiments thereof are listed in Table P78.

According to one embodiment, R⁷ and R⁸ together with the carbon atoms to which they are bound form a five-membered heteroaryl; wherein the heteroaryl contains one or two heteroatoms selected from N, O and S, and wherein the heteroaryl carries zero, one or two substituents (R⁷⁸)ₒ, as defined and preferably defined herein, wherein o is 0, 1 or 2. According to one specific embodiment, o is 0. According to a further embodiment, o is 1 or 2. Particular embodiments thereof are listed in Table P78.

According to one embodiment, R⁷ and R⁸ together with the carbon atoms to which they are bound form a five-membered heteroaryl; wherein the heteroaryl contains one or two heteroatoms N, and wherein the heteroaryl carries zero, one or two substituents (R⁷⁸)ₒ, as defined and preferably defined herein, wherein o is 0, 1 or 2. According to one specific embodiment, o is 0. According to a further embodiment, o is 1 or 2. Particular embodiments thereof are listed in Table P78.

According to one embodiment, R⁷ and R⁸ together with the carbon atoms to which they are bound form a five-membered heteroaryl; wherein the heteroaryl contains one or two heteroatoms selected from O and S, and wherein the heteroaryl carries zero, one or two substituents (R⁷⁸)ₒ, as defined and preferably defined herein, wherein o is 0, 1 or 2. According to one specific embodiment, o is 0. According to a further embodiment, o is 1 or 2. Particular embodiments thereof are listed in Table P78.

According to one embodiment, R⁷ and R⁸ together with the carbon atoms to which they are bound form a five-membered heteroaryl; wherein the heteroaryl contains one heteroatom S, and wherein the heteroaryl carries zero, one or two substituents (R⁷⁸)ₒ, as defined and preferably defined herein, wherein o is 0, 1 or 2. According to one specific embodiment, o is 0. According to a further embodiment, o is 1 or 2.

According to one embodiment, R⁷ and R⁸ together with the carbon atoms to which they are bound form a five-membered heteroaryl; wherein the heteroaryl contains one heteroatom O, and wherein the heteroaryl carries zero, one or two substituents (R⁷⁸)ₒ, as defined and preferably defined herein, wherein o is 0, 1 or 2. According to one specific embodiment, o is 0. According to a further embodiment, o is 1 or 2. Particular embodiments thereof are listed in Table P78.

According to a further embodiment, R⁷ and R⁸ together with the carbon atoms to which they are bound form a six-membered heteroaryl; wherein the heteroaryl contains one or two heteroatoms selected from N, O and S, and wherein the heteroaryl carries zero, one or two substituents (R⁷⁸)ₒ, as defined and preferably defined herein, wherein o is 0, 1 or 2. According to one specific embodiment, o is 0. According to a further embodiment, o is 1 or 2. Particular embodiments thereof are listed in Table P78.

According to a further embodiment, R⁷ and R⁸ together with the carbon atoms to which they are bound form a six-membered heteroaryl; wherein the heteroaryl contains one or two heteroatoms N, and wherein the heteroaryl carries zero, one or two substituents (R⁷⁸)ₒ, as defined and preferably defined herein, wherein o is 0, 1 or 2. According to one specific embodiment, o is 0. According to a further embodiment, o is 1 or 2. Particular embodiments thereof are listed in Table P78.

According to the invention, there can be zero, one, two or three R⁷⁸ present, namely for o is 0, 1, 2 or 3.

According to one embodiment, o is 0.

According to a further embodiment, o is 1.

According to a further embodiment, o is 2 or 3. According to one specific embodiment thereof, o is 2, according to a further specific embodiment, o is 3.

For every R⁷⁸ that is present in the inventive compounds, the following embodiments and preferences apply independently of the meaning of any other R⁷⁸ that may be present in the ring. Furthermore, the particular embodiments and preferences given herein for R⁷⁸ apply independently for each of o=1, o=2 and o=3.

According to one specific embodiment, R⁷⁸ is halogen, in particular F, Cl, Br or I, more specifically F, Cl or Br, in particular F or Cl.

According to a further specific embodiment, R⁷⁸ is OH.

According to a further specific embodiment, R⁷⁸ is CN.

According to a further specific embodiment, R⁷⁸ is C₁-C₆-alkyl, in particular C₁-C₄-alkyl, such as CH₃. or C₂H₅, in particular CH₃.

According to a further specific embodiment, R⁷⁸ is C₁-C₆-halogenalkyl, in particular C₁-C₄-halogenalkyl, such as CF₃, CHF₂, CH₂F, CCl₃, CHCl₂ and CH₂Cl.

According to still a further embodiment, R⁷⁸ is C₂-C₆-alkenyl, in particular C₂-C₄-alkenyl, such as CH=CH₂.

According to still a further embodiment, R⁷⁸ is C₃-C₆-cycloalkyl-C₂-C₆-alkenyl, in particular C₃-C₆-cycloalkyl-C₂-C₄-alkenyl, more specifically C₃-C₆-cycloalkyl-C₂-C₃-alkenyl, such as C₃H₅-CH=CH₂.

According to a further specific embodiment, R⁷⁸ is C₂-C₆-halogenalkenyl, in particular C₂-C₄-halogenalkenyl, more specifically C₂-C₃-halogenalkenyl.

According to still a further embodiment, R⁷⁸ is C₂-C₆-alkynyl, in particular C₂-C₄-alkynyl, more specifically C₂-C₃-alkynyl, such as C=CH.

According to still a further embodiment, R⁷⁸ is C₂-C₆-halogenalkynyl, in particular C₂-C₄-halogenalkynyl, more specifically C₂-C₃-halogenalkynyl.

According to a further specific embodiment, R⁷⁸ is C₁-C₆-alkoxy, in particular C₁-C₄-alkoxy, more specifically C₁-C₂-alkoxy such as OCH₃ or OCH₂CH₃.

According to a further specific embodiment, R⁷⁸ is C₁-C₆-halogenalkoxy, in particular C₁-C₄-halogenalkoxy, more specifically C₁-C₂-halogenalkoxy such as OCF₃, OCHF₂, OCH₂F, OCCl₃, OCHCl₂, OCH₂Cl and OCF₂CHF₂, in particular OCF₃, OCHF₂ and OCF₂CHF₂.

According to still a further specific embodiment, R⁷⁸ is unsubstituted phenyl or phenyl that is substituted by one, two, three or four R^{78b}, as defined and preferably herein. In particular, R⁷⁸ is unsubstituted phenyl or phenyl that is substituted by one, two, three or four R^{78b}, as defined herein. In one embodiment R⁷⁸ is unsubstituted phenyl.

According to one further embodiment, R⁷⁸ is in each case independently selected from halogen, CN, C₁-C₆-alkyl, C₂-C₆-alkenyl, C₂-C₆-alkynyl, C₁-C₆-alkoxy, C₃-C₆-alkenyloxy, C₃-C₆-alkynyloxy, C₃-C₆-cycloalkyl, three-, four-, five- or six-membered saturated or partially unsaturated heterocycle, five- or six-membered heteroaryl and phenyl; wherein the heterocycle or heteroaryl contains one, two or three heteroatoms selected from N, O and S; and wherein the aliphatic moieties of R⁷⁸ are not further substituted or carry one, two, three or up to the maximum possible number of identical or different groups R^{78a} as defined and preferably defined herein, and wherein the heterocyclic, alicyclic, phenyl and heteroaryl moieties of R⁷⁸ are not further substituted or carry one, two, three, four, five or up to the maximum number of identical or different groups R^{78b} as defined and preferably defined herein.

According to one further embodiment, R⁷⁸ is in each case independently selected from halogen, CN, C₁-C₆-alkyl, C₂-C₆-alkenyl, C₂-C₆-alkynyl, C₁-C₆-alkoxy, C₁-C₆-halogenalkoxy, C₃-C₆-alkenyloxy, C₃-C₆-alkynyloxy, C₃-C₆-cycloalkyl, three-, four-, five- or six-membered saturated or partially unsaturated heterocycle, five- or six-membered heteroaryl and phenyl; wherein the heterocycle or heteroaryl contains one, two or three heteroatoms selected from N, O and S; and wherein the aliphatic moieties of R⁷⁸ are not further substituted or carry one, two, three or up to the maximum possible number of identical or different groups R^{78a} as defined and preferably defined herein, and wherein the heterocyclic, alicyclic, phenyl and heteroaryl moieties of R⁷⁸ are not further substituted or carry one, two, three, four, five or up to the maximum number of identical or different groups R^{78b} as defined and preferably defined herein. Accordingto one specific embodiment, the aliphatic and cyclic moieties of R⁷⁸ are not further substituted, according to another embodiment, the aliphatic moieties of R⁷⁸ carry one, two, three or four identical or different groups R^{78a} as defined and preferably defined herein.

According to a further embodiment, R⁷⁸ is in each case independently selected from halogen, CN, C₁-C₆-alkyl, C₂-C₆-alkenyl, C₂-C₆-alkynyl, C₁-C₆-alkoxy, C₃-C₆-alkenyloxy, C₃-C₆-alkynyloxy and C₃-C₆-cycloalkyl, wherein the aliphatic moieties of R⁷⁸ are not further substituted or carry one, two, three or up to the maximum possible number of identical or different groups R^{78a} as defined and preferably defined herein, and wherein the cycloalkyl moieties of R⁷⁸ are not further substituted or carry one, two, three, four, five or up to the maximum number of identical or different groups R^{78b} as defined and preferably defined herein.

According to a further embodiment, R⁷⁸ is in each case independently selected from halogen, CN, C₁-C₆-alkyl, C₂-C₆-alkenyl, C₂-C₆-alkynyl, C₁-C₆-alkoxy, C₁-C₆-halogenalkoxy, C₃-C₆-alkenyloxy, C₃-C₆-alkynyloxy and C₃-C₆-cycloalkyl, wherein the aliphatic moieties of R⁷⁸ are not further substituted or carry one, two, three or up to the maximum possible number of identical or different groups R^{78a} as defined and preferably defined herein, and wherein the cycloalkyl moieties of R⁷⁸ are not further substituted or carry one, two, three, four, five or up to the maximum number of identical or different groups R^{78b} as defined and preferably defined herein. Accordingto one specific embodiment, the aliphatic and cyclic moieties of R⁷⁸ are not further substituted, according to another embodiment, the aliphatic moieties of R⁷⁸ carry one, two, three or four identical or different groups R^{78a} as defined and preferably defined herein.

According to still a further embodiment, R⁷⁸ is in each case independently selected from halogen, C₁-C₆-alkyl and C₁-C₆-alkoxy, wherein the aliphatic moieties of R⁷⁸ are not further substituted or carry one, two, three or up to the maximum possible number of identical or different groups R^{78a} defined and preferably defined herein.

According to still a further embodiment, R⁷⁸ is in each case independently selected from halogen, C₁-C₆-alkyl, C₁-C₆-alkoxy and C₁-C₆-halogenalkoxy, wherein the aliphatic moieties of R⁷⁸ are not further substituted or carry one, two, three or up to the maximum possible number of identical or different groups R^{78a} defined and preferably defined herein. Accordingto one specific embodiment, the aliphatic and cyclic moieties of R⁷⁸ are not further substituted, according to another embodiment, the aliphatic moieties of R⁷⁸ carry one, two, three or four identical or different groups R^{78a} as defined and preferably defined herein.

R^{78a} are the possible substituents for the aliphatic moieties of R⁷⁸. R^{78a} is independently selected from halogen, OH, CN, C₁-C₆-alkoxy, C₃-C₆-cycloalkyl, C₃-C₆-cycloalkenyl, C₃-C₆-halogencycloalkyl, C₃-C₆-halogencycloalkenyl, C₁-C₄-halogenalkoxy, C₁-C₆-alkylthio, five- or six-membered heteroaryl, phenyl and phenoxy, wherein the heterorayl, phenyl and phenoxy group is unsubstituted or carries one, two, three, four or five substituents R^{78a'} selected from the group consisting of halogen, OH, C₁-C₄-alkyl, C₁-C₄-halogenalkyl, C₁-C₄-alkoxy and C₁-C₄-halogenalkoxy.

According to one embodiment R^{78a} is independently selected from halogen, C₁-C₆-alkoxy, C₃-C₆-cycloalkyl, C₃-C₆-halogencycloalkyl and C₁-C₄-halogenalkoxy. Specifically, R^{78a} is independently selected from F, Cl, Br, I, C₁-C₂-alkoxy, cyclopropyl, 1-F-cyclopropyl, 1-Cl-cyclopropyl and C₁-C₂-halogenalkoxy.

According to a further embodiment, R^{78a} is independently halogen, in particular selected from F, Cl, Br and I, more specifically F, Cl and Br.

R^{78b} are the possible substituents for the cycloalkyl, heterocyclyl, heteroaryl and phenyl moieties of R⁷⁸. R^{78b} according to the invention is independently selected from halogen, OH, CN, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy and C₁-C₆-alkylthio.

According to one embodiment thereof R^{78b} is independently selected from halogen, CN, C₁-C₄-alkyl, C₁-C₄-alkoxy, C₁-C₄-halogenalkyl and C₁-C₄-halogenalkoxy, in particular halogen, C₁-C₄-alkyl and C₁-C₄-alkoxy. Specifically, R^{78b} is independently selected from F, Cl, CN, CH₃, OCH₃ and halogenmethoxy.

Particularly preferred embodiments of R⁷ and R⁸, optionally substituted by (R⁷⁸)ₒ, according to the invention are in Table P78 below, wherein each line of lines P78-1 to P78-40 corresponds to one particular embodiment of the invention, wherein P78-1 to P78-40 are also in any combination with one another a preferred embodiment of the present invention. Thereby, the positions of the heteroaryls marked with "#" represents the connection points (carbon atoms 5' and 6' in formula I) with the remaining skeleton of the compounds of formula I:

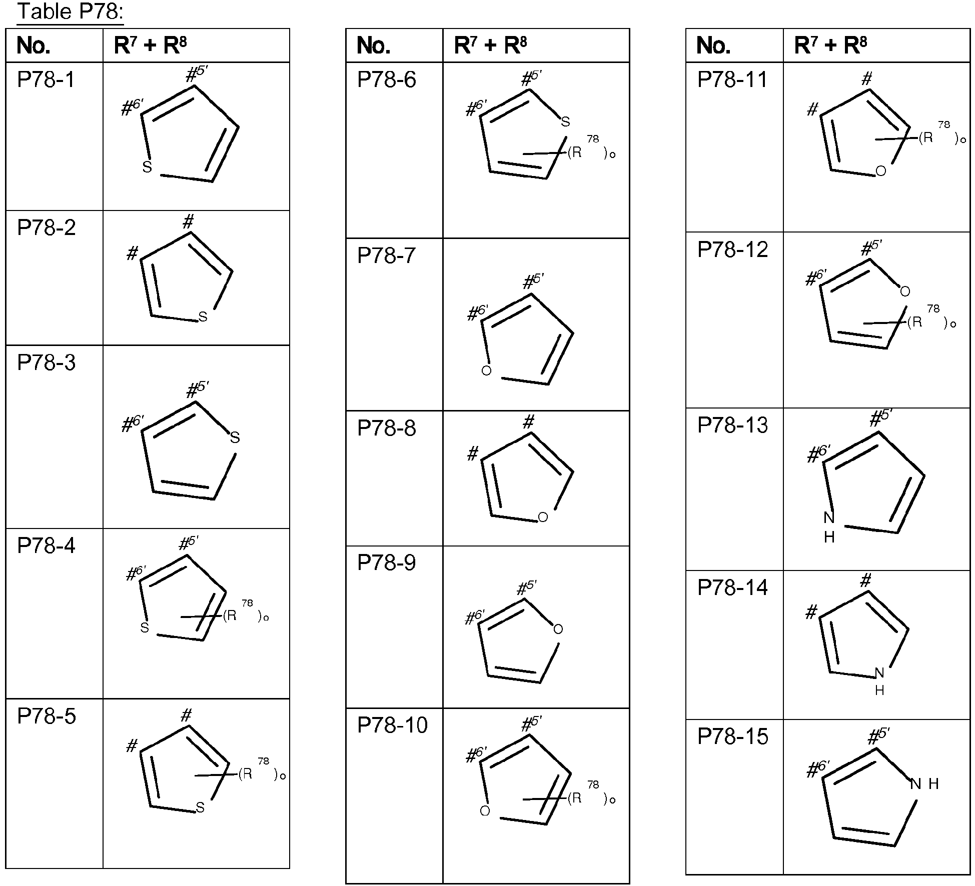

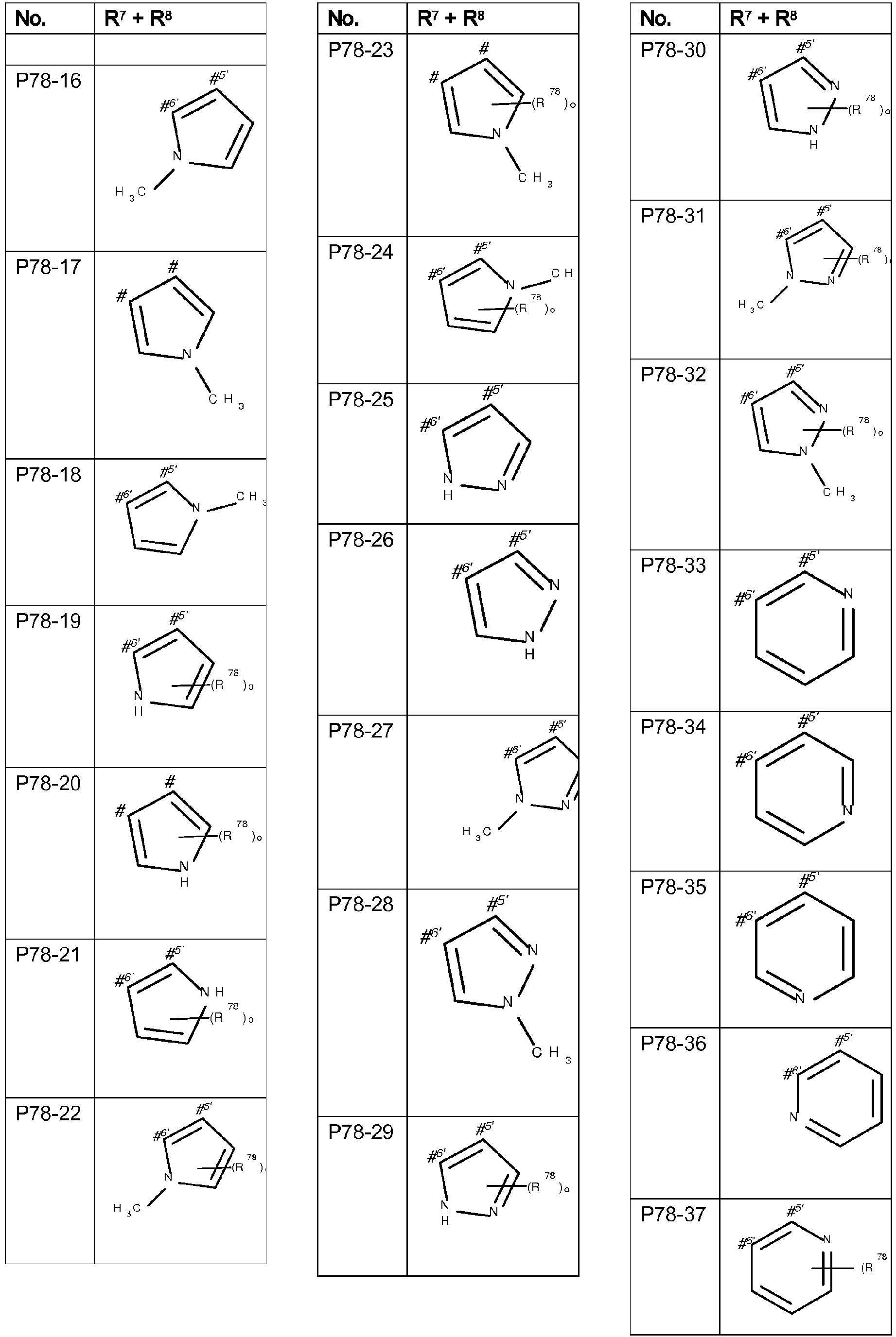

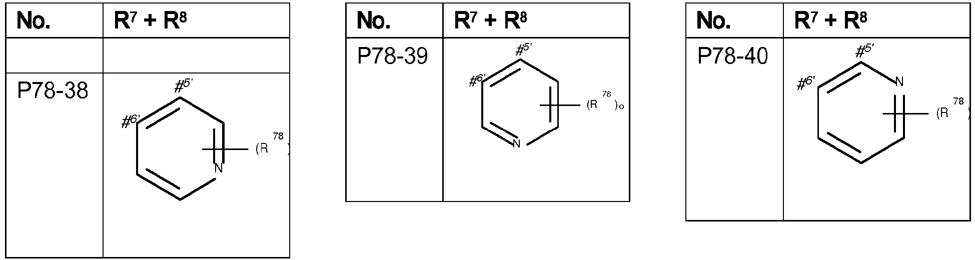

Preferred embodiments of the present invention are the following compounds I.A, I.B, I.C, I.D, I.E, I.F, I.G, I.H, I.I, I.J and I.K. In these formulae, the substituents R¹, m, R², n, R³, R⁴, R⁵, R⁶, R⁷⁸ and o are independently as defined or preferably defined herein:

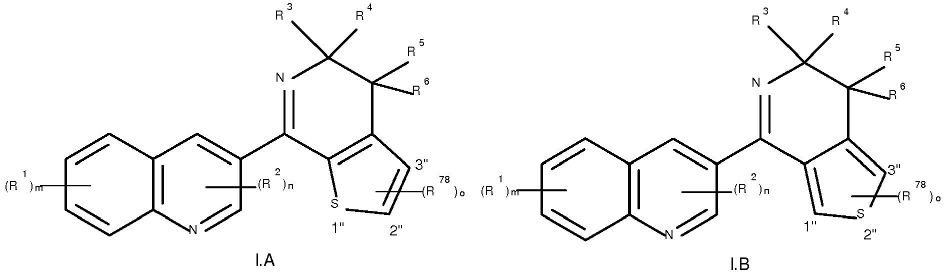

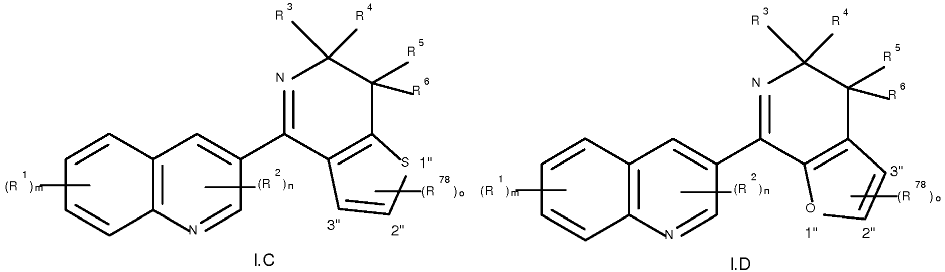

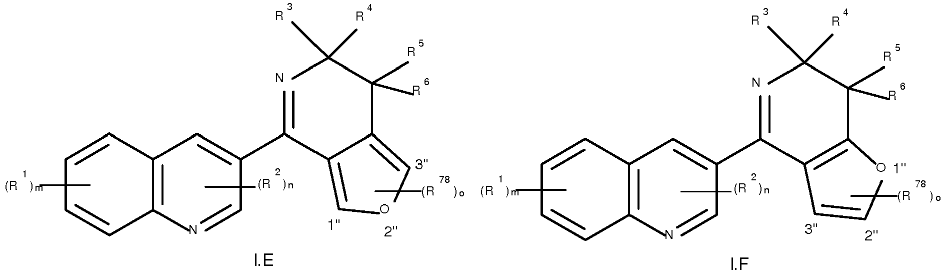

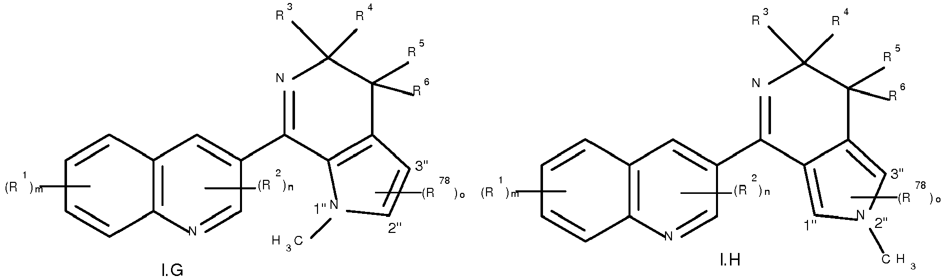

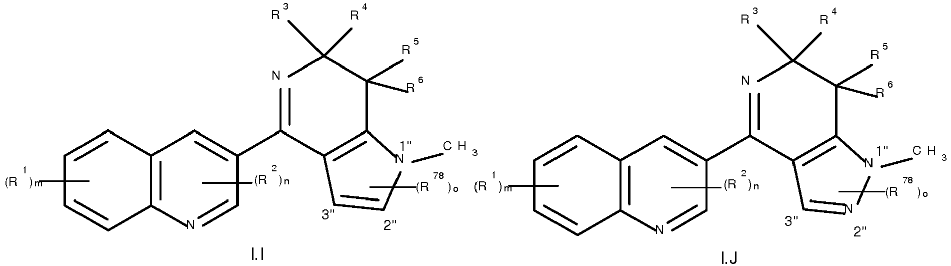

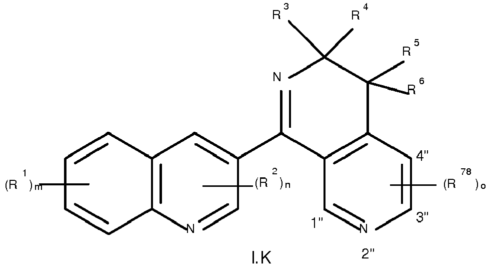

According to one embodiment, o in each of the formulae I.A, I.B, I.C, I.D, I.E, I.F, I.G, I.H, I.I, I.J and I.K, respectively, is 0, i.e. the heteroaryl is not substituted. These compounds are named I.A.1, I.B.1, I.C.1, I.D.1, I.E.1, I.F.1, I.G.1, I.H.1, I.I.1, I.J.1 and I.K.1, respectively.

Further preferred compounds I are the following compounds I.L, I.M, I.N, I.O, I.P, I.Q, I.R, I.S, I.T and I.U. In these formulae, the substituents R¹, m, R², n, R³, R⁴, R⁵, R⁶, R⁷⁸ and o are independently as defined or preferably defined herein:

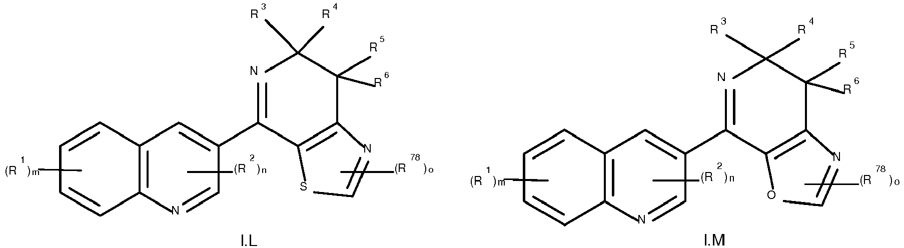

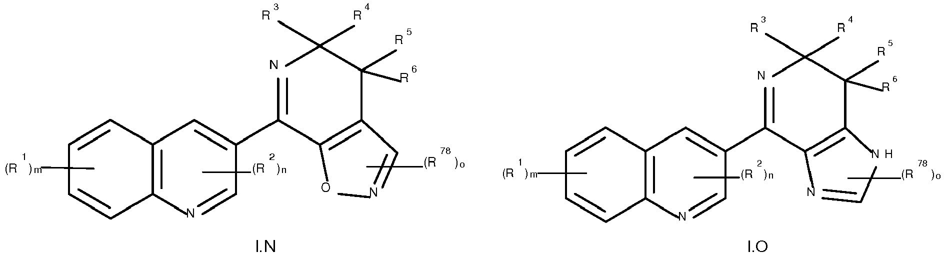

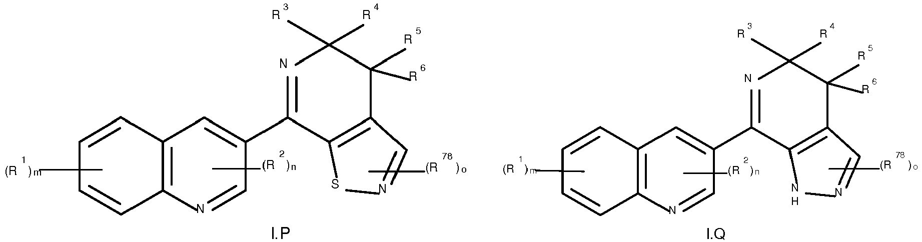

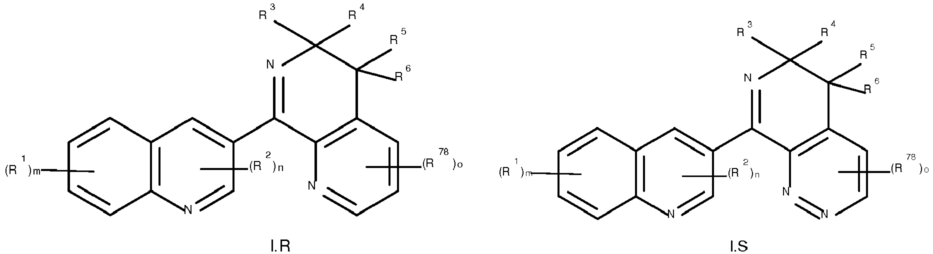

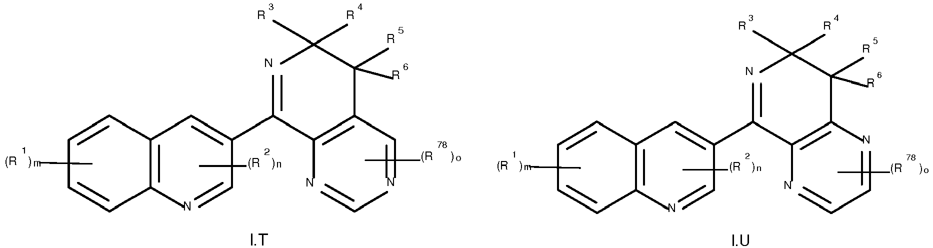

According to one embodiment, o in each of the formulae I.L, I.M, I.N, I.O, I.P, I.Q, I.R, I.S, I.T and I.U, respectively, is 0, i.e. the heteroaryl is not substituted. These compounds are named I.L.1, I.M.1, I.N.1, I.O.1, I.P.1, I.Q.1, I.R.1, I.S.1, I.T.1 and I.U.1, respectively.

In particular with a view to their use, according to one embodiment, preference is given to the compounds of the formulae I.A, I.B, I.C, I.D, I.E, I.F, I.G, I.H, I.I, I.J and I.K that are compiled in the Tables 1a to 7a, Tables 1b to 7b, Tables 1c to 7c, Tables 1d to 7d, Tables 1e to 7e, Tables 1f to 7f, Tables 1g to 7g, Tables 1h to 7h, Tables 1i to 7i, Tables 1j to 7j and Tables 1k to 7k. Each of the groups mentioned for a substituent in the tables is furthermore per se, independently of the combination in which it is mentioned, a particularly preferred aspect of the substituent in question.
Table 1a Compounds of the formula I.A in which o is 0 and the meaning for the combination of (R¹)ₘ, (R²)ₙ, R³, R⁴, R⁵ and R⁶ for each individual compound corresponds in each case to one line of Table B (compounds I.A.1a.B-1 to I.A.1a.B-1680).
Table 2a Compounds of the formula I.A in which o is 1, R⁷⁸ is 2"-F and the meaning for the combination of (R¹)ₘ, (R²)ₙ, R³, R⁴, R⁵ and R⁶ for each individual compound corresponds in each case to one line of Table B (compounds I.A.2a.B-1 to I.A.2a.B-1680).
Table 3a Compounds of the formula I.A in which o is 1, R⁷⁸ is 2"-CI and the meaning for the combination of (R¹)ₘ, (R²)ₙ, R³, R⁴, R⁵ and R⁶ for each individual compound corresponds in each case to one line of Table B (compounds I.A.3a.B-1 to I.A.3a.B-1680).
Table 4a Compounds of the formula I.A in which o is 1, R⁷⁸ is 2"-Br and the meaning for the combination of (R¹)ₘ, (R²)ₙ, R³, R⁴, R⁵ and R⁶ for each individual compound corresponds in each case to one line of Table B (compounds I.A.4a.B-1 to I.A.4a.B-1680).
Table 5a Compounds of the formula I.A in which o is 1, R⁷⁸ is 2"-CH₃ and the meaning for the combination of (R¹)ₘ, (R²)ₙ, R³, R⁴, R⁵ and R⁶ for each individual compound corresponds in each case to one line of Table B (compounds I.A.5a.B-1 to I.A.5a.B-1680).
Table 6a Compounds of the formula I.A in which o is 1, R⁷⁸ is 2"-OCH₃ and the meaning for the combination of (R¹)ₘ, (R²)ₙ, R³, R⁴, R⁵ and R⁶ for each individual compound corresponds in each case to one line of Table B (compounds I.A.6a.B-1 to I.A.6a.B-1680).
Table 7a Compounds of the formula I.A in which o is 1, R⁷⁸ is 2"-OCHF₂ and the meaning for the combination of (R¹)ₘ, (R²)ₙ, R³, R⁴, R⁵ and R⁶ for each individual compound corresponds in each case to one line of Table B (compounds I.A.7a.B-1 to I.A.7a.B-1680).
Table 8a Compounds of the formula I.A in which o is 1, R⁷⁸ is 2"-C₆H₅ and the meaning for the combination of (R¹)ₘ, (R²)ₙ, R³, R⁴, R⁵ and R⁶ for each individual compound corresponds in each case to one line of Table B (compounds I.A.8a.B-1 to I.A.8a.B-1680).
Table 9a Compounds of the formula I.A in which o is 1, R⁷⁸ is 3"-F and the meaning for the combination of (R¹)ₘ, (R²)ₙ, R³, R⁴, R⁵ and R⁶ for each individual compound corresponds in each case to one line of Table B (compounds I.A.9a.B-1 to I.A.9a.B-1680).
Table 10a Compounds of the formula I.A in which o is 1, R⁷⁸ is 3"-Cl and the meaning for the combination of (R¹)ₘ, (R²)ₙ, R³, R⁴, R⁵ and R⁶ for each individual compound corresponds in each case to one line of Table B (compounds I.A.10a.B-1 to I.A.10a.B-1680).
Table 11a Compounds of the formula I.A in which o is 1, R⁷⁸ is 3"-Br and the meaning for the combination of (R¹)ₘ, (R²)ₙ, R³, R⁴, R⁵ and R⁶ for each individual compound corresponds in each case to one line of Table B (compounds I.A.11a.B-1 to I.A.11a.B-1680).
Table 12a Compounds of the formula I.A in which o is 1, R⁷⁸ is 3"-CH₃ and the meaning for the combination of (R¹)ₘ, (R²)ₙ, R³, R⁴, R⁵ and R⁶ for each individual compound corresponds in each case to one line of Table B (compounds I.A.12a.B-1 to I.A.12a.B-1680).
Table 13a Compounds of the formula I.A in which o is 1, R⁷⁸ is 3"-OCH₃ and the meaning for the combination of (R¹)ₘ, (R²)ₙ, R³, R⁴, R⁵ and R⁶ for each individual compound corresponds in each case to one line of Table B (compounds I.A.13a.B-1 to I.A.13a.B-1680).
Table 14a Compounds of the formula I.A in which o is 1, R⁷⁸ is 3"-OCHF₂ and the meaning for the combination of (R¹)ₘ, (R²)ₙ, R³, R⁴, R⁵ and R⁶ for each individual compound corresponds in each case to one line of Table B (compounds I.A.14a.B-1 to I.A.14a.B-1680).
Table 15a Compounds of the formula I.A in which o is 1, R⁷⁸ is 3"-C₆H₅ and the meaning for the combination of (R¹)ₘ, (R²)ₙ, R³, R⁴, R⁵ and R⁶ for each individual compound corresponds in each case to one line of Table B (compounds I.A.15a.B-1 to I.A.15a.B-1680).
Table 1b Compounds of the formula I.B in which o is 0 and the meaning for the combination of (R¹)ₘ, (R²)ₙ, R³, R⁴, R⁵ and R⁶ for each individual compound corresponds in each case to one line of Table B (compounds I.B.1b.B-1 to I.B.1b.B-1680).
Table 2b Compounds of the formula I.B in which o is 1, R⁷⁸ is 1"-F and the meaning for the combination of (R¹)ₘ, (R²)ₙ, R³, R⁴, R⁵ and R⁶ for each individual compound corresponds in each case to one line of Table B (compounds I.B.2b.B-1 to I.B.2b.B-1680).
Table 3b Compounds of the formula I.B in which o is 1, R⁷⁸ is 1"-Cl and the meaning for the combination of (R¹)ₘ, (R²)ₙ, R³, R⁴, R⁵ and R⁶ for each individual compound corresponds in each case to one line of Table B (compounds I.B.3b.B-1 to I.B.3b.B-1680).
Table 4b Compounds of the formula I.B in which o is 1, R⁷⁸ is 1"-Br and the meaning for the combination of (R¹)ₘ, (R²)ₙ, R³, R⁴, R⁵ and R⁶ for each individual compound corresponds in each case to one line of Table B (compounds I.B.4b.B-1 to I.B.4b.B-1680).
Table 5b Compounds of the formula I.B in which o is 1, R⁷⁸ is 1"-CH₃ and the meaning for the combination of (R¹)ₘ, (R²)ₙ, R³, R⁴, R⁵ and R⁶ for each individual compound corresponds in each case to one line of Table B (compounds I.B.5b.B-1 to I.B.5b.B-1680).
Table 6b Compounds of the formula I.B in which o is 1, R⁷⁸ is 1"-OCH₃ and the meaning for the combination of (R¹)ₘ, (R²)ₙ, R³, R⁴, R⁵ and R⁶ for each individual compound corresponds in each case to one line of Table B (compounds I.B.6b.B-1 to I.B.6b.B-1680).
Table 7b Compounds of the formula I.B in which o is 1, R⁷⁸ is 1"-OCHF₂ and the meaning for the combination of (R¹)ₘ, (R²)ₙ, R³, R⁴, R⁵ and R⁶ for each individual compound corresponds in each case to one line of Table B (compounds I.B.7b.B-1 to I.B.7b.B-1680).
Table 8b Compounds of the formula I.B in which o is 1, R⁷⁸ is 1"-C₆H₅ and the meaning for the combination of (R¹)ₘ, (R²)ₙ, R³, R⁴, R⁵ and R⁶ for each individual compound corresponds in each case to one line of Table B (compounds I.B.8b.B-1 to I.B.8b.B-1680).
Table 9b Compounds of the formula I.B in which o is 1, R⁷⁸ is 3"-F and the meaning for the combination of (R¹)ₘ, (R²)ₙ, R³, R⁴, R⁵ and R⁶ for each individual compound corresponds in each case to one line of Table B (compounds I.B.9b.B-1 to I.B.9b.B-1680).
Table 10b Compounds of the formula I.B in which o is 1, R⁷⁸ is 3"-Cl and the meaning for the combination of (R¹)ₘ, (R²)ₙ, R³, R⁴, R⁵ and R⁶ for each individual compound corresponds in each case to one line of Table B (compounds I.B.10b.B-1 to I.B.10b.B-1680).
Table 11b Compounds of the formula I.B in which o is 1, R⁷⁸ is 3"-Br and the meaning for the combination of (R¹)ₘ, (R²)ₙ, R³, R⁴, R⁵ and R⁶ for each individual compound corresponds in each case to one line of Table B (compounds I.B.11b.B-1 to I.B.11b.B-1680).
Table 12b Compounds of the formula I.B in which o is 1, R⁷⁸ is 3"-CH₃ and the meaning for the combination of (R¹)ₘ, (R²)ₙ, R³, R⁴, R⁵ and R⁶ for each individual compound corresponds in each case to one line of Table B (compounds I.B.12b.B-1 to I.B.12b.B-1680).
Table 13b Compounds of the formula I.B in which o is 1, R⁷⁸ is 3"-OCH₃ and the meaning for the combination of (R¹)ₘ, (R²)ₙ, R³, R⁴, R⁵ and R⁶ for each individual compound corresponds in each case to one line of Table B (compounds I.B.13b.B-1 to I.B.13b.B-1680).
Table 14b Compounds of the formula I.B in which o is 1, R⁷⁸ is 3"-OCHF₂ and the meaning for the combination of (R¹)ₘ, (R²)ₙ, R³, R⁴, R⁵ and R⁶ for each individual compound corresponds in each case to one line of Table B (compounds I.B.14b.B-1 to I.B.14b.B-1680).
Table 15b Compounds of the formula I.B in which o is 1, R⁷⁸ is 3"-C₆H₅ and the meaning for the combination of (R¹)ₘ, (R²)ₙ, R³, R⁴, R⁵ and R⁶ for each individual compound corresponds in each case to one line of Table B (compounds I.B.15b.B-1 to I.B.15b.B-1680).
Table 1c Compounds of the formula I.C in which o is 0 and the meaning for the combination of (R¹)ₘ, (R²)ₙ, R³, R⁴, R⁵ and R⁶ for each individual compound corresponds in each case to one line of Table B (compounds I.C.1c.B-1 to I.C.1c.B-1680).
Table 2c Compounds of the formula I.C in which o is 1, R⁷⁸ is 2"-F and the meaning for the combination of (R¹)ₘ, (R²)ₙ, R³, R⁴, R⁵ and R⁶ for each individual compound corresponds in each case to one line of Table B (compounds I.C.2c.B-1 to I.C.2c.B-1680).
Table 3c Compounds of the formula I.C in which o is 1, R⁷⁸ is 2"-CI and the meaning for the combination of (R¹)ₘ, (R²)ₙ, R³, R⁴, R⁵ and R⁶ for each individual compound corresponds in each case to one line of Table B (compounds I.C.3c.B-1 to I.C.3c.B-1680).
Table 4c Compounds of the formula I.C in which o is 1, R⁷⁸ is 2"-Br and the meaning for the combination of (R¹)ₘ, (R²)ₙ, R³, R⁴, R⁵ and R⁶ for each individual compound corresponds in each case to one line of Table B (compounds I.C.4c.B-1 to I.C.4c.B-1680).
Table 5c Compounds of the formula I.C in which o is 1, R⁷⁸ is 2"-CH₃ and the meaning for the combination of (R¹)ₘ, (R²)ₙ, R³, R⁴, R⁵ and R⁶ for each individual compound corresponds in each case to one line of Table B (compounds I.C.5c.B-1 to I.C.5c.B-1680).
Table 6c Compounds of the formula I.C in which o is 1, R⁷⁸ is 2"-OCH₃ and the meaning for the combination of (R¹)ₘ, (R²)ₙ, R³, R⁴, R⁵ and R⁶ for each individual compound corresponds in each case to one line of Table B (compounds I.C.6c.B-1 to I.C.6c.B-1680).
Table 7c Compounds of the formula I.C in which o is 1, R⁷⁸ is 2"-OCHF₂ and the meaning for the combination of (R¹)ₘ, (R²)ₙ, R³, R⁴, R⁵ and R⁶ for each individual compound corresponds in each case to one line of Table B (compounds I.C.7c.B-1 to I.C.7c.B-1680).
Table 8c Compounds of the formula I.C in which o is 1, R⁷⁸ is 2"-C₆H₅ and the meaning for the combination of (R¹)ₘ, (R²)ₙ, R³, R⁴, R⁵ and R⁶ for each individual compound corresponds in each case to one line of Table B (compounds I.C.8c.B-1 to I.C.8c.B-1680).
Table 9c Compounds of the formula I.C in which o is 1, R⁷⁸ is 3"-F and the meaning for the combination of (R¹)ₘ, (R²)ₙ, R³, R⁴, R⁵ and R⁶ for each individual compound corresponds in each case to one line of Table B (compounds I.C.9c.B-1 to I.C.9c.B-1680).
Table 10c Compounds of the formula I.C in which o is 1, R⁷⁸ is 3"-Cl and the meaning for the combination of (R¹)ₘ, (R²)ₙ, R³, R⁴, R⁵ and R⁶ for each individual compound corresponds in each case to one line of Table B (compounds I.C.10c.B-1 to I.C.10c.B-1680).
Table 11c Compounds of the formula I.C in which o is 1, R⁷⁸ is 3"-Br and the meaning for the combination of (R¹)ₘ, (R²)ₙ, R³, R⁴, R⁵ and R⁶ for each individual compound corresponds in each case to one line of Table B (compounds I.C.11c.B-1 to I.C.11c.B-1680).
Table 12c Compounds of the formula I.C in which o is 1, R⁷⁸ is 3"-CH₃ and the meaning for the combination of (R¹)ₘ, (R²)ₙ, R³, R⁴, R⁵ and R⁶ for each individual compound corresponds in each case to one line of Table B (compounds I.C.12c.B-1 to I.C.12c.B-1680).
Table 13c Compounds of the formula I.C in which o is 1, R⁷⁸ is 3"-OCH₃ and the meaning for the combination of (R¹)ₘ, (R²)ₙ, R³, R⁴, R⁵ and R⁶ for each individual compound corresponds in each case to one line of Table B (compounds I.C.13c.B-1 to I.C.13c.B-1680).
Table 14c Compounds of the formula I.C in which o is 1, R⁷⁸ is 3"-OCHF₂ and the meaning for the combination of (R¹)ₘ, (R²)ₙ, R³, R⁴, R⁵ and R⁶ for each individual compound corresponds in each case to one line of Table B (compounds I.C.14c.B-1 to I.C.14c.B-1680).
Table 15c Compounds of the formula I.C in which o is 1, R⁷⁸ is 3"-C₆H₅ and the meaning for the combination of (R¹)ₘ, (R²)ₙ, R³, R⁴, R⁵ and R⁶ for each individual compound corresponds in each case to one line of Table B (compounds I.C.15c.B-1 to I.C.15c.B-1680).
Table 1d Compounds of the formula I.D in which o is 0 and the meaning for the combination of (R¹)ₘ, (R²)ₙ, R³, R⁴, R⁵ and R⁶ for each individual compound corresponds in each case to one line of Table B (compounds I.D.1d.B-1 to I.D.1d.B-1680).
Table 2d Compounds of the formula I.D in which o is 1, R⁷⁸ is 2"-F and the meaning for the combination of (R¹)ₘ, (R²)ₙ, R³, R⁴, R⁵ and R⁶ for each individual compound corresponds in each case to one line of Table B (compounds I.D.2d.B-1 to I.D.2d.B-1680).
Table 3d Compounds of the formula I.D in which o is 1, R⁷⁸ is 2"-CI and the meaning for the combination of (R¹)ₘ, (R²)ₙ, R³, R⁴, R⁵ and R⁶ for each individual compound corresponds in each case to one line of Table B (compounds I.D.3d.B-1 to I.D.3d.B-1680).
Table 4d Compounds of the formula I.D in which o is 1, R⁷⁸ is 2"-Br and the meaning for the combination of (R¹)ₘ, (R²)ₙ, R³, R⁴, R⁵ and R⁶ for each individual compound corresponds in each case to one line of Table B (compounds I.D.4d.B-1 to I.D.4d.B-1680).
Table 5d Compounds of the formula I.D in which o is 1, R⁷⁸ is 2"-CH₃ and the meaning for the combination of (R¹)ₘ, (R²)ₙ, R³, R⁴, R⁵ and R⁶ for each individual compound corresponds in each case to one line of Table B (compounds I.D.5d.B-1 to I.D.5d.B-1680).
Table 6d Compounds of the formula I.D in which o is 1, R⁷⁸ is 2"-OCH₃ and the meaning for the combination of (R¹)ₘ, (R²)ₙ, R³, R⁴, R⁵ and R⁶ for each individual compound corresponds in each case to one line of Table B (compounds I.D.6d.B-1 to I.D.6d.B-1680).
Table 7d Compounds of the formula I.D in which o is 1, R⁷⁸ is 2"-OCHF₂ and the meaning for the combination of (R¹)ₘ, (R²)ₙ, R³, R⁴, R⁵ and R⁶ for each individual compound corresponds in each case to one line of Table B (compounds I.D.7d.B-1 to I.D.7d.B-1680).
Table 8d Compounds of the formula I.D in which o is 1, R⁷⁸ is 2"-C₆H₅ and the meaning for the combination of (R¹)ₘ, (R²)ₙ, R³, R⁴, R⁵ and R⁶ for each individual compound corresponds in each case to one line of Table B (compounds I.D.8d.B-1 to I.D.8d.B-1680).
Table 9d Compounds of the formula I.D in which o is 1, R⁷⁸ is 3"-F and the meaning for the combination of (R¹)ₘ, (R²)ₙ, R³, R⁴, R⁵ and R⁶ for each individual compound corresponds in each case to one line of Table B (compounds I.D.9d.B-1 to I.D.9d.B-1680).
Table 10d Compounds of the formula I.D in which o is 1, R⁷⁸ is 3"-Cl and the meaning for the combination of (R¹)ₘ, (R²)ₙ, R³, R⁴, R⁵ and R⁶ for each individual compound corresponds in each case to one line of Table B (compounds I.D.10d.B-1 to I.D.10d.B-1680).
Table 11d Compounds of the formula I.D in which o is 1, R⁷⁸ is 3"-Br and the meaning for the combination of (R¹)ₘ, (R²)ₙ, R³, R⁴, R⁵ and R⁶ for each individual compound corresponds in each case to one line of Table B (compounds I.D.11d.B-1 to I.D.11d.B-1680).
Table 12d Compounds of the formula I.D in which o is 1, R⁷⁸ is 3"-CH₃ and the meaning for the combination of (R¹)ₘ, (R²)ₙ, R³, R⁴, R⁵ and R⁶ for each individual compound corresponds in each case to one line of Table B (compounds I.D.12d.B-1 to I.D.12d.B-1680).
Table 13d Compounds of the formula I.D in which o is 1, R⁷⁸ is 3"-OCH₃ and the meaning for the combination of (R¹)ₘ, (R²)ₙ, R³, R⁴, R⁵ and R⁶ for each individual compound corresponds in each case to one line of Table B (compounds I.D.13d.B-1 to I.D.13d.B-1680).
Table 14d Compounds of the formula I.D in which o is 1, R⁷⁸ is 3"-OCHF₂ and the meaning for the combination of (R¹)ₘ, (R²)ₙ, R³, R⁴, R⁵ and R⁶ for each individual compound corresponds in each case to one line of Table B (compounds I.D.14d.B-1 to I.D.14d.B-1680).
Table 15d Compounds of the formula I.D in which o is 1, R⁷⁸ is 3"-C₆H₅ and the meaning for the combination of (R¹)ₘ, (R²)ₙ, R³, R⁴, R⁵ and R⁶ for each individual compound corresponds in each case to one line of Table B (compounds I.D.15d.B-1 to I.D.15d.B-1680).
Table 1e Compounds of the formula I.E in which o is 0 and the meaning for the combination of (R¹)ₘ, (R²)ₙ, R³, R⁴, R⁵ and R⁶ for each individual compound corresponds in each case to one line of Table B (compounds I.E.1e.B-1 to I.E.1e.B-1680).
Table 2e Compounds of the formula I.E in which o is 1, R⁷⁸ is 1"-F and the meaning for the combination of (R¹)ₘ, (R²)ₙ, R³, R⁴, R⁵ and R⁶ for each individual compound corresponds in each case to one line of Table B (compounds I.E.2e.B-1 to I.E.2e.B-1680).
Table 3e Compounds of the formula I.E in which o is 1, R⁷⁸ is 1"-Cl and the meaning for the combination of (R¹)ₘ, (R²)ₙ, R³, R⁴, R⁵ and R⁶ for each individual compound corresponds in each case to one line of Table B (compounds I.E.3e.B-1 to I.E.3e.B-1680).
Table 4e Compounds of the formula I.E in which o is 1, R⁷⁸ is 1"-Br and the meaning for the combination of (R¹)ₘ, (R²)ₙ, R³, R⁴, R⁵ and R⁶ for each individual compound corresponds in each case to one line of Table B (compounds I.E.4e.B-1 to I.E.4e.B-1680).
Table 5e Compounds of the formula I.E in which o is 1, R⁷⁸ is 1"-CH₃ and the meaning for the combination of (R¹)ₘ, (R²)ₙ, R³, R⁴, R⁵ and R⁶ for each individual compound corresponds in each case to one line of Table B (compounds I.E.5e.B-1 to I.E.5e.B-1680).
Table 6e Compounds of the formula I.E in which o is 1, R⁷⁸ is 1"-OCH₃ and the meaning for the combination of (R¹)ₘ, (R²)ₙ, R³, R⁴, R⁵ and R⁶ for each individual compound corresponds in each case to one line of Table B (compounds I.E.6e.B-1 to I.E.6e.B-1680).
Table 7e Compounds of the formula I.E in which o is 1, R⁷⁸ is 1"-OCHF₂ and the meaning for the combination of (R¹)ₘ, (R²)ₙ, R³, R⁴, R⁵ and R⁶ for each individual compound corresponds in each case to one line of Table B (compounds I.E.7e.B-1 to I.E.7e.B-1680).
Table 8e Compounds of the formula I.E in which o is 1, R⁷⁸ is 1"-C₆H₅ and the meaning for the combination of (R¹)ₘ, (R²)ₙ, R³, R⁴, R⁵ and R⁶ for each individual compound corresponds in each case to one line of Table B (compounds I.E.8e.B-1 to I.E.8e.B-1680).
Table 9e Compounds of the formula I.E in which o is 1, R⁷⁸ is 3"-F and the meaning for the combination of (R¹)ₘ, (R²)ₙ, R³, R⁴, R⁵ and R⁶ for each individual compound corresponds in each case to one line of Table B (compounds I.E.9e.B-1 to I.E.9e.B-1680).
Table 10e Compounds of the formula I.E in which o is 1, R⁷⁸ is 3"-Cl and the meaning for the combination of (R¹)ₘ, (R²)ₙ, R³, R⁴, R⁵ and R⁶ for each individual compound corresponds in each case to one line of Table B (compounds I.E.10e.B-1 to I.E.10e.B-1680).
Table 11e Compounds of the formula I.E in which o is 1, R⁷⁸ is 3"-Br and the meaning for the combination of (R¹)ₘ, (R²)ₙ, R³, R⁴, R⁵ and R⁶ for each individual compound corresponds in each case to one line of Table B (compounds I.E.11e.B-1 to I.E.11e.B-1680).
Table 12e Compounds of the formula I.E in which o is 1, R⁷⁸ is 3"-CH₃ and the meaning for the combination of (R¹)ₘ, (R²)ₙ, R³, R⁴, R⁵ and R⁶ for each individual compound corresponds in each case to one line of Table B (compounds I.E.12e.B-1 to I.E.12e.B-1680).
Table 13e Compounds of the formula I.E in which o is 1, R⁷⁸ is 3"-OCH₃ and the meaning for the combination of (R¹)ₘ, (R²)ₙ, R³, R⁴, R⁵ and R⁶ for each individual compound corresponds in each case to one line of Table B (compounds I.E.13e.B-1 to I.E.13e.B-1680).
Table 14e Compounds of the formula I.E in which o is 1, R⁷⁸ is 3"-OCHF₂ and the meaning for the combination of (R¹)ₘ, (R²)ₙ, R³, R⁴, R⁵ and R⁶ for each individual compound corresponds in each case to one line of Table B (compounds I.E.14e.B-1 to I.E.14e.B-1680).
Table 15e Compounds of the formula I.E in which o is 1, R⁷⁸ is 3"-C₆H₅ and the meaning for the combination of (R¹)ₘ, (R²)ₙ, R³, R⁴, R⁵ and R⁶ for each individual compound corresponds in each case to one line of Table B (compounds I.E.15e.B-1 to I.E.15e.B-1680).
Table 1f Compounds of the formula I.F in which o is 0 and the meaning for the combination of (R¹)ₘ, (R²)ₙ, R³, R⁴, R⁵ and R⁶ for each individual compound corresponds in each case to one line of Table B (compounds I.F.1f.B-1 to I.F.1f.B-1680).
Table 2f Compounds of the formula I.F in which o is 1, R⁷⁸ is 2"-F and the meaning for the combination of (R¹)ₘ, (R²)ₙ, R³, R⁴, R⁵ and R⁶ for each individual compound corresponds in each case to one line of Table B (compounds I.F.2f.B-1 to I.F.2f.B-1680).
Table 3f Compounds of the formula I.F in which o is 1, R⁷⁸ is 2"-CI and the meaning for the combination of (R¹)ₘ, (R²)ₙ, R³, R⁴, R⁵ and R⁶ for each individual compound corresponds in each case to one line of Table B (compounds I.F.3f.B-1 to I.F.3f.B-1680).
Table 4f Compounds of the formula I.F in which o is 1, R⁷⁸ is 2"-Br and the meaning for the combination of (R¹)ₘ, (R²)ₙ, R³, R⁴, R⁵ and R⁶ for each individual compound corresponds in each case to one line of Table B (compounds I.F.4f.B-1 to I.F.4f.B-1680).
Table 5f Compounds of the formula I.F in which o is 1, R⁷⁸ is 2"-CH₃ and the meaning for the combination of (R¹)ₘ, (R²)ₙ, R³, R⁴, R⁵ and R⁶ for each individual compound corresponds in each case to one line of Table B (compounds I.F.5f.B-1 to I.F.5f.B-1680).
Table 6f Compounds of the formula I.F in which o is 1, R⁷⁸ is 2"-OCH₃ and the meaning for the combination of (R¹)ₘ, (R²)ₙ, R³, R⁴, R⁵ and R⁶ for each individual compound corresponds in each case to one line of Table B (compounds I.F.6f.B-1 to I.F.6f.B-1680).
Table 7f Compounds of the formula I.F in which o is 1, R⁷⁸ is 2"-OCHF₂ and the meaning for the combination of (R¹)ₘ, (R²)ₙ, R³, R⁴, R⁵ and R⁶ for each individual compound corresponds in each case to one line of Table B (compounds I.F.7f.B-1 to I.F.7f.B-1680).
Table 8f Compounds of the formula I.F in which o is 1, R⁷⁸ is 2"-C₆H₅ and the meaning for the combination of (R¹)ₘ, (R²)ₙ, R³, R⁴, R⁵ and R⁶ for each individual compound corresponds in each case to one line of Table B (compounds I.F.8f.B-1 to I.F.8f.B-1680).
Table 9f Compounds of the formula I.F in which o is 1, R⁷⁸ is 3"-F and the meaning for the combination of (R¹)ₘ, (R²)ₙ, R³, R⁴, R⁵ and R⁶ for each individual compound corresponds in each case to one line of Table B (compounds I.F.9f.B-1 to I.F.9f.B-1680).
Table 10f Compounds of the formula I.F in which o is 1, R⁷⁸ is 3"-Cl and the meaning for the combination of (R¹)ₘ, (R²)ₙ, R³, R⁴, R⁵ and R⁶ for each individual compound corresponds in each case to one line of Table B (compounds I.F.10f.B-1 to I.F.10f.B-1680).
Table 11f Compounds of the formula I.F in which o is 1, R⁷⁸ is 3"-Br and the meaning for the combination of (R¹)ₘ, (R²)ₙ, R³, R⁴, R⁵ and R⁶ for each individual compound corresponds in each case to one line of Table B (compounds I.F.11f.B-1 to I.F.11f.B-1680).
Table 12f Compounds of the formula I.F in which o is 1, R⁷⁸ is 3"-CH₃ and the meaning for the combination of (R¹)ₘ, (R²)ₙ, R³, R⁴, R⁵ and R⁶ for each individual compound corresponds in each case to one line of Table B (compounds I.F.12f.B-1 to I.F.12f.B-1680).
Table 13f Compounds of the formula I.F in which o is 1, R⁷⁸ is 3"-OCH₃ and the meaning for the combination of (R¹)ₘ, (R²)ₙ, R³, R⁴, R⁵ and R⁶ for each individual compound corresponds in each case to one line of Table B (compounds I.F.13f.B-1 to I.F.13f.B-1680).
Table 14f Compounds of the formula I.F in which o is 1, R⁷⁸ is 3"-OCHF₂ and the meaning for the combination of (R¹)ₘ, (R²)ₙ, R³, R⁴, R⁵ and R⁶ for each individual compound corresponds in each case to one line of Table B (compounds I.F.14f.B-1 to I.F.14f.B-1680).
Table 15f Compounds of the formula I.F in which o is 1, R⁷⁸ is 3"-C₆H₅ and the meaning for the combination of (R¹)ₘ, (R²)ₙ, R³, R⁴, R⁵ and R⁶ for each individual compound corresponds in each case to one line of Table B (compounds I.F.15f.B-1 to I.F.15f.B-1680).
Table 1g Compounds of the formula I.G in which o is 0 and the meaning for the combination of (R¹)ₘ, (R²)ₙ, R³, R⁴, R⁵ and R⁶ for each individual compound corresponds in each case to one line of Table B (compounds I.G.1g.B-1 to I.G.1g.B-1680).
Table 2g Compounds of the formula I.G in which o is 1, R⁷⁸ is 2"-F and the meaning for the combination of (R¹)ₘ, (R²)ₙ, R³, R⁴, R⁵ and R⁶ for each individual compound corresponds in each case to one line of Table B (compounds I.G.2g.B-1 to I.G.2g.B-1680).
Table 3g Compounds of the formula I.G in which o is 1, R⁷⁸ is 2"-Cl and the meaning for the combination of (R¹)ₘ, (R²)ₙ, R³, R⁴, R⁵ and R⁶ for each individual compound corresponds in each case to one line of Table B (compounds I.G.3g.B-1 to I.G.3g.B-1680).
Table 4g Compounds of the formula I.G in which o is 1, R⁷⁸ is 2"-Br and the meaning for the combination of (R¹)ₘ, (R²)ₙ, R³, R⁴, R⁵ and R⁶ for each individual compound corresponds in each case to one line of Table B (compounds I.G.4g.B-1 to I.G.4g.B-1680).
Table 5g Compounds of the formula I.G in which o is 1, R⁷⁸ is 2"-CH₃ and the meaning for the combination of (R¹)ₘ, (R²)ₙ, R³, R⁴, R⁵ and R⁶ for each individual compound corresponds in each case to one line of Table B (compounds I.G.5g.B-1 to I.G.5g.B-1680).
Table 6g Compounds of the formula I.G in which o is 1, R⁷⁸ is 2"-OCH₃ and the meaning for the combination of (R¹)ₘ, (R²)ₙ, R³, R⁴, R⁵ and R⁶ for each individual compound corresponds in each case to one line of Table B (compounds I.G.6g.B-1 to I.G.6g.B-1680).
Table 7g Compounds of the formula I.G in which o is 1, R⁷⁸ is 2"-OCHF₂ and the meaning for the combination of (R¹)ₘ, (R²)ₙ, R³, R⁴, R⁵ and R⁶ for each individual compound corresponds in each case to one line of Table B (compounds I.G.7g.B-1 to I.G.7g.B-1680).
Table 8g Compounds of the formula I.G in which o is 1, R⁷⁸ is 2"-C₆H₅ and the meaning for the combination of (R¹)ₘ, (R²)ₙ, R³, R⁴, R⁵ and R⁶ for each individual compound corresponds in each case to one line of Table B (compounds I.G.8g.B-1 to I.G.8g.B-1680).
Table 9g Compounds of the formula I.G in which o is 1, R⁷⁸ is 3"-F and the meaning for the combination of (R¹)ₘ, (R²)ₙ, R³, R⁴, R⁵ and R⁶ for each individual compound corresponds in each case to one line of Table B (compounds I.G.9g.B-1 to I.G.9g.B-1680).
Table 10g Compounds of the formula I.G in which o is 1, R⁷⁸ is 3"-Cl and the meaning for the combination of (R¹)ₘ, (R²)ₙ, R³, R⁴, R⁵ and R⁶ for each individual compound corresponds in each case to one line of Table B (compounds I.G.10g.B-1 to I.G.10g.B-1680).
Table 11g Compounds of the formula I.G in which o is 1, R⁷⁸ is 3"-Br and the meaning for the combination of (R¹)ₘ, (R²)ₙ, R³, R⁴, R⁵ and R⁶ for each individual compound corresponds in each case to one line of Table B (compounds I.G.11g.B-1 to I.G.11g.B-1680).
Table 12g Compounds of the formula I.G in which o is 1, R⁷⁸ is 3"-CH₃ and the meaning for the combination of (R¹)ₘ, (R²)ₙ, R³, R⁴, R⁵ and R⁶ for each individual compound corresponds in each case to one line of Table B (compounds I.G.12g.B-1 to I.G.12g.B-1680).
Table 13g Compounds of the formula I.G in which o is 1, R⁷⁸ is 3"-OCH₃ and the meaning for the combination of (R¹)ₘ, (R²)ₙ, R³, R⁴, R⁵ and R⁶ for each individual compound corresponds in each case to one line of Table B (compounds I.G.13g.B-1 to I.G.13g.B-1680).
Table 14g Compounds of the formula I.G in which o is 1, R⁷⁸ is 3"-OCHF₂ and the meaning for the combination of (R¹)ₘ, (R²)ₙ, R³, R⁴, R⁵ and R⁶ for each individual compound corresponds in each case to one line of Table B (compounds I.G.14g.B-1 to I.G.14g.B-1680).
Table 15g Compounds of the formula I.G in which o is 1, R⁷⁸ is 3"-C₆H₅ and the meaning for the combination of (R¹)ₘ, (R²)ₙ, R³, R⁴, R⁵ and R⁶ for each individual compound corresponds in each case to one line of Table B (compounds I.G.15g.B-1 to I.G.15g.B-1680).
Table 1h Compounds of the formula I.H in which o is 0 and the meaning for the combination of (R¹)ₘ, (R²)ₙ, R³, R⁴, R⁵ and R⁶ for each individual compound corresponds in each case to one line of Table B (compounds I.H.1h.B-1 to I.H.1h.B-1680).
Table 2h Compounds of the formula I.H in which o is 1, R⁷⁸ is 1 "-F and the meaning for the combination of (R¹)ₘ, (R²)ₙ, R³, R⁴, R⁵ and R⁶ for each individual compound corresponds in each case to one line of Table B (compounds I.H.2h.B-1 to I.H.2h.B-1680).
Table 3h Compounds of the formula I.H in which o is 1, R⁷⁸ is 1"-Cl and the meaning for the combination of (R¹)ₘ, (R²)ₙ, R³, R⁴, R⁵ and R⁶ for each individual compound corresponds in each case to one line of Table B (compounds I.H.3h.B-1 to I.H.3h.B-1680).
Table 4h Compounds of the formula I.H in which o is 1, R⁷⁸ is 1"-Br and the meaning for the combination of (R¹)ₘ, (R²)ₙ, R³, R⁴, R⁵ and R⁶ for each individual compound corresponds in each case to one line of Table B (compounds I.H.4h.B-1 to I.H.4h.B-1680).
Table 5h Compounds of the formula I.H in which o is 1, R⁷⁸ is 1"-CH₃ and the meaning for the combination of (R¹)ₘ, (R²)ₙ, R³, R⁴, R⁵ and R⁶ for each individual compound corresponds in each case to one line of Table B (compounds I.H.5h.B-1 to I.H.5h.B-1680).
Table 6h Compounds of the formula I.H in which o is 1, R⁷⁸ is 1"-OCH₃ and the meaning for the combination of (R¹)ₘ, (R²)ₙ, R³, R⁴, R⁵ and R⁶ for each individual compound corresponds in each case to one line of Table B (compounds I.H.6h.B-1 to I.H.6h.B-1680).
Table 7h Compounds of the formula I.H in which o is 1, R⁷⁸ is 1"-OCHF₂ and the meaning for the combination of (R¹)ₘ, (R²)ₙ, R³, R⁴, R⁵ and R⁶ for each individual compound corresponds in each case to one line of Table B (compounds I.H.7h.B-1 to I.H.7h.B-1680).
Table 8h Compounds of the formula I.H in which o is 1, R⁷⁸ is 1"-C₆H₅ and the meaning for the combination of (R¹)ₘ, (R²)ₙ, R³, R⁴, R⁵ and R⁶ for each individual compound corresponds in each case to one line of Table B (compounds I.H.8h.B-1 to I.H.8h.B-1680).
Table 9h Compounds of the formula I.H in which o is 1, R⁷⁸ is 3"-F and the meaning for the combination of (R¹)ₘ, (R²)ₙ, R³, R⁴, R⁵ and R⁶ for each individual compound corresponds in each case to one line of Table B (compounds I.H.9h.B-1 to I.H.9h.B-1680).
Table 10h Compounds of the formula I.H in which o is 1, R⁷⁸ is 3"-Cl and the meaning for the combination of (R¹)ₘ, (R²)ₙ, R³, R⁴, R⁵ and R⁶ for each individual compound corresponds in each case to one line of Table B (compounds I.H.10h.B-1 to I.H.10h.B-1680).
Table 11h Compounds of the formula I.H in which o is 1, R⁷⁸ is 3"-Br and the meaning for the combination of (R¹)ₘ, (R²)ₙ, R³, R⁴, R⁵ and R⁶ for each individual compound corresponds in each case to one line of Table B (compounds I.H.11h.B-1 to I.H.11h.B-1680).
Table 12h Compounds of the formula I.H in which o is 1, R⁷⁸ is 3"-CH₃ and the meaning for the combination of (R¹)ₘ, (R²)ₙ, R³, R⁴, R⁵ and R⁶ for each individual compound corresponds in each case to one line of Table B (compounds I.H.12h.B-1 to I.H.12h.B-1680).
Table 13h Compounds of the formula I.H in which o is 1, R⁷⁸ is 3"-OCH₃ and the meaning for the combination of (R¹)ₘ, (R²)ₙ, R³, R⁴, R⁵ and R⁶ for each individual compound corresponds in each case to one line of Table B (compounds I.H.13h.B-1 to I.H.13h.B-1680).
Table 14h Compounds of the formula I.H in which o is 1, R⁷⁸ is 3"-OCHF₂ and the meaning for the combination of (R¹)ₘ, (R²)ₙ, R³, R⁴, R⁵ and R⁶ for each individual compound corresponds in each case to one line of Table B (compounds I.H.14h.B-1 to I.H.14h.B-1680).
Table 15h Compounds of the formula I.H in which o is 1, R⁷⁸ is 3"-C₆H₅ and the meaning for the combination of (R¹)ₘ, (R²)ₙ, R³, R⁴, R⁵ and R⁶ for each individual compound corresponds in each case to one line of Table B (compounds I.H.15h.B-1 to I.H.15h.B-1680).
Table 1i Compounds of the formula I.I in which o is 0 and the meaning for the combination of (R¹)ₘ, (R²)ₙ, R³, R⁴, R⁵ and R⁶ for each individual compound corresponds in each case to one line of Table B (compounds I.I.1i.B-1 to I.I.1i.B-1680).
Table 2i Compounds of the formula I.I in which o is 1, R⁷⁸ is 2"-F and the meaning for the combination of (R¹)ₘ, (R²)ₙ, R³, R⁴, R⁵ and R⁶ for each individual compound corresponds in each case to one line of Table B (compounds I.I.2i.B-1 to I.I.2i.B-1680).
Table 3i Compounds of the formula I.I in which o is 1, R⁷⁸ is 2"-Cl and the meaning for the combination of (R¹)ₘ, (R²)ₙ, R³, R⁴, R⁵ and R⁶ for each individual compound corresponds in each case to one line of Table B (compounds I.I.3i.B-1 to I.I.3i.B-1680).
Table 4i Compounds of the formula I.I in which o is 1, R⁷⁸ is 2"-Br and the meaning for the combination of (R¹)ₘ, (R²)ₙ, R³, R⁴, R⁵ and R⁶ for each individual compound corresponds in each case to one line of Table B (compounds I.I.4i.B-1 to I.I.4i.B-1680).
Table 5i Compounds of the formula I.I in which o is 1, R⁷⁸ is 2"-CH₃ and the meaning for the combination of (R¹)ₘ, (R²)ₙ, R³, R⁴, R⁵ and R⁶ for each individual compound corresponds in each case to one line of Table B (compounds I.I.5i.B-1 to I.I.5i.B-1680).
Table 6i Compounds of the formula I.I in which o is 1, R⁷⁸ is 2"-OCH₃ and the meaning for the combination of (R¹)ₘ, (R²)ₙ, R³, R⁴, R⁵ and R⁶ for each individual compound corresponds in each case to one line of Table B (compounds I.I.6i.B-1 to I.I.6i.B-1680).
Table 7i Compounds of the formula I.I in which o is 1, R⁷⁸ is 2"-OCHF₂ and the meaning for the combination of (R¹)ₘ, (R²)ₙ, R³, R⁴, R⁵ and R⁶ for each individual compound corresponds in each case to one line of Table B (compounds I.I.7i.B-1 to I.I.7i.B-1680).
Table 8i Compounds of the formula I.I in which o is 1, R⁷⁸ is 2"-C₆H₅ and the meaning for the combination of (R¹)ₘ, (R²)ₙ, R³, R⁴, R⁵ and R⁶ for each individual compound corresponds in each case to one line of Table B (compounds I.I.8i.B-1 to I.I.8i.B-1680).
Table 9i Compounds of the formula I.I in which o is 1, R⁷⁸ is 3"-F and the meaning for the combination of (R¹)ₘ, (R²)ₙ, R³, R⁴, R⁵ and R⁶ for each individual compound corresponds in each case to one line of Table B (compounds I.I.9i.B-1 to I.I.9i.B-1680).
Table 10i Compounds of the formula I.I in which o is 1, R⁷⁸ is 3"-Cl and the meaning for the combination of (R¹)ₘ, (R²)ₙ, R³, R⁴, R⁵ and R⁶ for each individual compound corresponds in each case to one line of Table B (compounds I.I.10i.B-1 to I.I.10i.B-1680).
Table 11i Compounds of the formula I.I in which o is 1, R⁷⁸ is 3"-Br and the meaning for the combination of (R¹)ₘ, (R²)ₙ, R³, R⁴, R⁵ and R⁶ for each individual compound corresponds in each case to one line of Table B (compounds I.I.1.1i.B-1 to I.I.11i.B-1680).
Table 12i Compounds of the formula I.I in which o is 1, R⁷⁸ is 3"-CH₃ and the meaning for the combination of (R¹)ₘ, (R²)ₙ, R³, R⁴, R⁵ and R⁶ for each individual compound corresponds in each case to one line of Table B (compounds I.I.12i.B-1 to I.I.12i.B-1680).
Table 13i Compounds of the formula I.I in which o is 1, R⁷⁸ is 3"-OCH₃ and the meaning for the combination of (R¹)ₘ, (R²)ₙ, R³, R⁴, R⁵ and R⁶ for each individual compound corresponds in each case to one line of Table B (compounds I.I.13i.B-1 to I.I.13i.B-1680).
Table 14i Compounds of the formula I.I in which o is 1, R⁷⁸ is 3"-OCHF₂ and the meaning for the combination of (R¹)ₘ, (R²)ₙ, R³, R⁴, R⁵ and R⁶ for each individual compound corresponds in each case to one line of Table B (compounds I.I.14i.B-1 to I.I.14i.B-1680).
Table 15i Compounds of the formula I.I in which o is 1, R⁷⁸ is 3"-C₆H₅ and the meaning for the combination of (R¹)ₘ, (R²)ₙ, R³, R⁴, R⁵ and R⁶ for each individual compound corresponds in each case to one line of Table B (compounds I.I.15i.B-1 to I.I.15i.B-1680).
Table 1j Compounds of the formula I.J in which o is 0 and the meaning for the combination of (R¹)ₘ, (R²)ₙ, R³, R⁴, R⁵ and R⁶ for each individual compound corresponds in each case to one line of Table B (compounds I.J.1j.B-1 to I.J.1j.B-1680).
Table 2j Compounds of the formula I.J in which o is 1, R⁷⁸ is 3"-F and the meaning for the combination of (R¹)ₘ, (R²)ₙ, R³, R⁴, R⁵ and R⁶ for each individual compound corresponds in each case to one line of Table B (compounds I.J.2j.B-1 to I.J.2j.B-1680).
Table 3j Compounds of the formula I.J in which o is 1, R⁷⁸ is 3"-Cl and the meaning for the combination of (R¹)ₘ, (R²)ₙ, R³, R⁴, R⁵ and R⁶ for each individual compound corresponds in each case to one line of Table B (compounds I.J.3j.B-1 to I.J.3j.B-1680).
Table 4j Compounds of the formula I.J in which o is 1, R⁷⁸ is 3"-Br and the meaning for the combination of (R¹)ₘ, (R²)ₙ, R³, R⁴, R⁵ and R⁶ for each individual compound corresponds in each case to one line of Table B (compounds I.J.4j.B-1 to I.J.4j.B-1680).
Table 5j Compounds of the formula I.J in which o is 1, R⁷⁸ is 3"-CH₃ and the meaning for the combination of (R¹)ₘ, (R²)ₙ, R³, R⁴, R⁵ and R⁶ for each individual compound corresponds in each case to one line of Table B (compounds I.J.5j.B-1 to I.J.5j.B-1680).
Table 6j Compounds of the formula I.J in which o is 1, R⁷⁸ is 3"-OCH₃ and the meaning for the combination of (R¹)ₘ, (R²)ₙ, R³, R⁴, R⁵ and R⁶ for each individual compound corresponds in each case to one line of Table B (compounds I.J.6j.B-1 to I.J.6j.B-1680).
Table 7j Compounds of the formula I.J in which o is 1, R⁷⁸ is 3"-OCHF₂ and the meaning for the combination of (R¹)ₘ, (R²)ₙ, R³, R⁴, R⁵ and R⁶ for each individual compound corresponds in each case to one line of Table B (compounds I.J.7j.B-1 to I.J.7j.B-1680).
Table 8j Compounds of the formula I.J in which o is 1, R⁷⁸ is 3"-C₆H₅ and the meaning for the combination of (R¹)ₘ, (R²)ₙ, R³, R⁴, R⁵ and R⁶ for each individual compound corresponds in each case to one line of Table B (compounds I.J.8j.B-1 to I.J.8j.B-1680).
Table 1k Compounds of the formula I.K in which o is 0 and the meaning for the combination of (R¹)ₘ, (R²)ₙ, R³, R⁴, R⁵ and R⁶ for each individual compound corresponds in each case to one line of Table B (compounds I.K.1k.B-1 to I.K.1k.B-1680).
Table 2k Compounds of the formula I.K in which o is 1, R⁷⁸ is 1"-F and the meaning for the combination of (R¹)ₘ, (R²)ₙ, R³, R⁴, R⁵ and R⁶ for each individual compound corresponds in each case to one line of Table B (compounds I.K.2k.B-1 to I.K.2k.B-1680).
Table 3k Compounds of the formula I.K in which o is 1, R⁷⁸ is 1 "-Cl and the meaning for the combination of (R¹)ₘ, (R²)ₙ, R³, R⁴, R⁵ and R⁶ for each individual compound corresponds in each case to one line of Table B (compounds I.K.3k.B-1 to I.K.3k.B-1680).
Table 4k Compounds of the formula I.K in which o is 1, R⁷⁸ is 1"-Br and the meaning for the combination of (R¹)ₘ, (R²)ₙ, R³, R⁴, R⁵ and R⁶ for each individual compound corresponds in each case to one line of Table B (compounds I.K.4k.B-1 to I.K.4k.B-1680).
Table 5k Compounds of the formula I.K in which o is 1, R⁷⁸ is 1"-CH₃ and the meaning for the combination of (R¹)ₘ, (R²)ₙ, R³, R⁴, R⁵ and R⁶ for each individual compound corresponds in each case to one line of Table B (compounds I.K.5k.B-1 to I.K.5k.B-1680).
Table 6k Compounds of the formula I.K in which o is 1, R⁷⁸ is 1"-OCH₃ and the meaning for the combination of (R¹)ₘ, (R²)ₙ, R³, R⁴, R⁵ and R⁶ for each individual compound corresponds in each case to one line of Table B (compounds I.K.6k.B-1 to I.K.6k.B-1680).
Table 7k Compounds of the formula I.K in which o is 1, R⁷⁸ is 1"-OCHF₂ and the meaning for the combination of (R¹)ₘ, (R²)ₙ, R³, R⁴, R⁵ and R⁶ for each individual compound corresponds in each case to one line of Table B (compounds I.K.7k.B-1 to I.K.7k.B-1680).
Table 8k Compounds of the formula I.K in which o is 1, R⁷⁸ is 1"-C₆H₅ and the meaning for the combination of (R¹)ₘ, (R²)ₙ, R³, R⁴, R⁵ and R⁶ for each individual compound corresponds in each case to one line of Table B (compounds I.K.8k.B-1 to I.K.8k.B-1680).
Table 9k Compounds of the formula I.K in which o is 1, R⁷⁸ is 3"-F and the meaning for the combination of (R¹)ₘ, (R²)ₙ, R³, R⁴, R⁵ and R⁶ for each individual compound corresponds in each case to one line of Table B (compounds I.K.9k.B-1 to I.K.9k.B-1680).
Table 10k Compounds of the formula I.K in which o is 1, R⁷⁸ is 3"-Cl and the meaning for the combination of (R¹)ₘ, (R²)ₙ, R³, R⁴, R⁵ and R⁶ for each individual compound corresponds in each case to one line of Table B (compounds I.K.10k.B-1 to I.K.10k.B-1680).
Table 11k Compounds of the formula I.K in which o is 1, R⁷⁸ is 3"-Br and the meaning for the combination of (R¹)ₘ, (R²)ₙ, R³, R⁴, R⁵ and R⁶ for each individual compound corresponds in each case to one line of Table B (compounds I.K.11 k.B-1 to I.K.11 k.B-1680).
Table 12k Compounds of the formula I.K in which o is 1, R⁷⁸ is 3"-CH₃ and the meaning for the combination of (R¹)ₘ, (R²)ₙ, R³, R⁴, R⁵ and R⁶ for each individual compound corresponds in each case to one line of Table B (compounds I.K.12k.B-1 to I.K.12k.B-1680).
Table 13k Compounds of the formula I.K in which o is 1, R⁷⁸ is 3"-OCH₃ and the meaning for the combination of (R¹)ₘ, (R²)ₙ, R³, R⁴, R⁵ and R⁶ for each individual compound corresponds in each case to one line of Table B (compounds I.K.13k.B-1 to I.K.13k.B-1680).
Table 14k Compounds of the formula I.K in which o is 1, R⁷⁸ is 3"-OCHF₂ and the meaning for the combination of (R¹)ₘ, (R²)ₙ, R³, R⁴, R⁵ and R⁶ for each individual compound corresponds in each case to one line of Table B (compounds I.K.14k.B-1 to I.K.14k.B-1680).
Table 15k Compounds of the formula I.K in which o is 1, R⁷⁸ is 3"-C₆H₅ and the meaning for the combination of (R¹)ₘ, (R²)ₙ, R³, R⁴, R⁵ and R⁶ for each individual compound corresponds in each case to one line of Table B (compounds I.K.15k.B-1 to I.K.15k.B-1680).
Table 16k Compounds of the formula I.K in which o is 1, R⁷⁸ is 4"-F and the meaning for the combination of (R¹)ₘ, (R²)ₙ, R³, R⁴, R⁵ and R⁶ for each individual compound corresponds in each case to one line of Table B (compounds I.K.16k.B-1 to I.K.16k.B-1680).
Table 17k Compounds of the formula I.K in which o is 1, R⁷⁸ is 4"-Cl and the meaning for the combination of (R¹)ₘ, (R²)ₙ, R³, R⁴, R⁵ and R⁶ for each individual compound corresponds in each case to one line of Table B (compounds I.K.17k.B-1 to I.K.17k.B-1680).
Table 18k Compounds of the formula I.K in which o is 1, R⁷⁸ is 4"-Br and the meaning for the combination of (R¹)ₘ, (R²)ₙ, R³, R⁴, R⁵ and R⁶ for each individual compound corresponds in each case to one line of Table B (compounds I.K.18k.B-1 to I.K.18k.B-1680).
Table 19k Compounds of the formula I.K in which o is 1, R⁷⁸ is 4"-CH₃ and the meaning for the combination of (R¹)ₘ, (R²)ₙ, R³, R⁴, R⁵ and R⁶ for each individual compound corresponds in each case to one line of Table B (compounds I.K.19k.B-1 to I.K.19k.B-1680).
Table 20k Compounds of the formula I.K in which o is 1, R⁷⁸ is 4"-OCH₃ and the meaning for the combination of (R¹)ₘ, (R²)ₙ, R³, R⁴, R⁵ and R⁶ for each individual compound corresponds in each case to one line of Table B (compounds I.K.20k.B-1 to I.K.20k.B-1680).
Table 21k Compounds of the formula I.K in which o is 1, R⁷⁸ is 4"-OCHF₂ and the meaning for the combination of (R¹)ₘ, (R²)ₙ, R³, R⁴, R⁵ and R⁶ for each individual compound corresponds in each case to one line of Table B (compounds I.K.21 k.B-1 to I.K.21 k.B-1680).
Table 22k Compounds of the formula I.K in which o is 1, R⁷⁸ is 4"-C₆H₅ and the meaning for the combination of (R¹)ₘ, (R²)ₙ, R³, R⁴, R⁵ and R⁶ for each individual compound corresponds in each case to one line of Table B (compounds I.K.22k.B-1 to I.K.22k.B-1680).

In the Table B the following abbreviations are used: C1= 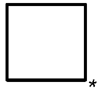 ; C2= 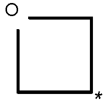 ; Me=CH₃; Et=C₂H₅

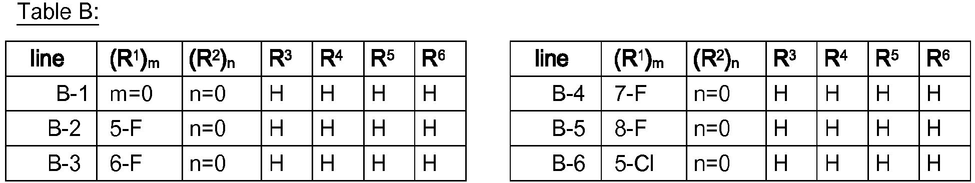

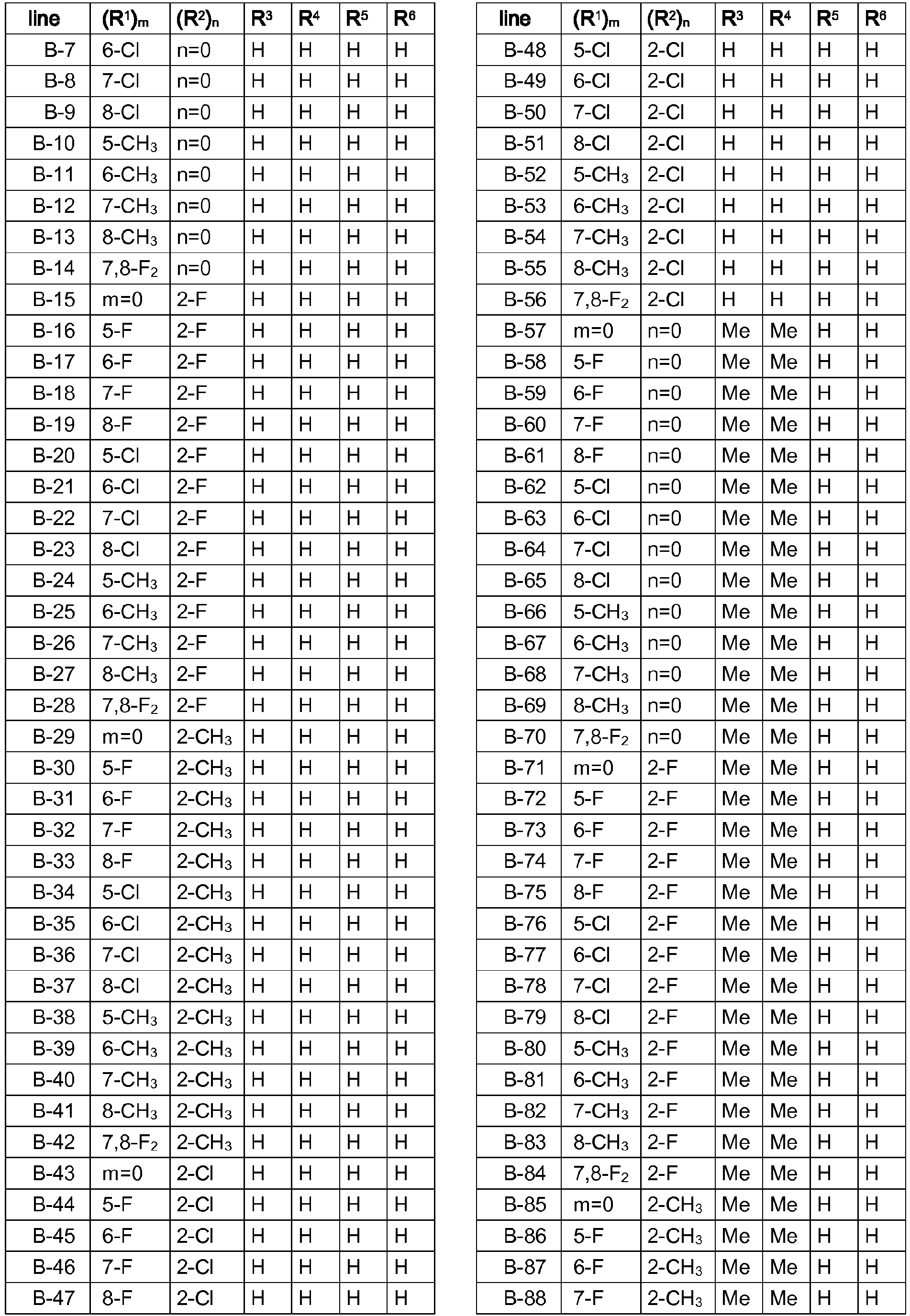

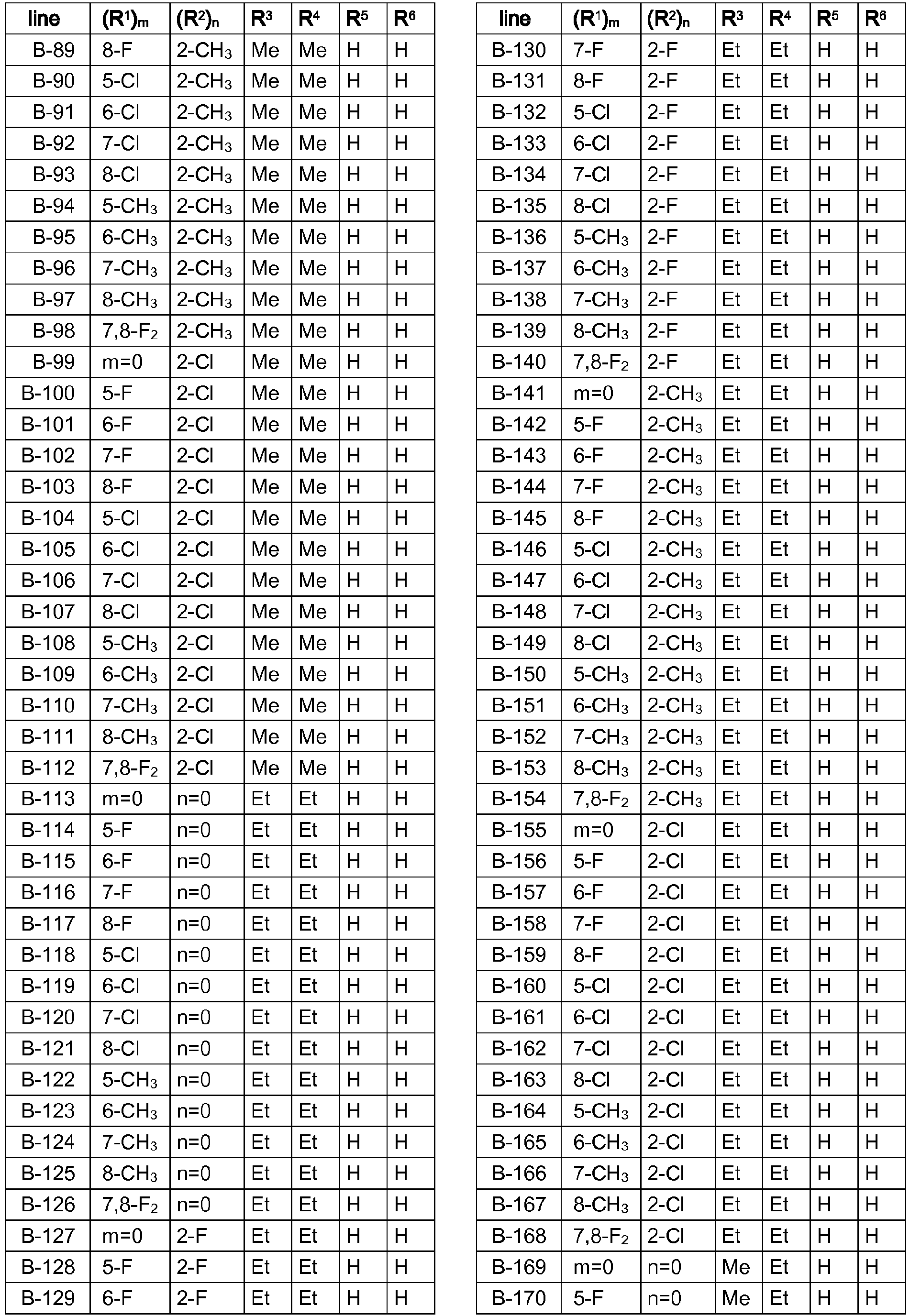

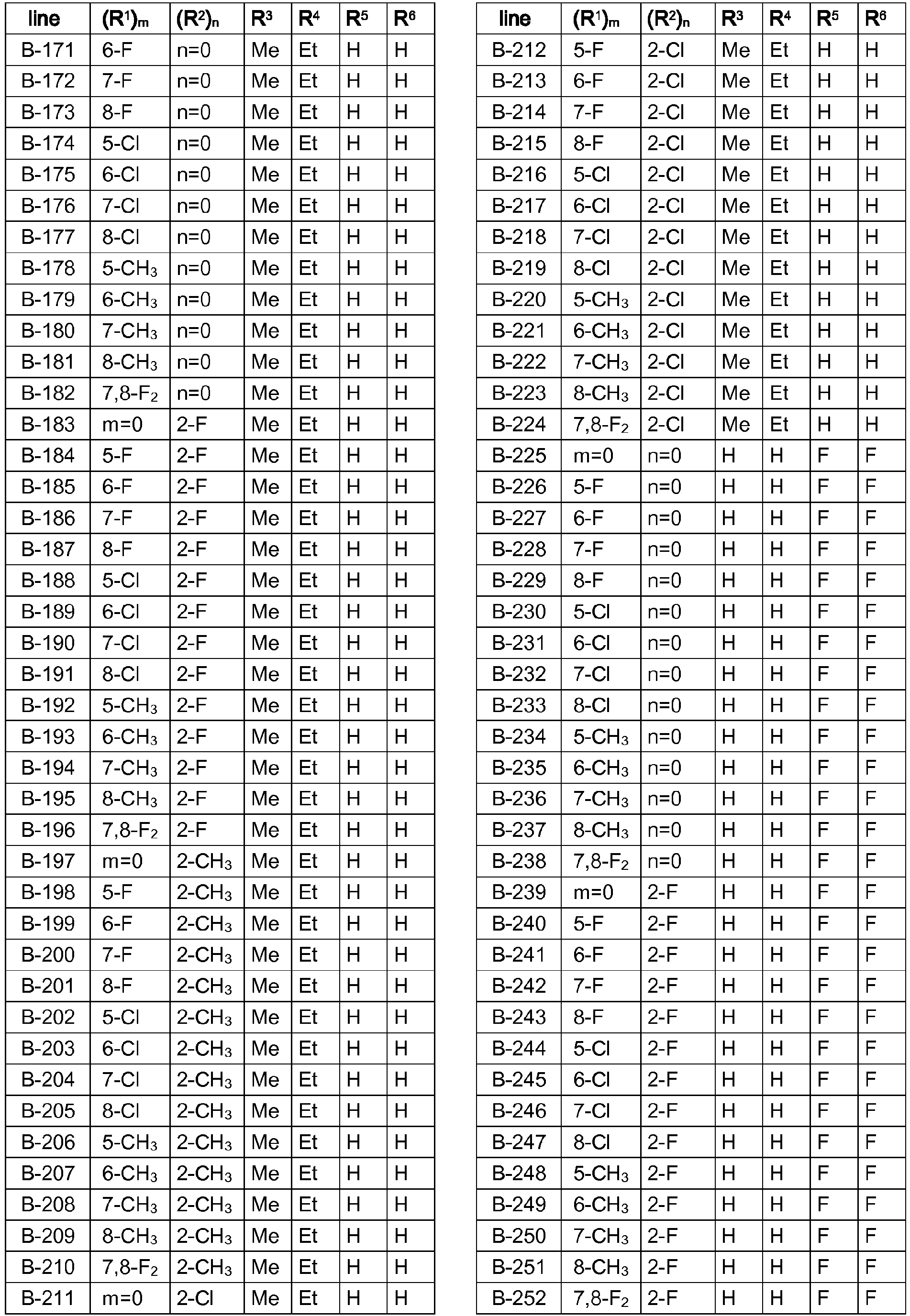

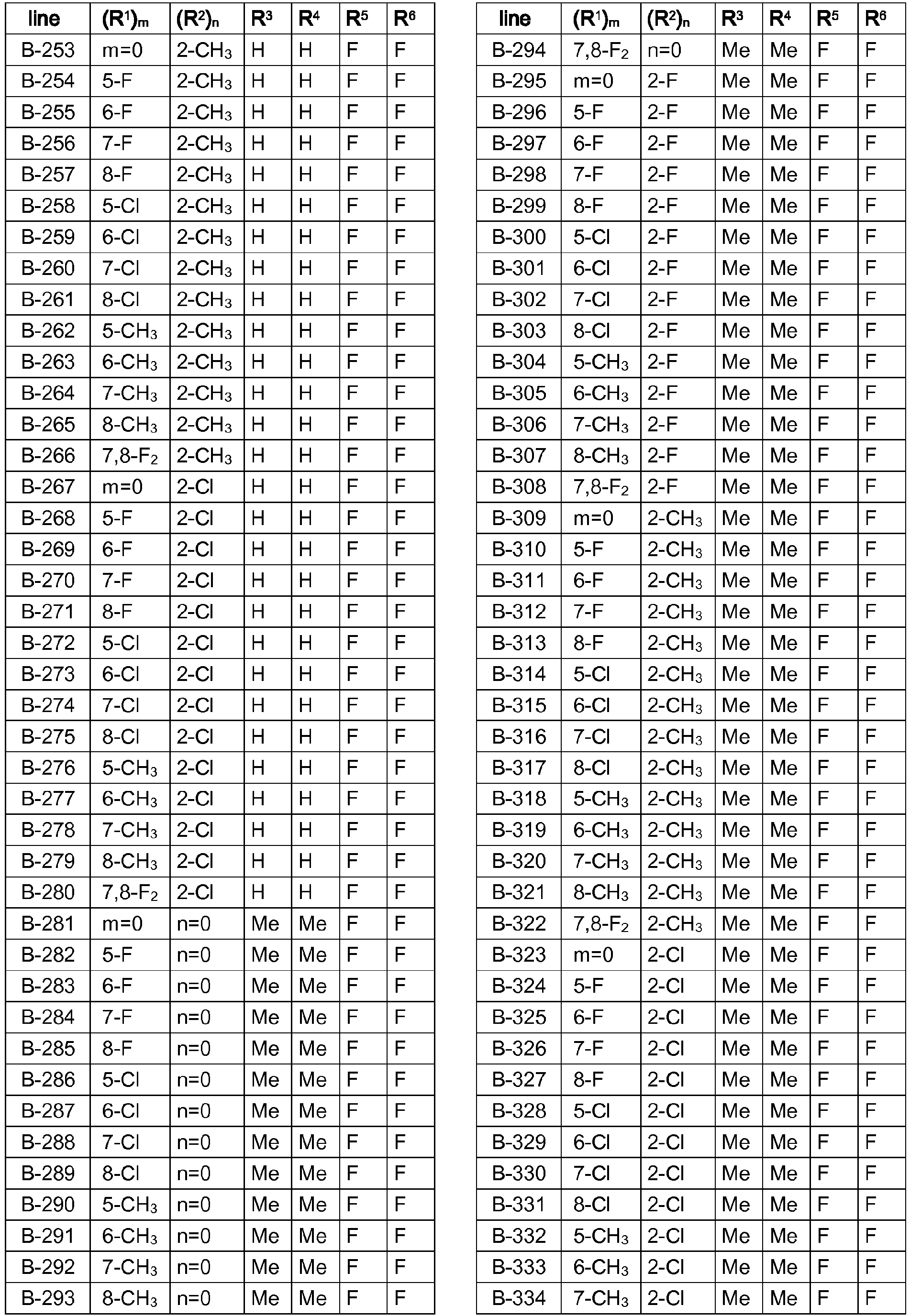

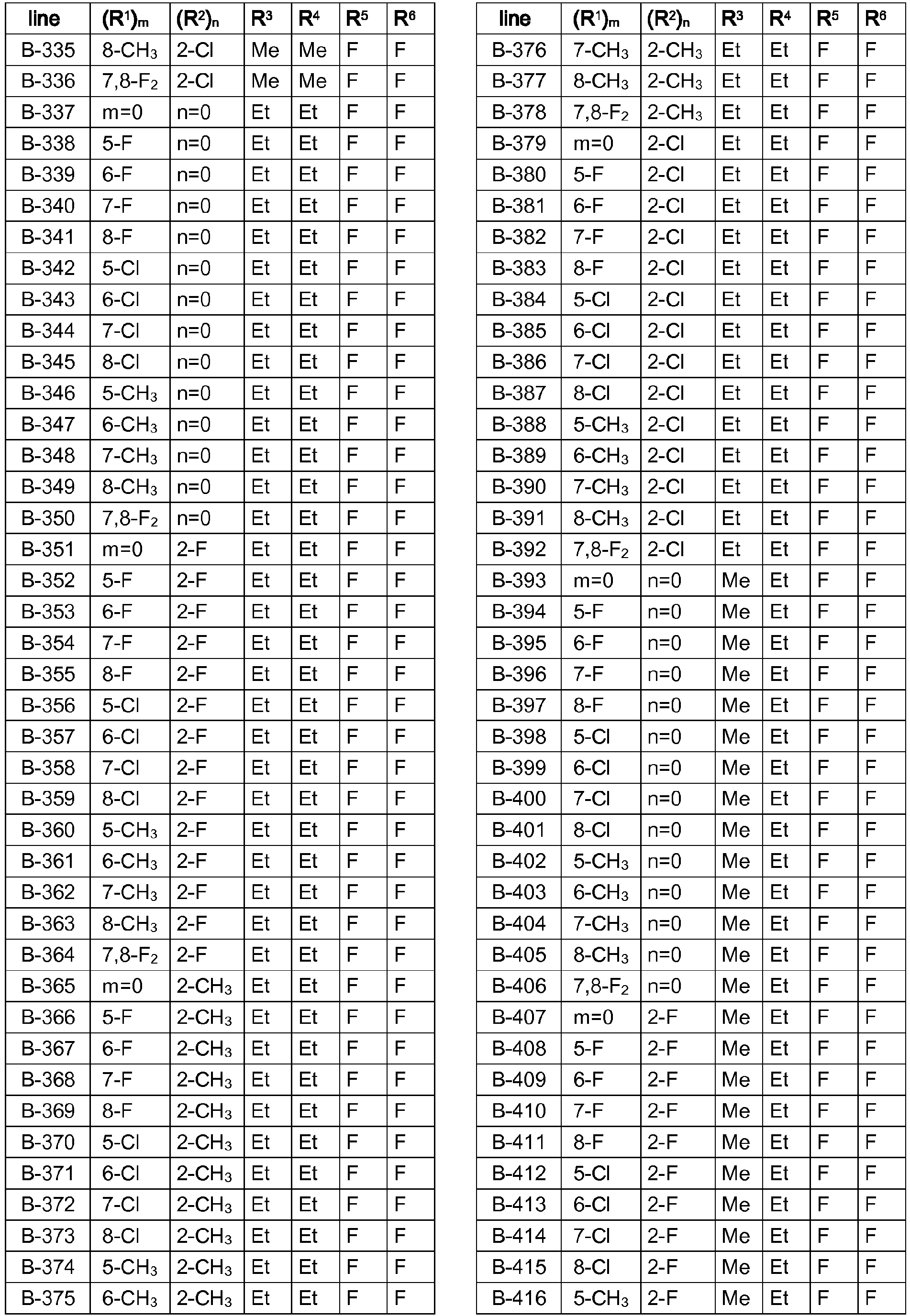

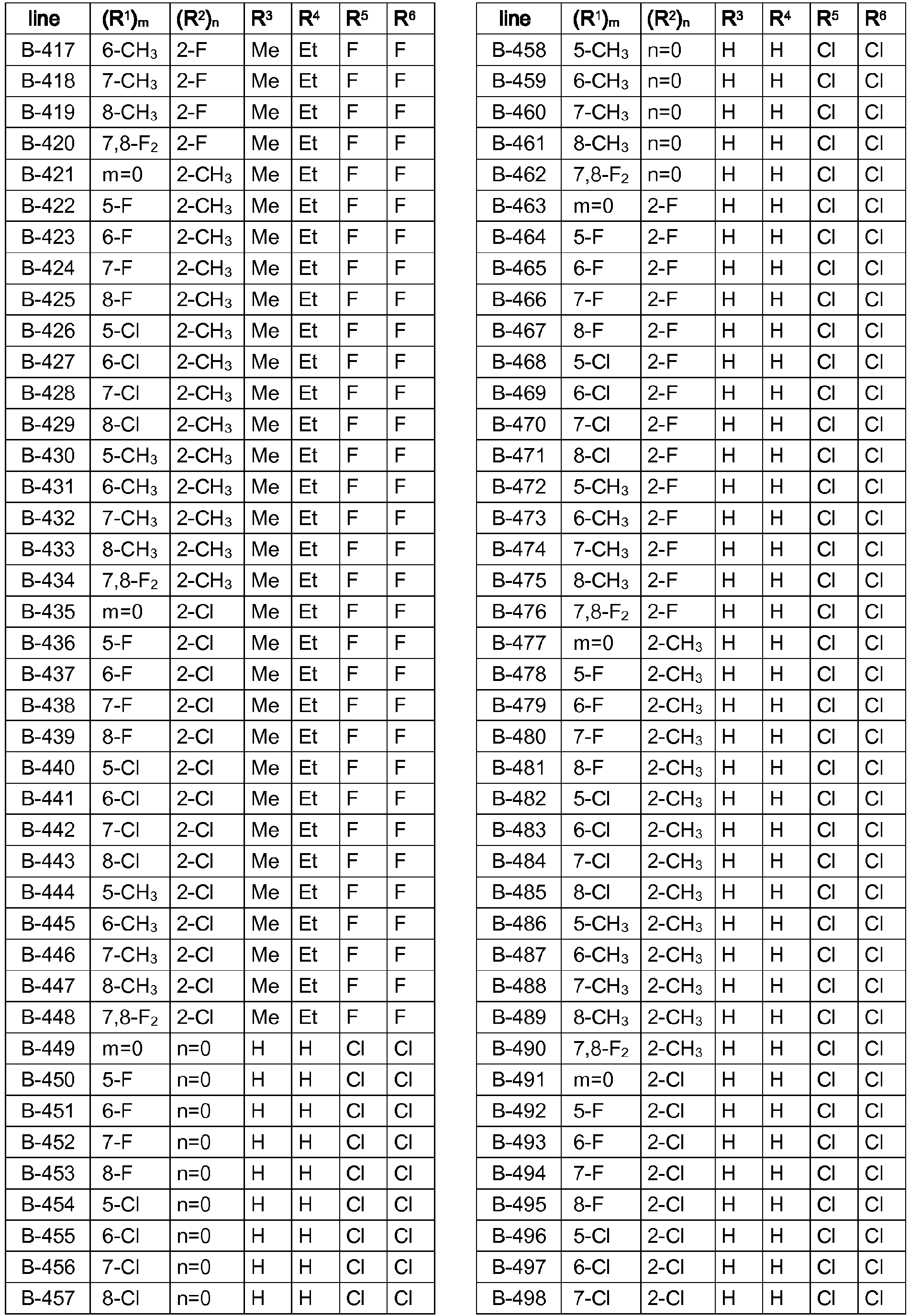

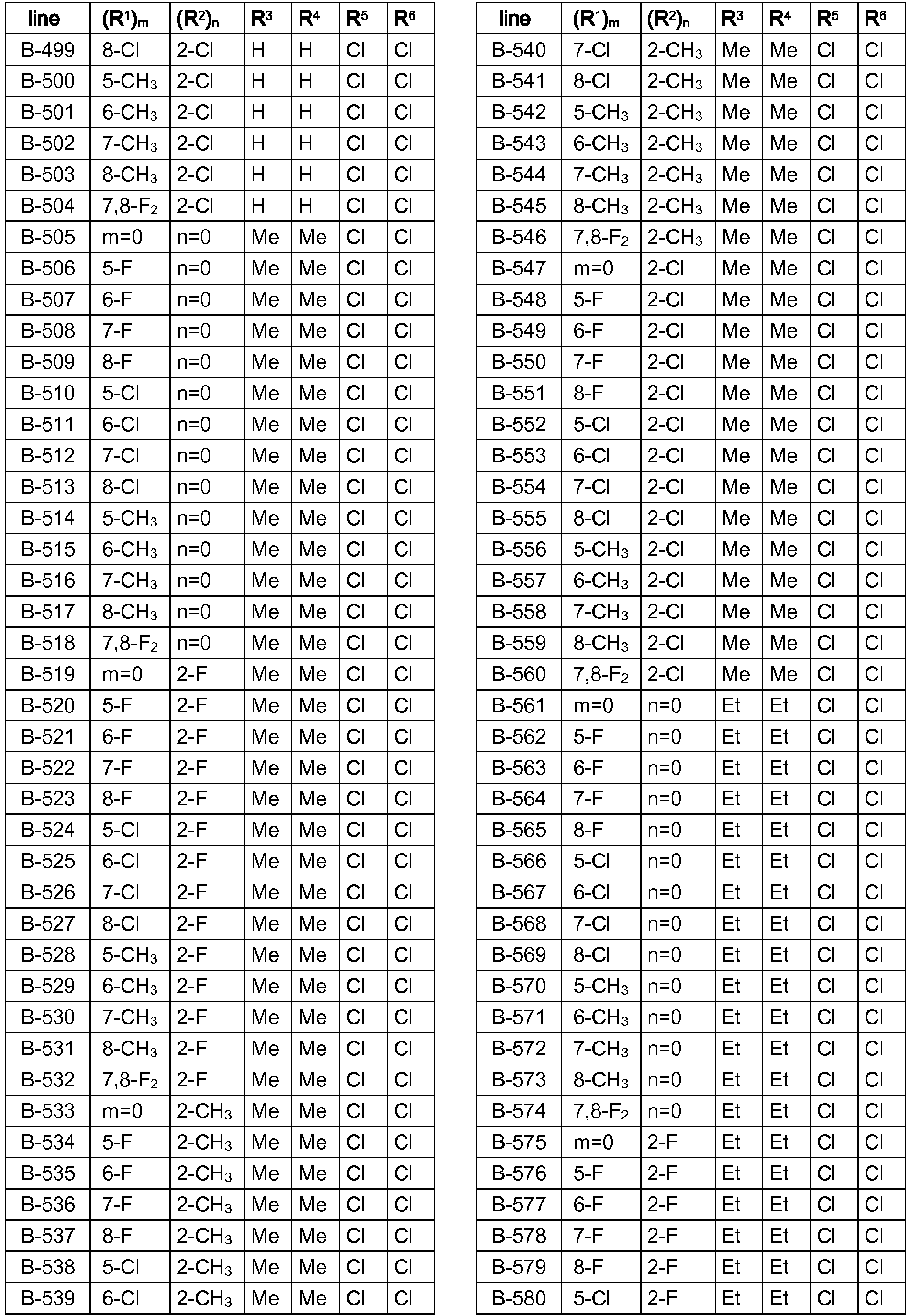

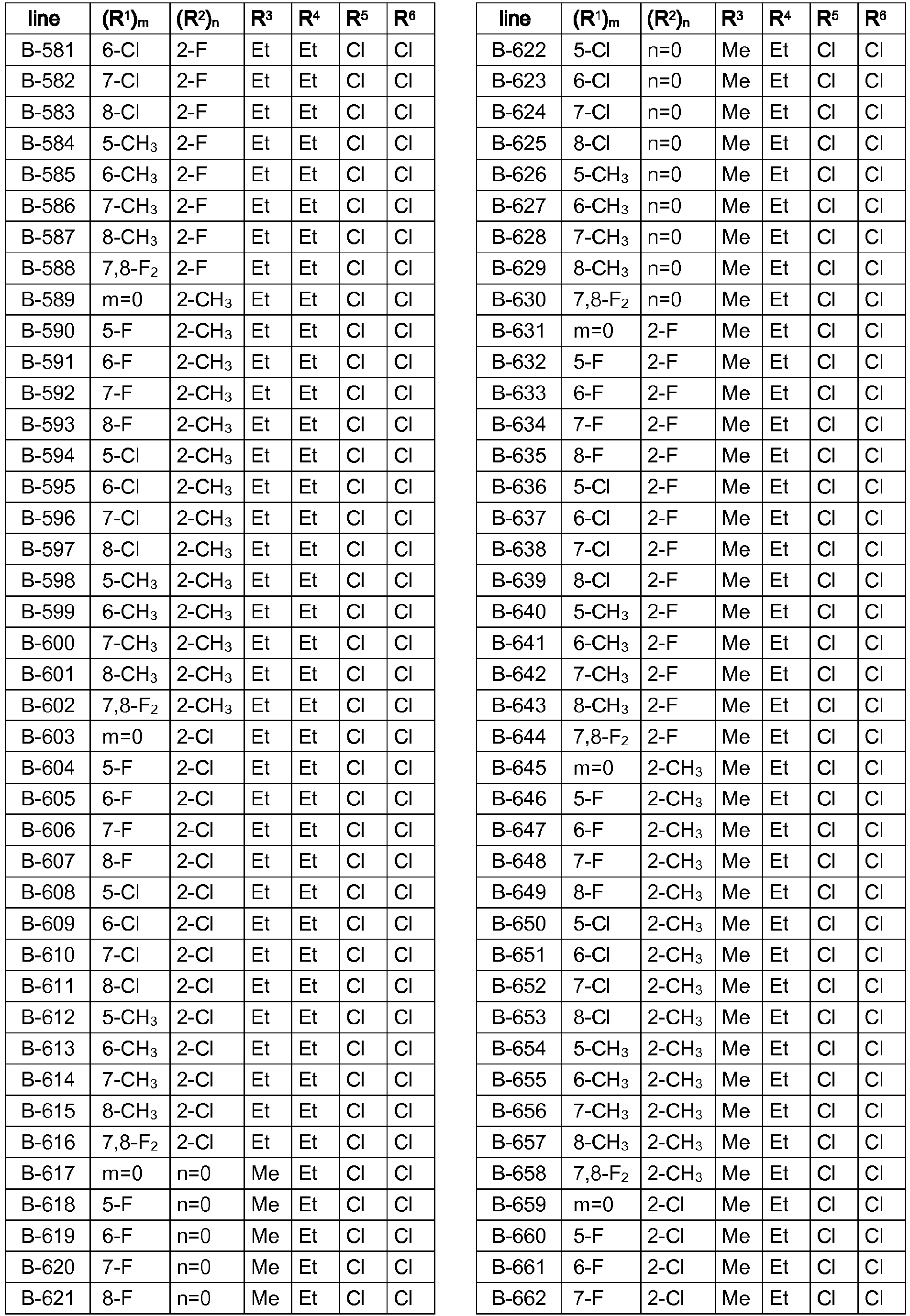

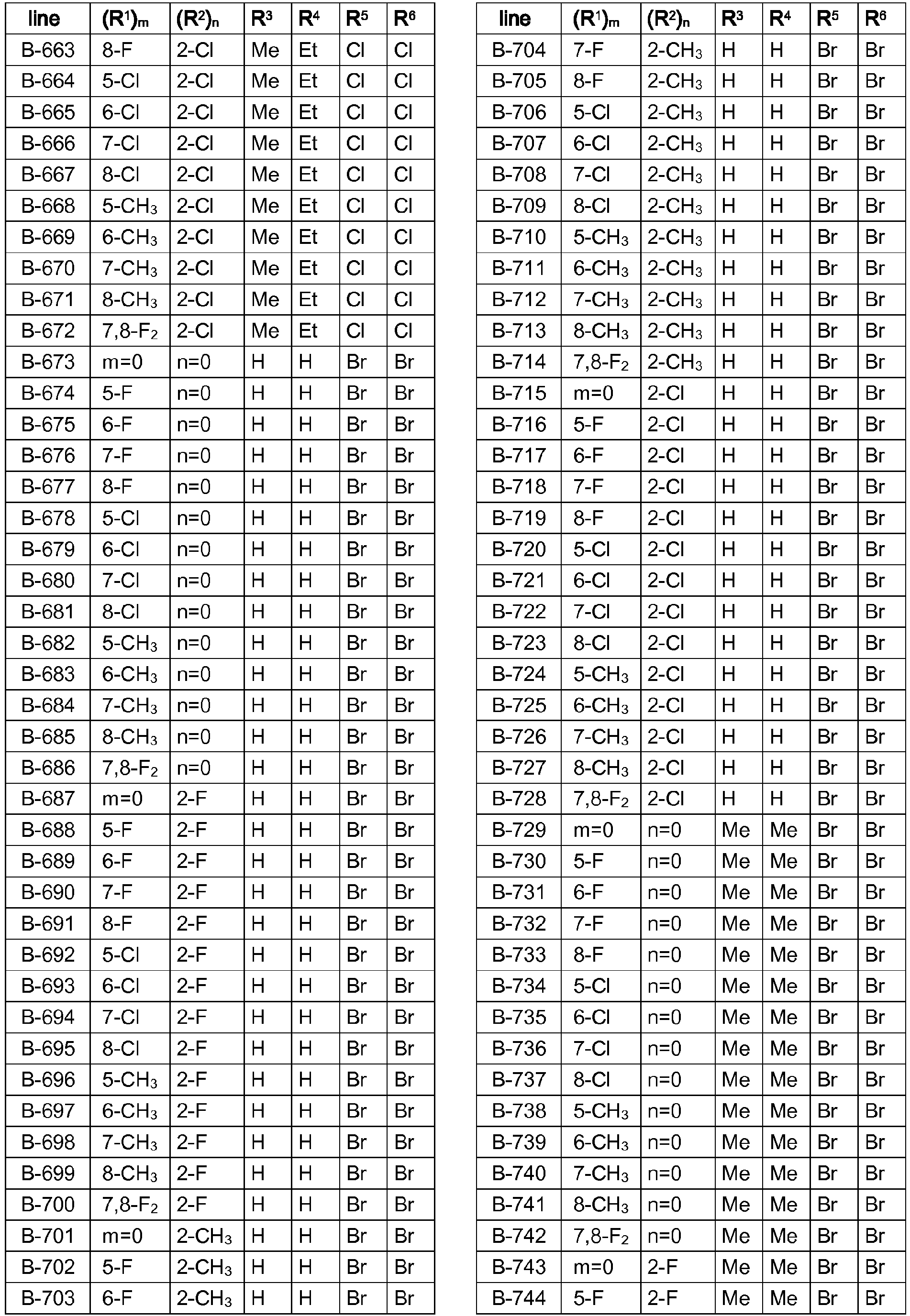

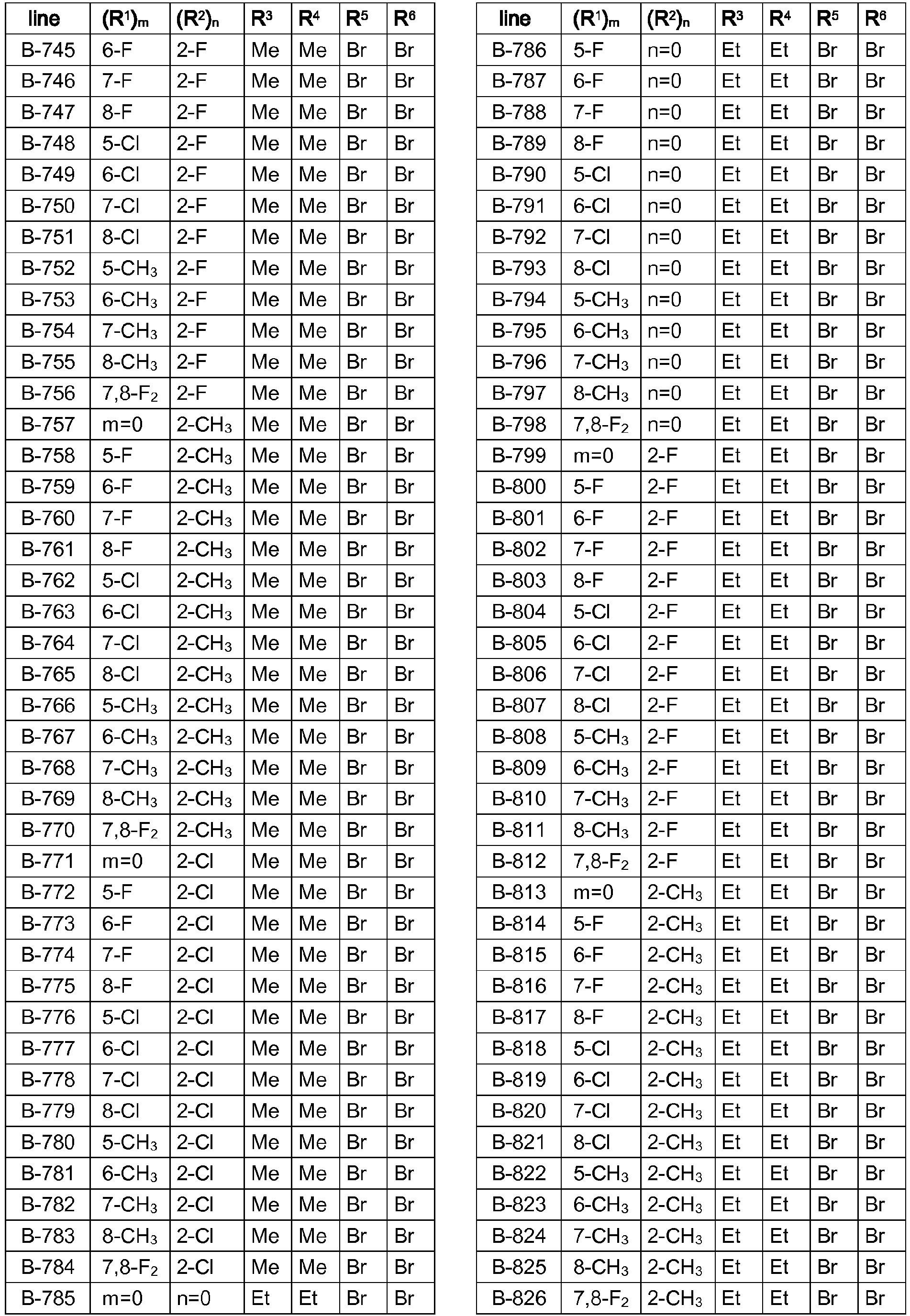

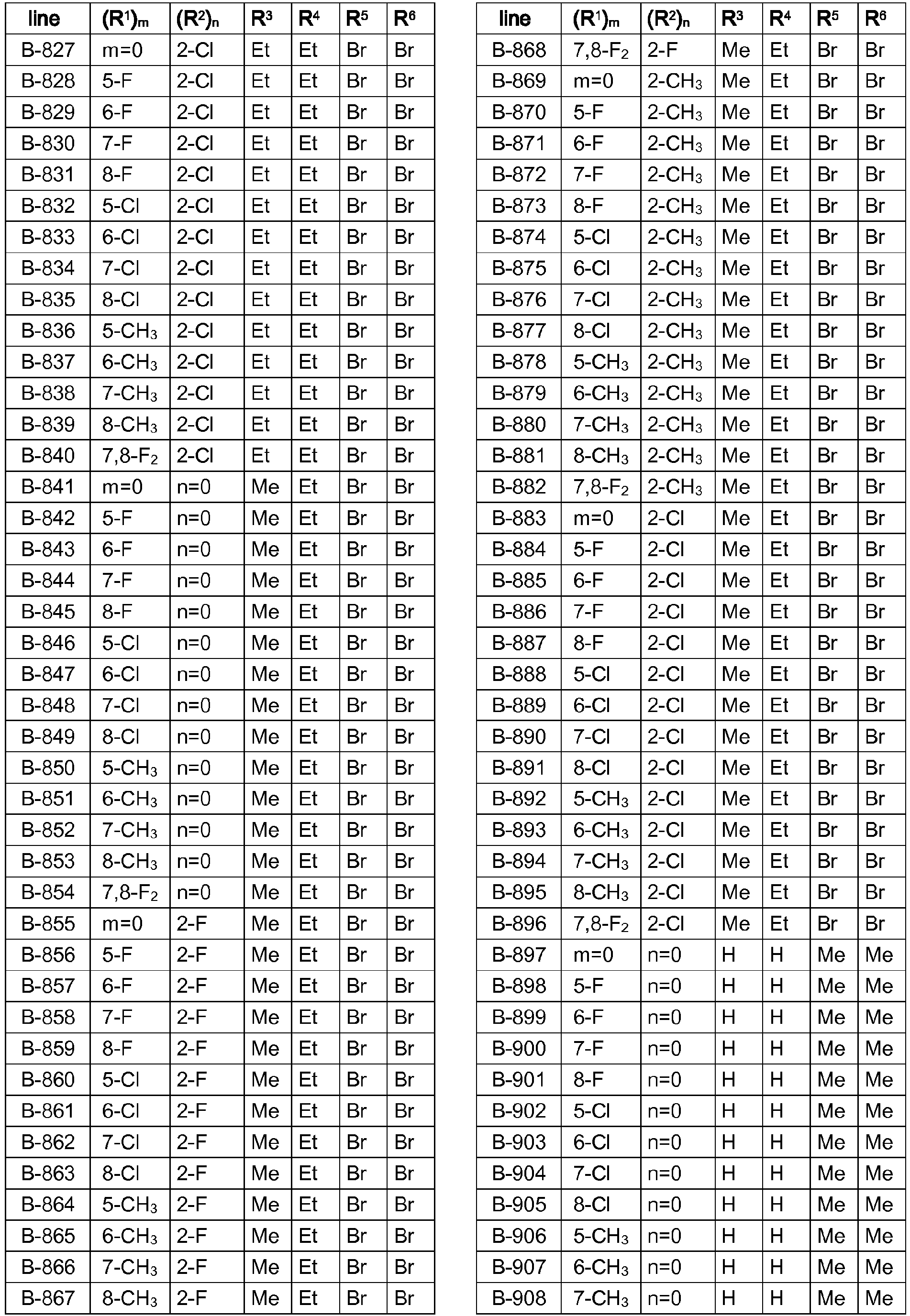

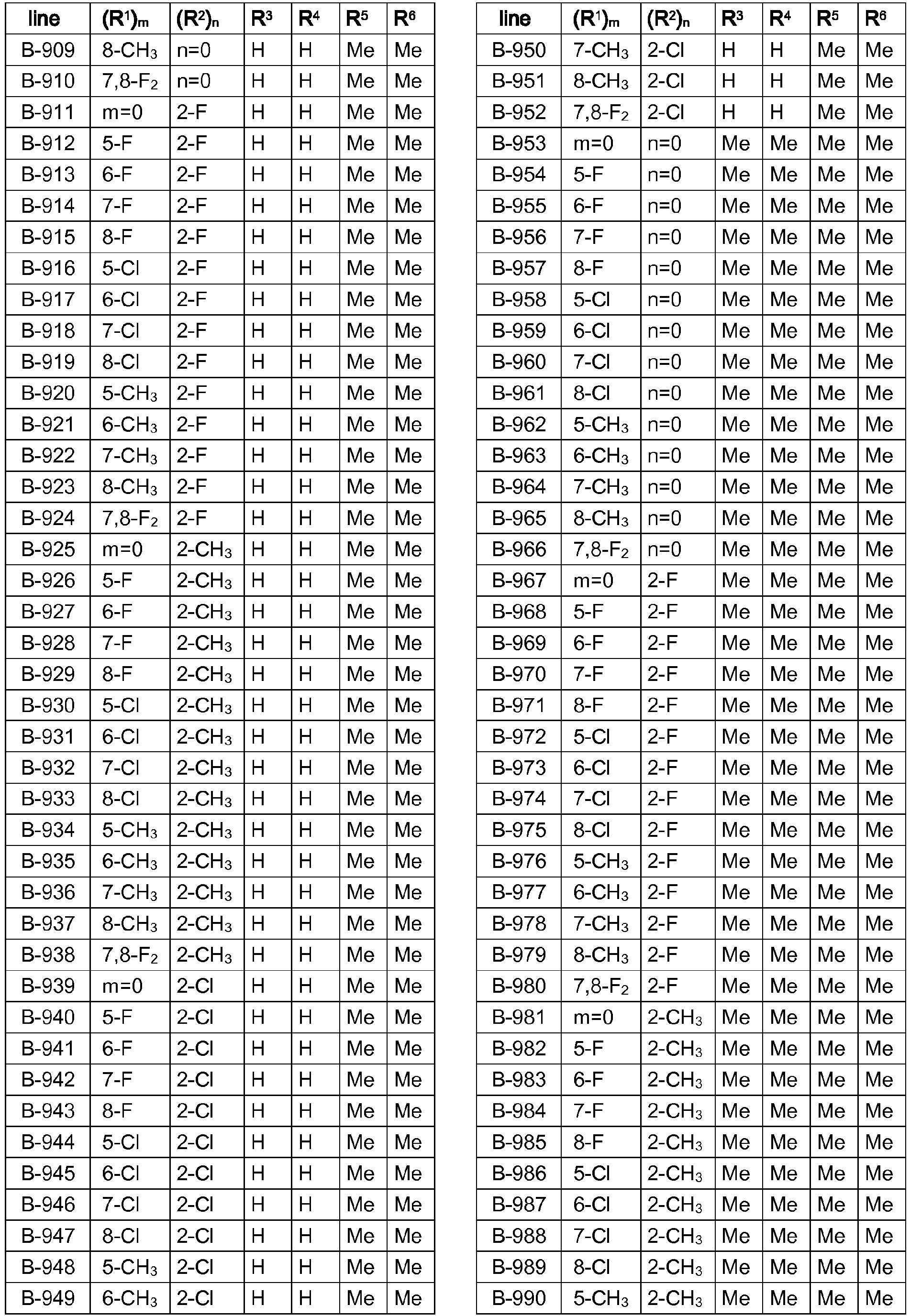

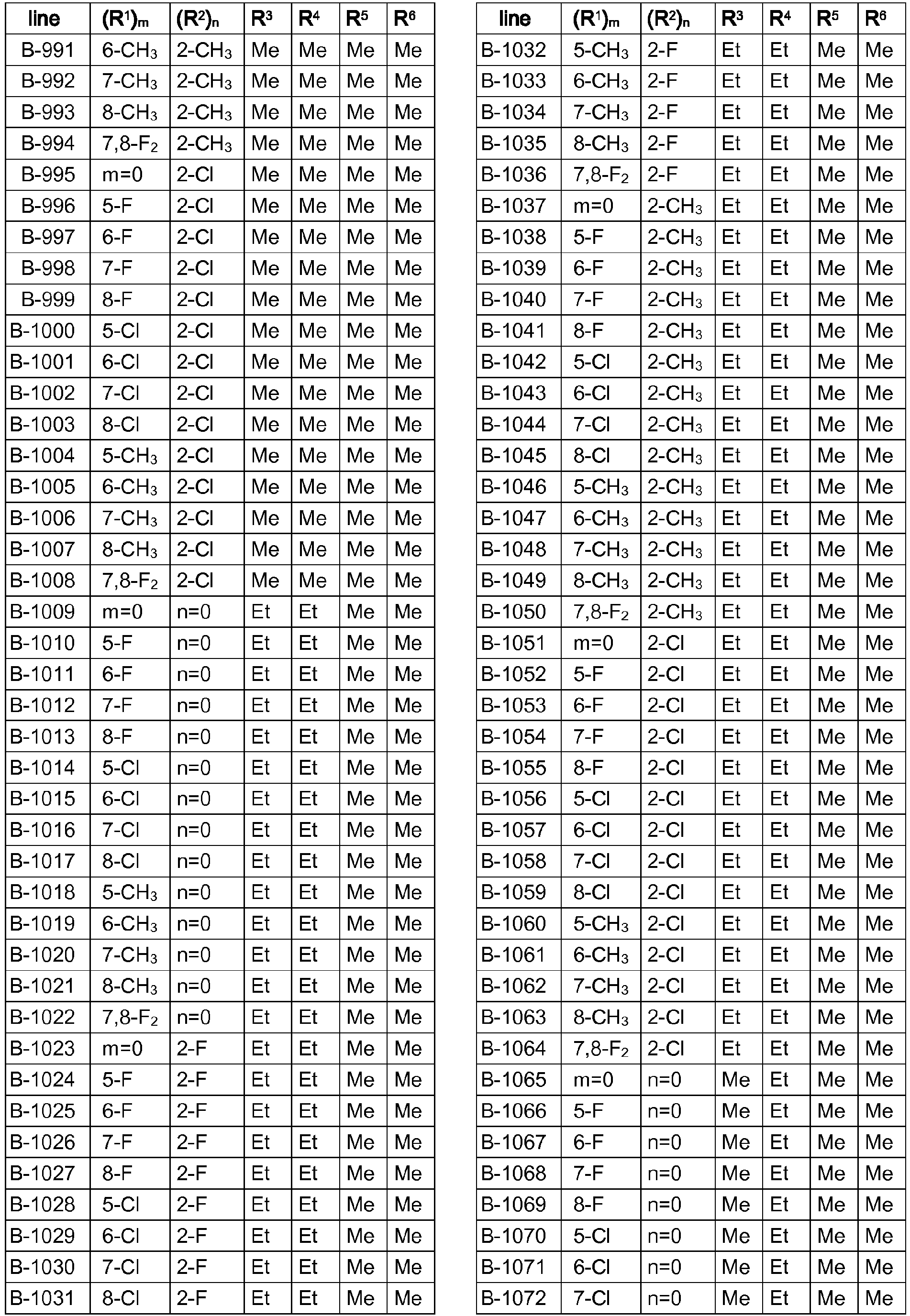

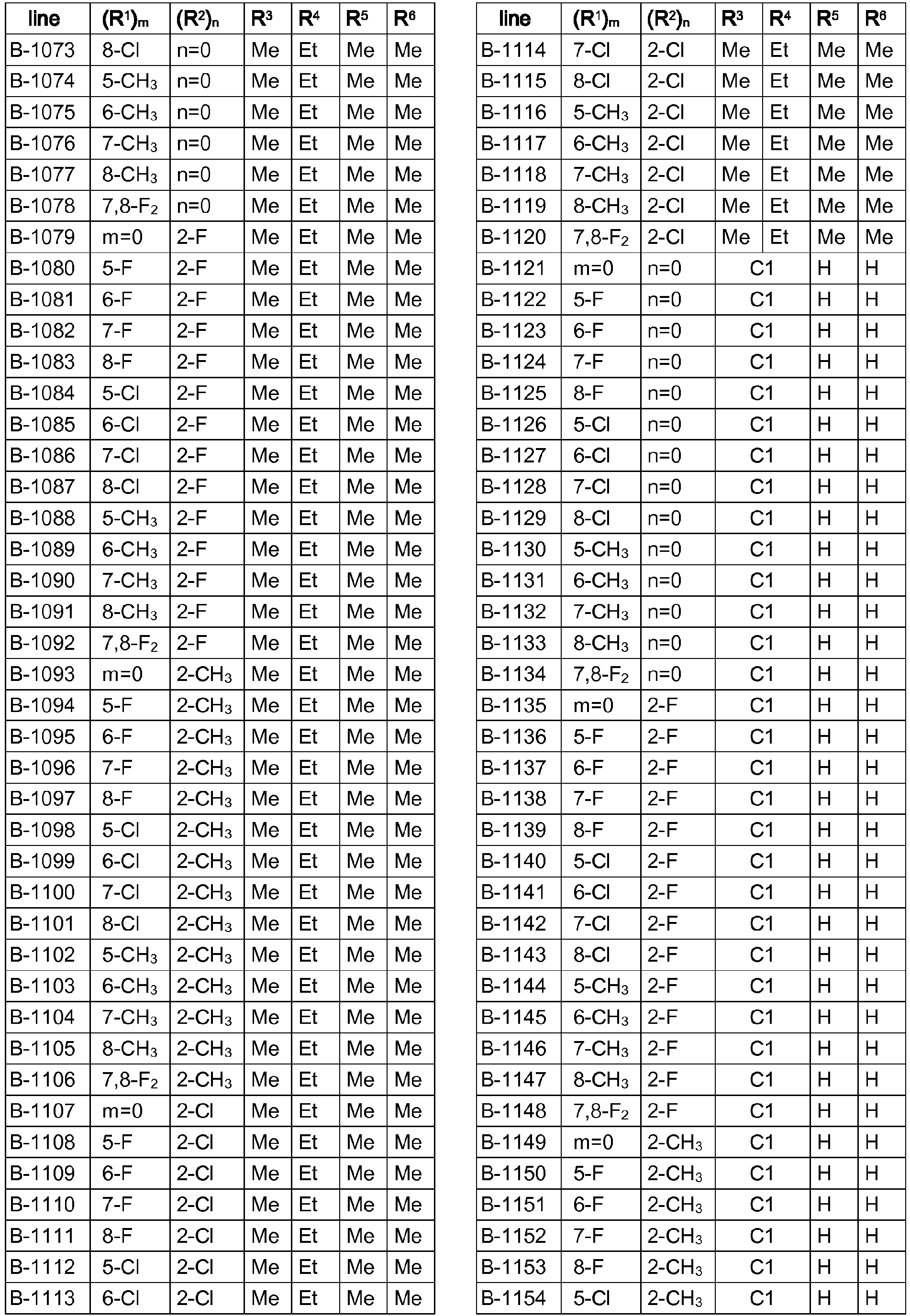

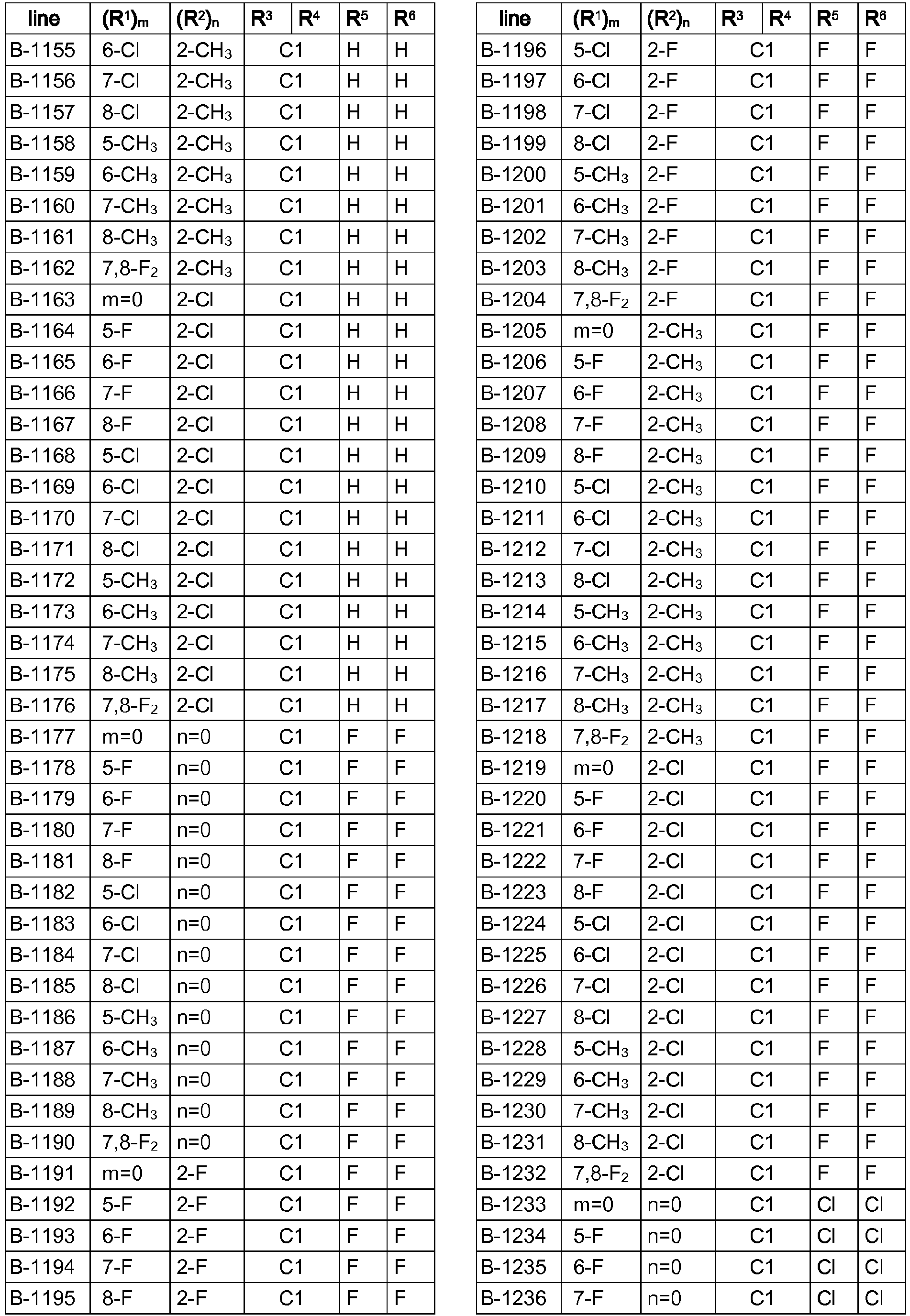

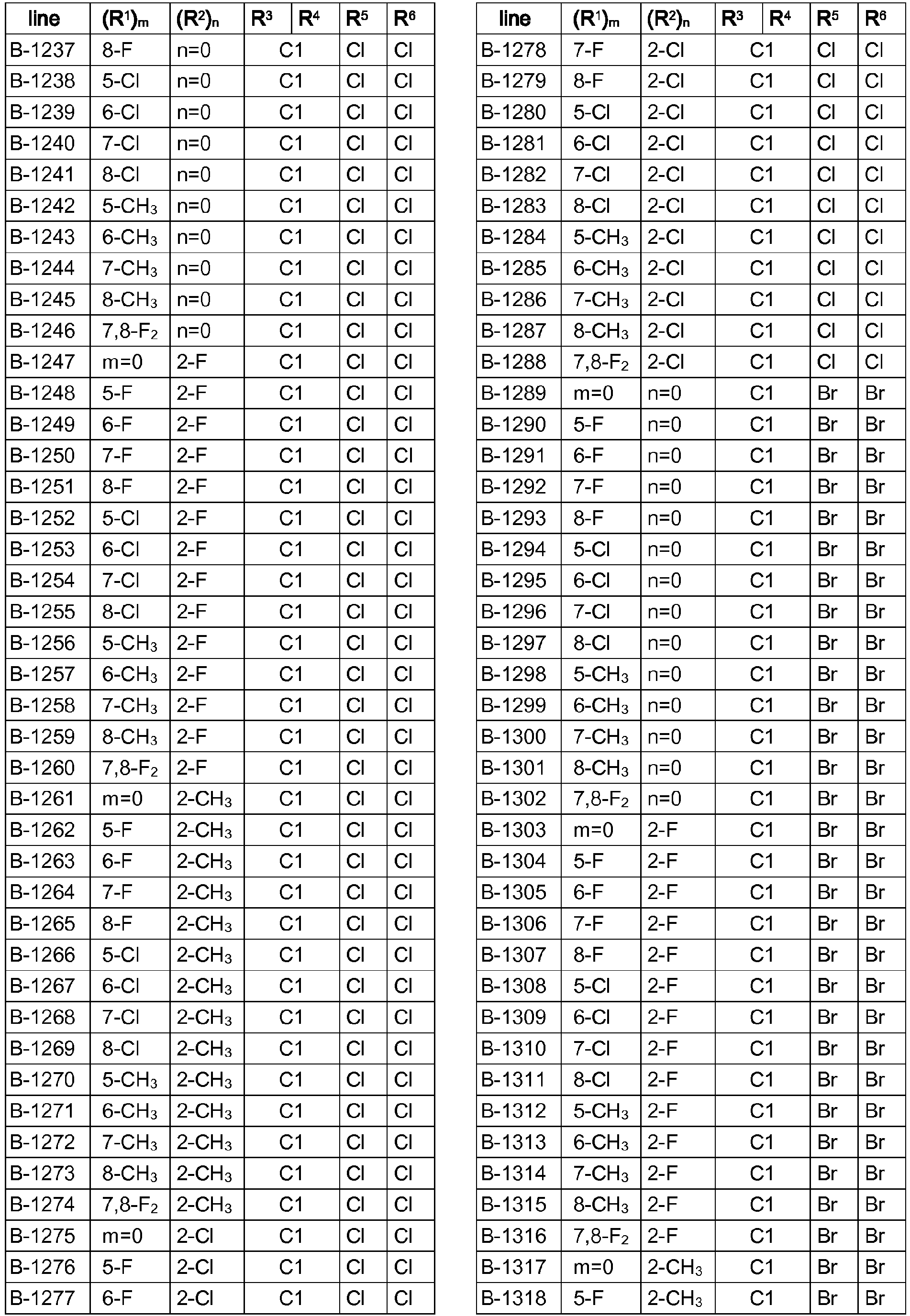

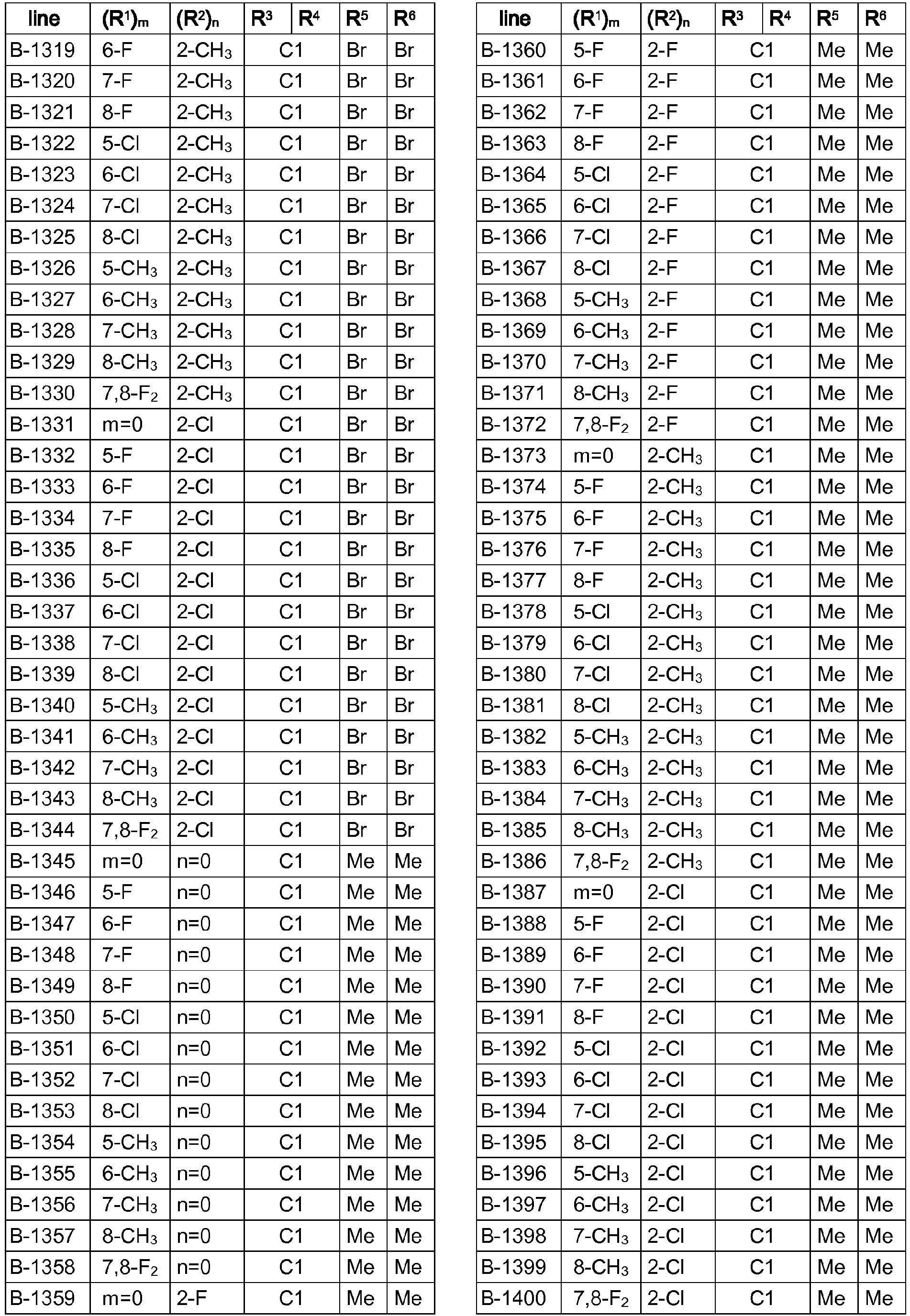

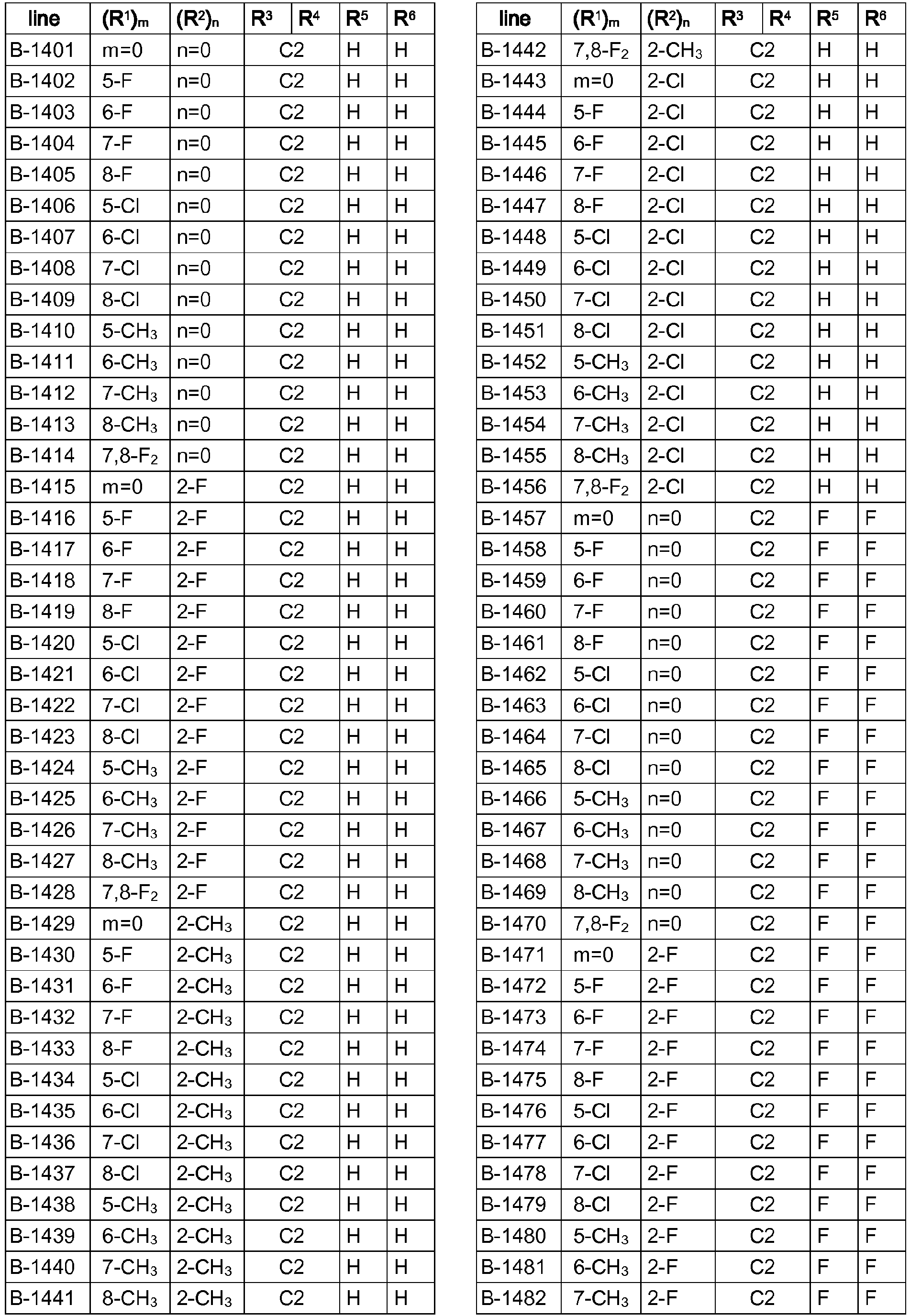

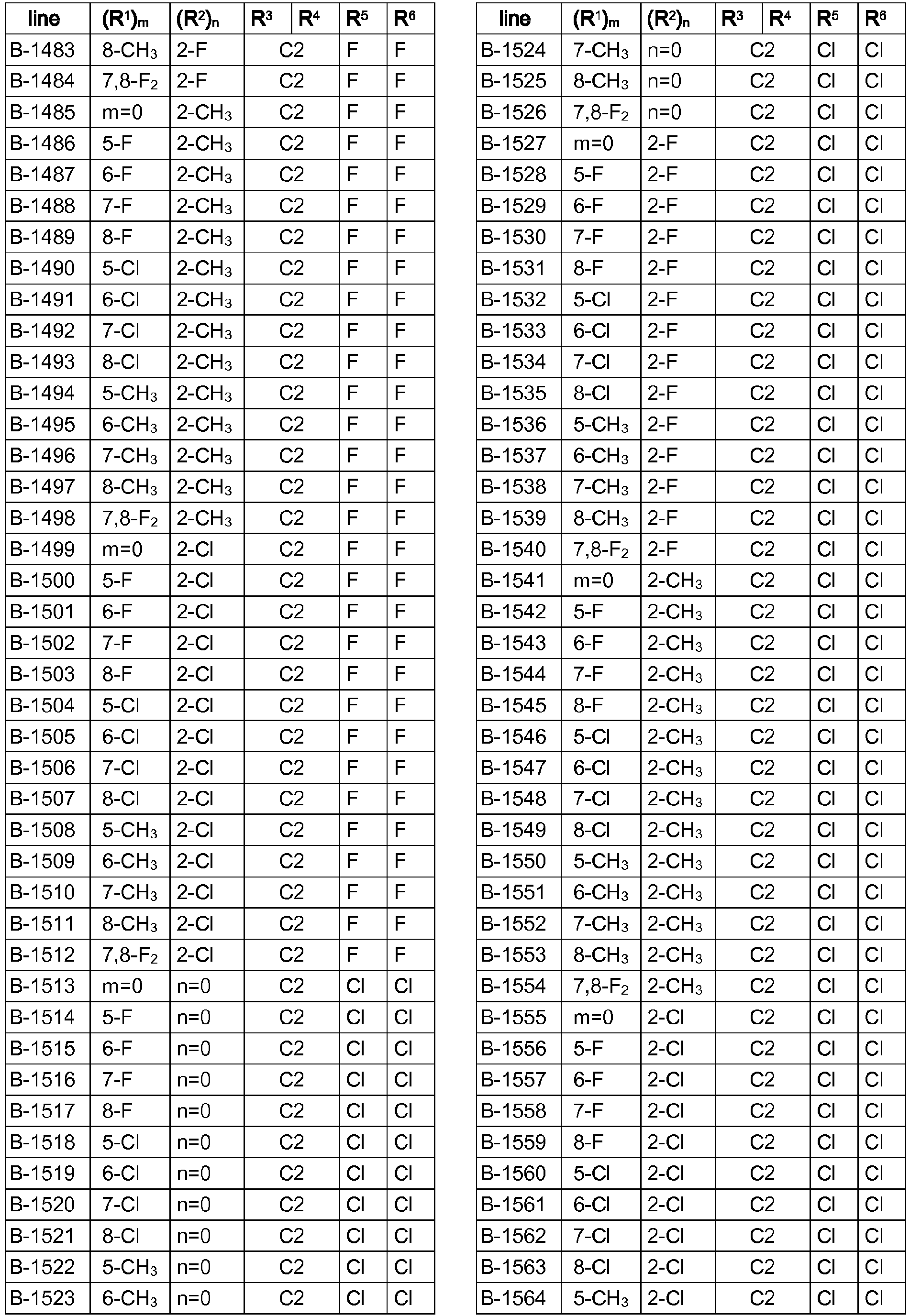

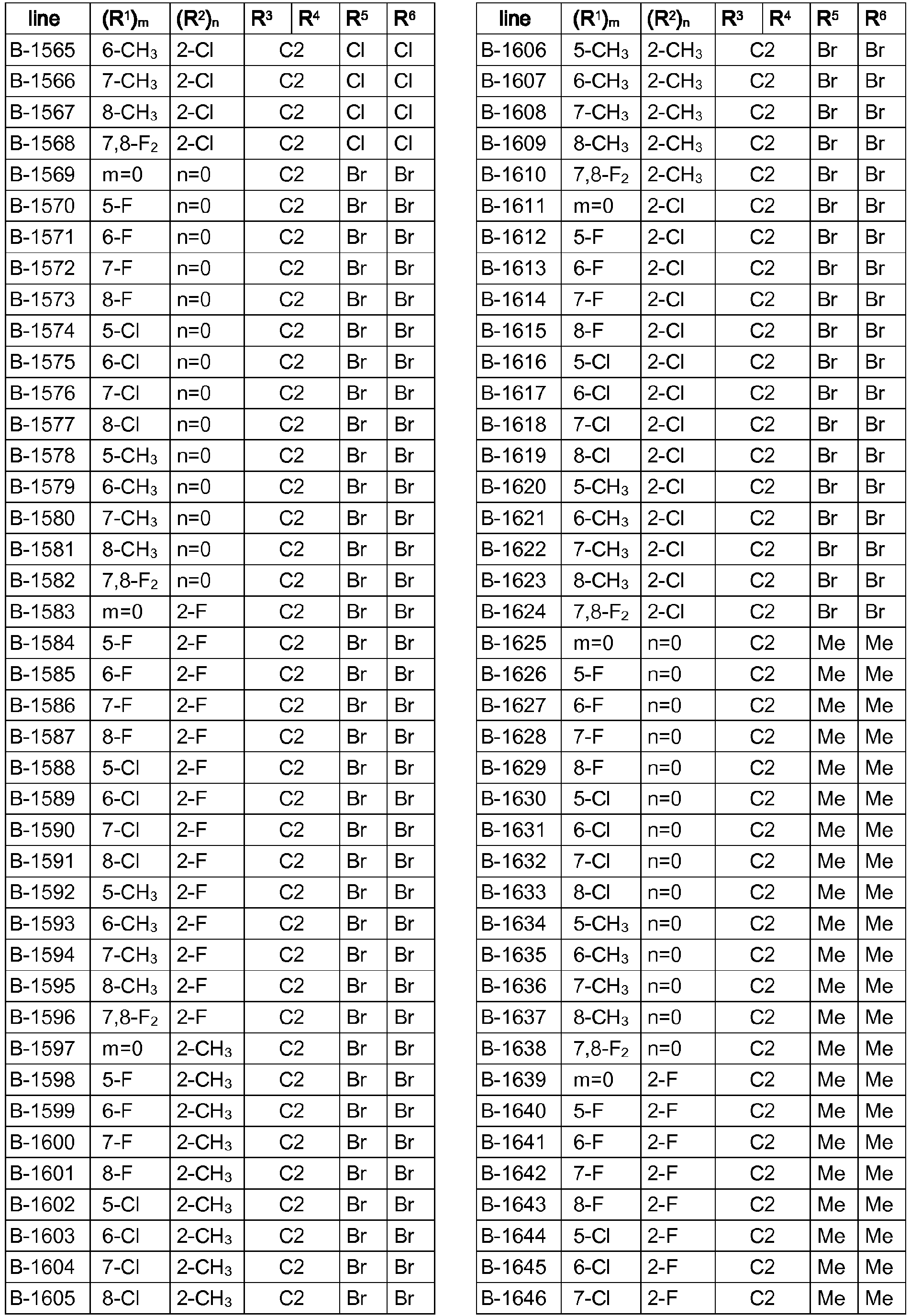

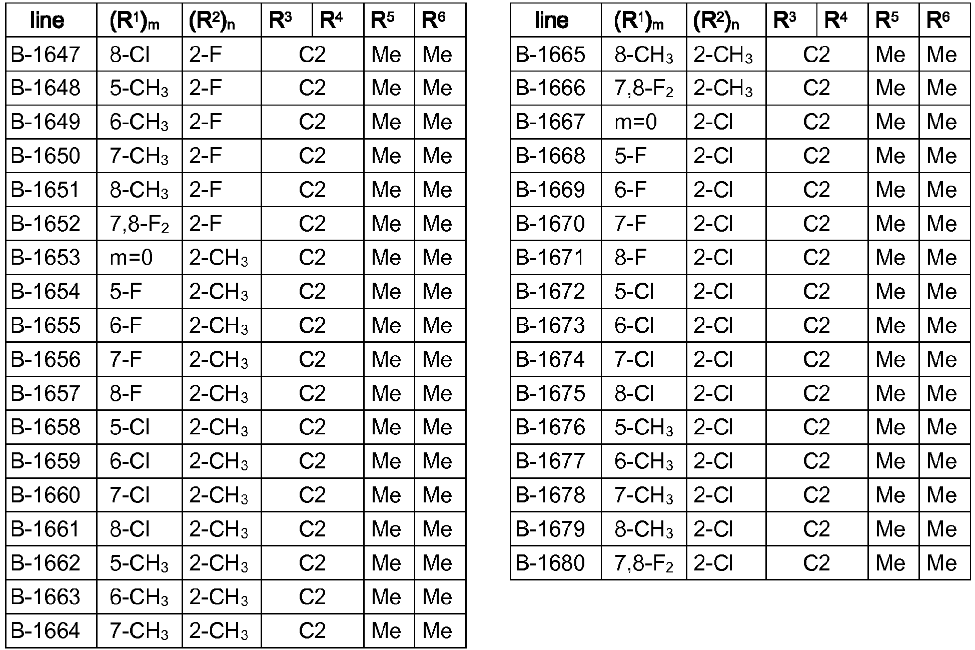

Particular compounds of the formula I according to the present invention are the following compounds I.Aa ,I.Ba and I.Ca:

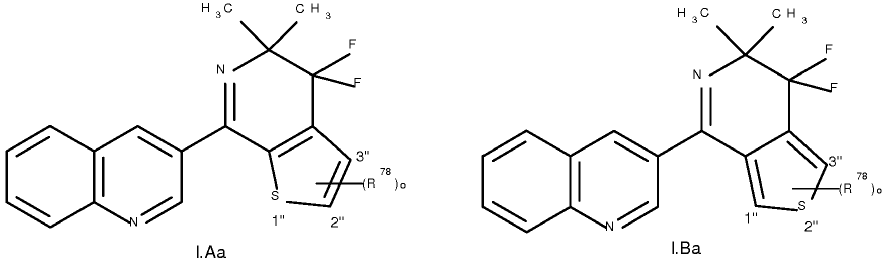

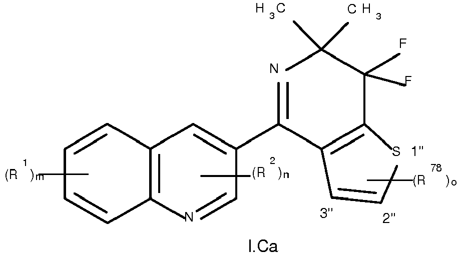

Particular embodiments thereof are compiled in the following Table B, wherein (R⁷⁸)ₒ is specified:

**Table C: Specific compounds I.Aa (I.Aa-1 to I.Aa-5), I.Ba (I.Ba-1) and I.Ca (I-Ca-1 to I.Ca-7):**

| **compound No.** | **(R⁷⁸)ₒ** |
|---|---|
| I.Aa-1 | o=0 |
| I.Aa-2 | 2"-F |
| I.Aa-3 | 2"-Cl |
| I.Aa-4 | 2"-CHF₂ |
| I.Aa-5 | 3"-Cl |
| I.Aa-6 | 3"-F |
| I.Aa-7 | 3"-CHF₂ |
| I.Ba-1 | 3"-Cl |
| I.Ba-2 | o=0 |
| I.Ba-3 | 3"=F |
| I.Ba-4 | 3"=CHF₂ |
| I.Ca-1 | o=0 |
| I.Ca-2 | 2"-F |
| I.Ca-3 | 2"-Cl |
| I.Ca-4 | 2"-CHF₂ |
| I.Ca-5 | 3"-F |
| I.Ca-6 | 3"-Cl |
| I.Ca-7 | 3"-CHF₂ |

The compounds I and the compositions according to the invention, respectively, are suitable as fungicides. They are distinguished by an outstanding effectiveness against a broad spectrum of phytopathogenic fungi, including soil-borne fungi, which derive especially from the classes of the Plasmodiophoromycetes, Peronosporomycetes (syn. Oomycetes), Chytridiomycetes, Zygomycetes, Ascomycetes, Basidiomycetes and Deuteromycetes (syn. Fungi imperfecti). Some are systemically effective and they can be used in crop protection as foliar fungicides, fungicides for seed dressing and soil fungicides. Moreover, they are suitable for controlling harmful fungi, which inter alia occur in wood or roots of plants.

The compounds I and the compositions according to the invention are particularly important in the control of a multitude of phytopathogenic fungi on various cultivated plants, such as cereals, e. g. wheat, rye, barley, triticale, oats or rice; beet, e. g. sugar beet or fodder beet; fruits, such as pomes, stone fruits or soft fruits, e. g. apples, pears, plums, peaches, almonds, cherries, strawberries, raspberries, blackberries or gooseberries; leguminous plants, such as lentils, peas, alfalfa or soybeans; oil plants, such as rape, mustard, olives, sunflowers, coconut, cocoa beans, castor oil plants, oil palms, ground nuts or soybeans; cucurbits, such as squashes, cucumber or melons; fiber plants, such as cotton, flax, hemp or jute; citrus fruit, such as oranges, lemons, grapefruits or mandarins; vegetables, such as spinach, lettuce, asparagus, cabbages, carrots, onions, tomatoes, potatoes, cucurbits or paprika; lauraceous plants, such as avocados, cinnamon or camphor; energy and raw material plants, such as corn, soybean, rape, sugar cane or oil palm; corn; tobacco; nuts; coffee; tea; bananas; vines (table grapes and grape juice grape vines); hop; turf; sweet leaf (also called Stevia); natural rubber plants or ornamental and forestry plants, such as flowers, shrubs, broad-leaved trees or evergreens, e. g. conifers; and on the plant propagation material, such as seeds, and the crop material of these plants.

Preferably, compounds I and compositions thereof, respectively are used for controlling a multitude of fungi on field crops, such as potatoes sugar beets, tobacco, wheat, rye, barley, oats, rice, corn, cotton, soybeans, rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. These young plants may also be protected before transplantation by a total or partial treatment by immersion or pouring.

Preferably, treatment of plant propagation materials with compounds I and compositions thereof, respectively, is used for controlling a multitude of fungi on cereals, such as wheat, rye, barley and oats; rice, corn, cotton and soybeans.

The term "cultivated plants" is to be understood as including plants which have been modified by breeding, mutagenesis or genetic engineering including but not limiting to agricultural biotech products on the market or in development (cf. http://cera-gmc.org/, see GM crop database therein). Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-translational modification of protein(s), oligo- or polypeptides e. g. by glycosylation or polymer additions such as prenylated, acetylated or farnesylated moieties or PEG moieties.

Plants that have been modified by breeding, mutagenesis or genetic engineering, e. g. have been rendered tolerant to applications of specific classes of herbicides, such as auxin herbicides such as dicamba or 2,4-D; bleacher herbicides such as hydroxylphenylpyruvate dioxy-genase (HPPD) inhibitors or phytoene desaturase (PDS) inhibitors; acetolactate synthase (ALS) inhibitors such as sulfonyl ureas or imidazolinones; enolpyruvylshikimate-3-phosphate synthase (EPSPS) inhibitors, such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering. Furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are e. g. described in Pest Managem. Sci. 61, 2005, 246; 61, 2005, 258; 61, 2005, 277; 61, 2005, 269; 61, 2005, 286; 64, 2008, 326; 64, 2008, 332; Weed Sci. 57, 2009, 108; Austral. J. Agricult. Res. 58, 2007, 708; Science 316, 2007, 1185; and references quoted therein. Several cultivated plants have been rendered tolerant to herbicides by conventional methods of breeding (mutagenesis), e. g. Clearfield® summer rape (Canola, BASF SE, Germany) being tolerant to imidazolinones, e. g. imazamox, or ExpressSun® sunflowers (DuPont, USA) being tolerant to sulfonyl ureas, e. g. tribenuron. Genetic engineering methods have been used to render cultivated plants such as soybean, cotton, corn, beets and rape, tolerant to herbicides such as glyphosate and glufosinate, some of which are commercially available under the trade names RoundupReady® (glyphosate-tolerant, Monsanto, U.S.A.), Cultivance® (imidazolinone tolerant, BASF SE, Germany) and LibertyLink® (glufosinate-tolerant, Bayer CropScience, Germany).

Furthermore, plants are also covered that are by the use of recombinant DNA techniques capable to synthesize one or more insecticidal proteins, especially those known from the bacterial genus *Bacillus*, particularly from *Bacillus thuringiensis*, such as δ-endotoxins, e. g. CryIA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. or *Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdyster-oid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilbene synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of arthropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard® (corn cultivars producing the Cry1Ab toxin), YieldGard® Plus (corn cultivars producing Cry1Ab and Cry3Bb1 toxins), Starlink® (corn cultivars producing the Cry9c toxin), Herculex® RW (corn cultivars producing Cry34Ab1, Cry35Ab1 and the enzyme phosphinothricin-N-acetyltransferase [PAT]); NuCOTN® 33B (cotton cultivars producing the Cry1Ac toxin), Bollgard® I (cotton cultivars producing the Cry1Ac toxin), Bollgard® II (cotton cultivars producing Cry1Ac and Cry2Ab2 toxins); VIPCOT® (cotton cultivars producing a VIP-toxin); NewLeaf® (potato cultivars producing the Cry3A toxin); Bt-Xtra®, NatureGard®, KnockOut®, BiteGard®, Protecta®, Bt11 (e. g. Agrisure® CB) and Bt176 from Syngenta Seeds SAS, France, (corn cultivars producing the Cry1Ab toxin and PAT enyzme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e. g. EP-A 392 225), plant disease resistance genes (e. g. potato cultivars, which express resistance genes acting against *Phytophthora infestans* derived from the Mexican wild potato *Solanum bulbocastanum*) or T4-lysozym (e. g. potato cultivars capable of synthesizing these proteins with increased resistance against bacteria such as *Erwinia amylvora*). The methods for producing such genetically modified plants are generally known to the person skilled in the art and are described, e. g. in the publications mentioned above.

Furthermore, plants are also covered that are by the use of recombinant DNA techniques capable to synthesize one or more proteins to increase the productivity (e. g. bio mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of substances of content or new substances of content, specifically to improve human or animal nutrition, e. g. oil crops that produce health-promoting long-chain omega-3 fatty acids or unsaturated omega-9 fatty acids (e. g. Nexera® rape, DOW Agro Sciences, Canada).

Furthermore, plants are also covered that contain by the use of recombinant DNA techniques a modified amount of substances of content or new substances of content, specifically to improve raw material production, e. g. potatoes that produce increased amounts of amylopectin (e. g. Amflora® potato, BASF SE, Germany).

The compounds I and compositions thereof, respectively, are particularly suitable for controlling the following plant diseases:
*Albugo* spp. (white rust) on ornamentals, vegetables (e. g. *A. candida*) and sunflowers (e. g. *A. tragopogonis*); *Alternaria* spp. (Alternaria leaf spot) on vegetables, rape (*A. brassicola* or *brassicae*), sugar beets (*A. tenuis*), fruits, rice, soybeans, potatoes (e. g. *A. solani* or *A. alternata*), tomatoes (e. g. *A. solani* or *A. alternata*) and wheat; *Aphanomyces* spp. on sugar beets and vegetables; *Ascochyta* spp. on cereals and vegetables, e. g. *A. tritici* (anthracnose) on wheat and *A. hordei* on barley; *Bipolaris* and *Drechslera* spp. (teleomorph: *Cochliobolus* spp.), e. g. Southern leaf blight (*D. maydis)* or Northern leaf blight (*B. zeicola*) on corn, e. g. spot blotch (*B. sorokiniana*) on cereals and e. g. *B. oryzae* on rice and turfs; *Blumeria* (formerly *Erysiphe*) *graminis* (powdery mildew) on cereals (e. g. on wheat or barley); *Botrytis cinerea* (teleomorph: *Botryotinia fuckeliana*: grey mold) on fruits and berries (e. g. strawberries), vegetables (e. g. lettuce, carrots, celery and cabbages), rape, flowers, vines, forestry plants and wheat; *Bremia lactucae* (downy mildew) on lettuce; *Ceratocystis* (syn. *Ophiostoma*) spp. (rot or wilt) on broad-leaved trees and evergreens, e. g. *C. ulmi* (Dutch elm disease) on elms; *Cercospora* spp. (Cercospora leaf spots) on corn (e. g. Gray leaf spot: *C. zeae-maydis*), rice, sugar beets (e. g. *C. beticola*), sugar cane, vegetables, coffee, soybeans (e. g. *C. sojina* or *C. kikuchii*) and rice; *Cladosporium* spp. on tomatoes (e. g. *C. fulvum*: leaf mold) and cereals, e. g. *C. herbarum* (black ear) on wheat; *Claviceps purpurea* (ergot) on cereals; *Cochliobolus* (anamorph: *Helminthosporium* of *Bipolaris*) spp. (leaf spots) on corn (*C. carbonum*)*,* cereals (e. g. *C. sativus,* anamorph: *B. sorokiniana*) and rice (e. g. *C. miyabeanus*, anamorph: *H. oryzae*); *Colletotrichum* (teleomorph: *Glomerella*) spp. (anthracnose) on cotton (e. g. *C. gossypii*)*,* corn (e. g. *C. graminicola:* Anthracnose stalk rot), soft fruits, potatoes (e. g. *C. coccodes*: black dot), beans (e. g. *C*. *lindemuthianum*) and soybeans (e. g. *C. truncatum* or *C. gloeosporioides*); *Corticium* spp., e. g. *C. sasakii* (*sheath* blight) on rice; *Corynespora cassiicola* (leaf spots) on soybeans and ornamentals; *Cycloconium* spp., e. g. *C. oleaginum* on olive trees; *Cylindrocarpon* spp. (e. g. fruit tree canker or young vine decline, teleomorph: *Nectria* or *Neonectria* spp.) on fruit trees, vines (e. g. *C. liriodendri*, teleomorph: *Neonectria liriodendri*: Black Foot Disease) and ornamentals; *Dematophora* (teleomorph: *Rosellinia*) *necatrix* (root and stem rot) on soybeans; *Diaporthe* spp., e. g. *D. phaseolorum* (damping off) on soybeans; *Drechslera* (syn. *Helminthosporium*, teleomorph: *Pyrenophora*) spp. on corn, cereals, such as barley (e. g. *D. teres*, net blotch) and wheat (e. g. *D. tritici-repentis*: tan spot), rice and turf; Esca (dieback, apoplexy) on vines, caused by *Formitiporia* (syn. *Phellinus*) *punctata, F. mediterranea, Phaeomoniella ch*/*amydospora* (earlier *Phaeoacremonium chlamydosporum*), *Phaeoacremonium aleophilum* and/or *Botryosphaeria obtusa*; *Elsinoe* spp. on pome fruits (*E. pyri*), soft fruits (*E. veneta*: anthracnose) and vines (*E. ampelina*: anthracnose); *Entyloma oryzae* (leaf smut) on rice; *Epicoccum* spp. (black mold) on wheat; *Erysiphe* spp. (powdery mildew) on sugar beets (*E*. *betae*), vegetables (e. g. *E. pisi*), such as cucurbits (e. g. *E. cichoracearum*), cabbages, rape (e. g. *E. cruciferarum*); *Eutypa lata* (Eutypa canker or dieback, anamorph: *Cytosporina lata*, syn. *Libertella blepharis*) on fruit trees, vines and ornamental woods; *Exserohilum* (syn. *Helminthosporium*) spp. on corn (e. g. *E. turcicum*); *Fusarium* (teleomorph: *Gibberella*) spp. (wilt, root or stem rot) on various plants, such as *F. graminearum* or *F. culmorum* (root rot, scab or head blight) on cereals (e. g. wheat or barley), *F. oxysporum* on tomatoes, *F. solani* (f*.* sp. *glycines* now syn. *F*. *virguliforme*) and *F. tucumaniae* and *F. brasiliense* each causing sudden death syndrome on soybeans, and *F. verticillioides* on corn; *Gaeumannomyces graminis* (take-all) on cereals (e. g. wheat or barley) and corn; *Gibberella* spp. on cereals (e. g. *G. zeae*) and rice (e. g. *G. fujikuroi*: Bakanae disease); *Glomerella cingulata* on vines, pome fruits and other plants and *G. gossypii* on cotton; Grainstaining complex on rice; *Guignardia bidwellii* (black rot) on vines; *Gymnosporangium* spp. on rosaceous plants and junipers, e. g. *G. sabinae* (rust) on pears; *Helminthosporium* spp. (syn. *Drechslera*, teleomorph: *Cochliobolus*) on corn, cereals and rice; *Hemileia* spp., e. g. *H. vastatrix* (*coffee* leaf rust) on coffee; *Isariopsis clavispora* (syn. *Cladosporium vitis*) on vines; *Macrophomina phaseolina* (syn. *phaseoli*) (root and stem rot) on soybeans and cotton; *Microdochium* (syn. *Fusarium*) *nivale* (pink snow mold) on cereals (e. g. wheat or barley); *Microsphaera diffusa* (powdery mildew) on soybeans; *Monilinia* spp., e. g. *M. laxa*, *M. fructicola* and *M. fructigena* (bloom and twig blight, brown rot) on stone fruits and other rosaceous plants; *Mycosphaerella* spp. on cereals, bananas, soft fruits and ground nuts, such as e. g. *M. graminicola* (anamorph: *Septoria tritici*, Septoria blotch) on wheat or *M. fijiensis* (black Sigatoka disease) on bananas; *Peronospora* spp. (downy mildew) on cabbage (e. g. *P. brassicae*), rape (e. g. *P. parasitica*), onions (e. g. *P. destructor*), tobacco (*P. tabacina)* and soybeans (e. g. *P. manshurica*); *Phakopsora pachyrhizi* and *P. meibomiae* (soybean rust) on soybeans; *Phialophora* spp. e. g. on vines (e. g. *P. tracheiphila* and *P. tetraspora*) and soybeans (e. g. *P. gregata*: stem rot); *Phoma lingam* (root and stem rot) on rape and cabbage and *P. betae* (root rot, leaf spot and damping-off) on sugar beets; *Phomopsis* spp. on sunflowers, vines (e. g. *P. viticola*: can and leaf spot) and soybeans (e. g. stem rot: *P. phaseoli*, teleomorph: *Diaporthe phaseolorum*); *Phy-soderma maydis* (brown spots) on corn; *Phytophthora* spp. (wilt, root, leaf, fruit and stem root) on various plants, such as paprika and cucurbits (e. g. *P. capsici*), soybeans (e. g. *P. megasperma*, syn. *P. sojae*), potatoes and tomatoes (e. g. *P. infestans*: late blight) and broadleaved trees (e. g. *P. ramorum*: sudden oak death); *Plasmodiophora brassicae* (club root) on cabbage, rape, radish and other plants; *Plasmopara* spp., e. g. *P. viticola* (grapevine downy mildew) on vines and *P. halstedii* on sunflowers; *Podosphaera* spp. (powdery mildew) on rosaceous plants, hop, pome and soft fruits, e. g. *P. leucotricha* on apples; *Polymyxa* spp., e. g. on cereals, such as barley and wheat (*P. graminis*) and sugar beets (*P. betae*) and thereby transmitted viral diseases; *Pseudocercosporella herpotrichoides* (eyespot, teleomorph: *Tapesia yal-lundae*) on cereals, e. g. wheat or barley; *Pseudoperonospora* (downy mildew) on various plants, e. g. *P. cubensis* on cucurbits or *P. humili* on hop; *Pseudopezicula tracheiphila* (red fire disease or ,rotbrenner', anamorph: *Phialophora*) on vines; *Puccinia* spp. (rusts) on various plants, e. g. *P. triticina* (brown or leaf rust), *P. striiformis* (stripe or yellow rust), *P. hordei* (dwarf rust), *P. graminis* (stem or black rust) or *P. recondita* (brown or leaf rust) on cereals, such as e. g. wheat, barley or rye, *P. kuehnii*(orange rust) on sugar cane and *P. asparagion* asparagus; *Pyrenophora* (anamorph: *Drechslera*) *tritici-repentis* (tan spot) on wheat or *P. teres* (net blotch) on barley; *Pyricularia* spp., e. g. *P. oryzae* (teleomorph: *Magnaporthe grisea*, rice blast) on rice and *P. grisea* on turf and cereals; *Pythium* spp. (damping-off) on turf, rice, corn, wheat, cotton, rape, sunflowers, soybeans, sugar beets, vegetables and various other plants (e. g. *P. ultimum* or *P. aphanidermatum*); *Ramularia* spp., e. g. *R. collo-cygni* (Ramularia leaf spots, Physiological leaf spots) on barley and *R. beticola* on sugar beets; *Rhizoctonia* spp. on cotton, rice, potatoes, turf, corn, rape, potatoes, sugar beets, vegetables and various other plants, e. g. *R. solani* (root and stem rot) on soybeans, *R. solani* (sheath blight) on rice or *R. cerealis* (Rhizoctonia spring blight) on wheat or barley; *Rhizopus stolonifer* (black mold, soft rot) on strawberries, carrots, cabbage, vines and tomatoes; *Rhynchosporium secalis* (scald) on barley, rye and triticale; *Sa-rocladium oryzae* and *S. attenuatum* (sheath rot) on rice; *Sclerotinia* spp. (stem rot or white mold) on vegetables and field crops, such as rape, sunflowers (e. g. *S. sclerotiorum*) and soybeans (e. g. *S. rolfsii* or *S. sclerotiorum*); *Septoria* spp. on various plants, e. g. *S. glycines* (brown spot) on soybeans, *S. tritici* (*Septoria* blotch) on wheat and *S.* (syn. *Stagonospora)* nodorum (Stagonospora blotch) on cereals; *Uncinula* (syn. *Erysiphe*) *necator* (powdery mildew, anamorph: *Oidium tuckeri*) on vines; *Setospaeria* spp. (leaf blight) on corn (e. g. *S. turcicum*, syn. *Helminthosporium turcicum*) and turf; *Sphacelotheca* spp. (smut) on corn, (e. g. *S. reiliana*: head smut), sorghum und sugar cane; *Sphaerotheca fuliginea* (powdery mildew) on cucurbits; *Spongospora subterranea* (powdery scab) on potatoes and thereby transmitted viral diseases; *Stagonospora* spp. on cereals, e. g. *S. nodorum* (Stagonospora blotch, teleomorph: *Lepto-sphaeria* [syn. *Phaeosphaeria*] *nodorum*) on wheat; *Synchytrium endobioticum* on potatoes (potato wart disease); *Taphrina* spp., e. g. *T. deformans* (leaf curl disease) on peaches and *T. pruni* (plum pocket) on plums; *Thielaviopsis* spp. (black root rot) on tobacco, pome fruits, vegetables, soybeans and cotton, e. g. *T. basicola* (syn. *Chalara elegans*); *Tilletia* spp. (common bunt or stinking smut) on cereals, such as e. g. *T. tritici* (syn. *T. caries*, wheat bunt) and *T. controversa* (dwarf bunt) on wheat; *Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendiculatus*, syn. *U. phaseoli*) and sugar beets (e. g. *U. betae*); *Ustilago* spp. (loose smut) on cereals (e. g. *U. nuda* and *U. avaenae*), corn (e. g. *U. maydis*: corn smut) and sugar cane; *Venturia* spp. (scab) on apples (e. g. *V. inaequalis*) and pears; and *Verticillium* spp. (wilt) on various plants, such as fruits and ornamentals, vines, soft fruits, vegetables and field crops, e. g. *V. dahliae* on strawberries, rape, potatoes and tomatoes.

The compounds I and compositions thereof, respectively, are also suitable for controlling harmful fungi in the protection of stored products or harvest and in the protection of materials.

The term "protection of materials" is to be understood to denote the protection of technical and non-living materials, such as adhesives, glues, wood, paper and paperboard, textiles, leather, paint dispersions, plastics, cooling lubricants, fiber or fabrics, against the infestation and destruction by harmful microorganisms, such as fungi and bacteria. As to the protection of wood and other materials, the particular attention is paid to the following harmful fungi: Ascomycetes such as *Ophiostoma* spp., *Ceratocystis* spp., *Aureobasidium pullulans, Sclerophoma* spp., *Chaetomium* spp., *Humicola* spp., *Petriella* spp., *Trichurus* spp.; Basidiomycetes such as *Coni-ophora* spp., *Coriolus* spp., *Gloeophyllum* spp., *Lentinus* spp., *Pleurotus* spp., *Poria* spp., *Ser-pula* spp. and *Tyromyces* spp., Deuteromycetes such as *Aspergillus* spp., *Cladosporium* spp., *Penicillium* spp., *Trichoderma* spp., *Alternaria* spp., *Paecilomyces* spp. and Zygomycetes such as *Mucorspp.,* and in addition in the protection of stored products and harvest the following yeast fungi are worthy of note: *Candida* spp. and *Saccharomyces cerevisae.*

The method of treatment according to the invention can also be used in the field of protecting stored products or harvest against attack of fungi and microorganisms. According to the present invention, the term "stored products" is understood to denote natural substances of plant or animal origin and their processed forms, which have been taken from the natural life cycle and for which long-term protection is desired. Stored products of crop plant origin, such as plants or parts thereof, for example stalks, leafs, tubers, seeds, fruits or grains, can be protected in the freshly harvested state or in processed form, such as pre-dried, moistened, comminuted, ground, pressed or roasted, which process is also known as post-harvest treatment. Also falling under the definition of stored products is timber, whether in the form of crude timber, such as construction timber, electricity pylons and barriers, or in the form of finished articles, such as furniture or objects made from wood. Stored products of animal origin are hides, leather, furs, hairs and the like. The combinations according the present invention can prevent disadvantageous effects such as decay, discoloration or mold. Preferably "stored products" is understood to denote natural substances of plant origin and their processed forms, more preferably fruits and their processed forms, such as pomes, stone fruits, soft fruits and citrus fruits and their processed forms.

The compounds I and compositions thereof, respectively, may be used for improving the health of a plant. The invention also relates to a method for improving plant health by treating a plant, its propagation material and/or the locus where the plant is growing or is to grow with an effective amount of compounds I and compositions thereof, respectively.

The term "plant health" is to be understood to denote a condition of the plant and/or its products which is determined by several indicators alone or in combination with each other such as yield (e. g. increased biomass and/or increased content of valuable ingredients), plant vigor (e. g. improved plant growth and/or greener leaves ("greening effect")), quality (e. g. improved content or composition of certain ingredients) and tolerance to abiotic and/or biotic stress. The above identified indicators for the health condition of a plant may be interdependent or may result from each other.

The compounds of formula I can be present in different crystal modifications whose biological activity may differ. They are likewise subject matter of the present invention.

The compounds I are employed as such or in form of compositions by treating the fungi or the plants, plant propagation materials, such as seeds, soil, surfaces, materials or rooms to be protected from fungal attack with a fungicidally effective amount of the active substances. The application can be carried out both before and after the infection of the plants, plant propagation materials, such as seeds, soil, surfaces, materials or rooms by the fungi.

Plant propagation materials may be treated with compounds I as such or a composition comprising at least one compound I prophylactically either at or before planting or transplanting.

The invention also relates to agrochemical compositions comprising an auxiliary and at least one compound I according to the invention.

An agrochemical composition comprises a fungicidally effective amount of a compound I. The term "effective amount" denotes an amount of the composition or of the compounds I, which is sufficient for controlling harmful fungi on cultivated plants or in the protection of materials and which does not result in a substantial damage to the treated plants. Such an amount can vary in a broad range and is dependent on various factors, such as the fungal species to be controlled, the treated cultivated plant or material, the climatic conditions and the specific compound I used.

The compounds I, their N-oxides and salts can be converted into customary types of agrochemical compositions, e. g. solutions, emulsions, suspensions, dusts, powders, pastes, granules, pressings, capsules, and mixtures thereof. Examples for composition types are suspensions (e. g. SC, OD, FS), emulsifiable concentrates (e. g. EC), emulsions (e. g. EW, EO, ES, ME), capsules (e. g. CS, ZC), pastes, pastilles, wettable powders or dusts (e. g. WP, SP, WS, DP, DS), pressings (e. g. BR, TB, DT), granules (e. g. WG, SG, GR, FG, GG, MG), insecticidal articles (e. g. LN), as well as gel formulations for the treatment of plant propagation materials such as seeds (e. g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e. g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e. g. ethanol, propanol, butanol, benzyl alcohol, cyclohexanol; glycols; DMSO; ketones, e. g. cyclohexanone; esters, e. g. lactates, carbonates, fatty acid esters, gamma-butyrolactone; fatty acids; phosphonates; amines; amides, e. g. N-methyl pyrrolidone, fatty acid dimethyl amides; and mixtures thereof.

Suitable solid carriers or fillers are mineral earths, e. g. silicates, silica gels, talc, kaolins, limestone, lime, chalk, clays, dolomite, diatomaceous earth, bentonite, calcium sulfate, magnesium sulfate, magnesium oxide; polysaccharides, e. g. cellulose, starch; fertilizers, e. g. ammonium sulfate, ammonium phosphate, ammonium nitrate, ureas; products of vegetable origin, e. g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylaryl sulfonates, diphenyl sulfonates, alpha-olefin sulfonates, lignin sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkyl naphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or al-kylpolyglucosides. Examples of polymeric surfactants are home- or copolymers of vinyl pyrrolidone, vinyl alcohols, or vinyl acetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinyl amines or polyethylene amines.

Suitable adjuvants are compounds, which have a negligible or even no pesticidal activity themselves, and which improve the biological performance of the compound I on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e. g. xanthan gum, carboxymethyl cellulose), inorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e. g. in red, blue, or green) are pigments of low water solubility and water-soluble dyes. Examples are inorganic colorants (e. g. iron oxide, titan oxide, iron hexacyanoferrate) and organic colorants (e. g. alizarin-, azo- and phthalocyanine colorants).

Suitable tackifiers or binders are polyvinyl pyrrolidones, polyvinyl acetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I and 5-15 wt% wetting agent (e. g. alcohol alkoxylates) are dissolved in water and/or in a water-soluble solvent (e. g. alcohols) ad 100 wt%. The active substance dissolves upon dilution with water.
ii) Dispersible concentrates (DC)
   5-25 wt% of a compound I and 1-10 wt% dispersant (e. g. polyvinyl pyrrolidone) are dissolved in organic solvent (e. g. cyclohexanone) ad 100 wt%. Dilution with water gives a dispersion.
iii) Emulsifiable concentrates (EC)
   15-70 wt% of a compound I and 5-10 wt% emulsifiers (e. g. calcium dodecylbenzenesulfonate and castor oil ethoxylate) are dissolved in water-insoluble organic solvent (e. g. aromatic hydrocarbon) ad 100 wt%. Dilution with water gives an emulsion.
iv) Emulsions (EW, EO, ES)
   5-40 wt% of a compound I and 1-10 wt% emulsifiers (e. g. calcium dodecylbenzenesulfonate and castor oil ethoxylate) are dissolved in 20-40 wt% water-insoluble organic solvent (e. g. aromatic hydrocarbon). This mixture is introduced into water ad 100 wt% by means of an emulsifying machine and made into a homogeneous emulsion. Dilution with water gives an emulsion.
v) Suspensions (SC, OD, FS)
   In an agitated ball mill, 20-60 wt% of a compound I are comminuted with addition of 2-10 wt% dispersants and wetting agents (e. g. sodium lignosulfonate and alcohol ethoxylate), 0.1-2 wt% thickener (e. g. xanthan gum) and water ad 100 wt% to give a fine active substance suspension. Dilution with water gives a stable suspension of the active substance. For FS type composition up to 40 wt% binder (e. g. polyvinyl alcohol) is added.
vi) Water-dispersible granules and water-soluble granules (WG, SG)
   50-80 wt% of a compound I are ground finely with addition of dispersants and wetting agents (e. g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound I are ground in a rotor-stator mill with addition of 1-5 wt% dispersants (e. g. sodium lignosulfonate), 1-3 wt% wetting agents (e. g. alcohol ethoxylate) and solid carrier (e. g. silica gel) ad 100 wt%. Dilution with water gives a stable dispersion or solution of the active substance.
viii) Gel (GW, GF)
   In an agitated ball mill, 5-25 wt% of a compound I are comminuted with addition of 3-10 wt% dispersants (e. g. sodium lignosulfonate), 1-5 wt% thickener (e. g. carboxymethyl cellulose) and water ad 100 wt% to give a fine suspension of the active substance. Dilution with water gives a stable suspension of the active substance.
ix) Microemulsion (ME)
   5-20 wt% of a compound I are added to 5-30 wt% organic solvent blend (e. g. fatty acid dimethyl amide and cyclohexanone), 10-25 wt% surfactant blend (e. g. alcohol ethoxylate and arylphenol ethoxylate), and water ad 100 %. This mixture is stirred for 1 h to produce spontaneously a thermodynamically stable microemulsion.
x) Microcapsules (CS)
   An oil phase comprising 5-50 wt% of a compound I, 0-40 wt% water insoluble organic solvent (e. g. aromatic hydrocarbon), 2-15 wt% acrylic monomers (e. g. methylmethacrylate, methacrylic acid and a di- or triacrylate) are dispersed into an aqueous solution of a protective colloid (e. g. polyvinyl alcohol). Radical polymerization results in the formation of poly(meth)acrylate microcapsules. Alternatively, an oil phase comprising 5-50 wt% of a compound I according to the invention, 0-40 wt% water insoluble organic solvent (e. g. aromatic hydrocarbon), and an isocyanate monomer (e. g. diphenylmethene-4,4'-diisocyanatae) are dispersed into an aqueous solution of a protective colloid (e. g. polyvinyl alcohol). The addition of a polyamine (e. g. hexamethylenediamine) results in the formation of polyurea microcapsules. The monomers amount to 1-10 wt%. The wt% relate to the total CS composition.
xi) Dustable powders (DP, DS)
   1-10 wt% of a compound I are ground finely and mixed intimately with solid carrier (e. g. finely divided kaolin) ad 100 wt%.
xii) Granules (GR, FG)
   0.5-30 wt% of a compound I is ground finely and associated with solid carrier (e. g. silicate) ad 100 wt%. Granulation is achieved by extrusion, spray-drying or fluidized bed.
xiii) Ultra-low volume liquids (UL)
   1-50 wt% of a compound I are dissolved in organic solvent (e. g. aromatic hydrocarbon) ad 100 wt%.

The compositions types i) to xiii) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, more preferably between 1 and 70%, and in particular between 10 and 60%, by weight of active substance. The active substances are employed in a purity of from 90% to 100%, preferably from 95% to 100% (according to NMR spectrum).

For the purposes of treatment of plant propagation materials, particularly seeds, solutions for seed treatment (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC), and gels (GF) are usually employed. The compositions in question give, after two-to-tenfold dilution, active substance concentrations of from 0.01 to 60% by weight, preferably from 0.1 to 40%, in the ready-to-use preparations. Application can be carried out before or during sowing. Methods for applying compound I and compositions thereof, respectively, onto plant propagation material, especially seeds, include dressing, coating, pelleting, dusting, and soaking as well as in-furrow application methods. Preferably, compound I or the compositions thereof, respectively, are applied on to the plant propagation material by a method such that germination is not induced, e. g. by seed dressing, pelleting, coating and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e. g. herbicides, insecticides, fungicides, growth regulators, safeners, biopesticides) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

A pesticide is generally a chemical or biological agent (such as pestidal active ingredient, compound, composition, virus, bacterium, antimicrobial or disinfectant) that through its effect deters, incapacitates, kills or otherwise discourages pests. Target pests can include insects, plant pathogens, weeds, mollusks, birds, mammals, fish, nematodes (roundworms), and microbes that destroy property, cause nuisance, spread disease or are vectors for disease. The term "pesticide" includes also plant growth regulators that alter the expected growth, flowering, or reproduction rate of plants; defoliants that cause leaves or other foliage to drop from a plant, usually to facilitate harvest; desiccants that promote drying of living tissues, such as unwanted plant tops; plant activators that activate plant physiology for defense of against certain pests; safeners that reduce unwanted herbicidal action of pesticides on crop plants; and plant growth promoters that affect plant physiology e.g. to increase plant growth, biomass, yield or any other quality parameter of the harvestable goods of a crop plant.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a binary or ternary mixture may be mixed by the user himself in a spray tank or any other kind of vessel used for applications (e. g. seed treater drums, seed pelleting machinery, knapsack sprayer) and further auxiliaries may be added, if appropriate.

Consequently, one embodiment of the invention is a kit for preparing a usable pesticidal composition, the kit comprising a) a composition comprising component 1) as defined herein and at least one auxiliary; and b) a composition comprising component 2) as defined herein and at least one auxiliary; and optionally c) a composition comprising at least one auxiliary and optionally a further active component 3) as defined herein.

Mixing the compounds I or the compositions comprising them in the use form as fungicides with other fungicides results in many cases in an expansion of the fungicidal spectrum of activity being obtained or in a prevention of fungicide resistance development. Furthermore, in many cases, synergistic effects are obtained.

The following list of pesticides II (e. g. pesticidally-active substances and biopesticides), in conjunction with which the compounds I can be used, is intended to illustrate the possible combinations but does not limit them:
A) Respiration inhibitors
   - Inhibitors of complex III at Qₒ site: azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxystrobin (A.1.17), 2-(2-(3-(2,6-dichlorophenyl)-1-methyl-allylideneaminooxymethyl)-phenyl)-2-methoxyimino-N-methyl-acetamide (A.1.18), pyribencarb (A.1.19), triclopyricarb/chloro-dincarb (A.1.20), famoxadone (A.1.21), fenamidone (A.1.21), methyl-*N*-[2-[(1,4-dimethyl-5-phenyl-pyrazol-3-yl)oxylmethyl]phenyl]-N-methoxy-carbamate (A.1.22), 1-[3-chloro-2-[[1-(4-chlorophenyl)-1H-pyrazol-3-yl]oxymethyl]phenyl]-4-methyl-tetrazol-5-one (A.1.23), 1-[3-bromo-2-[[1-(4-chlorophenyl)pyrazol-3-yl]oxymethyl]phenyl]-4-methyl-tetrazol-5-one (A.1.24), 1-[2-[[1-(4-chlorophenyl)pyrazol-3-yl]oxymethyl]-3-methyl-phenyl]-4-methyl-tetrazol-5-one (A.1.25), 1-[2-[[1-(4-chlorophenyl)pyrazol-3-yl]oxymethyl]-3-fluoro-phenyl]-4-methyl-tetrazol-5-one (A.1.26), 1-[2-[[1-(2,4-dichlorophenyl)pyrazol-3-yl]oxymethyl]-3-fluoro-phenyl]-4-methyl-tetrazol-5-one (A.1.27), 1-[2-[[4-(4-chlorophenyl)thiazol-2-yl]oxymethyl]-3-methyl-phenyl]-4-methyl-tetrazol-5-one (A.1.28), 1-[3-chloro-2-[[4-(p-tolyl)thiazol-2-yl]-oxymethyl]phenyl]-4-methyl-tetrazol-5-one (A.1.29), 1-[3-cyclopropyl-2-[[2-methyl-4-(1-methylpyrazol-3-yl)phenoxy]methyl]phenyl]-4-methyl-tetrazol-5-one (A.1.30), 1-[3-(difluoromethoxy)-2-[[2-methyl-4-(1-methylpyrazol-3-yl)phenoxy]methyl]phenyl]-4-methyl-tetrazol-5-one (A.1.31), 1-methyl-4-[3-methyl-2-[[2-methyl-4-(1-methylpyrazol-3-yl)phen-oxy]methyl]phenyl]tetrazol-5-one (A.1.32), 1-methyl-4-[3-methyl-2-[[1-[3-(trifluoromethyl)-phenyl]-ethylideneamino]oxymethyl]phenyl]tetrazol-5-one (A.1.33), (*Z*,2*E*)-5-[1-(2,4-dichlorophenyl)pyrazol-3-yl]-oxy-2-methoxyimino-*N*,3-dimethyl-pent-3-enamide (A.1.34), (*Z*,2*E*)-5-[1-(4-chlorophenyl)pyrazol-3-yl]oxy-2-methoxyimino-*N*,3-dimethyl-pent-3-enamide (A.1.35), pyriminostrobin (A.1.36), bifujunzhi (A.1.37), 2-(ortho-((2,5-dimethylphenyl-oxy-methylen)phenyl)-3-methoxy-acrylic acid methylester (A.1.38);
   - inhibitors of complex III at Qᵢ site: cyazofamid (A.2.1), amisulbrom (A.2.2), [(6S,7R,8R)-8-benzyl-3-[(3-hydroxy-4-methoxy-pyridine-2-carbonyl)amino]-6-methyl-4,9-dioxo-1,5-dioxonan-7-yl] 2-methylpropanoate (A.2.3), [2-[[(7R,8R,9S)-7-benzyl-9-methyl-8-(2-methylpropanoyloxy)-2,6-dioxo-1,5-dioxonan-3-yl]carbamoyl]-4-methoxy-3-pyridyl]oxymethyl 2-methylpropanoate (A.2.4), [(6S,7R,8R)-8-benzyl-3-[[4-methoxy-3-(propanoyloxymethoxy)pyridine-2-carbonyl]amino]-6-methyl-4,9-dioxo-1,5-dioxonan-7-yl] 2-methylpropanoate (A.2.5);
   - inhibitors of complex II: benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.15), penthiopyrad (A.3.16), sedaxane (A.3.19), tecloftalam (A.3.20), thifluzamide (A.3.21), 3-(difluoromethyl)-1-methyl-N-(1,1,3-trimethylindan-4-yl)pyrazole-4-carboxamide (A.3.22), 3-(trifluoromethyl)-1-methyl-N-(1,1,3-trimethylindan-4-yl)pyrazole-4-carboxamide (A.3.23), 1,3-dimethyl-N-(1,1,3-trimethylindan-4-yl)pyrazole-4-carboxamide (A.3.24), 3-(trifluoromethyl)-1,5-dimethyl-N-(1,1,3-trimethylindan-4-yl)pyrazole-4-carboxamide (A.3.25), 1,3,5-trimethyl-N-(1,1,3-trimethylindan-4-yl)pyrazole-4-carboxamide (A.3.26), 3-(difluoromethyl)-1,5-dimethyl-N-(1,1,3-trimethylindan-4-yl)pyrazole-4-carboxamide (A.3.27), 3-(difluoromethyl)-N-(7-fluoro-1,1,3-trimethyl-indan-4-yl)-1-methyl-pyrazole-4-carboxamide (A.3.28), methyl (E)-2-[2-[(5-cyano-2-methyl-phenoxy)methyl]phenyl]-3-methoxy-prop-2-enoate (A.3.30);
   - other respiration inhibitors: diflumetorim (A.4.1); nitrophenyl derivates: binapacryl (A.4.2), dinobuton (A.4.3), dinocap (A.4.4), fluazinam (A.4.5), ferimzone (A.4.7); organometal compounds: fentin salts, e. g. fentin-acetate (A.4.8), fentin chloride (A.4.9) or fentin hydroxide (A.4.10); ametoctradin (A.4.11); silthiofam (A.4.12);
B) Sterol biosynthesis inhibitors (SBI fungicides)
   - C14 demethylase inhibitors: triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 1-[*rel*-(2*S*;3*R*)-3-(2-chlorophenyl)-2-(2,4-difluorophenyl)-oxiranylmethyl]-5-thiocyanato-1H-[1,2,4]triazole (B.1.31),2-[*rel*-(2*S*,3*R*)-3-(2-chlorophenyl)-2-(2,4-difluorophenyl)-oxiranylmethyl]-2H-[1,2,4]triazole-3-thiol (B.1.32), 2-[2-chloro-4-(4-chlorophenoxy)phenyl]-1-(1,2,4-triazol-1-yl)pentan-2-ol (B.1.33), 1-[4-(4-chlorophenoxy)-2-(trifluoromethyl)phenyl]-1-cyclopropyl-2-(1,2,4-triazol-1-yl)ethanol (B.1.34), 2-[4-(4-chlorophenoxy)-2-(trifluoromethyl)phenyl]-1-(1,2,4-triazol-1-yl)butan-2-ol (B.1.35), 2-[2-chloro-4-(4-chlorophenoxy)phenyl]-1-(1,2,4-triazol-1-yl)butan-2-ol (B.1.36), 2-[4-(4-chlorophenoxy)-2-(trifluoromethyl)phenyl]-3-methyl-1-(1,2,4-triazol-1-yl)butan-2-ol (B.1.37), 2-[4-(4-chlorophenoxy)-2-(trifluoromethyl)phenyl]-1-(1,2,4-triazol-1-yl)propan-2-ol (B.1.38), 2-[2-chloro-4-(4-chlorophenoxy)phenyl]-3-methyl-1-(1,2,4-triazol-1-yl)butan-2-ol (B.1.39), 2-[4-(4-chlorophenoxy)-2-(trifluoromethyl)phenyl]-1-(1,2,4-triazol-1-yl)pentan-2-ol (B.1.40), 2-[4-(4-fluorophenoxy)-2-(trifluoromethyl)phenyl]-1-(1,2,4-triazol-1-yl)propan-2-ol (B.1.41), 2-[2-chloro-4-(4-chlorophenoxy)phenyl]-1-(1,2,4-triazol-1-yl)pent-3-yn-2-ol (B.1.42), 2-(chloromethyl)-2-methyl-5-(p-tolylmethyl)-1-(1,2,4-triazol-1-ylmethyl)cyclopentanol (B.1.43); imidazoles: imazalil (B.1.44), pefurazoate (B.1.45), prochloraz (B.1.46), triflumizol (B.1.47); pyrimidines, pyridines and piperazines: fenarimol (B.1.49), pyrifenox (B.1.50), triforine (B.1.51), [3-(4-chloro-2-fluoro-phenyl)-5-(2,4-difluorophenyl)isoxazol-4-yl]-(3-pyridyl)methanol (B.1.52);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8);
   - Inhibitors of 3-keto reductase: fenhexamid (B.3.1);
   - Other Sterol biosynthesis inhibitors: chlorphenomizole (B.4.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - other nucleic acid synthesis inhibitors: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7), 5-fluoro-2-(4-chlorophenylmethoxy)pyrimidin-4 amine (C.2.8);
D) Inhibitors of cell division and cytoskeleton
   - tubulin inhibitors: benomyl (D.1.1), carbendazim (D.1.2), fuberidazole (D1.3), thiabendazole (D.1.4), thiophanate-methyl (D.1.5), 3-chloro-4-(2,6-difluorophenyl)-6-methyl-5-phenyl-pyridazine (D.1.6), 3-chloro-6-methyl-5-phenyl-4-(2,4,6-trifluorophenyl)pyridazine (D.1.7), N-ethyl-2-[(3-ethynyl-8-methyl-6-quinolyl)oxy]butanamide (D.1.8), N-ethyl-2-[(3-ethynyl-8-methyl-6-quinolyl)oxy]-2-methylsulfanyl-acetamide (D.1.9), 2-[(3-ethynyl-8-methyl-6-quinolyl)oxy]-N-(2-fluoroethyl)butanamide (D.1.10), 2-[(3-ethynyl-8-methyl-6-quinolyl)oxy]-N-(2-fluoroethyl)-2-methoxy-acetamide (D.1.11), 2-[(3-ethynyl-8-methyl-6-quinolyl)oxy]-N-propyl-butanamide (D.1.12), 2-[(3-ethynyl-8-methyl-6-quinolyl)oxy]-2-methoxy-N-propyl-acetamide (D.1.13), 2-[(3-ethynyl-8-methyl-6-quinolyl)oxy]-2-methylsulfanyl-N-propyl-acetamide (D.1.14), 2-[(3-ethynyl-8-methyl-6-quinolyl)oxy]-N-(2-fluoroethyl)-2-methylsulfanyl-acetamide (D.1.15), 4-(2-bromo-4-fluoro-phenyl)-N-(2-chloro-6-fluoro-phenyl)-2,5-dimethyl-pyrazol-3-amine (D.1.16);
   - other cell division inhibitors: diethofencarb (D.2.1), ethaboxam (D.2.2), pencycuron (D.2.3), fluopicolide (D.2.4), zoxamide (D.2.5), metrafenone (D.2.6), pyriofenone (D.2.7);
E) Inhibitors of amino acid and protein synthesis
   - methionine synthesis inhibitors: cyprodinil (E.1.1), mepanipyrim (E.1.2), pyrimethanil (E.1.3);
   - protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6);
F) Signal transduction inhibitors
   - MAP / histidine kinase inhibitors: fluoroimid (F.1.1), iprodione (F.1.2), procymidone (F.1.3), vinclozolin (F.1.4), fludioxonil (F.1.5);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7);
   - compounds affecting cell membrane permeability and fatty acides: propamocarb (G.4.1);
   - inhibitors of oxysterol binding protein: oxathiapiprolin (G.5.1), 2-{3-[2-(1-{[3,5-bis(difluoromethyl-1H-pyrazol-1-yl]acetyl}piperidin-4-yl)-1,3-thiazol-4-yl]-4,5-dihydro-1,2-oxazol-5-yl}-phenyl methanesulfonate (G.5.2), 2-{3-[2-(1-{[3,5-bis(difluoromethyl)-1H-pyrazol-1-yl]-acetyl}piperidin-4-yl) 1,3-thiazol-4-yl]-4,5-dihydro-1,2-oxazol-5-yl}-3-chlorophenyl methanesulfonate (G.5.3), 4-[1-[2-[3-(difluoromethyl)-5-methyl-pyrazol-1-yl]acetyl]-4-piperidyl]-N-tetralin-1-yl-pyridine-2-carboxamide (G.5.4), 4-[1-[2-[3,5-bis(difluoromethyl)pyrazol-1-yl]acetyl]-4-piperidyl]-N-tetralin-1-yl-pyridine-2-carboxamide (G.5.5), 4-[1-[2-[3-(difluoromethyl)-5-(trifluoromethyl)pyrazol-1-yl]acetyl]-4-piperidyl]-N-tetralin-1-yl-pyridine-2-carboxamide (G.5.6), 4-[1-[2-[5-cyclopropyl-3-(difluoromethyl)pyrazol-1-yl]acetyl]-4-piperidyl]-N-tetralin-1-yl-pyridine-2-carboxamide (G.5.7), 4-[1-[2-[5-methyl-3-(trifluoromethyl)pyrazol-1-yl]acetyl]-4-piperidyl]-N-tetralin-1-yl-pyridine-2-carboxamide (G.5.8), 4-[1-[2-[5-(difluoromethyl)-3-(trifluoromethyl)pyrazol-1-yl]acetyl]-4-piperidyl]-N-tetralin-1-yl-pyridine-2-carboxamide (G.5.9), 4-[1-[2-[3,5-bis(trifluoromethyl)pyrazol-1-yl]acetyl]-4-piperidyl]-N-tetralin-1-yl-pyridine-2-carboxamide (G.5.10), (4-[1-[2-[5-cyclopropyl-3-(trifluoromethyl)pyrazol-1-yl]acetyl]-4-piperidyl]-N-tetralin-1-yl-pyridine-2-carboxamide (G.5.11);
H) Inhibitors with Multi Site Action
   - inorganic active substances: Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds: anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1H,5H-[1,4]dithiino[2,3-c:5,6-c']dipyrrole-1,3,5,7(2H,6H)-tetraone (H.4.10);
I) Cell wall synthesis inhibitors
   - inhibitors of glucan synthesis: validamycin (I.1.1), polyoxin B (1.1.2);
   - melanin synthesis inhibitors: pyroquilon (1.2.1), tricyclazole (1.2.2), carpropamid (1.2.3), dicy-clomet (I.2.4), fenoxanil (1.2.5);
J) Plant defence inducers
   - acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), potassium or sodium bicarbonate (J.1.9);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclocymet (K.1.7), diclomezine (K.1.8), difenzoquat (K.1.9), difenzoquat-methylsulfate (K.1.10), diphenylamin (K.1.11), fenitropan (K.1.12), fenpyrazamine (K.1.13), flumetover (K.1.14), flusulfamide (K.1.15), flutianil (K.1.16), harpin (K.1.17), metha-sulfocarb (K.1.18), nitrapyrin (K.1.19), nitrothal-isopropyl (K.1.20), tolprocarb (K.1.21), oxin-copper (K.1.22), proquinazid (K.1.23), tebufloquin (K.1.24), tecloftalam (K.1.25), triazoxide (K.1.26), N'-(4-(4-chloro-3-trifluoromethyl-phenoxy)-2,5-dimethyl-phenyl)-N-ethyl-N-methyl formamidine (K.1.27), N'-(4-(4-fluoro-3-trifluoromethyl-phenoxy)-2,5-dimethyl-phenyl)-N-ethyl-N-methyl formamidine (K.1.28), N'-[4-[[3-[(4-chlorophenyl)methyl]-1,2,4-thiadiazol-5-yl]-oxy]-2,5-dimethyl-phenyl]-N-ethyl-N-methyl-formamidine (K.1.29), N'-(5-bromo-6-indan-2-yl-oxy-2-methyl-3-pyridyl)-N-ethyl-N-methyl-formamidine (K.1.30), N'-[5-bromo-6-[1-(3,5-difluorophenyl)ethoxy]-2-methyl-3-pyridyl]-N-ethyl-N-methyl-formamidine (K.1.31), N'-[5-bromo-6-(4-isopropylcyclohexoxy)-2-methyl-3-pyridyl]-N-ethyl-N-methyl-formamidine (K.1.32), N'-[5-bromo-2-methyl-6-(1-phenylethoxy)-3-pyridyl]-N-ethyl-N-methyl-formamidine (K.1.33), N'-(2-methyl-5-trifluoromethyl-4-(3-trimethylsilanyl-propoxy)-phenyl)-N-ethyl-N-methyl formamidine (K.1.34), N'-(5-difluoromethyl-2-methyl-4-(3-trimethylsilanyl-propoxy)-phenyl)-N-ethyl-N-methyl formamidine (K.1.35), 2-(4-chloro-phenyl)-N-[4-(3,4-dimethoxy-phenyl)-isoxazol-5-yl]-2-prop-2-ynyloxy-acetamide (K.1.36), 3-[5-(4-chloro-phenyl)-2,3-dimethyl-isoxazolidin-3-yl]-pyridine (pyrisoxazole) (K.1.37), 3-[5-(4-methylphenyl)-2,3-dimethyl-isoxazolidin-3 yl]-pyridine (K.1.38), 5-chloro-1-(4,6-dimethoxy-pyrimidin-2-yl)-2-methyl-1H-benzoimidazole (K.1.39), ethyl (Z)-3-amino-2-cyano-3-phenyl-prop-2-enoate (K.1.40), picarbutrazox (K.1.41), pentyl N-[6-[[(Z)-[(1-methyltetrazol-5-yl)-phenyl-methylene]amino]oxymethyl]-2-pyridyl]carbamate (K.1.42), 2-[2-[(7,8-difluoro-2-methyl-3-quinolyl)oxy]-6-fluoro-phenyl]propan-2-ol (K.1.44), 2-[2-fluoro-6-[(8-fluoro-2-methyl-3-quinolyl)oxy]phen-yl]propan-2-ol (K.1.45), 3-(5-fluoro-3,3,4,4-tetramethyl-3,4-dihydroisoquinolin-1-yl)quinoline (K.1.46), 3-(4,4-difluoro-3,3-dimethyl-3,4-dihydroisoquinolin-1-yl)quinoline (K.1.47), 3-(4,4,5-trifluoro-3,3-dimethyl-3,4-dihydroisoquinolin-1-yl)quinoline (K.1.48), 9-fluoro-2,2-dimethyl-5-(3-quinolyl)-3H-1,4-benzoxazepine (K.1.49), 2-(6-benzyl-2-pyridyl)quinazoline (K.1.50), 2-[6-(3-fluoro-4-methoxy-phenyl)-5-methyl-2-pyridyl]quinazoline (K.1.51), 3-[(3,4-dichloroisothiazol-5-yl)methoxy]-1,2-benzothiazole 1,1-dioxide (K.1.52), N'-(2,5-dimethyl-4-phenoxy-phenyl)-N-ethyl-N-methyl-formamidine (K.1.53);
M) Growth regulators
   abscisic acid (M.1.1), amidochlor, ancymidol, 6-benzylaminopurine, brassinolide, butralin, chlormequat, chlormequat chloride, choline chloride, cyclanilide, daminozide, dikegulac, dimethipin, 2,6-dimethylpuridine, ethephon, flumetralin, flurprimidol, fluthiacet, forchlorfenuron, gib-berellic acid, inabenfide, indole-3-acetic acid, maleic hydrazide, mefluidide, mepiquat, mepiquat chloride, naphthaleneacetic acid, N-6-benzyladenine, paclobutrazol, prohexadione, prohexadione-calcium, prohydrojasmon, thidiazuron, triapenthenol, tributyl phosphorotrithioate, 2,3,5-tri-iodobenzoic acid, trinexapac-ethyl and uniconazole;
N) Herbicides from classes N.1 to N.15
   N.1 Lipid biosynthesis inhibitors: alloxydim, alloxydim-sodium, butroxydim, clethodim, clodinafop, clodinafop-propargyl, cycloxydim, cyhalofop, cyhalofop-butyl, diclofop, diclofop-methyl, fenoxaprop, fenoxaprop-ethyl, fenoxaprop-P, fenoxaprop-P-ethyl, fluazifop, fluazifop-butyl, fluazifop-P, fluazifop-P-butyl, haloxyfop, haloxyfop-methyl, haloxyfop-P, haloxyfop-P-methyl, metamifop, pinoxaden, profoxydim, propaquizafop, quizalofop, quizalofop-ethyl, quizalofop-tefuryl, quizalofop-P, quizalofop-P-ethyl, quizalofop-P-tefuryl, sethoxydim, tepra-loxydim, tralkoxydim, 4-(4'-chloro-4-cyclo¬propyl-2'-fluoro[1,1'-biphenyl]-3-yl)-5-hydroxy-2,2,6,6-tetramethyl-2H-pyran-3(6H)-one (CAS 1312337-72-6); 4-(2',4'-dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(acetyloxy)-4-(2',4'-dichloro-4-cyclopropyl- [1,1'-biphenyl]-3-yl)-3,6-dihydro-2,2,6,6-tetramethyl-2H-pyran-3-one; 5-(acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-dichloro-4-cyclopropyl- [1,1'-biphenyl]-3-yl)-5,6-dihydro-2,2,6,6-tetramethyl-5-oxo-2H-pyran-3-yl carbonic acid methyl ester; 4-(4'-chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-dichloro-4-ethyl¬[1,1'-biphenyl]-3-yl)-5,6-dihydro-2,2,6,6-tetramethyl-5-oxo-2H-pyran-3-yl carbonic acid methyl ester (CAS 1033760-58-5); benfuresate, butylate, cycloate, dalapon, dimepiperate, EPTC, esprocarb, ethofumesate, flupropanate, molinate, orbencarb, pebulate, prosulfocarb, TCA, thiobencarb, tiocarbazil, triallate and vernolate;
   N.2 ALS inhibitors: amidosulfuron, azimsulfuron, bensulfuron, bensulfuron-methyl, chlorimuron, chlorimuron-ethyl, chlorsulfuron, cinosulfuron, cyclosulfamuron, ethametsulfuron, ethametsulfuron-methyl, ethoxysulfuron, flazasulfuron, flucetosulfuron, flupyrsulfuron, flupyrsulfuron-methyl-sodium, foramsulfuron, halosulfuron, halosulfuron-methyl, imazosulfuron, iodosulfuron, iodosulfuron-methyl-sodium, iofensulfuron, iofensulfuron-sodium, mesosulfuron, metazosulfuron, metsulfuron, metsulfuron-methyl, nicosulfuron, orthosulfamuron, oxasulfuron, primisulfuron, primisulfuron-methyl, propyrisulfuron, prosulfuron, pyrazosulfuron, pyrazosulfuron-ethyl, rimsulfuron, sulfometuron, sulfometuron-methyl, sulfosulfuron, thifensulfuron, thifensulfuron-methyl, triasulfuron, tribenuron, tribenuron-methyl, trifloxysulfuron, triflusulfuron, triflusulfuron-methyl, tritosulfuron, imazamethabenz, imazamethabenz-methyl, imazamox, imazapic, imazapyr, imazaquin, imazethapyr; cloransulam, cloransulam-methyl, diclosulam, flumetsulam, florasulam, metosulam, penoxsulam, pyrimisulfan and pyroxsulam; bispyribac, bispyribac-sodium, pyribenzoxim, pyriftalid, pyriminobac, pyriminobac-methyl, pyrithiobac, pyrithiobac-sodium, 4-[[[2-[(4,6-dimethoxy-2-pyrimidinyl)oxy]phenyl]methyl]amino]-benzoic acid-1-methy¬ethyl ester (CAS 420138-41-6), 4-[[[2-[(4,6-dimethoxy-2-pyrimidinyl)oxy]phenyl]¬methyl]amino]-benzoic acid propyl ester (CAS 420138-40-5), N-(4-bromophenyl)-2-[(4,6-dimethoxy-2-pyrimidinyl)oxy]benzenemethanamine (CAS 420138-01-8); flucarbazone, flucarbazone-sodium, propoxycarbazone, propoxycarbazone-sodium, thiencarbazone, thiencarbazone-methyl; triafamone;
   N.3 Photosynthesis inhibitors: amicarbazone; chlorotriazine; ametryn, atrazine, chloridazone, cyanazine, desmetryn, dimethametryn,hexazinone, metribuzin, prometon, prometryn, propazine, simazine, simetryn, terbumeton, terbuthylazin, terbutryn, trietazin; chlorobromuron, chlorotoluron, chloroxuron, dimefuron, diuron, fluometuron, isoproturon, isouron, linuron, metamitron, methabenzthiazuron, metobenzuron, metoxuron, monolinuron, neburon, siduron, tebuthiuron, thiadiazuron, desmedipham, karbutilat, phenmedipham, phenmedipham-ethyl, bromofenoxim, bromoxynil and its salts and esters, ioxynil and its salts and esters, bromacil, lenacil, terbacil, bentazon, bentazon-sodium, pyridate, pyridafol, pentanochlor, propanil; diquat, diquat-dibromide, paraquat, paraquat-dichloride, paraquat-dimetilsulfate;
   N.4 protoporphyrinogen-IX oxidase inhibitors: acifluorfen, acifluorfen-sodium, azafenidin, bencarbazone, benzfendizone, bifenox, butafenacil, carfentrazone, carfentrazone-ethyl, chlormethoxyfen, cinidon-ethyl, fluazolate, flufenpyr, flufenpyr-ethyl, flumiclorac, flumicloracpentyl, flumioxazin, fluoroglycofen, fluoroglycofen-ethyl, fluthiacet, fluthiacet-methyl, fomesafen, halosafen, lactofen, oxadiargyl, oxadiazon, oxyfluorfen, pentoxazone, profluazol, pyraclonil, pyraflufen, pyraflufen-ethyl, saflufenacil, sulfentrazone, thidiazimin, tiafenacil, trifludimoxazin, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6), N-ethyl-3-(2,6-dichloro-4-trifluoro-methylphenoxy)-5-methyl-1H-pyrazole-1-carboxamide (CAS 452098-92-9), N tetrahydrofurfuryl-3-(2,6-dichloro-4-trifluoromethylphenoxy)-5-methyl-1H-pyrazole-1-carboxamide (CAS 915396-43-9), N-ethyl-3-(2-chloro-6-fluoro-4-trifluoromethyl¬phenoxy)-5-methyl-1H-pyrazole-1-carboxamide (CAS 452099-05-7), N tetrahydro¬furfuryl-3-(2-chloro-6-fluoro-4-trifluoro¬methylphenoxy)-5-methyl-1H-pyrazole-1-carboxamide (CAS 452100-03-7), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione (CAS 451484-50-7), 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0), 1-methyl-6-trifluoro¬methyl-3-(2,2,7-tri-fluoro-3-oxo-4-prop-2-ynyl-3,4-dihydro-2H-ben- zo[1,4]oxazin-6-yl)-1H-pyrimidine-2,4-dione (CAS 1304113-05-0), methyl (E)-4-[2-chloro-5-[4-chloro-5-(difluoromethoxy)-1H-methyl-pyrazol-3-yl]-4-fluoro-phenoxy]-3-methoxy-but-2-enoate (CAS 948893-00-3), 3-[7-chloro-5-fluoro-2-(trifluoromethyl)-1H-benzimidazol-4-yl]-1-methyl-6-(trifluoromethyl)-1H-pyrimidine-2,4-dione (CAS 212754-02-4);
   N.5 Bleacher herbicides: beflubutamid, diflufenican, fluridone, flurochloridone, flurtamone, norflurazon, picolinafen, 4-(3-trifluoromethyl¬phenoxy)-2-(4-trifluoromethylphenyl)¬pyrimidine (CAS 180608-33-7); benzobicyclon, benzofenap, bicyclopyrone, clomazone, fenquintrione, isoxaflutole, mesotrione, pyrasulfotole, pyrazolynate, pyrazoxyfen, sulcotrione, tefuryltrione, tembotrione, tolpyralate, topramezone; aclonifen, amitrole, flumeturon;
   N.6 EPSP synthase inhibitors: glyphosate, glyphosate-isopropylammonium, glyposate-potassium, glyphosate-trimesium (sulfosate);
   N.7 Glutamine synthase inhibitors: bilanaphos (bialaphos), bilanaphos-sodium, glufosinate, glufosinate-P, glufosinate-ammonium;
   N.8 DHP synthase inhibitors: asulam;
   N.9 Mitosis inhibitors: benfluralin, butralin, dinitramine, ethalfluralin, fluchloralin, oryzalin, pendimethalin, prodiamine, trifluralin; amiprophos, amiprophos-methyl, butamiphos; chlorthal, chlorthal-dimethyl, dithiopyr, thiazopyr, propyzamide, tebutam; carbetamide, chlorpropham, flamprop, flamprop-isopropyl, flamprop-methyl, flamprop-M-isopropyl, flamprop-M-methyl, propham;
   N.10 VLCFA inhibitors: acetochlor, alachlor, butachlor, dimethachlor, dimethenamid, dimethenamid-P, metazachlor, metolachlor, metolachlor-S, pethoxamid, pretilachlor, propachlor, propisochlor, thenylchlor, flufenacet, mefenacet, diphenamid, naproanilide, napropamide, napropamide-M, fentrazamide, anilofos, cafenstrole, fenoxasulfone, ipfencarbazone, piperophos, pyroxasulfone, isoxazoline compounds of the formulae 11.1, II.2, II.3, II.4, II.5, II.6, II.7, II.8 and II.9 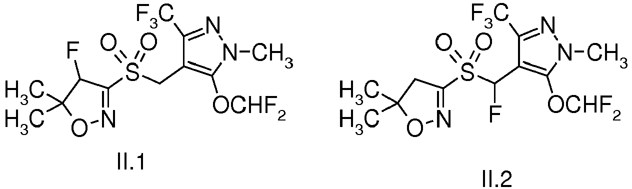 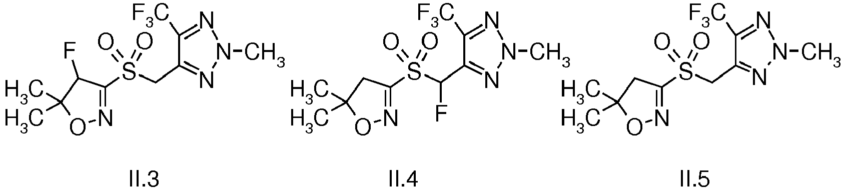 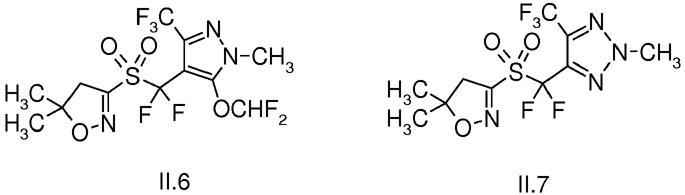 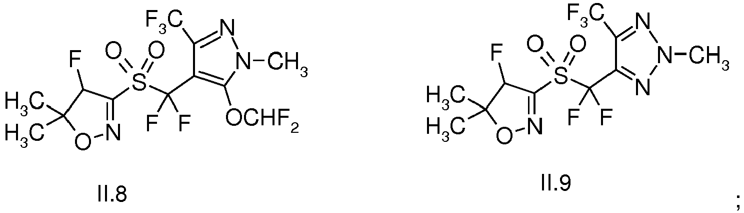
   N.11 Cellulose biosynthesis inhibitors: chlorthiamid, dichlobenil, flupoxam, indaziflam, isoxaben, triaziflam, 1-cyclohexyl-5-pentafluorphenyloxy-14-[1,2,4,6]thiatriazin-3-ylamine (CAS 175899-01-1);
   N.12 Decoupler herbicides: dinoseb, dinoterb, DNOC and its salts;
   N.13 Auxinic herbicides: 2,4-D and its salts and esters, clacyfos, 2,4-DB and its salts and esters, aminocyclopyrachlor and its salts and esters, aminopyralid and its salts such as aminopyralid-dimethylammonium, aminopyralid-tris(2-hydroxypropyl)ammonium and its esters, benazolin, benazolin-ethyl, chloramben and its salts and esters, clomeprop, clopyralid and its salts and esters, dicamba and its salts and esters, dichlorprop and its salts and esters, dichlorprop-P and its salts and esters, fluroxypyr, fluroxypyr-butometyl, fluroxypyr-meptyl, halauxifen and its salts and esters (CAS 943832-60-8); MCPA and its salts and esters, MCPAthioethyl, MCPB and its salts and esters, mecoprop and its salts and esters, mecoprop-P and its salts and esters, picloram and its salts and esters, quinclorac, quinmerac, TBA (2,3,6) and its salts and esters, triclopyr and its salts and esters, 4-amino-3-chloro-6-(4-chloro-2-fluoro-3-methoxyphenyl)-5-fluoropyridine-2-carboxylic acid, benzyl 4-amino-3-chloro-6-(4-chloro-2-fluoro-3-methoxyphenyl)-5-fluoropyridine-2-carboxylate (CAS 1390661-72-9);
   N.14 Auxin transport inhibitors: diflufenzopyr, diflufenzopyr-sodium, naptalam and naptalam-sodium;
   N.15 Other herbicides: bromobutide, chlorflurenol, chlorflurenol-methyl, cinmethylin, cumyluron, cyclopyrimorate (CAS 499223-49-3) and its salts and esters, dalapon, dazomet, difenzoquat, difenzoquat-metilsulfate, dimethipin, DSMA, dymron, endothal and its salts, etobenzanid, flurenol, flurenol-butyl, flurprimidol, fosamine, fosamine-ammonium, indanofan, maleic hydrazide, mefluidide, metam, methiozolin (CAS 403640-27-7), methyl azide, methyl bromide, methyl-dymron, methyl iodide, MSMA, oleic acid, oxaziclomefone, pelargonic acid, pyributicarb, quinoclamine, tridiphane;
O) Insecticides from classes O.1 to O.29
   O.1 Acetylcholine esterase (AChE) inhibitors: aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb and triazamate; acephate, azamethiphos, azinphos-ethyl, azinphosmethyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothio-phosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosa- lone, phosmet, phosphamidon, phoxim, pirimiphos- methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion;
   O.2 GABA-gated chloride channel antagonists: endosulfan, chlordane; ethiprole, fipronil, flufiprole, pyrafluprole, pyriprole;
   O.3 Sodium channel modulators: acrinathrin, allethrin, d-cis-trans allethrin, d-trans allethrin, bifenthrin, bioallethrin, bioallethrin S-cylclopentenyl, bioresmethrin, cycloprothrin, cyfluthrin, beta-cyfluthrin, cyhalothrin, lambda-cyhalothrin, gamma-cyhalothrin, cypermethrin, alphacypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, heptafluthrin, imiprothrin, meperfluthrin, metofluthrin, momfluorothrin, permethrin, phenothrin, prallethrin, profluthrin, pyrethrin (pyrethrum), resmethrin, silafluofen, tefluthrin, tetramethylfluthrin, tetramethrin, tralomethrin and transfluthrin; DDT, methoxychlor;
   O.4 Nicotinic acetylcholine receptor agonists (nAChR): acetamiprid, clothianidin, cycloxaprid, dinotefuran, imidacloprid, nitenpyram, thiacloprid, thiamethoxam; (2E)-1-[(6-chloropyridin-3-yl)methyl]-N'-nitro-2-pentylidenehydrazinecarboximidamide; 1-[(6-chloropyridin-3-yl)methyl]-7-methyl-8-nitro-5-propoxy-1,2,3,5,6,7-hexahydroimidazo[1,2-a]pyridine; nicotine;
   O.5 Nicotinic acetylcholine receptor allosteric activators: spinosad, spinetoram;
   O.6 Chloride channel activators: abamectin, emamectin benzoate, ivermectin, lepimectin, milbemectin;
   O.7 Juvenile hormone mimics: hydroprene, kinoprene, methoprene; fenoxycarb, pyriproxyfen;
   O.8 miscellaneous non-specific (multi-site) inhibitors: methyl bromide and other alkyl halides; chloropicrin, sulfuryl fluoride, borax, tartar emetic;
   O.9 Selective homopteran feeding blockers: pymetrozine, flonicamid;
   O.10 Mite growth inhibitors: clofentezine, hexythiazox, diflovidazin; etoxazole;
   O.11 Microbial disruptors of insect midgut membranes: *Bacillus thuringiensis, Bacillus sphaericus* and the insecticdal proteins they produce: *Bacillus thuringiensis* subsp. *israelensis*, *Bacillus sphaericus*, *Bacillus thuringiensis* subsp. *aizawai*, *Bacillus thuringiensis* subsp. *kurstaki*, *Bacillus thuringiensis* subsp. *tenebrionis*, the Bt crop proteins: Cry1Ab, Cry1Ac, Cry1Fa, Cry2Ab, mCry3A, Cry3Ab, Cry3Bb, Cry34/35Ab1;
   0.12 Inhibitors of mitochondrial ATP synthase: diafenthiuron; azocyclotin, cyhexatin, fenbutatin oxide, propargite, tetradifon;
   0.13 Uncouplers of oxidative phosphorylation via disruption of the proton gradient: chlorfenapyr, DNOC, sulfluramid;
   0.14 Nicotinic acetylcholine receptor (nAChR) channel blockers: bensultap, cartap hydrochloride, thiocyclam, thiosultap sodium;
   0.15 Inhibitors of the chitin biosynthesis type 0: bistrifluron, chlorfluazuron, diflubenzuron, flu-cycloxuron, flufenoxuron, hexaflumuron, lufenuron, novaluron, noviflumuron, teflubenzuron, triflumuron;
   0.16 Inhibitors of the chitin biosynthesis type 1: buprofezin;
   0.17 Moulting disruptors: cyromazine;
   0.18 Ecdyson receptor agonists: methoxyfenozide, tebufenozide, halofenozide, fufenozide, chromafenozide;
   O.19 Octopamin receptor agonists: amitraz;
   O.20 Mitochondrial complex III electron transport inhibitors: hydramethylnon, acequinocyl, fluacrypyrim;
   0.21 Mitochondrial complex I electron transport inhibitors: fenazaquin, fenpyroximate, pyrimidifen, pyridaben, tebufenpyrad, tolfenpyrad; rotenone;
   O.22 Voltage-dependent sodium channel blockers: indoxacarb, metaflumizone, 2-[2-(4-cyanophenyl)-1-[3-(trifluoromethyl)phenyl]ethylidene]-N-[4-(difluoromethoxy)phenyl]-hydrazine-carboxamide, N-(3-chloro-2-methylphenyl)-2-[(4-chlorophenyl)-[4-[methyl(methylsulfonyl)-amino]phenyl]methylene]-hydrazinecarboxamide;
   O.23 Inhibitors of the of acetyl CoA carboxylase: spirodiclofen, spiromesifen, spirotetramat; O.24 Mitochondrial complex IV electron transport inhibitors: aluminium phosphide, calcium phosphide, phosphine, zinc phosphide, cyanide;
   O.25 Mitochondrial complex II electron transport inhibitors: cyenopyrafen, cyflumetofen;
   O.28 Ryanodine receptor-modulators: flubendiamide, chlorantraniliprole, cyantraniliprole, cyclaniliprole, tetraniliprole; (R)-3-chloro-N1-{2-methyl-4-[1,2,2,2-tetrafluoro-1-(trifluoromethyl)-ethyl]phenyl}-N2-(1-methyl-2-methylsulfonylethyl)phthalamide, (S)-3-chloro-N1-{2-methyl-4-[1,2,2,2-tetrafluoro-1-(trifluoromethyl)ethyl]phenyl}-N2-(1-methyl-2-methylsulfonylethyl)-phthalamide, methyl-2-[3,5-dibromo-2-({[3-bromo-1-(3-chloropyridin-2-yl)-1H-pyrazol-5-yl]-carbonyl}amino)benzoyl]-1,2-dimethylhydrazinecarboxylate; N-[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide; N-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide; N-[4-chloro-2-[(di-2-propyl-lambda-4-sulfanylidene)carbamoyl]-6-methyl-phenyl]-2-(3-chloro-2-pyridyl)-5-(trifluoromethyl)pyrazole-3-carboxamide; N-[4,6-dichloro-2-[(di-2-propyl-lambda-4-sulfanylidene)carbamoyl]-phenyl]-2-(3-chloro-2-pyridyl)-5-(trifluoromethyl)pyrazole-3-carboxamide; N-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide; N-[2-(5-amino-1,3,4-thiadiazol-2-yl)-4-chloro-6-methylphenyl]-3-bromo-1-(3-chloro-2-pyridinyl)-1H-pyrazole-5-carboxamide; 3-chloro-1-(3-chloro-2-pyridinyl)-N-[2,4-dichloro-6-[[(1-cyano-1-methylethyl)amino]carbonyl]phenyl]-1H-pyrazole-5-carboxamide; 3-bromo-N-[2,4-dichloro-6-(methylcarbamoyl)phenyl]-1-(3,5-dichloro-2-pyridyl)-1H-pyrazole-5-carboxamide; N-[4-chloro-2-[[(1,1-dimethylethyl)amino]carbonyl]-6-methylphenyl]-1-(3-chloro-2-pyridinyl)-3-(fluoromethoxy)-1H-pyrazole-5-carboxamide; cyhalodiamide;
   O.29. insecticidal active compounds of unknown or uncertain mode of action: afidopyropen, afoxolaner, azadirachtin, amidoflumet, benzoximate, bifenazate, broflanilide, bromopropylate, chinomethionat, cryolite, dicloromezotiaz, dicofol, flufenerim, flometoquin, fluensulfone, fluhexafon, fluopyram, flupyradifurone, fluralaner, metoxadiazone, piperonyl butoxide, pyflubumide, pyridalyl, pyrifluquinazon, sulfoxaflor, tioxazafen, triflumezopyrim, 11-(4-chloro-2,6-dimethylphenyl)-12-hydroxy-1,4-dioxa-9-azadispiro[4.2.4.2]-tetradec-11-en-10-one, 3-(4'-fluoro-2,4-dimethylbiphenyl-3-yl)-4-hydroxy-8-oxa-1-azaspiro[4.5]dec-3-en-2-one, 1-[2-fluoro-4-methyl-5-[(2,2,2-trifluoroethyl)sulfinyl]phenyl]-3-(trifluoromethyl)-1H-1,2,4-triazole-5-amine, *Bacillus firmus*; (E/Z)-N-[1-[(6-chloro-3-pyridyl)methyl]-2-pyridylidene]-2,2,2-trifluoro-acetamide; (E/Z)-N-[1-[(6-chloro-5-fluoro-3-pyridyl)methyl]-2-pyridylidene]-2,2,2-trifluoro-acetamide; (E/Z)-2,2,2-trifluoro-N-[1-[(6-fluoro-3-pyridyl)methyl]-2-pyridyli- dene]acetamide; (E/Z)-N-[1-[(6-bromo-3-pyridyl)methyl]-2-pyridylidene]-2,2,2-trifluoro-acetamide; (E/Z)-N-[1-[1-(6-chloro-3-pyridyl)ethyl]-2-pyridylidene]-2,2,2-trifluoro-acetamide; (E/Z)-N-[1-[(6-chloro-3-pyridyl)methyl]-2-pyridylidene]-2,2-difluoro-acetamide; (E/Z)-2-chloro-N-[1-[(6-chloro-3-pyridyl)methyl]-2-pyridylidene]-2,2-difluoro-acetamide; (E/Z)-N-[1-[(2-chloropyrimidin-5-yl)methyl]-2-pyridylidene]-2,2,2-trifluoro-acetamide; (E/Z)-N-[1-[(6-chloro-3-pyridyl)methyl]-2-pyridylidene]-2,2,3,3,3-pentafluoro-propanamide.); N-[1-[(6-chloro-3-pyridyl)-methyl]-2-pyridylidene]-2,2,2-trifluoro-thioacetamide; N-[1-[(6-chloro-3-pyridyl)methyl]-2-pyridylidene]-2,2,2-trifluoro-N'-isopropyl-acetamidine; fluazaindolizine; 4-[5-(3,5-dichlorophenyl)-5-(trifluoromethyl)-4H-isoxazol-3-yl]-2-methyl-N-(1-oxothietan-3-yl)benzamide; fluxametamide; 5-[3-[2,6-dichloro-4-(3,3-dichloroallyloxy)phenoxy]propoxy]-1H-pyrazole; 3-(benzoyl-methylamino)-N-[2-bromo-4-[1,2,2,3,3,3-hexafluoro-1-(trifluoromethyl)propyl]-6-(trifluoromethyl)phenyl]-2-fluoro-benzamide; 3-(benzoylmethylamino)-2-fluoro-N-[2-iodo-4-[1,2,2,2-tetrafluoro-1-(trifluoromethyl)ethyl]-6-(trifluoromethyl)phenyl]-benzamide; N-[3-[[[2-iodo-4-[1,2,2,2-tetrafluoro-1-(trifluoromethyl)ethyl]-6-(trifluoromethyl)phenyl]amino]carbonyl]-phenyl]-N-methyl-benzamide; N-[3-[[[2-bromo-4-[1,2,2,2-tetrafluoro-1-(trifluoromethyl)ethyl]-6-(trifluoromethyl)phenyl]amino]carbonyl]-2-fluorophenyl]-4-fluoro-N-methyl-benzamide; 4-fluoro-N-[2-fluoro-3-[[[2-iodo-4-[1,2,2,2-tetrafluoro-1-(trifluoromethyl)ethyl]-6-(trifluoromethyl)phenyl]amino]carbonyl]phenyl]-N-methyl-benzamide; 3-fluoro-N-[2-fluoro-3-[[[2-iodo-4-[1,2,2,2-tetrafluoro-1-(trifluoromethyl)ethyl]-6(trifluoromethyl)phenyl]amino]carbonyl]phenyl]-N-methyl-benzamide; 2-chloro-N-[3-[[[2-iodo-4-[1,2,2,2-tetrafluoro-1-(trifluoromethyl)-ethyl]-6-(trifluoromethyl)phenyl]amino]carbonyl]phenyl]-3-pyridinecarboxamide; 4-cyano-N-[2-cyano-5-[[2,6-dibromo-4-[1,2,2,3,3,3-hexafluoro-1-(trifluoromethyl)propyl]phenyl]carbamoyl]phenyl]-2-methyl-benzamide; 4-cyano-3-[(4-cyano-2-methyl-benzoyl)amino]-N-[2,6-dichloro-4-[1,2,2,3,3,3-hexafluoro-1-(trifluoromethyl)propyl]phenyl]-2-fluoro-benzamide; N-[5-[[2-chloro-6-cyano-4-[1,2,2,3,3,3-hexafluoro-1-(trifluoromethyl)propyl]phenyl]carbamoyl]-2-cyano-phenyl]-4-cyano-2-methyl-benzamide; N-[5-[[2-bromo-6-chloro-4-[2,2,2-trifluoro-1-hydroxy-1-(trifluoromethyl)ethyl]phenyl]carbamoyl]-2-cyano-phenyl]-4-cyano-2-methyl-benzamide; N-[5-[[2-bromo-6-chloro-4-[1,2,2,3,3,3-hexafluoro-1-(trifluoromethyl)-propyl]phenyl]carbamoyl]-2-cyano-phenyl]-4-cyano-2-methyl-benzamide; 4-cyano-N-[2-cyano-5-[[2,6-dichloro-4-[1,2,2,3,3,3-hexafluoro-1-(trifluoromethyl)propyl]phenyl]carbamoyl]-phenyl]-2-methyl-benzamide; 4-cyano-N-[2-cyano-5-[[2,6-dichloro-4-[1,2,2,2-tetrafluoro-1-(trifluoromethyl)ethyl]phenyl]carbamoyl]phenyl]-2-methyl-benzamide; N-[5-[[2-bromo-6-chloro-4-[1,2,2,2-tetrafluoro-1-(trifluoromethyl)ethyl]phenyl]carbamoyl]-2-cyano-phenyl]-4-cyano-2-methyl-benzamide; 2-(1,3-dioxan-2-yl)-6-[2-(3-pyridinyl)-5-thiazolyl]-pyridine; 2-[6-[2-(5-fluoro-3-pyridinyl)-5-thiazolyl]-2-pyridinyl]-pyrimidine; 2-[6-[2-(3-pyridinyl)-5-thiazolyl]-2-pyridinyl]-pyrimidine; N-methylsulfonyl-6-[2-(3-pyridyl)thiazol-5-yl]pyridine-2-carboxamide; N-methylsulfonyl-6-[2-(3-pyridyl)thiazol-5-yl]pyridine-2-carboxamide; N-ethyl-N-[4-methyl-2-(3-pyridyl)thiazol-5-yl]-3-methylthio-propanamide; N-methyl-N-[4-methyl-2-(3-pyridyl)thiazol-5-yl]-3-methylthio-propanamide; N,2-dimethyl-N-[4-methyl-2-(3-pyridyl)-thiazol-5-yl]-3-methylthio-propanamide; N-ethyl-2-methyl-N-[4-methyl-2-(3-pyridyl)thiazol-5-yl]-3-methylthio-propanamide; N-[4-chloro-2-(3-pyridyl)thiazol-5-yl]-N-ethyl-2-methyl-3-methylthio-propanamide; N-[4-chloro-2-(3-pyridyl)thiazol-5-yl]-N,2-dimethyl-3-methylthio-propanamide; N-[4-chloro-2-(3-pyridyl)thiazol-5-yl]-N-methyl-3-methylthio-propanamide; N-[4-chloro-2-(3-pyridyl)thiazol-5-yl]-N-ethyl-3-methylthio-propanamide; 1-[(6-chloro-3-pyridinyl)methyl]-1,2,3,5,6,7-hexahydro-5-methoxy-7-methyl-8-nitro-imidazo[1,2-a]pyridine; 1-[(6-chloropyridin-3-yl)methyl]-7-methyl-8-nitro-1,2,3,5,6,7-hexahydroimidazo[1,2-a]pyridin-5-ol; 1-isopropyl-N,5-dimethyl-N-pyridazin-4-yl-pyrazole-4-carboxamide; 1-(1,2-dimethylpropyl)-N-ethyl-5-methyl-N-pyridazin-4-yl-pyrazole-4-carboxamide; N,5-dimethyl-N-pyridazin-4-yl-1-(2,2,2-trifluoro-1-methyl-ethyl)pyrazole-4-carboxamide; 1-[1-(1-cyanocyclopropyl)ethyl]-N-ethyl-5-methyl-N-pyridazin-4-yl-pyrazole-4-carboxamide; N-ethyl-1-(2-fluoro-1-methylpropyl)-5-methyl-N-pyridazin-4-yl-pyrazole-4-carboxamide; 1-(1,2-dimethylpropyl)-N,5-dimethyl-N-pyridazin-4-yl-pyrazole-4-carboxamide; 1-[1-(1-cyanocyclopropyl)ethyl]-N,5-dimethyl-N-pyridazin-4-yl-pyrazole-4-carboxamide; N-methyl-1-(2-fluoro-1-methyl-propyl]-5-methyl-N-pyridazin-4-yl-pyrazole-4-carboxamide; 1-(4,4-difluorocyclohexyl)-N-ethyl-5-methyl-N-pyridazin-4-yl-pyrazole-4-carboxamide; 1-(4,4-difluorocyclohexyl)-N,5-dimethyl-N-pyridazin-4-yl-pyrazole-4-carboxamide, N-(1-methylethyl)-2-(3-pyridinyl)-2H-indazole-4-carboxamide; N-cyclopropyl-2-(3-pyridinyl)-2H-indazole-4-carboxamide; N-cyclohexyl-2-(3-pyridinyl)-2H-indazole-4-carboxamide; 2-(3-pyridinyl)-N-(2,2,2-trifluoroethyl)-2H-indazole-4-carboxamide; 2-(3-pyridinyl)-N-[(tetrahydro-2-furanyl)methyl]-2H-indazole-5-carboxamide; methyl 2-[[2-(3-pyridinyl)-2H-indazol-5-yl]carbonyl]hydrazinecarboxylate; N-[(2,2-difluorocyclopropyl)methyl]-2-(3-pyridinyl)-2H-indazole-5-carboxamide; N-(2,2-difluoropropyl)-2-(3-pyridinyl)-2H-indazole-5-carboxamide; 2-(3-pyridinyl)-N-(2-pyrimidinylmethyl)-2H-indazole-5-carboxamide; N-[(5-methyl-2-pyrazinyl)methyl]-2-(3-pyridinyl)-2H-indazole-5-carboxamide, N-[3-chloro-1-(3-pyridyl)pyrazol-4-yl]-N-ethyl-3-(3,3,3-trifluoropropylsulfanyl)-propanamide; N-[3-chloro-1-(3-pyridyl)pyrazol-4-yl]-N-ethyl-3-(3,3,3-trifluoropropylsulfinyl)-propanamide; N-[3-chloro-1-(3-pyridyl)pyrazol-4-yl]-3-[(2,2-difluorocyclopropyl)methyl-sulfanyl]-N-ethyl-propanamide; N-[3-chloro-1-(3-pyridyl)pyrazol-4-yl]-3-[(2,2-difluorocyclo-propyl)methylsulfinyl]-N-ethyl-propanamide; sarolaner, lotilaner.

Preference is also given to mixtures comprising as component 2) at least one active substance selected from inhibitors of complex III at Qₒ site in group A), more preferably selected from compounds (A.1.1), (A.1.4), (A.1.8), (A.1.9), (A.1.10), (A.1.12), (A.1.13), (A.1.14), (A.1.17), (A.1.21), (A.1.24), (A.1.25), (A.1.26), (A.1.27), (A.1.28), (A.1.29), (A.1.30), (A.1.31), (A.1.32), (A.1.33), (A.1.34) and (A.1.35); particularly selected from (A.1.1), (A.1.4), (A.1.8), (A.1.9), (A.1.13), (A.1.14), (A.1.17), (A.1.24), (A.1.25), (A.1.26), (A.1.27), (A.1.28), (A.1.29), (A.1.30), (A.1.31), (A.1.32), (A.1.33), (A.1.34) and (A.1.35).

Preference is also given to mixtures comprising as component 2) at least one active substance selected from inhibitors of complex III at Qᵢ site in group A), more preferably selected from compounds (A.2.1), (A.2.3), (A.2.4) and (A.2.5); particularly selected from (A.2.3), (A.2.4) and (A.2.5).

Preference is also given to mixtures comprising as component 2) at least one active substance selected from inhibitors of complex II in group A), more preferably selected from compounds (A.3.2), (A.3.3), (A.3.4), (A.3.7), (A.3.9), (A.3.11), (A.3.12), (A.3.14), (A.3.16), (A.3.19), (A.3.20), (A.3.21), (A.3.22), (A.3.23), (A.3.24), (A.3.25), (A.3.27) and (A.3.28); particularly selected from (A.3.2), (A.3.3), (A.3.4), (A.3.7), (A.3.9), (A.3.12), (A.3.14), (A.3.17), (A.3.19), (A.3.22), (A.3.23), (A.3.24), (A.3.25), (A.3.27), (A.3.28) and (A.3.29).

Preference is also given to mixtures comprising as component 2) at least one active substance selected from other respiration nhibitors in group A), more preferably selected from compounds (A.4.4) and (A.4.11); in particular (A.4.11).

Preference is also given to mixtures comprising as component 2) at least one active substance selected from C14 demethylase inhibitors in group B), more preferably selected from compounds (B.1.4), (B.1.5), (B.1.8), (B.1.10), (B.1.11), (B.1.12), (B.1.13), (B.1.17), (B.1.18), (B.1.21), (B.1.22), (B.1.23), (B.1.25), (B.1.26), (B.1.29), (B.1.31), (B.1.32), (B.1.33), (B.1.34), (B.1.35), (B.1.36), (B.1.37), (B.1.38), (B.1.39), (B.1.40), (B.1.41), (B.1.42), (B.1.43) and (B.1.46); particularly selected from (B.1.5), (B.1.8), (B.1.10), (B.1.17), (B.1.23), (B.1.25), (B.1.31), (B.1.32), (B.1.33), (B.1.34), (B.1.35), (B.1.36), (B.1.37), (B.138), (B.1.39), (B.1.40), (B.1.41), (B.1.42), (B.1.43) and (B.1.46).

Preference is also given to mixtures comprising as component 2) at least one active substance selected from Delta14-reductase inhibitors in group B), more preferably selected from compounds (B.2.4), (B.2.5), (B.2.6) and (B.2.8); in particular (B.2.4).

Preference is also given to mixtures comprising as component 2) at least one active substance selected from phenylamides and acyl amino acid fungicides in group C), more preferably selected from compounds (C.1.1), (C.1.2), (C.1.4) and (C.1.5); particularly selected from (C.1.1) and (C.1.4).

Preference is also given to mixtures comprising as component 2) at least one active substance selected from other nucleic acid synthesis inhibitors in group C), more preferably selected from compounds (C.2.6) and (C.2.7).

Preference is also given to mixtures comprising as component 2) at least one active substance selected from group D), more preferably selected from compounds (D.1.1), (D.1.2), (D.1.5), (D.2.4) and (D.2.6); particularly selected from (D.1.2), (D.1.5) and (D.2.6).

Preference is also given to mixtures comprising as component 2) at least one active substance selected from group E), more preferably selected from compounds (E.1.1), (E.1.3), (E.2.2) and (E.2.3); in particular (E.1.3).

Preference is also given to mixtures comprising as component 2) at least one active substance selected from group F), more preferably selected from compounds (F.1.2), (F.1.4) and (F.1.5).

Preference is also given to mixtures comprising as component 2) at least one active substance selected from group G), more preferably selected from compounds (G.3.1), (G.3.3), (G.3.6), (G.5.1), (G.5.2) and (G.5.3); particularly selected from (G.3.1), (G.5.1), (G.5.2) and (G.5.3).

Preference is also given to mixtures comprising as component 2) at least one active substance selected from group H), more preferably selected from compounds (H.2.2), (H.2.3), (H.2.5), (H.2.7), (H.2.8), (H.3.2), (H.3.4), (H.3.5), (H.4.9) and (H.4.10); particularly selected from (H.2.2), (H.2.5), (H.3.2), (H.4.9) and (H.4.10).

Preference is also given to mixtures comprising as component 2) at least one active substance selected from group I), more preferably selected from compounds (1.2.2) and (1.2.5).

Preference is also given to mixtures comprising as component 2) at least one active substance selected from group J), more preferably selected from compounds (J.1.2), (J.1.5) and (J.1.8); in particular (J.1.5).

Preference is also given to mixtures comprising as component 2) at least one active substance selected from group K), more preferably selected from compounds (K.1.41), (K.1.42), (K.1.44), (K.1.45), (K.1.46), (K.1.47), (K.1.48) and (K.1.49); particularly selected from (K.1.41), (K.1.44), (K.1.45), (K.1.46), (K.1.47), (K.1.48) and (K.1.49).

Preferred two-component compositions comprising a compound of formula I according to the present invention are compiled in the following Table D:

**Table D: Individual preferred pesticidal compositions D-1 to D-504comprising a particular compound of formula I, namely one of I.Aa-1 to I.Aa-5,I.Ba-1 and I.Ca-1 as component I (from Table C above) and a particular second pesticidal compound as component II:**

| **composition** | **component I compound from Table B aove** | **component II** |
|---|---|---|
| D-1 | I.Aa-1 | Pyraclostrobin |
| D-2 | I.Aa-1 | Azoxystrobin |
| D-3 | I.Aa-1 | Trifloxystrobin |
| D-4 | I.Aa-1 | Picoxystrobin |
| D-5 | I.Aa-1 | Fluoxastrobin |
| D-6 | I.Aa-1 | Dimoxystrobin |
| D-7 | I.Aa-1 | Kresoxim-methyl |
| D-8 | I.Aa-1 | (2E,3Z)-5-[[1-(2,4-dichlorophenyl)-1H-pyrazol-3-yl]oxy]-2-(methoxyimino)-N,3-dimethyl-pent-3-enamide |
| D-9 | I.Aa-1 | (2E,3Z)-5-[[1-(4-chlorophenyl)-1H-pyrazol-3-yl]oxy]-2-(methoxyimino)-N,3-dimethyl-pent-3-enamide |
| D-10 | I.Aa-1 | [(3S,6S,7R,8R)-8-benzyl-3-[(3-acetoxy-4 methoxy-pyridine-2-carbonyl)amino]-6-methyl-4,9-dioxo-1,5-dioxonan-7-yl] 2 methylpropanoate |
| D-11 | I.Aa-1 | [(3S,6S,7R,8R)-8-benzyl-3-[[3-(acetoxymethoxy)-4-methoxy-pyridine-2 carbonyl]amino]-6-methyl-4,9-dioxo-1,5-dioxonan-7-yl] 2 methylpropanoate |
| D-12 | I.Aa-1 | [(3S,6S, 7R,8R)-8-benzyl-3-[(3-isobutoxycarbonyloxy-4-methoxy-pyridine-2 carbonyl)amino]-6-methyl-4,9-dioxo-1,5-dioxonan-7-yl] 2-methylpropanoate |
| D-13 | I.Aa-1 | [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate |
| D-14 | I.Aa-1 | (3S,6S,7R,8R)-3-[[(3-hydroxy-4-methoxy-2-pyridinyl)carbonyl]amino]-6 methyl-4,9-dioxo-8-(phenylmethyl)-1,5-dioxonan-7-yl 2-methylpropanoate |
| D-15 | I.Aa-1 | Fluxapyroxad |
| D-16 | I.Aa-1 | Boscalid |
| D-17 | I.Aa-1 | Bixafen |
| D-18 | I.Aa-1 | Isopyrazam |
| D-19 | I.Aa-1 | Benzovindiflupyr |
| D-20 | I.Aa-1 | Fluopyram |
| D-21 | I.Aa-1 | N-[(5-chloro-2-isopropyl-phenyl)methyl]-N-cyclopropyl-5-fluoro-1,3-dimethyl-pyrazole-4-carboxamide |
| D-22 | I.Aa-1 | Sedaxane |
| D-23 | I.Aa-1 | Penflufen |
| D-24 | I.Aa-1 | N-[2-(2,4-dichlorophenyl)-2-methoxy-1-methyl-ethyl]-3-(difluoromethyl)-1-methyl-pyrazole-4-carboxamide |
| D-25 | I.Aa-1 | 3 (difluoromethyl)-1-methyl-N-(1,1,3-trimethylindan-4-yl)pyrazole-4-carboxamide |
| D-26 | I.Aa-1 | 3 (trifluoromethyl)-1-methyl-N-(1,1,3-trimethylindan-4-yl)pyrazole-4-carboxamide |
| D-27 | I.Aa-1 | 1,3-dimethyl-N-(1,1,3-trimethylindan-4-yl)pyrazole-4-carboxamide |
| D-28 | I.Aa-1 | 3-(trifluoromethyl)-1,5-dimethyl-N-(1,1,3-trimethylindan-4-yl)pyrazole-4-carboxamide |
| D-29 | I.Aa-1 | 1,3,5-trimethyl-N-(1,1,3-trimethylindan-4-yl)pyrazole-4-carboxamide |
| D-30 | I.Aa-1 | 1-[3-chloro-2-[[[1-(4-chlorophenyl)-1H-pyrazol-3-yl]oxy]methyl]phenyl]-1,4-dihydro-4-methyl-5H-tetrazol-5-one |
| D-31 | I.Aa-1 | Ametoctradin |
| D-32 | I.Aa-1 | epoxiconazole |
| D-33 | I.Aa-1 | metconazole |
| D-34 | I.Aa-1 | prothioconazole |
| D-35 | I.Aa-1 | difenoconazole |
| D-36 | I.Aa-1 | fluquinconazole |
| D-37 | I.Aa-1 | propiconazole |
| D-38 | I.Aa-1 | tebuconazole |
| D-39 | I.Aa-1 | 2-[2-chloro-4-(4-chlorophenoxy)phenyl]-1 (1,2,4-triazol-1-yl)pentan-2-ol |
| D-40 | I.Aa-1 | 1-[4-(4-chlorophenoxy)-2-(trifluoromethyl)phenyl]-1 cyclopropyl-2-(1,2,4-triazol-1-yl)ethanol |
| D-41 | I.Aa-1 | 2-[4-(4-chlorophenoxy)-2-(trifluoromethyl)phenyl]-1 -(1,2,4-triazol-1 - yl)butan-2-ol |
| D-42 | I.Aa-1 | 2-[2-chloro-4-(4-chlorophenoxy)phenyl]-1-(1,2,4-triazol-1-yl)butan-2-ol |
| D-43 | I.Aa-1 | 2-[4-(4-chlorophenoxy)-2-(trifluoromethyl)phenyl]-3-methyl-1-(1,2,4-triazol-1-yl)butan-2-ol |
| D-44 | I.Aa-1 | 2-[4-(4-chlorophenoxy)-2-(trifluoromethyl)phenyl]-1-(1,2,4-triazol-1-yl)propan-2-ol |
| D-45 | I.Aa-1 | 2-[2-chloro-4-(4-chlorophenoxy)phenyl]-3-methyl-1-(1,2,4-triazol-1-yl)butan-2-ol |
| D-46 | I.Aa-1 | 2-[4-(4-chlorophenoxy)-2-(trifluoromethyl)phenyl]-1-(1,2,4-triazol-1-yl)pentan-2-ol |
| D-47 | I.Aa-1 | 2-[4-(4-fluorophenoxy)-2-(trifluoromethyl)phenyl]-1-(1,2,4-triazol-1-yl)propan-2-ol |
| D-48 | I.Aa-1 | 2-[2-chloro-4-(4-chlorophenoxy)phenyl]-1-(1,2,4-triazol-1-yl)pent-3-yn-2-ol |
| D-49 | I.Aa-1 | prochloraz |
| D-50 | I.Aa-1 | [3-(4-chloro-2-fluoro-phenyl)-5-(2,4-difluorophenyl)isoxazol-4-yl]-(3-pyridyl)methanol |
| D-51 | I.Aa-1 | fenpropimorph |
| D-52 | I.Aa-1 | Metalaxyl |
| D-53 | I.Aa-1 | Benalaxyl |
| D-54 | I.Aa-1 | Thiophanate-methyl |
| D-55 | I.Aa-1 | Carbendazim |
| D-56 | I.Aa-1 | Metrafenone |
| D-57 | I.Aa-1 | Pyrimethanil |
| D-58 | I.Aa-1 | Iprodione |
| D-59 | I.Aa-1 | Vinclozolin |
| D-60 | I.Aa-1 | Fludioxonil |
| D-61 | I.Aa-1 | dimethomorph |
| D-62 | I.Aa-1 | oxathiapiprolin |
| D-63 | I.Aa-1 | metiram |
| D-64 | I.Aa-1 | mancozeb |
| D-65 | I.Aa-1 | chlorothalonil |
| D-66 | I.Aa-1 | dithianon |
| D-67 | I.Aa-1 | Dipymetitrone |
| D-68 | I.Aa-1 | prohexadione-calcium |
| D-69 | I.Aa-1 | 3-(5-fluoro-3,3,4,4-tetramethyl-3,4-dihydroisoquinolin-1-yl)quinoline |
| D-70 | I.Aa-1 | 3-(4,4-difluoro-3,3-dimethyl-3,4-dihydroisoquinolin-1-yl)quinoline |
| D-71 | I.Aa-1 | 3-(4,4,5-trifluoro-3,3-dimethyl-3,4-dihydroisoquinolin-1-yl)quinoline |
| D-72 | I.Aa-1 | 9-fluoro-2,2-dimethyl-5-(3-quinolyl)-3H 1,4-benzoxazepine |
| D-73 | I.Aa-2 | Pyraclostrobin |
| D-74 | I.Aa-2 | Azoxystrobin |
| D-75 | I.Aa-2 | Trifloxystrobin |
| D-76 | I.Aa-2 | Picoxystrobin |
| D-77 | I.Aa-2 | Fluoxastrobin |
| D-78 | I.Aa-2 | Dimoxystrobin |
| D-79 | I.Aa-2 | Kresoxim-methyl |
| D-80 | I.Aa-2 | (2E,3Z)-5-[[1-(2,4-dichlorophenyl)-1H-pyrazol-3-yl]oxy]-2-(methoxyimino)-N,3-dimethyl-pent-3-enamide |
| D-81 | I.Aa-2 | (2E,3Z)-5-[[1-(4-chlorophenyl)-1H-pyrazol-3-yl]oxy]-2-(methoxyimino)-N,3-dimethyl-pent-3-enamide |
| D-82 | I.Aa-2 | [(3S,6S,7R,8R)-8-benzyl-3-[(3-acetoxy-4 methoxy-pyridine-2-carbonyl)amino]-6-methyl-4,9-dioxo-1,5-dioxonan-7-yl] 2 methylpropanoate |
| D-83 | I.Aa-2 | [(3S,6S,7R,8R)-8-benzyl-3-[[3-(acetoxymethoxy)-4-methoxy-pyridine-2 carbonyl]amino]-6-methyl-4,9-dioxo-1,5-dioxonan-7-yl] 2 methylpropanoate |
| D-84 | I.Aa-2 | [(3S,6S,7R,8R)-8-benzyl-3-[(3-isobutoxycarbonyloxy-4-methoxy-pyridine-2 carbonyl)amino]-6-methyl-4,9-dioxo-1,5-dioxonan-7-yl] 2-methylpropanoate |
| D-85 | I.Aa-2 | [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate |
| D-86 | I.Aa-2 | (3S,6S,7R,8R)-3-[[(3-hydroxy-4-methoxy-2-pyridinyl)carbonyl]amino]-6 methyl-4,9-dioxo-8-(phenylmethyl)-1,5-dioxonan-7-yl 2-methylpropanoate |
| D-87 | I.Aa-2 | Fluxapyroxad |
| D-88 | I.Aa-2 | Boscalid |
| D-89 | I.Aa-2 | Bixafen |
| D-90 | I.Aa-2 | Isopyrazam |
| D-91 | I.Aa-2 | Benzovindiflupyr |
| D-92 | I.Aa-2 | Fluopyram |
| D-93 | I.Aa-2 | N-[(5-chloro-2-isopropyl-phenyl)methyl]-N-cyclopropyl-5-fluoro-1,3-dimethyl-pyrazole-4-carboxamide |
| D-94 | I.Aa-2 | Sedaxane |
| D-95 | I.Aa-2 | Penflufen |
| D-96 | I.Aa-2 | N-[2-(2,4-dichlorophenyl)-2-methoxy-1-methyl-ethyl]-3-(difluoromethyl)-1-methyl-pyrazole-4-carboxamide |
| D-97 | I.Aa-2 | 3 (difluoromethyl)-1-methyl-N-(1,1,3-trimethylindan-4-yl)pyrazole-4-carboxamide |
| D-98 | I.Aa-2 | 3 (trifluoromethyl)-1-methyl-N-(1,1,3-trimethylindan-4-yl)pyrazole-4-carboxamide |
| D-99 | I.Aa-2 | 1,3-dimethyl-N-(1,1,3-trimethylindan-4-yl)pyrazole-4-carboxamide |
| D-100 | I.Aa-2 | 3-(trifluorometh¬yl)-1,5-dimethyl-N-(1,1,3-trimethylindan-4-yl)pyrazole-4-carboxamide |
| D-101 | I.Aa-2 | 1,3,5-trimethyl-N-(1,1,3-trimethylindan-4-yl)pyrazole-4-carboxamide |
| D-102 | I.Aa-2 | 1-[3-chloro-2-[[[1-(4-chlorophenyl)-1 H-pyrazol-3-yl]oxy]methyl]phenyl]-1,4-dihydro-4-methyl-5H-tetrazol-5-one |
| D-103 | I.Aa-2 | Ametoctradin |
| D-104 | I.Aa-2 | epoxiconazole |
| D-105 | I.Aa-2 | metconazole |
| D-106 | I.Aa-2 | prothioconazole |
| D-107 | I.Aa-2 | difenoconazole |
| D-108 | I.Aa-2 | fluquinconazole |
| D-109 | I.Aa-2 | propiconazole |
| D-110 | I.Aa-2 | tebuconazole |
| D-111 | I.Aa-2 | 2-[2-chloro-4-(4-chlorophenoxy)phenyl]-1 (1,2,4-triazol-1-yl)pentan-2-ol |
| D-112 | I.Aa-2 | 1-[4-(4-chlorophenoxy)-2-(trifluoromethyl)phenyl]-1 cyclopropyl-2-(1,2,4-triazol-1-yl)ethanol |
| D-113 | I.Aa-2 | 2-[4-(4-chlorophenoxy)-2-(trifluoromethyl)phenyl]-1-(1,2,4-triazol-1-yl)butan-2-ol |
| D-114 | I.Aa-2 | 2-[2-chloro-4-(4-chlorophenoxy)phenyl]-1-(1,2,4-triazol-1-yl)butan-2-ol |
| D-115 | I.Aa-2 | 2-[4-(4-chlorophenoxy)-2-(trifluoromethyl)phenyl]-3-methyl-1-(1,2,4-triazol-1-yl)butan-2-ol |
| D-116 | I.Aa-2 | 2-[4-(4-chlorophenoxy)-2-(trifluoromethyl)phenyl]-1-(1,2,4-triazol-1-yl)propan-2-ol |
| D-117 | I.Aa-2 | 2-[2-chloro-4-(4-chlorophenoxy)phenyl]-3-methyl-1-(1,2,4-triazol-1-yl)butan-2-ol |
| D-118 | I.Aa-2 | 2-[4-(4-chlorophenoxy)-2-(trifluoromethyl)phenyl]-1-(1,2,4-triazol-1-yl)pentan-2-ol |
| D-119 | I.Aa-2 | 2-[4-(4-fluorophenoxy)-2-(trifluoromethyl)phenyl]-1-(1,2,4-triazol-1-yl)propan-2-ol |
| D-120 | I.Aa-2 | 2-[2-chloro-4-(4-chlorophenoxy)phenyl]-1-(1,2,4-triazol-1-yl)pent-3-yn-2-ol |
| D-121 | I.Aa-2 | prochloraz |
| D-122 | I.Aa-2 | [3-(4-chloro-2-fluoro-phenyl)-5-(2,4-difluorophenyl)isoxazol-4-yl]-(3-pyridyl)methanol |
| D-123 | I.Aa-2 | fenpropimorph |
| D-124 | I.Aa-2 | Metalaxyl |
| D-125 | I.Aa-2 | Benalaxyl |
| D-126 | I.Aa-2 | Thiophanate-methyl |
| D-127 | I.Aa-2 | Carbendazim |
| D-128 | I.Aa-2 | Metrafenone |
| D-129 | I.Aa-2 | Pyrimethanil |
| D-130 | I.Aa-2 | Iprodione |
| D-131 | I.Aa-2 | Vinclozolin |
| D-132 | I.Aa-2 | Fludioxonil |
| D-133 | I.Aa-2 | dimethomorph |
| D-134 | I.Aa-2 | oxathiapiprolin |
| D-135 | I.Aa-2 | metiram |
| D-136 | I.Aa-2 | mancozeb |
| D-137 | I.Aa-2 | chlorothalonil |
| D-138 | I.Aa-2 | dithianon |
| D-139 | I.Aa-2 | Dipymetitrone |
| D-140 | I.Aa-2 | prohexadione-calcium |
| D-141 | I.Aa-2 | 3-(5-fluoro-3,3,4,4-tetramethyl-3,4-dihydroisoquinolin-1-yl)quinoline |
| D-142 | I.Aa-2 | 3-(4,4-difluoro-3,3-dimethyl-3,4-dihydroisoquinolin-1-yl)quinoline |
| D-143 | I.Aa-2 | 3-(4,4,5-trifluoro-3,3-dimethyl-3,4-dihydroisoquinolin-1-yl)quinoline |
| D-144 | I.Aa-2 | 9-fluoro-2,2-dimethyl-5-(3-quinolyl)-3H 1,4-benzoxazepine |
| D-145 | I.Aa-3 | Pyraclostrobin |
| D-146 | I.Aa-3 | Azoxystrobin |
| D-147 | I.Aa-3 | Trifloxystrobin |
| D-148 | I.Aa-3 | Picoxystrobin |
| D-149 | I.Aa-3 | Fluoxastrobin |
| D-150 | I.Aa-3 | Dimoxystrobin |
| D-151 | I.Aa-3 | Kresoxim-methyl |
| D-152 | I.Aa-3 | (2E,3Z)-5-[[1-(2,4-dichlorophenyl)-1H-pyrazol-3-yl]oxy]-2-(methoxyimino)-N,3-dimethyl-pent-3-enamide |
| D-153 | I.Aa-3 | (2E,3Z)-5-[[1-(4-chlorophenyl)-1H-pyrazol-3-yl]oxy]-2-(methoxyimino)-N,3-dimethyl-pent-3-enamide |
| D-154 | I.Aa-3 | [(3S,6S,7R,8R)-8-benzyl-3-[(3-acetoxy-4 methoxy-pyridine-2-carbonyl)amino]-6-methyl-4,9-dioxo-1,5-dioxonan-7-yl] 2 methylpropanoate |
| D-155 | I.Aa-3 | [(3S,6S,7R,8R)-8-benzyl-3-[[3-(acetoxymethoxy)-4-methoxy-pyridine-2 carbonyl]amino]-6-methyl-4,9-dioxo-1,5-dioxonan-7-yl] 2 methylpropanoate |
| D-156 | I.Aa-3 | [(3S,6S, 7R,8R)-8-benzyl-3-[(3-isobutoxycarbonyloxy-4-methoxy-pyridine-2 carbonyl)amino]-6-methyl-4,9-dioxo-1,5-dioxonan-7-yl] 2-methylpropanoate |
| D-157 | I.Aa-3 | [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate |
| D-158 | I.Aa-3 | (3S,6S,7R,8R)-3-[[(3-hydroxy-4-methoxy-2-pyridinyl)carbonyl]amino]-6 methyl-4,9-dioxo-8-(phenylmethyl)-1 ,5-dioxonan-7-yl 2-methylpropanoate |
| D-159 | I.Aa-3 | Fluxapyroxad |
| D-160 | I.Aa-3 | Boscalid |
| D-161 | I.Aa-3 | Bixafen |
| D-162 | I.Aa-3 | Isopyrazam |
| D-163 | I.Aa-3 | Benzovindiflupyr |
| D-164 | I.Aa-3 | Fluopyram |
| D-165 | I.Aa-3 | N-[(5-chloro-2-isopropyl-phenyl)methyl]-N-cyclopropyl-5-fluoro-1,3-dimethyl-pyrazole-4-carboxamide |
| D-166 | I.Aa-3 | Sedaxane |
| D-167 | I.Aa-3 | Penflufen |
| D-168 | I.Aa-3 | N-[2-(2,4-dichlorophenyl)-2-methoxy-1-methyl-ethyl]-3-(difluoromethyl)-1-methyl-pyrazole-4-carboxamide |
| D-169 | I.Aa-3 | 3 (difluoromethyl)-1-methyl-N-(1,1,3-trimethylindan-4-yl)pyrazole-4-carboxamide |
| D-170 | I.Aa-3 | 3 (trifluoromethyl)-1-methyl-N-(1,1,3-trimethylindan-4-yl)pyrazole-4-carboxamide |
| D-171 | I.Aa-3 | 1,3-dimethyl-N-(1,1,3-trimethylindan-4-yl)pyrazole-4-carboxamide |
| D-172 | I.Aa-3 | 3-(trifluorometh¬yl)-1,5-dimethyl-N-(1,1,3-trimethylindan-4-yl)pyrazole-4-carboxamide |
| D-173 | I.Aa-3 | 1,3,5-trimethyl-N-(1,1,3-trimethylindan-4-yl)pyrazole-4-carboxamide |
| D-174 | I.Aa-3 | 1-[3-chloro-2-[[[1-(4-chlorophenyl)-1H-pyrazol-3-yl]oxy]methyl]phenyl]-1,4-dihydro-4-methyl-5H-tetrazol-5-one |
| D-175 | I.Aa-3 | Ametoctradin |
| D-176 | I.Aa-3 | epoxiconazole |
| D-177 | I.Aa-3 | metconazole |
| D-178 | I.Aa-3 | prothioconazole |
| D-179 | I.Aa-3 | difenoconazole |
| D-180 | I.Aa-3 | fluquinconazole |
| D-181 | I.Aa-3 | propiconazole |
| D-182 | I.Aa-3 | tebuconazole |
| D-183 | I.Aa-3 | 2-[2-chloro-4-(4-chlorophenoxy)phenyl]-1 (1,2,4-triazol-1-yl)pentan-2-ol |
| D-184 | I.Aa-3 | 1-[4-(4-chlorophenoxy)-2-(trifluoromethyl)phenyl]-1 cyclopropyl-2-(1,2,4-triazol-1-yl)ethanol |
| D-185 | I.Aa-3 | 2-[4-(4-chlorophenoxy)-2-(trifluoromethyl)phenyl]-1-(1,2,4-triazol-1 - yl)butan-2-ol |
| D-186 | I.Aa-3 | 2-[2-chloro-4-(4-chlorophenoxy)phenyl]-1-(1,2,4-triazol-1-yl)butan-2-ol |
| D-187 | I.Aa-3 | 2-[4-(4-chlorophenoxy)-2-(trifluoromethyl)phenyl]-3-methyl-1-(1,2,4-triazol-1-yl)butan-2-ol |
| D-188 | I.Aa-3 | 2-[4-(4-chlorophenoxy)-2-(trifluoromethyl)phenyl]-1-(1,2,4-triazol-1-yl)propan-2-ol |
| D-189 | I.Aa-3 | 2-[2-chloro-4-(4-chlorophenoxy)phenyl]-3-methyl-1-(1,2,4-triazol-1-yl)butan-2-ol |
| D-190 | I.Aa-3 | 2-[4-(4-chlorophenoxy)-2-(trifluoromethyl)phenyl]-1-(1,2,4-triazol-1-yl)pentan-2-ol |
| D-191 | I.Aa-3 | 2-[4-(4-fluorophenoxy)-2-(trifluoromethyl)phenyl]-1-(1,2,4-triazol-1-yl)propan-2-ol |
| D-192 | I.Aa-3 | 2-[2-chloro-4-(4-chlorophenoxy)phenyl]-1-(1,2,4-triazol-1-yl)pent-3-yn-2-ol |
| D-193 | I.Aa-3 | prochloraz |
| D-194 | I.Aa-3 | [3-(4-chloro-2-fluoro-phenyl)-5-(2,4-difluorophenyl)isoxazol-4-yl]-(3-pyridyl)methanol |
| D-195 | I.Aa-3 | fenpropimorph |
| D-196 | I.Aa-3 | Metalaxyl |
| D-197 | I.Aa-3 | Benalaxyl |
| D-198 | I.Aa-3 | Thiophanate-methyl |
| D-199 | I.Aa-3 | Carbendazim |
| D-200 | I.Aa-3 | Metrafenone |
| D-201 | I.Aa-3 | Pyrimethanil |
| D-202 | I.Aa-3 | Iprodione |
| D-203 | I.Aa-3 | Vinclozolin |
| D-204 | I.Aa-3 | Fludioxonil |
| D-205 | I.Aa-3 | dimethomorph |
| D-206 | I.Aa-3 | oxathiapiprolin |
| D-207 | I.Aa-3 | metiram |
| D-208 | I.Aa-3 | mancozeb |
| D-209 | I.Aa-3 | chlorothalonil |
| D-210 | I.Aa-3 | dithianon |
| D-211 | I.Aa-3 | Dipymetitrone |
| D-212 | I.Aa-3 | prohexadione-calcium |
| D-213 | I.Aa-3 | 3-(5-fluoro-3,3,4,4-tetramethyl-3,4-dihydroisoquinolin-1-yl)quinoline |
| D-214 | I.Aa-3 | 3-(4,4-difluoro-3,3-dimethyl-3,4-dihydroisoquinolin-1-yl)quinoline |
| D-215 | I.Aa-3 | 3-(4,4,5-trifluoro-3,3-dimethyl-3,4-dihydroisoquinolin-1-yl)quinoline |
| D-216 | I.Aa-3 | 9-fluoro-2,2-dimethyl-5-(3-quinolyl)-3H 1,4-benzoxazepine |
| D-217 | I.Aa-4 | Pyraclostrobin |
| D-218 | I.Aa-4 | Azoxystrobin |
| D-219 | I.Aa-4 | Trifloxystrobin |
| D-220 | I.Aa-4 | Picoxystrobin |
| D-221 | I.Aa-4 | Fluoxastrobin |
| D-222 | I.Aa-4 | Dimoxystrobin |
| D-223 | I.Aa-4 | Kresoxim-methyl |
| D-224 | I.Aa-4 | (2E,3Z)-5-[[1-(2,4-dichlorophenyl)-1H-pyrazol-3-yl]oxy]-2-(methoxyimino)-N,3-dimethyl-pent-3-enamide |
| D-225 | I.Aa-4 | (2E,3Z)-5-[[1-(4-chlorophenyl)-1H-pyrazol-3-yl]oxy]-2-(methoxyimino)-N,3-dimethyl-pent-3-enamide |
| D-226 | I.Aa-4 | [(3S,6S,7R,8R)-8-benzyl-3-[(3-acetoxy-4 methoxy-pyridine-2-carbonyl)amino]-6-methyl-4,9-dioxo-1,5-dioxonan-7-yl] 2 methylpropanoate |
| D-227 | I.Aa-4 | [(3S,6S,7R,8R)-8-benzyl-3-[[3-(acetoxymethoxy)-4-methoxy-pyridine-2 carbonyl]amino]-6-methyl-4,9-dioxo-1,5-dioxonan-7-yl] 2 methylpropanoate |
| D-228 | I.Aa-4 | [(3S,6S, 7R,8R)-8-benzyl-3-[(3-isobutoxycarbonyloxy-4-methoxy-pyridine-2 carbonyl)amino]-6-methyl-4,9-dioxo-1,5-dioxonan-7-yl] 2-methylpropanoate |
| D-229 | I.Aa-4 | [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate |
| D-230 | I.Aa-4 | (3S,6S,7R,8R)-3-[[(3-hydroxy-4-methoxy-2-pyridinyl)carbonyl]amino]-6 methyl-4,9-dioxo-8-(phenylmethyl)-1,5-dioxonan-7-yl 2-methylpropanoate |
| D-231 | I.Aa-4 | Fluxapyroxad |
| D-232 | I.Aa-4 | Boscalid |
| D-233 | I.Aa-4 | Bixafen |
| D-234 | I.Aa-4 | Isopyrazam |
| D-235 | I.Aa-4 | Benzovindiflupyr |
| D-236 | I.Aa-4 | Fluopyram |
| D-237 | I.Aa-4 | N-[(5-chloro-2-isopropyl-phenyl)methyl]-N-cyclopropyl-5-fluoro-1,3-dimethyl-pyrazole-4-carboxamide |
| D-238 | I.Aa-4 | Sedaxane |
| D-239 | I.Aa-4 | Penflufen |
| D-240 | I.Aa-4 | N-[2-(2,4-dichlorophenyl)-2-methoxy-1-methyl-ethyl]-3-(difluoromethyl)-1-methyl-pyrazole-4-carboxamide |
| D-241 | I.Aa-4 | 3 (difluoromethyl)-1-methyl-N-(1,1,3-trimethylindan-4-yl)pyrazole-4-carboxamide |
| D-242 | I.Aa-4 | 3 (trifluoromethyl)-1-methyl-N-(1,1,3-trimethylindan-4-yl)pyrazole-4-carboxamide |
| D-243 | I.Aa-4 | 1,3-dimethyl-N-(1,1 ,3-trimethylindan-4-yl)pyrazole-4-carboxamide |
| D-244 | I.Aa-4 | 3-(trifluorometh¬yl)-1,5-dimethyl-N-(1,1,3-trimethylindan-4-yl)pyrazole-4-carboxamide |
| D-245 | I.Aa-4 | 1,3,5-trimethyl-N-(1,1,3-trimethylindan-4-yl)pyrazole-4-carboxamide |
| D-246 | I.Aa-4 | 1-[3-chloro-2-[[[1-(4-chlorophenyl)-1H-pyrazol-3-yl]oxy]methyl]phenyl]-1,4-dihydro-4-methyl-5H-tetrazol-5-one |
| D-247 | I.Aa-4 | Ametoctradin |
| D-248 | I.Aa-4 | epoxiconazole |
| D-249 | I.Aa-4 | metconazole |
| D-250 | I.Aa-4 | prothioconazole |
| D-251 | I.Aa-4 | difenoconazole |
| D-252 | I.Aa-4 | fluquinconazole |
| D-253 | I.Aa-4 | propiconazole |
| D-254 | I.Aa-4 | tebuconazole |
| D-255 | I.Aa-4 | 2-[2-chloro-4-(4-chlorophenoxy)phenyl]-1 (1,2,4-triazol-1-yl)pentan-2-ol |
| D-256 | I.Aa-4 | 1-[4-(4-chlorophenoxy)-2-(trifluoromethyl)phenyl]-1 cyclopropyl-2-(1,2,4-triazol-1-yl)ethanol |
| D-257 | I.Aa-4 | 2-[4-(4-chlorophenoxy)-2-(trifluoromethyl)phenyl]-1-(1,2,4-triazol-1-yl)butan-2-ol |
| D-258 | I.Aa-4 | 2-[2-chloro-4-(4-chlorophenoxy)phenyl]-1-(1,2,4-triazol-1-yl)butan-2-ol |
| D-259 | I.Aa-4 | 2-[4-(4-chlorophenoxy)-2-(trifluoromethyl)phenyl]-3-methyl-1-(1,2,4-triazol-1-yl)butan-2-ol |
| D-260 | I.Aa-4 | 2-[4-(4-chlorophenoxy)-2-(trifluoromethyl)phenyl]-1-(1,2,4-triazol-1-yl)propan-2-ol |
| D-261 | I.Aa-4 | 2-[2-chloro-4-(4-chlorophenoxy)phenyl]-3-methyl-1-(1,2,4-triazol-1-yl)butan-2-ol |
| D-262 | I.Aa-4 | 2-[4-(4-chlorophenoxy)-2-(trifluoromethyl)phenyl]-1-(1,2,4-triazol-1-yl)pentan-2-ol |
| D-263 | I.Aa-4 | 2-[4-(4-fluorophenoxy)-2-(trifluoromethyl)phenyl]-1-(1,2,4-triazol-1-yl)propan-2-ol |
| D-264 | I.Aa-4 | 2-[2-chloro-4-(4-chlorophenoxy)phenyl]-1-(1,2,4-triazol-1-yl)pent-3-yn-2-ol |
| D-265 | I.Aa-4 | prochloraz |
| D-266 | I.Aa-4 | [3-(4-chloro-2-fluoro-phenyl)-5-(2,4-difluorophenyl)isoxazol-4-yl]-(3-pyridyl)methanol |
| D-267 | I.Aa-4 | fenpropimorph |
| D-268 | I.Aa-4 | Metalaxyl |
| D-269 | I.Aa-4 | Benalaxyl |
| D-270 | I.Aa-4 | Thiophanate-methyl |
| D-271 | I.Aa-4 | Carbendazim |
| D-272 | I.Aa-4 | Metrafenone |
| D-273 | I.Aa-4 | Pyrimethanil |
| D-274 | I.Aa-4 | Iprodione |
| D-275 | I.Aa-4 | Vinclozolin |
| D-276 | I.Aa-4 | Fludioxonil |
| D-277 | I.Aa-4 | dimethomorph |
| D-278 | I.Aa-4 | oxathiapiprolin |
| D-279 | I.Aa-4 | metiram |
| D-280 | I.Aa-4 | mancozeb |
| D-281 | I.Aa-4 | chlorothalonil |
| D-282 | I.Aa-4 | dithianon |
| D-283 | I.Aa-4 | Dipymetitrone |
| D-284 | I.Aa-4 | prohexadione-calcium |
| D-285 | I.Aa-4 | 3-(5-fluoro-3,3,4,4-tetramethyl-3,4-dihydroisoquinolin-1-yl)quinoline |
| D-286 | I.Aa-4 | 3-(4,4-difluoro-3,3-dimethyl-3,4-dihydroisoquinolin-1-yl)quinoline |
| D-287 | I.Aa-4 | 3-(4,4,5-trifluoro-3,3-dimethyl-3,4-dihydroisoquinolin-1-yl)quinoline |
| D-288 | I.Aa-4 | 9-fluoro-2,2-dimethyl-5-(3-quinolyl)-3H 1,4-benzoxazepine |
| D-289 | I.Aa-5 | Pyraclostrobin |
| D-290 | I.Aa-5 | Azoxystrobin |
| D-291 | I.Aa-5 | Trifloxystrobin |
| D-292 | I.Aa-5 | Picoxystrobin |
| D-293 | I.Aa-5 | Fluoxastrobin |
| D-294 | I.Aa-5 | Dimoxystrobin |
| D-295 | I.Aa-5 | Kresoxim-methyl |
| D-296 | I.Aa-5 | (2E,3Z)-5-[[1-(2,4-dichlorophenyl)-1H-pyrazol-3-yl]oxy]-2-(methoxyimino)-N,3-dimethyl-pent-3-enamide |
| D-297 | I.Aa-5 | (2E,3Z)-5-[[1-(4-chlorophenyl)-1H-pyrazol-3-yl]oxy]-2-(methoxyimino)-N,3-dimethyl-pent-3-enamide |
| D-298 | I.Aa-5 | [(3S,6S,7R,8R)-8-benzyl-3-[(3-acetoxy-4 methoxy-pyridine-2-carbonyl)amino]-6-methyl-4,9-dioxo-1,5-dioxonan-7-yl] 2 methylpropanoate |
| D-299 | I.Aa-5 | [(3S,6S,7R,8R)-8-benzyl-3-[[3-(acetoxymethoxy)-4-methoxy-pyridine-2 carbonyl]amino]-6-methyl-4,9-dioxo-1,5-dioxonan-7-yl] 2 methylpropanoate |
| D-300 | I.Aa-5 | [(3S,6S,7R,8R)-8-benzyl-3-[(3-isobutoxycarbonyloxy-4-methoxy-pyridine-2 carbonyl)amino]-6-methyl-4,9-dioxo-1,5-dioxonan-7-yl] 2-methylpropanoate |
| D-301 | I.Aa-5 | [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate |
| D-302 | I.Aa-5 | (3S,6S,7R,8R)-3-[[(3-hydroxy-4-methoxy-2-pyridinyl)carbonyl]amino]-6 methyl-4,9-dioxo-8-(phenylmethyl)-1 ,5-dioxonan-7-yl 2-methylpropanoate |
| D-303 | I.Aa-5 | Fluxapyroxad |
| D-304 | I.Aa-5 | Boscalid |
| D-305 | I.Aa-5 | Bixafen |
| D-306 | I.Aa-5 | Isopyrazam |
| D-307 | I.Aa-5 | Benzovindiflupyr |
| D-308 | I.Aa-5 | Fluopyram |
| D-309 | I.Aa-5 | N-[(5-chloro-2-isopropyl-phenyl)methyl]-N-cyclopropyl-5-fluoro-1,3-dimethyl-pyrazole-4-carboxamide |
| D-310 | I.Aa-5 | Sedaxane |
| D-311 | I.Aa-5 | Penflufen |
| D-312 | I.Aa-5 | N-[2-(2,4-dichlorophenyl)-2-methoxy-1-methyl-ethyl]-3-(difluoromethyl)-1-methyl-pyrazole-4-carboxamide |
| D-313 | I.Aa-5 | 3 (difluoromethyl)-1-methyl-N-(1,1,3-trimethylindan-4-yl)pyrazole-4-carboxamide |
| D-314 | I.Aa-5 | 3 (trifluoromethyl)-1-methyl-N-(1,1,3-trimethylindan-4-yl)pyrazole-4-carboxamide |
| D-315 | I.Aa-5 | 1,3-dimethyl-N-(1,1,3-trimethylindan-4-yl)pyrazole-4-carboxamide |
| D-316 | I.Aa-5 | 3-(trifluorometh¬yl)-1,5-dimethyl-N-(1,1,3-trimethylindan-4-yl)pyrazole-4-carboxamide |
| D-317 | I.Aa-5 | 1,3,5-trimethyl-N-(1,1,3-trimethylindan-4-yl)pyrazole-4-carboxamide |
| D-318 | I.Aa-5 | 1-[3-chloro-2-[[[1-(4-chlorophenyl)-1H-pyrazol-3-yl]oxy]methyl]phenyl]-1,4-dihydro-4-methyl-5H-tetrazol-5-one |
| D-319 | I.Aa-5 | Ametoctradin |
| D-320 | I.Aa-5 | epoxiconazole |
| D-321 | I.Aa-5 | metconazole |
| D-322 | I.Aa-5 | prothioconazole |
| D-323 | I.Aa-5 | difenoconazole |
| D-324 | I.Aa-5 | fluquinconazole |
| D-325 | I.Aa-5 | propiconazole |
| D-326 | I.Aa-5 | tebuconazole |
| D-327 | I.Aa-5 | 2-[2-chloro-4-(4-chlorophenoxy)phenyl]-1 (1,2,4-triazol-1-yl)pentan-2-ol |
| D-328 | I.Aa-5 | 1-[4-(4-chlorophenoxy)-2-(trifluoromethyl)phenyl]-1cyclopropyl-2-(1,2,4-triazol-1-yl)ethanol |
| D-329 | I.Aa-5 | 2-[4-(4-chlorophenoxy)-2-(trifluoromethyl)phenyl]-1-(1,2,4-triazol-1-yl)butan-2-ol |
| D-330 | I.Aa-5 | 2-[2-chloro-4-(4-chlorophenoxy)phenyl]-1-(1,2,4-triazol-1-yl)butan-2-ol |
| D-331 | I.Aa-5 | 2-[4-(4-chlorophenoxy)-2-(trifluoromethyl)phenyl]-3-methyl-1-(1,2,4-triazol-1-yl)butan-2-ol |
| D-332 | I.Aa-5 | 2-[4-(4-chlorophenoxy)-2-(trifluoromethyl)phenyl]-1-(1,2,4-triazol-1-yl)propan-2-ol |
| D-333 | I.Aa-5 | 2-[2-chloro-4-(4-chlorophenoxy)phenyl]-3-methyl-1-(1,2,4-triazol-1-yl)butan-2-ol |
| D-334 | I.Aa-5 | 2-[4-(4-chlorophenoxy)-2-(trifluoromethyl)phenyl]-1-(1,2,4-triazol-1-yl)pentan-2-ol |
| D-335 | I.Aa-5 | 2-[4-(4-fluorophenoxy)-2-(trifluoromethyl)phenyl]-1-(1,2,4-triazol-1-yl)propan-2-ol |
| D-336 | I.Aa-5 | 2-[2-chloro-4-(4-chlorophenoxy)phenyl]-1-(1,2,4-triazol-1-yl)pent-3-yn-2-ol |
| D-337 | I.Aa-5 | prochloraz |
| D-338 | I.Aa-5 | [3-(4-chloro-2-fluoro-phenyl)-5-(2,4-difluorophenyl)isoxazol-4-yl]-(3-pyridyl)methanol |
| D-339 | I.Aa-5 | fenpropimorph |
| D-340 | I.Aa-5 | Metalaxyl |
| D-341 | I.Aa-5 | Benalaxyl |
| D-342 | I.Aa-5 | Thiophanate-methyl |
| D-343 | I.Aa-5 | Carbendazim |
| D-344 | I.Aa-5 | Metrafenone |
| D-345 | I.Aa-5 | Pyrimethanil |
| D-346 | I.Aa-5 | Iprodione |
| D-347 | I.Aa-5 | Vinclozolin |
| D-348 | I.Aa-5 | Fludioxonil |
| D-349 | I.Aa-5 | dimethomorph |
| D-350 | I.Aa-5 | oxathiapiprolin |
| D-351 | I.Aa-5 | metiram |
| D-352 | I.Aa-5 | mancozeb |
| D-353 | I.Aa-5 | chlorothalonil |
| D-354 | I.Aa-5 | dithianon |
| D-355 | I.Aa-5 | Dipymetitrone |
| D-356 | I.Aa-5 | prohexadione-calcium |
| D-357 | I.Aa-5 | 3-(5-fluoro-3,3,4,4-tetramethyl-3,4-dihydroisoquinolin-1-yl)quinoline |
| D-358 | I.Aa-5 | 3-(4,4-difluoro-3,3-dimethyl-3,4-dihydroisoquinolin-1-yl)quinoline |
| D-359 | I.Aa-5 | 3-(4,4,5-trifluoro-3,3-dimethyl-3,4-dihydroisoquinolin-1-yl)quinoline |
| D-360 | I.Aa-5 | 9-fluoro-2,2-dimethyl-5-(3-quinolyl)-3H 1,4-benzoxazepine |
| D-361 | I.Ba-1 | Pyraclostrobin |
| D-362 | I.Ba-1 | Azoxystrobin |
| D-363 | I.Ba-1 | Trifloxystrobin |
| D-364 | I.Ba-1 | Picoxystrobin |
| D-365 | I.Ba-1 | Fluoxastrobin |
| D-366 | I.Ba-1 | Dimoxystrobin |
| D-367 | I.Ba-1 | Kresoxim-methyl |
| D-368 | I.Ba-1 | (2E,3Z)-5-[[1-(2,4-dichlorophenyl)-1H-pyrazol-3-yl]oxy]-2-(methoxyimino)-N,3-dimethyl-pent-3-enamide |
| D-369 | I.Ba-1 | (2E,3Z)-5-[[1-(4-chlorophenyl)-1H-pyrazol-3-yl]oxy]-2-(methoxyimino)-N,3-dimethyl-pent-3-enamide |
| D-370 | I.Ba-1 | [(3S,6S,7R,8R)-8-benzyl-3-[(3-acetoxy-4 methoxy-pyridine-2-carbonyl)amino]-6-methyl-4,9-dioxo-1,5-dioxonan-7-yl] 2 methylpropanoate |
| D-371 | I.Ba-1 | [(3S,6S,7R,8R)-8-benzyl-3-[[3-(acetoxymethoxy)-4-methoxy-pyridine-2 carbonyl]amino]-6-methyl-4,9-dioxo-1,5-dioxonan-7-yl] 2 methylpropanoate |
| D-372 | I.Ba-1 | [(3S,6S,7R,8R)-8-benzyl-3-[(3-isobutoxycarbonyloxy-4-methoxy-pyridine-2 carbonyl)amino]-6-methyl-4,9-dioxo-1,5-dioxonan-7-yl] 2-methylpropanoate |
| D-373 | I.Ba-1 | [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate |
| D-374 | I.Ba-1 | (3S,6S,7R,8R)-3-[[(3-hydroxy-4-methoxy-2-pyridinyl)carbonyl]amino]-6 methyl-4,9-dioxo-8-(phenylmethyl)-1,5-dioxonan-7-yl 2-methylpropanoate |
| D-375 | I.Ba-1 | Fluxapyroxad |
| D-376 | I.Ba-1 | Boscalid |
| D-377 | I.Ba-1 | Bixafen |
| D-378 | I.Ba-1 | Isopyrazam |
| D-379 | I.Ba-1 | Benzovindiflupyr |
| D-380 | I.Ba-1 | Fluopyram |
| D-381 | I.Ba-1 | N-[(5-chloro-2-isopropyl-phenyl)methyl]-N-cyclopropyl-5-fluoro-1,3-dimethyl-pyrazole-4-carboxamide |
| D-382 | I.Ba-1 | Sedaxane |
| D-383 | I.Ba-1 | Penflufen |
| D-384 | I.Ba-1 | N-[2-(2,4-dichlorophenyl)-2-methoxy-1-methyl-ethyl]-3-(difluoromethyl)-1-methyl-pyrazole-4-carboxamide |
| D-385 | I.Ba-1 | 3 (difluoromethyl)-1-methyl-N-(1,1,3-trimethylindan-4-yl)pyrazole-4-carboxamide |
| D-386 | I.Ba-1 | 3 (trifluoromethyl)-1-methyl-N-(1,1,3-trimethylindan-4-yl)pyrazole-4-carboxamide |
| D-387 | I.Ba-1 | 1,3-dimethyl-N-(1,1,3-trimethylindan-4-yl)pyrazole-4-carboxamide |
| D-388 | I.Ba-1 | 3-(trifluorometh¬yl)-1,5-dimethyl-N-(1,1,3-trimethylindan-4-yl)pyrazole-4-carboxamide |
| D-389 | I.Ba-1 | 1,3,5-trimethyl-N-(1,1,3-trimethylindan-4-yl)pyrazole-4-carboxamide |
| D-390 | I.Ba-1 | 1-[3-chloro-2-[[[1-(4-chlorophenyl)-1H-pyrazol-3-yl]oxy]methyl]phenyl]-1,4-dihydro-4-methyl-5H-tetrazol-5-one |
| D-391 | I.Ba-1 | Ametoctradin |
| D-392 | I.Ba-1 | epoxiconazole |
| D-393 | I.Ba-1 | metconazole |
| D-394 | I.Ba-1 | prothioconazole |
| D-395 | I.Ba-1 | difenoconazole |
| D-396 | I.Ba-1 | fluquinconazole |
| D-397 | I.Ba-1 | propiconazole |
| D-398 | I.Ba-1 | tebuconazole |
| D-399 | I.Ba-1 | 2-[2-chloro-4-(4-chlorophenoxy)phenyl]-1 (1,2,4-triazol-1-yl)pentan-2-ol |
| D-400 | I.Ba-1 | 1-[4-(4-chlorophenoxy)-2-(trifluoromethyl)phenyl]-1 cyclopropyl-2-(1,2,4-triazol-1-yl)ethanol |
| D-401 | I.Ba-1 | 2-[4-(4-chlorophenoxy)-2-(trifluoromethyl)phenyl]-1-(1,2,4-triazol-1-yl)butan-2-ol |
| D-402 | I.Ba-1 | 2-[2-chloro-4-(4-chlorophenoxy)phenyl]-1-(1,2,4-triazol-1-yl)butan-2-ol |
| D-403 | I.Ba-1 | 2-[4-(4-chlorophenoxy)-2-(trifluoromethyl)phenyl]-3-methyl-1-(1,2,4-triazol-1-yl)butan-2-ol |
| D-404 | I.Ba-1 | 2-[4-(4-chlorophenoxy)-2-(trifluoromethyl)phenyl]-1-(1,2,4-triazol-1-yl)propan-2-ol |
| D-405 | I.Ba-1 | 2-[2-chloro-4-(4-chlorophenoxy)phenyl]-3-methyl-1-(1,2,4-triazol-1-yl)butan-2-ol |
| D-406 | I.Ba-1 | 2-[4-(4-chlorophenoxy)-2-(trifluoromethyl)phenyl]-1-(1,2,4-triazol-1-yl)pentan-2-ol |
| D-407 | I.Ba-1 | 2-[4-(4-fluorophenoxy)-2-(trifluoromethyl)phenyl]-1-(1 ,2,4-triazol-1-yl)propan-2-ol |
| D-408 | I.Ba-1 | 2-[2-chloro-4-(4-chlorophenoxy)phenyl]-1-(1,2,4-triazol-1-yl)pent-3-yn-2-ol |
| D-409 | I.Ba-1 | prochloraz |
| D-410 | I.Ba-1 | [3-(4-chloro-2-fluoro-phenyl)-5-(2,4-difluorophenyl)isoxazol-4-yl]-(3-pyridyl)methanol |
| D-411 | I.Ba-1 | fenpropimorph |
| D-412 | I.Ba-1 | Metalaxyl |
| D-413 | I.Ba-1 | Benalaxyl |
| D-414 | I.Ba-1 | Thiophanate-methyl |
| D-415 | I.Ba-1 | Carbendazim |
| D-416 | I.Ba-1 | Metrafenone |
| D-417 | I.Ba-1 | Pyrimethanil |
| D-418 | I.Ba-1 | Iprodione |
| D-419 | I.Ba-1 | Vinclozolin |
| D-420 | I.Ba-1 | Fludioxonil |
| D-421 | I.Ba-1 | dimethomorph |
| D-422 | I.Ba-1 | oxathiapiprolin |
| D-423 | I.Ba-1 | metiram |
| D-424 | I.Ba-1 | mancozeb |
| D-425 | I.Ba-1 | chlorothalonil |
| D-426 | I.Ba-1 | dithianon |
| D-427 | I.Ba-1 | Dipymetitrone |
| D-428 | I.Ba-1 | prohexadione-calcium |
| D-429 | I.Ba-1 | 3-(5-fluoro-3,3,4,4-tetramethyl-3,4-dihydroisoquinolin-1-yl)quinoline |
| D-430 | I.Ba-1 | 3-(4,4-difluoro-3,3-dimethyl-3,4-dihydroisoquinolin-1-yl)quinoline |
| D-431 | I.Ba-1 | 3-(4,4,5-trifluoro-3,3-dimethyl-3,4-dihydroisoquinolin-1-yl)quinoline |
| D-432 | I.Ba-1 | 9-fluoro-2,2-dimethyl-5-(3-quinolyl)-3H 1,4-benzoxazepine |
| D-433 | I.Ca-1 | Pyraclostrobin |
| D-434 | I.Ca-1 | Azoxystrobin |
| D-435 | I.Ca-1 | Trifloxystrobin |
| D-436 | I.Ca-1 | Picoxystrobin |
| D-437 | I.Ca-1 | Fluoxastrobin |
| D-438 | I.Ca-1 | Dimoxystrobin |
| D-439 | I.Ca-1 | Kresoxim-methyl |
| D-440 | I.Ca-1 | (2E,3Z)-5-[[1-(2,4-dichlorophenyl)-1H-pyrazol-3-yl]oxy]-2-(methoxyimino)-N,3-dimethyl-pent-3-enamide |
| D-441 | I.Ca-1 | (2E,3Z)-5-[[1-(4-chlorophenyl)-1H-pyrazol-3-yl]oxy]-2-(methoxyimino)-N,3-dimethyl-pent-3-enamide |
| D-442 | I.Ca-1 | [(3S,6S,7R,8R)-8-benzyl-3-[(3-acetoxy-4 methoxy-pyridine-2-carbonyl)amino]-6-methyl-4,9-dioxo-1,5-dioxonan-7-yl] 2 methylpropanoate |
| D-443 | I.Ca-1 | [(3S,6S,7R,8R)-8-benzyl-3-[[3-(acetoxymethoxy)-4-methoxy-pyridine-2 carbonyl]amino]-6-methyl-4,9-dioxo-1,5-dioxonan-7-yl] 2 methylpropanoate |
| D-444 | I.Ca-1 | [(3S,6S,7R,8R)-8-benzyl-3-[(3-isobutoxycarbonyloxy-4-methoxy-pyridine-2 carbonyl)amino]-6-methyl-4,9-dioxo-1,5-dioxonan-7-yl] 2-methylpropanoate |
| D-445 | I.Ca-1 | [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate |
| D-446 | I.Ca-1 | (3S,6S,7R,8R)-3-[[(3-hydroxy-4-methoxy-2-pyridinyl)carbonyl]amino]-6 methyl-4,9-dioxo-8-(phenylmethyl)-1 ,5-dioxonan-7-yl 2-methylpropanoate |
| D-447 | I.Ca-1 | Fluxapyroxad |
| D-448 | I.Ca-1 | Boscalid |
| D-449 | I.Ca-1 | Bixafen |
| D-450 | I.Ca-1 | Isopyrazam |
| D-451 | I.Ca-1 | Benzovindiflupyr |
| D-452 | I.Ca-1 | Fluopyram |
| D-453 | I.Ca-1 | N-[(5-chloro-2-isopropyl-phenyl)methyl]-N-cyclopropyl-5-fluoro-1,3-dimethyl-pyrazole-4-carboxamide |
| D-454 | I.Ca-1 | Sedaxane |
| D-455 | I.Ca-1 | Penflufen |
| D-456 | I.Ca-1 | N-[2-(2,4-dichlorophenyl)-2-methoxy-1-methyl-ethyl]-3-(difluoromethyl)-1-methyl-pyrazole-4-carboxamide |
| D-457 | I.Ca-1 | 3 (difluoromethyl)-1-methyl-N-(1,1,3-trimethylindan-4-yl)pyrazole-4-carboxamide |
| D-458 | I.Ca-1 | 3 (trifluoromethyl)-1-methyl-N-(1,1,3-trimethylindan-4-yl)pyrazole-4-carboxamide |
| D-459 | I.Ca-1 | 1,3-dimethyl-N-(1,1,3-trimethylindan-4-yl)pyrazole-4-carboxamide |
| D-460 | I .Ca-1 | 3-(trifluorometh¬yl)-1,5-dimethyl-N-(1,1,3-trimethylindan-4-yl)pyrazole-4-carboxamide |
| D-461 | I.Ca-1 | 1,3,5-trimethyl-N-(1,1,3-trimethylindan-4-yl)pyrazole-4-carboxamide |
| D-462 | I.Ca-1 | 1-[3-chloro-2-[[[1-(4-chlorophenyl)-1H-pyrazol-3-yl]oxy]methyl]phenyl]-1,4-dihydro-4-methyl-5H-tetrazol-5-one |
| D-463 | I.Ca-1 | Ametoctradin |
| D-464 | I.Ca-1 | epoxiconazole |
| D-465 | I.Ca-1 | metconazole |
| D-466 | I.Ca-1 | prothioconazole |
| D-467 | I.Ca-1 | difenoconazole |
| D-468 | I.Ca-1 | fluquinconazole |
| D-469 | I.Ca-1 | propiconazole |
| D-470 | I.Ca-1 | tebuconazole |
| D-471 | I.Ca-1 | 2-[2-chloro-4-(4-chlorophenoxy)phenyl]-1 (1,2,4-triazol-1-yl)pentan-2-ol |
| D-472 | I.Ca-1 | 1-[4-(4-chlorophenoxy)-2-(trifluoromethyl)phenyl]-1 cyclopropyl-2-(1,2,4-triazol-1-yl)ethanol |
| D-473 | I.Ca-1 | 2-[4-(4-chlorophenoxy)-2-(trifluoromethyl)phenyl]-1-(1,2,4-triazol-1-yl)butan-2-ol |
| D-474 | I.Ca-1 | 2-[2-chloro-4-(4-chlorophenoxy)phenyl]-1-(1,2,4-triazol-1-yl)butan-2-ol |
| D-475 | I.Ca-1 | 2-[4-(4-chlorophenoxy)-2-(trifluoromethyl)phenyl]-3-methyl-1-(1,2,4-triazol-1-yl)butan-2-ol |
| D-476 | I.Ca-1 | 2-[4-(4-chlorophenoxy)-2-(trifluoromethyl)phenyl]-1-(1,2,4-triazol-1-yl)propan-2-ol |
| D-477 | I.Ca-1 | 2-[2-chloro-4-(4-chlorophenoxy)phenyl]-3-methyl-1-(1,2,4-triazol-1-yl)butan-2-ol |
| D-478 | I.Ca-1 | 2-[4-(4-chlorophenoxy)-2-(trifluoromethyl)phenyl]-1-(1,2,4-triazol-1-yl)pentan-2-ol |
| D-479 | I.Ca-1 | 2-[4-(4-fluorophenoxy)-2-(trifluoromethyl)phenyl]-1-(1,2,4-triazol-1-yl)propan-2-ol |
| D-480 | I.Ca-1 | 2-[2-chloro-4-(4-chlorophenoxy)phenyl]-1-(1,2,4-triazol-1-yl)pent-3-yn-2-ol |
| D-481 | I.Ca-1 | prochloraz |
| D-482 | I.Ca-1 | [3-(4-chloro-2-fluoro-phenyl)-5-(2,4-difluorophenyl)isoxazol-4-yl]-(3-pyridyl)methanol |
| D-483 | I.Ca-1 | fenpropimorph |
| D-484 | I.Ca-1 | Metalaxyl |
| D-485 | I.Ca-1 | Benalaxyl |
| D-486 | I.Ca-1 | Thiophanate-methyl |
| D-487 | I.Ca-1 | Carbendazim |
| D-488 | I.Ca-1 | Metrafenone |
| D-489 | I.Ca-1 | Pyrimethanil |
| D-490 | I.Ca-1 | Iprodione |
| D-491 | I.Ca-1 | Vinclozolin |
| D-492 | I.Ca-1 | Fludioxonil |
| D-493 | I.Ca-1 | dimethomorph |
| D-494 | I.Ca-1 | oxathiapiprolin |
| D-495 | I.Ca-1 | metiram |
| D-496 | I.Ca-1 | mancozeb |
| D-497 | I.Ca-1 | chlorothalonil |
| D-498 | I.Ca-1 | dithianon |
| D-499 | I.Ca-1 | Dipymetitrone |
| D-500 | I.Ca-1 | prohexadione-calcium |
| D-501 | I.Ca-1 | 3-(5-fluoro-3,3,4,4-tetramethyl-3,4-dihydroisoquinolin-1-yl)quinoline |
| D-502 | I.Ca-1 | 3-(4,4-difluoro-3,3-dimethyl-3,4-dihydroisoquinolin-1-yl)quinoline |
| D-503 | I.Ca-1 | 3-(4,4,5-trifluoro-3,3-dimethyl-3,4-dihydroisoquinolin-1-yl)quinoline |
| D-504 | I.Ca-1 | 9-fluoro-2,2-dimethyl-5-(3-quinolyl)-3H 1,4-benzoxazepine |

The active substances referred to as component 2, their preparation and their activity e. g. against harmful fungi is known (cf.: http://www.alanwood.net/pesticides/); these substances are commercially available. The compounds described by IUPAC nomenclature, their preparation and their pesticidal activity are also known (cf. Can. J. Plant Sci. 48(6), 587-94, 1968; EP-A 141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 10/139271, WO 11/028657, WO 12/168188, WO 07/006670, WO 11/77514; WO 13/047749, WO 10/069882, WO 13/047441, WO 03/16303, WO 09/90181, WO 13/007767, WO 13/010862, WO 13/127704, WO 13/024009, WO 13/24010, WO 13/047441, WO 13/162072, WO 13/092224, WO 11/135833, CN 1907024, CN 1456054, CN 103387541, CN 1309897, WO 12/84812, CN 1907024, WO 09094442, WO 14/60177, WO 13/116251, WO 08/013622, WO 15/65922, WO 94/01546, EP 2865265, WO 07/129454, WO 12/165511, WO 11/081174, WO 13/47441).

The present invention furthermore relates to agrochemical compositions comprising a mixture of at least one compound I (component 1) and at least one further active substance useful for plant protection, e. g. selected from the groups A) to O) (component 2), in particular one further fungicide, e. g. one or more fungicide from the groups A) to K), as described above, and if desired one suitable solvent or solid carrier. Those mixtures are of particular interest, since many of them at the same application rate show higher efficiencies against harmful fungi. Furthermore, combating harmful fungi with a mixture of compounds I and at least one fungicide from groups A) to K), as described above, is more efficient than combating those fungi with individual compounds I or individual fungicides from groups A) to K).

By applying compounds I together with at least one active substance from groups A) to O) a synergistic effect can be obtained, i.e. more then simple addition of the individual effects is obtained (synergistic mixtures).

This can be obtained by applying the compounds I and at least one further active substance simultaneously, either jointly (e. g. as tank-mix) or seperately, or in succession, wherein the time interval between the individual applications is selected to ensure that the active substance applied first still occurs at the site of action in a sufficient amount at the time of application of the further active substance(s). The order of application is not essential for working of the present invention.

When applying compound I and a pesticide II sequentially the time between both applications may vary e. g. between 2 hours to 7 days. Also a broader range is possible ranging from 0.25 hour to 30 days, preferably from 0.5 hour to 14 days, particularly from 1 hour to 7 days or from 1.5 hours to 5 days, even more preferred from 2 hours to 1 day.

In the binary mixtures and compositions according to the invention the weight ratio of the component 1) and the component 2) generally depends from the properties of the active components used, usually it is in the range of from 1:10,000 to 10,000:1, often it is in the range of from 1:100 to 100:1, regularly in the range of from 1:50 to 50:1, preferably in the range of from 1:20 to 20:1, more preferably in the range of from 1:10 to 10:1, even more preferably in the range of from 1:4 to 4:1 and in particular in the range of from 1:2 to 2:1.

According to further embodiments of the binary mixtures and compositions, the weight ratio of the component 1) and the component 2) usually is in the range of from 1000:1 to 1:1, often in the range of from 100: 1 to 1:1, regularly in the range of from 50:1 to 1:1, preferably in the range of from 20:1 to 1:1, more preferably in the range of from 10:1 to 1:1, even more preferably in the range of from 4:1 to 1:1 and in particular in the range of from 2:1 to 1:1.

According to a further embodiments of the binary mixtures and compositions, the weight ratio of the component 1) and the component 2) usually is in the range of from 1:1 to 1:1000, often in the range of from 1:1 to 1:100, regularly in the range of from 1:1 to 1:50, preferably in the range of from 1:1 to 1:20, more preferably in the range of from 1:1 to 1:10, even more preferably in the range of from 1:1 to 1:4 and in particular in the range of from 1:1 to 1:2.

In the ternary mixtures, i.e. compositions according to the invention comprising the component 1) and component 2) and a compound III (component 3), the weight ratio of component 1) and component 2) depends from the properties of the active substances used, usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:4 to 4:1, and the weight ratio of component 1) and component 3) usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:4 to 4:1.

Any further active components are, if desired, added in a ratio of from 20:1 to 1:20 to the component 1).

These ratios are also suitable for inventive mixtures applied by seed treatment.

### I. Synthesis examples

With due modification of the starting compounds, the procedures shown in the synthesis examples below were used to obtain further compounds I. The resulting compounds, together with physical data, are listed in Table I below.

### Example 1 Synthesis of 4,4-difluoro-5,5-dimethyl-7-(3-quinolyl)thieno[2,3-c]pyridine (I-3)

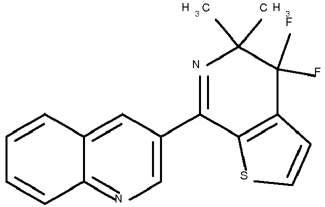

### Step 1.1 2-Methyl-1-(3-thienyl)propan-2-ol

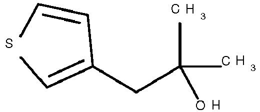

13.5 ml (33.7 mmol) 2.5 m Butyllithium (in hexanes) were added dropwise to a solution of 5 g (30.7 mmol) 3-bromo-thiophen in 15 ml diethylether keeping the reaction temperature below - 50°C. After 1 hour 2.43 g (30.7 mmol) dimethyloxirane were added dropwise at -50°C and the reaction mixture was allowed to warm to -30°C. Then the reaction mixture was cooled with an ice/water-bath and 25 ml 1 n hydrochloric acid were added dropwise. The organic layer was separated and the aqueous layer was extracted twice with methyl-t-butylether. The combined organic layers were washed with brine, dried over sodium sulfate and concentrated. The residue was purified via silica gel chromatography with heptane/methyl-t-butylether-mixtures to yield 0.32 g (6.7 %) of the title compound as a brown oil. ¹H-NMR(CDCl₃, δ in ppm): 7,4 (dd, 1H); 7,05 (d, broad, 1H); 6,95 (d, 1H); 2,8 (s, 2H); 1,45 (s, broad, 1H); 1,25 (s, 6H)

### Step 1.2 5,5-Dimethyl-7-(3-quinolyl)-4H-thieno[2,3-c]pyridine (I-1)

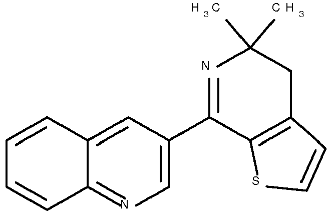

Upon cooling with ice 0.92 g (6.1 mmol) trifluoromethylsulfonic acid were added to a mixture of 0.33 g (2.05 mmol) 3-cyanoquinoline and 0.32 g (2.05 mmol) 2-methyl-1-(3-thienyl)propan-2-ol in 8 ml dichloroethane. After 17 hours at room temperature the reaction mixture was dropped unto cold sodium hydrogen carbonate solution. The aqueous phase was extracted three times with methyl-t-butylether. The combined organic layers were extracted with brine and water and concentrated. The residue was purified via silica gel chromatography with heptane/ethyl acetate mixtures to yield 0.56 g (40 %) of the title compound as a brown oil. ¹H-NMR(CDCl₃, δ in ppm): 9,35 (s, 1H); 8,55 (s, 1H); 8,15 (d, 1H); 7,9 (d, 1H); 7,75 (t, 1H); 7,6 (t, 1H); 7,45 (d, 1H); 7,0 (d, 1H); 2,9 (s, 2H); 1,4 (s, 6H)

### Step 1.3 4,4-Dibromo-5,5-dimethyl-7-(3-quinolyl)thieno[2,3-c]pyridine (I-5)

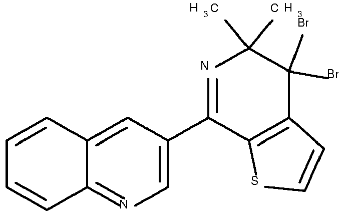

A mixture of 0.93 g (3.09 mmol) 5,5-dimethyl-7-(3-quinolyl)-4H-thieno[2,3-c]pyridine, 0.97 g (3.39 mmol) dibromo-dimethyl-hydantoin and 0.16 g (0.96 mmol) azo-isobutyro-dinitrile in 10 ml carbon tetrachloride was heated to reflux. After 1 hour the reaction mixture was cooled to 15°C upon which a crystalline solid precipitated. The solid was filtered off, the solvent was evaporated and the residue was treated with an ethyl acetate/hexane mixture. More solid precipitated which was filtered off. The mother liquor was concentrated and the residue (0.52 g) was used in the subsequent reaction without further purification. HPLC-MS of this residue indicated hydrolysis of the title compound, probably occurring during the HPLC-MS run.

### Step 1.4 4,4-Difluoro-5,5-dimethyl-7-(3-quinolyl)thieno[2,3-c]pyridine (I-3)

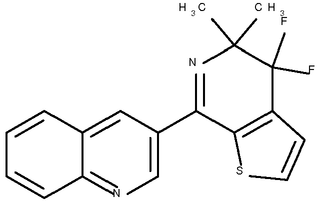

At room temperature 0.69 g (4.04 mmol) □rimethylamine x 3 HF was added dropwise to a mixture of 0.52 g (1.16 mmol) 4,4-dibromo-5,5-dimethyl-7-(3-quinolyl)thieno[2,3-c]pyridine in 20 ml acetonitrile. Afterwards the reaction mixture was heated to reflux and kept at this temperature for 3 hours. Subsequently the reaction mixture was cooled to room temperature and 30 ml saturated sodium hydrogen carbonate solution was added. The aqueous phase was extracted three times with methyl-t-butyl ether and the combined organic layers were extracted with water and brine, dried over sodium sulfate and concentrated The residue was purified via silica gel chromatography with heptane/ethyl acetate mixtures to yield 0.11 g (30 %) of the title compound as a yellow oil. ¹H-NMR(CDCl₃, δ in ppm): 9,35 (s, 1H); 8,65 (s, 1H); 8,2 (d, 1H); 7,95 (d, 1H); 7,85 (t, 1H); 7,65 (m, 2H); 7,55 (d, 1H); 1,6 (s, 6H)

### Example 2 Synthesis of 7,7-difluoro-6,6-dimethyl-4-(3-quinolyl)thieno[3,2-c]pyridine

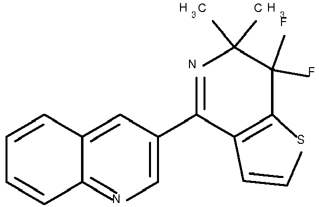

### Step 2.1. 5-(3-bromo-2-thienyl)-4,4-dimethyl-oxazolidin-2-one

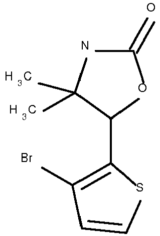

At -78°C lithium diisopropylamide (147 mL, 0.295 mol) was added dropwise to a solution of 3-bromothiophene (43.58 g, 0.267 mol) in tetrahydrofuran (700 mL) and the mixture was stirred for 30 min.

Subsequently tert.-butyl N-(1,1-dimethyl-2-oxo-ethyl)carbamate (25 g, 0.134 mol) in tetrahydrofuran (100 mL) was added dropwise at -78°C. The reaction mixture was allowed to warm up and was stirred for 16 h at ambient temperature. Afterwards the reaction mixture was quenched with aq. NH₄Cl (800 mL) and extracted with ethyl acetate. (500 mL x 2). The combines organic layers were washed with brine (800 mL), dried over sodium sulfate and concentrated. The residue was purified via silica gel chromatography with petrol ether - ethyl acetate mixtures to give the title compound (59 g, 80%) as a yellow solid.

¹H NMR (CDCl₃, δ in ppm): 1.10 (s, 3 H) 1.61 (s, 3 H) 5.66 (s, 1 H) 5.94 (br. s., 1 H) 7.00 (d, J=5.15 Hz, 1 H) 7.36 (d, J=5.14 Hz, 1 H)

### Step 2.2. Methyl 2-(4,4-dimethyl-2-oxo-oxazolidin-5-yl)thiophene-3-carboxylate

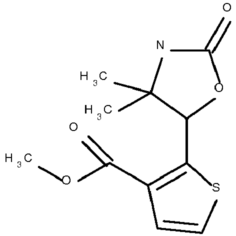

A solution of 5-(3-bromo-2-thienyl)-4,4-dimethyl-oxazolidin-2-one (59 g, 0.214 mol), sodium methanolate (23 g, 0.428 mol) and Pd(dppf)Cl₂ ([1,1'-Bis(diphenylphosphino)ferrocene]dichloro-palladium(II)) (6 g) in methanol (1200 mL) was purged with carbon monoxide gas and stirred at 100°C in a steel autoclave under carbon monoxide (2 MPa) for 16 h. The reaction mixture was cooled to room temperature and filtered through celite. The filtrate was concentrated and the residue was purified via silica gel chromatography with petrol ether - ethyl acetate mixtures to give the title compound (48 g, 88%) as a yellow solid.

¹H-NMR (MeOD, δ in ppm): 0.89 (s, 3 H) 1.57 (s, 3 H) 3.86 (s, 3 H) 6.28 (s, 1 H) 7.46 (s, 1 H)

### Step 2.3. 2-(2-Amino-1-hydroxy-2-methyl-propyl)thiophene-3-carboxylic acid

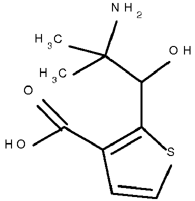

To a mixture of methyl 2-(4,4-dimethyl-2-oxo-oxazolidin-5-yl)thiophene-3-carboxylate

(19 g, 0.0745 mol) in methanol\water (300mL\100 mL) was added potassium hydroxide (41.7 g, 0.745 mol) upon stirring. The resulting mixture was stirred and heated to reflux at 80°C for 16 h. Subsequently methanol was evaporated under reduced pressure and the remaining aqueous phase was extracted with MTBE (200 mL). Afterwards the aqueous phase was acidified with concentrated HCl to pH=2 and the so formed solid was collected, washed with toluene (100 mL) and dried in vacuum to give the title compound (39 g, 91%) as a yellow solid.

¹H-NMR (DMSO-d6, δ in ppm): 1.19 (d, J=8.66 Hz, 6 H) 5.85 (s, 1 H) 6.98 (br. s., 1 H) 7.35 (d, J=5.27 Hz, 1 H) 7.58 (d, J=5.27 Hz, 1 H) 8.06 (br. s., 1 H)

### Step 2.4. 7-Hydroxy-6,6-dimethyl-5,7-dihydrothieno[3,2-c]pyridin-4-one

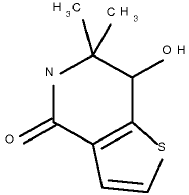

To a solution of 7-hydroxy-6,6-dimethyl-5,7-dihydrothieno[3,2-c]pyridin-4-one (35.5 g, 0.156 mol) ii tetrahydrofuran (1.2 L) was added triethylamine (31.5 g, 0.312 mol) and HATU (1-**[Bis(dimethylamino)methylene]-1H-1,2,3-triazolo[4,5-b]pyridinium 3-oxid hexafluorophosphate)** (88.75 g, 0.234 mol). The resulting mixture was heated to reflux and stirred at 80°C for 16 h. The reaction mixture was concentrated and the residue was purified via silica gel chromatography with petrol ether - ethyl acetate mixtures to give the title compound (40 g, crude) as a yellow solid. ¹H NMR (MeOD, δ in ppm): 1.29 (d, J=3.97 Hz, 6 H) 4.71 (s, 1 H) 7.32 (d, J=5.29 Hz, 1 H) 7.36 - 7.41 (m, 1 H)

### Step 2.5. 6,6-Dimethyl-5H-thieno[3,2-c]pyridine-4,7-dione

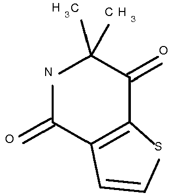

To a solution of 7-hydroxy-6,6-dimethyl-5,7-dihydrothieno[3,2-c]pyridin-4-one (40 g, 0.203 mol) in tetrahydrofuran (1200 mL) was added manganese dioxide (265 g, 3.05 mol) and the resulting mixture was stirred and heated to reflux at 80°C for 16 h.

Afterwards the reaction mixture was filtered through celite, the filtrate was concentrated and the residue was purified via silica gel chromatography with petrol ether - ethyl acetate mixtures to give the title compound (30 g, 75%) as a yellow solid.

¹H-NMR: GV-39238-81-1a MeOD 400MHz δ: 1.54 (s, 6 H) 7.61 (d, J=5.02 Hz, 1 H) 8.12 (d, J=5.02 Hz, 1 H)

### Step 2.6. (6,6-Dimethyl-7-oxo-thieno[3,2-c]pyridin-4-yl) trifluoromethanesulfonate

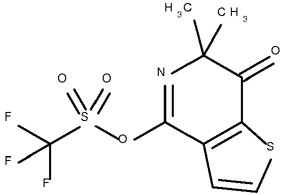

Trifluoromethyl sulfonic anhydride (116 g, 0.41 mol) was added dropwise to a solution of 6,6-dimethyl-5H-thieno[3,2-c]pyridine-4,7-dione (20 g, 0.103 mol) and pyridine (65 g, 0.824 mol) in dichloromethane (500 mL) at 0°C. The reaction mixture was stirred for 1 h at 0°C and afterwards water (300 mL) was added. The aqueous phase was extracted with dichloromethane (500 mL x 2) and the combined organic phases were separated, washed with brine (300 mL), dried over sodium sulfate and concentrated. The residue was purified via silica gel chromatography with petrol ether - ethyl acetate mixtures to give 14,2 g (42 %) of the title compound as a yellow solid.

¹H-NMR (CDCl₃, δ in ppm): 1.99 (s, 6 H) 7.73 (d, J=5.29 Hz, 1 H) 7.95 (d, J=4.85 Hz, 1 H)

### Step 2.7. (7,7-difluoro-6,6-dimethyl-thieno[3,2-c]pyridin-4-yl) trifluoromethanesulfonate

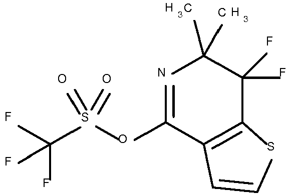

A solution of (6,6-Dimethyl-7-oxo-thieno[3,2-c]pyridin-4-yl) trifluoromethanesulfonate (2.5 g, 7.65 mmol) in BAST (bis(2-methoxyethyl)aminosulfur trifluoride, 13 mL, neat) was heated to 100°C under N2 for 2 h. The reaction mixture was quenched with water (100 mL) and extracted with methyl-t-butylether (100 mL). The organic phase was washed with sodium hydrogen carbonate-solution (100 mL) and brine (100 mL), dried over sodium sulfate and concentrated. The resulting residue was was purified via silica gel chromatography with petrol ether - ethyl acetate mixtures to give the title compound (0,615 g, 23%) as a yellow solid.

¹H-NMR (CDCl₃, δ in ppm): 1.81 (s, 6 H) 7.53 - 7.60 (m, 2 H)

### Step 2.8. 7,7-Difluoro-6,6-dimethyl-4-(3-quinolyl)thieno[3,2-c]pyridine

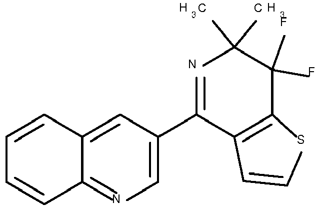

3-Quinolylboronic acid (149 mg, 0.86 mmol), potassium carbonate (158 mg, 1.146 mmol) and Pd(PPh3)4 (20 mg) were added to a solution of ((7,7-difluoro-6,6-dimethyl-thieno[3,2-c]pyridin-4-yl) trifluoromethanesulfonate) (0.2 g, 0.573 mmol) in toluene (4 mL) under an athmosphere of nitrogen. The resulting mixture was heated to 160°C upon microwave irradiation for 1 h. Subsequently the reaction mixture was filtered and concentrated. The residue was purified via silica gel chromatography with petrol ether - ethyl acetate mixtures to give the crude product, which was then purified by preparative HPLC (neutral) to give the title compound (12 mg, 6.3%) as a yellow solid.

¹H NMR (CDCl₃, δ in ppm): 1.52 (s, 6 H) 7.06 - 7.13 (m, 1 H) 7.54 (d, J=4.85 Hz, 1 H) 7.59 - 7.67 (m, 1 H) 7.76 - 7.85 (m, 1 H) 7.91 (d, J=7.94 Hz, 1 H) 8.18 (d, J=8.38 Hz, 1 H) 8.48 (d, J=1.76 Hz, 1 H) 9.25 (d, J=2.21 Hz, 1 H)

**Table I:**

| The positions of the heteroaryls given as "R⁷+R⁸" marked with "#" represents the connection points (carbon atoms 5' and 6' in formula I) with the remaining skeleton of the compounds of formula | | | | | | | | | |
|---|---|---|---|---|---|---|---|---|---|
| **No.** | **(R¹)ₘ** | **(R²)ₙ** | **R³** | **R⁴** | **R⁵** | **R⁶** | **R⁷+R⁸** | **(R⁷⁸)ₒ** | **¹H-NMR (δ in ppm);** |
| | | | | | | | | | **HPLC-MS* (EI (M⁺+H)** |
| | | | | | | | | | **Rₜ [min], Mp (°C)** |
| I-1 | m=0 | n=0 | CH₃ | CH₃ | H | H | 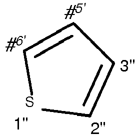 | o=0 | ¹H-NMR (CDCl₃): 9,35 (s, 1H); 8,55 (s, 1H); 8,15 (d, 1H); 7,9 (d, 1H); 7,75 (t, 1H); 7,6 (t, 1H); 7,45 (d, 1H); 7,0 (d, 1H); 2,9 (s, 2H); 1,4 (s, 6H) |
| | | | | | | | | | M⁺+H: 292.9; |
| | | | | | | | | | Rₜ = 0.770 |
| I-2 | m=0 | n=0 | CH₃ | CH₃ | H | H | 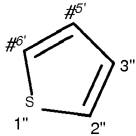 | 2"-CH₃ | M⁺+H: 307.1; |
| | | | | | | | | | Rₜ = 0.837 |
| I-3 | m=0 | n=0 | CH₃ | CH₃ | F | F | 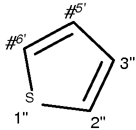 | o=0 | ¹H-NMR (CDCl₃): 9,35 (s, 1H); 8,65 (s, 1H); 8,2 (d, 1H); 7,95 (d, 1H); 7,85 (t, 1H); 7,65 (m, 2H); 7,55 (d, 1H); 1,6 (s, 6H) |
| | | | | | | | | | M⁺+H: 329.1 |
| | | | | | | | | | Rₜ = 1.066 |
| IIA-1 | m=0 | n=0 | CH₃ | CH₃ | =O | | 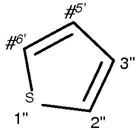 | o=0 | M⁺+H: 307.1; |
| | | | | | | | | | Rₜ = 0.936 |
| I-4 | m=0 | n=0 | CH₃ | CH₃ | H | H | 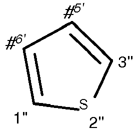 | 3"-Cl | M⁺+H: 326; |
| | | | | | | | | | Rₜ = 0,862 |
| I-5 | m=0 | n=0 | CH₃ | CH₃ | Br | Br | 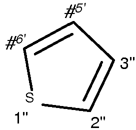 | o=0 | oil |
| I-6 | m=0 | n=0 | CH₃ | CH₃ | CH₃ | CH₃ | 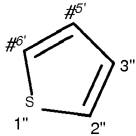 | o=0 | 127 |
| I-7 | m=0 | n=0 | CH₃ | CH₃ | H | H | 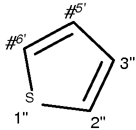 | 3"-F | 9,25 (s, 1 H); 9,0 (s, 1H); 8,3 (m, 2H); 8,2 (d, 1H); 8,0 (t, 1H); 7,8 (t, 1H); 3,2 (s, 2H); 1,55 (s, 6H) |
| I-8 | m=0 | n=0 | CH₃ | CH₃ | CH₃ | H | 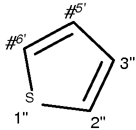 | o=0 | M⁺+H: 307,2 |
| | | | | | | | | | Rₜ = 0,804 |
| I-9 | 8-F | n=0 | CH₃ | CH₃ | F | F | 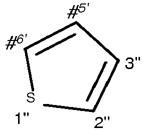 | o=0 | 95 |
| I-10 | 8-Cl | n=0 | CH₃ | CH₃ | F | F | 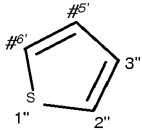 | o=0 | 102 |
| I-11 | 7-F 8-FI | n=0 | CH₃ | CH₃ | F | F | 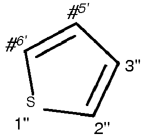 | o=0 | 109 |
| I-12 | m=0 | n=0 | CH₃ | CH₃ | F | H | 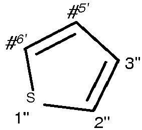 | 3"-F | 9,3 (s, 1H); 9,25 (s, 1H); 8,3 (m, 2H); 8,2 (m, 1H); 7,9 (m, 2H); 6 (2s, 1H); 1,8 (s, 3H); 1,4 (s, 3H) |
| I-13 | m=0 | n=0 | CH₃ | CH₃ | F | F | 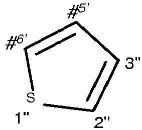 | 3"-F | 9,25 (s,1H); 8,9 (s, 1H); 8,15 (m, 2H); 7,95 (t, 1H); 7,8 (t, 1H); 7,5 (s, 1H); 1,5 (s, 6H) |
| I-14 | m=0 | n=0 | CH₃ | CH₃ | H | H | 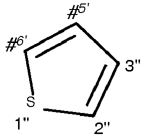 | 2"-Cl | M⁺+H: 327 |
| | | | | | | | | | Rₜ = 0,844 |
| | | | | | | | | | |
| I-15 | m=0 | n=0 | CH₃ | CH₃ | F | F | 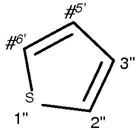 | 2"-Cl | 74 |
| I-16 | m=0 | n=0 | CH₃ | CH₃ | H | H | | 2"-CHF₂ | M⁺+H: 342,8 |
| | | | | | | | 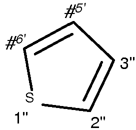 | | Rₜ = 0,875 |
| I-17 | m=0 | n=0 | CH₃ | CH₃ | H | H | 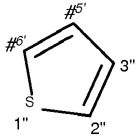 | 3"-Cl | 9,3 (s, 1H); 8,55 (s, 1H) 8,2 (d, 1H); 7,9 (d, 1H); 7,8 (t, 1H); 7,6 (t, 1H); 7,3 (s, 1H); 2,8 (s, 2H); 1,4 (s, 6H) |
| I-18 | m=0 | n=0 | CH₃ | CH₃ | OH | H | 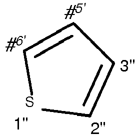 | 3"-Cl | 9,1 (s, 1H); 8,4 (s, 1H); 8,1 (d, 1H); 7,8 (d, 1H); 7,7 (t, 1H); 7,5 (t, 1H); 7,3 (s, 1H); |
| I-19 | m=0 | n=0 | CH₃ | CH₃ | H | H | 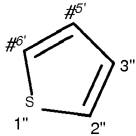 | 3"-Br | 9,3 (s, 1H); 8,55 (s, 1H); 8,2 (d, 1H); 7,9 (d, 2H); 7,8 (t, 1H); 7,6 (t, 2H); 7,4 (s, 1H); 2,8 (s, 2H); 1,4 (s, 6H) |
| I-20 | m=0 | n=0 | CH₃ | CH₃ | F | F | 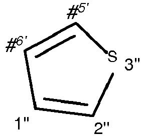 | o=0 | 9,25 (s, 1H); 8,5 (s, 1H); 8,2 (d, 1H); 7,9 (d, 1H); 7,8 (t, 1H); 7,6 (t, 1H); 7,55 (d, 1H); 7,1 (d, 1H); 1,5 (s, 6H) |
| I-21 | m=0 | n=0 | CH₃ | CH₃ | Br | H | 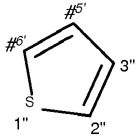 | 3"-Cl | 9,35 (s, 1H); 8,s (s, 1 H); 8,2 (d, 1H); 7,95 (d, 1H); 7,85 (t, 1H); 7,65 (t, 1H); 7,4 (s, 1H); 5,4 (s, 1H); 1,95 (s, 3H); 1,45 (s, 3H) |
| I-22 | m=0 | n=0 | CH₃ | CH₃ | H | H | 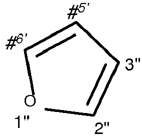 | o=0 | M⁺+H: 277,2 |
| | | | | | | | | | Rₜ = 0,746 |
| I-23 | m=0 | n=0 | CH₃ | CH₃ | H | H | 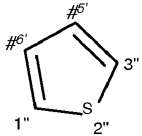 | 3"-Br | M⁺+H: 370,9 |
| | | | | | | | | | Rₜ = 0,950 |

| | | | | | | | | | |
|---|---|---|---|---|---|---|---|---|---|
| *HPLC-MS: HPLC-column Kinetex XB C18 1,7µ (50 x 2,1 mm); eluent: acetonitrile / water + | | | | | | | | | |

0.1% TFA (5 gradient from 5:95 to 100 : 0 in 1.5 min at 60°C, flow gradient from 0.8 to 1.0 ml/min in 1.5 min). MS: Quadrupol Electrospray lonisation, 80 V (positive mode).

### II. Biological trials

### Microtest

The active compounds were formulated separately as a stock solution having a concentration of 10000 ppm in dimethyl sulfoxide.

### Example 1 - Activity against the grey mold Botrytis cinerea in the microtiterplate test

The stock solutions were mixed according to the ratio, pipetted onto a micro titer plate (MTP) and diluted with water to the stated concentrations. A spore suspension of *Botrci cinerea* in a DOB medium solution was then added. The plates were placed in a water vapor-saturated chamber at a temperature of 18°C. Using an absorption photometer, the MTPs were measured at 405 nm 9 days after the inoculation.

In this test, the samples which had been treated with 31 ppm of the active substance from examples I-6, I-7, I-8, I-9, I-10, I-11, I-12, I-13 and I-20 respectively, showed up to at most 5 % growth of the pathogen.

### Example 2 - Activity against rice blast Pyricu/aria oryzae in the microtiterplate test

The stock solutions were mixed according to the ratio, pipetted onto a micro titer plate (MTP) and diluted with water to the stated concentrations. A spore suspension of *Pyricularia oryzae* in a DOB medium solution was then added. The plates were placed in a water vapor-saturated chamber at a temperature of 18°C. Using an absorption photometer, the MTPs were measured at 405 nm 9 days after the inoculation.

In this test, the samples which had been treated with 31 ppm of the active substance from examples I-1, I-2, I-3, I-4, I-6, I-7, I-8, I-9, I-10, I-11, I-12, I-13, I-14, I-15, I-16, I-17, I-19, I-20 and IIA-1 respectively, showed up to at most 8 % growth of the pathogen.

### Example 3 - Activity against leaf blotch on wheat caused by Septoria tritici (

The stock solutions were mixed according to the ratio, pipetted onto a micro titer plate (MTP) and diluted with water to the stated concentrations. A spore suspension of *Septoria tritici* in a DOB medium solution was then added. The plates were placed in a water vapor-saturated chamber at a temperature of 18°C. Using an absorption photometer, the MTPs were measured at 405 nm 9 days after the inoculation.

In this test, the samples which had been treated with 31 ppm of the active substance from examples I-3, I-4, I-6, I-7, I-8, I-9, I-10, I-11, 1-12, 1-13, I-14, I-15, I-16, I-17, I-19, I-20 and IIA-1 respectively, showed up to at most 17 % growth of the pathogen.

### Example 4 - Activity against early blight caused by Alternaria solani

The stock solutions were mixed according to the ratio, pipetted onto a micro titer plate (MTP) and diluted with water to the stated concentrations. A spore suspension of *Alternaria solani* in a DOB medium solution was then added. The plates were placed in a water vapor-saturated chamber at a temperature of 18°C. Using an absorption photometer, the MTPs were measured at 405 nm 9 days after the inoculation.

In this test, the samples which had been treated with 31 ppm of the active substance from examples I-1, I-3, I-4, I-6, I-8, I-9, I-10, I-11, I-12, I-13, I-14, I-15, I-16, I-17, I-19, I-20 and IIA-1 respectively, showed up to at most 17 % growth of the pathogen.

### Example 5 - Activity against wheat leaf spots caused by Leptosphaeria nodorum

The stock solutions were mixed according to the ratio, pipetted onto a micro titer plate (MTP) and diluted with water to the stated concentrations. A spore suspension of *Leptosphaeria nodorum* in a DOB medium solution was then added. The plates were placed in a water vapor-saturated chamber at a temperature of 18°C. Using an absorption photometer, the MTPs were measured at 405 nm 9 days after the inoculation.

In this test, the samples which had been treated with 31 ppm of the active substance from examples I-8, I-9, I-10, I-11, I-12, I-13 and I-20 respectively, showed up to at most 9 % growth of the pathogen.

The measured parameters were compared to the growth of the active compound-free control variant (100%) and the fungus-free and active compound-free blank value to determine the relative growth in % of the pathogens in the respective active compounds.

### III. Comparative Examples

### Microtest

The active compounds were formulated separately as a stock solution having a concentration of 10000 ppm in dimethyl sulfoxide.

### Example 1 - Activity against the late blight pathogen Phytophthora infestans in the microtiter test

The stock solutions were mixed according to the ratio, pipetted onto a micro titer plate (MTP) and diluted with water to the stated concentrations. A spore suspension of *Phytophtora infestans* containing a pea juice-based aqueous nutrient medium or DDC medium was then added. The plates were placed in a water vapor-saturated chamber at a temperature of 18°C. Using an absorption photometer, the MTPs were measured at 405 nm 7 days after the inoculation.

| **Compound** | **Structure** | **Growth (%) at 31 ppm** |
|---|---|---|
| EP 1736471 | 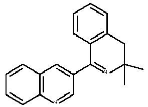 | 33 |
| Acc. to the invention | 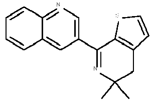 | 17 |

### Example 2 - Activity against wheat leaf spots caused by Leptosphaeria nodorum

The stock solutions were mixed according to the ratio, pipetted onto a micro titer plate (MTP) and diluted with water to the stated concentrations. A spore suspension of *Leptosphaeria nodorum* in a DOB medium solution was then added. The plates were placed in a water vapor-saturated chamber at a temperature of 18°C. Using an absorption photometer, the MTPs were measured at 405 nm 9 days after the inoculation.

| **Compound** | **Structure** | **Growth (%) at 2 ppm** |
|---|---|---|
| EP 1736471) | 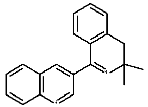 | 83 |
| Acc. to the invention | 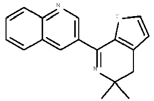 | 65 |

### Example 3 - Activity against leaf blotch on wheat caused by Septoria tritici

The stock solutions were mixed according to the ratio, pipetted onto a micro titer plate (MTP) and diluted with water to the stated concentrations. A spore suspension of *Septoria tritici* in a DOB medium solution was then added. The plates were placed in a water vapor-saturated chamber at a temperature of 18°C. Using an absorption photometer, the MTPs were measured at 405 nm 9 days after the inoculation.

| **Compound** | **Structure** | **Growth (%) at 8 ppm** |
|---|---|---|
| EP 1736471 | 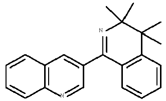 | 74 |
| Acc. to the invention | 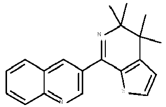 | 53 |

## Claims

1. Compounds of the formula I 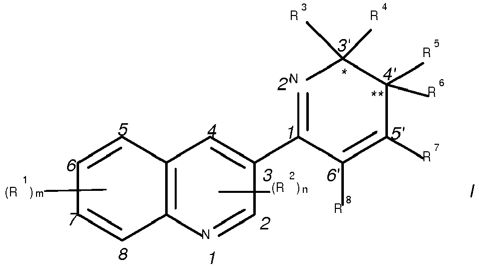 wherein
m is 0, 1, 2, 3 or 4;
R¹ is in each case independently selected from halogen, OH, CN, NO₂, SH, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl, C₁-C₆-alkoxy, C₃-C₆-cycloalkyl, five- or six-membered heteroaryl and aryl; wherein the heteroaryl contains one, two or three heteroatoms selected from N, O and S; and wherein
R^{x} is C₁-C₄-alkyl, C₁-C₄-halogenalkyl, unsubstituted aryl or aryl that is substituted by one, two, three, four or five substituents R^{x1} independently selected from C₁-C₄-alkyl;
wherein the aliphatic moieties of R¹ are not further substituted or carry one, two, three or up to the maximum possible number of identical or different groups R^{1a} which independently of one another are selected from:
R^{1a} halogen, OH, CN, C₁-C₆-alkoxy, C₃-C₆-cycloalkyl, C₃-C₆-halogencycloalkyl, C₁-C₄-halogenalkoxy, C₁-C₆-alkylthio and phenoxy, wherein the phenyl group is unsubstituted or carries one, two, three, four or five substituents R^{11a} selected from the group consisting of halogen, OH, C₁-C₄-alkyl, C₁-C₄-halogenalkyl, C₁-C₄-alkoxy and C₁-C₄-halogenalkoxy;
wherein the cycloalkyl, heteroaryl and aryl moieties of R¹ are not further substituted or carry one, two, three, four, five or up to the maximum number of identical or different groups R^{1b} which independently of one another are selected from:
R^{1b} halogen, OH, CN, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy and C₁-C₆-alkylthio;
n is 0, 1 or 2;
R² is in each case independently selected from the substituents as defined for R¹, wherein the possible substituents for R² are R^{2a} and R^{2b}, respectively, which correspond to R^{1a} and R^{1b}, respectively;
R³,R⁴ are independently selected from hydrogen, halogen, OH, CN, NO₂, SH, C₁-C₆-alkylthio, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl, C₁-C₆-alkoxy, C₁-C₆-halogenalkoxy, a saturated or partially unsaturated three-, four-, five-, six-, seven-, eight-, nine-, or ten-membered carbocycle or heterocycle, wherein in each case one or two CH₂ groups of the carbo- and heterocycle may be replaced by a group independently selected from C(=O) and C(=S), five- or six-membered heteroaryl and aryl; wherein the heterocycle and the heteroaryl contain independently one, two, three or four heteroatoms selected from N, O and S;
wherein the aliphatic moieties of R³ and R⁴ are independently not further substituted or carry one, two, three or up to the maximum possible number of identical or different groups R^{3a} or R^{4a}, respectively, which independently of one another are selected from:
R^{3a},R^{4a} halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, C₁-C₆-alkoxy, C₃-C₆-cycloalkyl, C₃-C₆-halogencycloalkyl, C₁-C₄-halogenalkoxy, C₁-C₆-alkylthio, aryl and phenoxy, wherein the aryl and phenyl groups are independently unsubstituted or carry one, two, three, four or five substituents selected from the group consisting of halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, NH-SO₂-R^{x}, C₁-C₆-alkylthio, C₁-C₄-alkyl, C₁-C₄-halogenalkyl, C₁-C₄-alkoxy and C₁-C₄-halogenalkoxy;
wherein the carbocyclic, heterocyclic, heteroaryl and aryl moieties of R³ and R⁴ are independently not further substituted or carry one, two, three, four, five or up to the maximum number of identical or different groups R^{3b} or R^{4b}, respectively, which independently of one another are selected from:
R^{3b},R^{4b} halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, NH-SO₂-R^{x}, C₁-C₄-alkyl, C₁-C₄-alkoxy, C₁-C₄-halogenalkyl, C₃-C₆-CyCloalkyl, C₃-C₆-halogencycloalkyl, C₁-C₄-halogenalkoxy, C₁-C₆-alkylthio, C₁-C₆-halogenalkylthio, C₁-C₄-alkoxy-C₁-C₄-alkyl, phenyl and phenoxy, wherein the phenyl groups are unsubstituted or carry one, two, three, four or five substituents selected from the group consisting of halogen, OH, C₁-C₄-alkyl, C₁-C₄-halogenalkyl, C₁-C₄-alkoxy and C₁-C₄-halogenalkoxy;
and wherein R^{x} is as defined above;
or R³, R⁴ together with the carbon atom to which they are bound (marked with * in formula I) form a saturated or partially unsaturated three-, four-, five-, six-, seven-, eight-, nine-, or ten-membered carbocycle or heterocycle; wherein the heterocycle contains one, two, three or four heteroatoms selected from N, O and S, wherein the heteroatom N may carry one substituent R^{N} selected from C₁-C₄-alkyl, C₁-C₄-halogenalkyl and SO₂Ph, wherein Ph is unsubstituted phenyl or phenyl that is substituted by one, two or three substituents selected from C₁-C₄-alkyl, and wherein the heteroatom S may be in the form of its oxide SO or SO₂, and wherein the carbocycle or heterocycle is unsubstituted or carries one, two, three or four substituents R³⁴ independently selected from halogen, OH, CN, NO₂, SH, NH₂, C₁-C₆-alkyl, C₁-C₆-halogenalkyl, C₁-C₆-alkoxy, C₁-C₆-halogenalkoxy, C₁-C₆-alkylthio, C₁-C₆-halogenalkylthio, C₁-C₄-alkoxy-C₁-C₄-alkyl, phenyl and phenoxy, wherein the phenyl groups are unsubstituted or carry one, two, three, four or five substituents R^{34a} selected from the group consisting of halogen, OH, C₁-C₄-alkyl, C₁-C₄-halogenalkyl, C₁-C₄-alkoxy and C₁-C₄-halogenalkoxy; and wherein in each case one or two CH₂ groups of the carbo- or heterocycle may be replaced by a group independently selected from C(=O) and C(=S);
R⁵ is hydrogen, halogen, OH, CN, NO₂, SH, NH₂, NH(C₁-C₄-alkyl), N(C₁-C₄-alkyl)₂, NH-SO₂-R^{x}, C₁-C₆-alkyl, C₂-C₆-alkenyl, C₂-C₆-alkynyl, C₁-C₆-alkoxy, C₃-C₆-CyCloalkyl, saturated or partially unsaturated three-, four-, five-, six-, seven-, eight-, nine-, or ten-membered heterocycle, five- or six-membered heteroaryl or aryl; wherein the heterocycle or heteroaryl contains one, two or three heteroatoms selected from N, O and S; and wherein R^{x} is defined above; and
wherein the aliphatic moieties of R⁵ are not further substituted or carry one, two, three or up to the maximum possible number of identical or different groups R^{5a} which independently of one another are selected from:
R^{5a} halogen, OH, CN, C₁-C₆-alkoxy, C₃-C₆-CyCloalkyl, C₃-C₆-halogencycloalkyl, C₁-C₄-halogenalkoxy, C₁-C₆-alkylthio and phenoxy, wherein the phenyl group is unsubstituted or carries one, two, three, four or five substituents R^{55a} selected from the group consisting of halogen, OH, C₁-C₄-alkyl, C₁-C₄-halogenalkyl, C₁-C₄-alkoxy and C₁-C₄-halogenalkoxy;
wherein the cycloalkyl, heterocycle, heteroaryl and aryl moieties of R⁵ are not further substituted or carry one, two, three, four, five or up to the maximum number of identical or different groups R^{5b} which independently of one another are selected from:
R^{5b} halogen, OH, CN, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy and C₁-C₆-alkylthio;
R⁶ is independently selected from the substituents as defined for R⁵, wherein the possible substituents for R⁶ are R^{6a}, R^{66a} and R^{6b}, respectively, which correspond to R^{5a}, R^{55a} and R^{5b}, respectively;
or R⁵ and R⁶ together with the carbon atom to which they are bound (marked with C** in formula I) form a saturated or partially unsaturated three-, four-, five-, six-, seven-, eight-, nine-, or ten-membered carbo- or heterocycle; wherein the heterocycle contains one, two, three or four heteroatoms selected from N, O and S, and wherein the carbocycle or heterocycle is unsubstituted or carries one, two, three or four substituents R⁵⁶ independently selected from halogen, OH, CN, NO₂, SH, NH₂, C₁-C₆-alkyl, C₁-C₆-halogenalkyl, C₁-C₆-alkoxy, C₁-C₆-halogenalkoxy, C₁-C₆-alkylthio, C₁-C₆-halogenalkylthio, C₁-C₄-alkoxy-C₁-C₄-alkyl, phenyl and phenoxy; wherein the phenyl groups are unsubstituted or carry one, two, three, four or five substituents R^{56a} selected from the group consisting of halogen, OH, C₁-C₄-alkyl, C₁-C₄-halogenalkyl, C₁-C₄-alkoxy and C₁-C₄-halogenalkoxy; and wherein in each case one or two CH₂ groups of the carbo- or heterocycle may be replaced by a group independently selected from C(=O) and C(=S); and
R⁷ and R⁸ together with the carbon atoms to which they are bound form a five- or six-membered heteroaryl; wherein the heteroaryl contains one, two or three heteroatoms selected from N, O and S, and wherein the heteroaryl carries zero, one, two, three or four substituents (R⁷⁸)ₒ, wherein
o is 0, 1, 2 or 3; and
R⁷⁸ are independently selected from halogen, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, NH-SO₂-R^{x}, CH(=O), C(=O)C₁-C₆-alkyl, C(=O)NH(C₁-C₆-alkyl), CR'=NOR", C₁-C₆-alkyl, C₂-C₆-alkenyl, C₂-C₆-alkynyl, C₁-C₆-alkoxy, C₁-C₆-halogenalkoxy, C₂-C₆-alkenyloxy, C₂-C₆-alkynyloxy, C₃-C₆-cycloalkyl, C₃-C₆-cycloalkenyl, three-, four-, five- or six-membered saturated or partially unsaturated heterocycle, five- or six-membered heteroaryl and phenyl; wherein the heterocycle or heteroaryl contains one, two or three heteroatoms selected from N, O and S; wherein R' and R" are independently selected from C₁-C₄-alkyl, C₂-C₆-alkenyl, C₂-C₆-alkynyl, C₃-C₆-cycloalkyl, saturated or partially unsaturated three-, four-, five-, six-, seven-, eight-, nine-, or ten-membered heterocycle, five- or six-membered heteroaryl or aryl; wherein the heterocycle or heteroaryl contains one, two or three heteroatoms selected from N, O and S, and wherein R' and/or R" are independently unsubstituted or carry one, two or three R'" independently selected from halogen, OH, CN, NO₂, SH, NH₂, NH(C₁-C₄-alkyl), N(C₁-C₄-alkyl)₂, NH-SO₂-R^{x}, C₁-C₆-alkyl, C₁-C₆-halogenalkyl, C₂-C₆-alkenyl, C₂-C₆-halogenalkenyl, C₂-C₆-alkynyl, C₂-C₆-halogenalkynyl, C₁-C₆-alkoxy, C₁-C₆-halogenalkoxy, C₃-C₆-cycloalkyl, C₃-C₆-halogencycloalkyl and phenyl; and wherein R^{x} is defined above;
and
wherein the aliphatic moieties of R⁷⁸ are not further substituted or carry one, two, three or up to the maximum possible number of identical or different groups R^{78a} which independently of one another are selected from:
R^{78a} halogen, OH, CN, C₁-C₆-alkoxy, C₃-C₆-CyCloalkyl, C₃-C₆-cycloalkenyl, C₃-C₆-halogencycloalkyl, C₃-C₆-halogencycloalkenyl, C₁-C₄-halogenalkoxy, C₁-C₆-alkylthio, five- or six-membered heteroaryl, phenyl and phenoxy, wherein the heterorayl, phenyl and phenoxy group is unsubstituted or carries one, two, three, four or five substituents R^{78a'} selected from the group consisting of halogen, OH, C₁-C₄-alkyl, C₁-C₄-halogenalkyl, C₁-C₄-alkoxy and C₁-C₄-halogenalkoxy; wherein the alicyclic, phenyl, heterocyclic and heteroaryl moieties of R⁷⁸ are not further substituted or carry one, two, three, four, five or up to the maximum number of identical or different groups R^{78b} which independently of one another are selected from:
R^{78b} halogen, OH, CN, C₁-C₄-alkyl, C₁-C₄-alkoxy, C₁-C₄-halogenalkyl, C₃-C₆-cycloalkyl, C₃-C₆-halogencycloalkyl, C₁-C₄-halogenalkoxy and C₁-C₆-alkylthio;
and the N-oxides and the agriculturally acceptable salts thereof.

2. The compounds of claim 1, wherein m is 0.

3. The compounds of claim 1 or 2, wherein n is 0.

4. The compounds of any one of claims 1 to 3, wherein R³ and R⁴ are independently selected from C₁-C₄-alkyl.

5. The compounds of any one of claims 1 to 4, wherein R⁵ and R⁶ independently are selected from hydrogen, F, Cl, Br and C₁-C₆-alkyl, or R⁵ and R⁶ together with the carbon atom to which they are bound form a saturated three-, four-, five- or six-membered unsubstituted or substituted carbocycle as defined in claim 1.

6. The compounds of any one of claims 1 to 5, wherein o is 0.

7. The compounds of any one of claims 1 to 5, wherein R⁶ and R⁷ together with the carbon atoms to which they are bound form a five-membered heteroaryl; wherein the heteroaryl contains one, two or three heteroatoms selected from N, O and S, and wherein the heteroaryl carries zero, one, two, three or four substituents (R⁷⁸)ₒ.

8. The compounds of any one of claims 1 to 5, wherein R⁶ and R⁷ together with the carbon atoms to which they are bound form a six-membered heteroaryl; wherein the heteroaryl contains one, two or three heteroatoms selected from N, O and S, and wherein the heteroaryl carries zero, one, two, three or four substituents (R⁷⁸)ₒ.

9. A process for the synthesis of compounds I of claim 1, wherein R⁵ and R⁶ are halogen, comprising the step of:
a) reacting a compound IIA 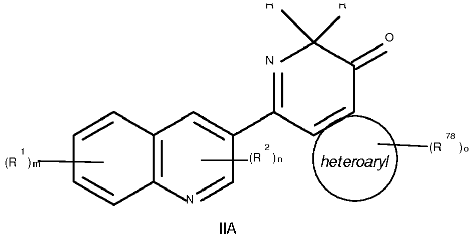 wherein the optionally substituted heteroaryl formed by R⁷ and R⁸ is sketched by a circle named "heteroaryl" and wherein the variables are defined as for compound I in any one of claims 1 to 4 and 6 to 8, with a halogenation agent.

10. The intermediate compounds IIA as defined in claim 9.

11. A composition, comprising one compound of formula I, as defined in any of the claims 1 to 8, an N-oxide or an agriculturally acceptable salt thereof.

12. The composition according to claim 11, comprising additionally a further active substance.

13. A use of a compound of the formula I, as defined in any of the claims 1 to 8, and/or of an agriculturally acceptable salt thereof or of the compositions, as defined in any of the claims 11 or 12, for combating phytopathogenic fungi, excluding the use in human or animal body.

14. A method for combating phytopathogenic fungi, comprising treating the fungi or the materials, plants, the soil or seeds to be protected against fungal attack with an effective amount of at least one compound of formula I, as defined in any of the claims 1 to 8 or with a composition, as defined in any of the claims 11 or 12, excluding therapeutic methods of treatment of the human or animal body.

15. Seed, coated with at least one compound of the formula I, as defined in any of the claims 1 to 8, and/or an agriculturally acceptable salt thereof or with a composition, as defined in any of the claims 11 or 12, in an amount of from 0.1 to 10 kg per 100 kg of seed.

## Patentansprüche

1. Verbindungen der Formel I 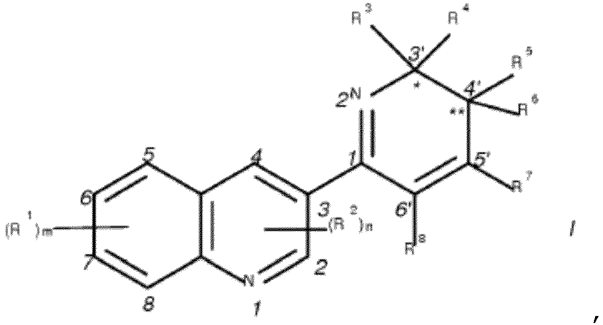 wobei
m für 0, 1, 2, 3 oder 4 steht,
R¹ jeweils unabhängig aus Halogen, OH, CN, NO₂, SH, NH₂, NH(C₁-C₄-Alkyl), N(C₁-C₄-Alkyl)₂, NH-SO₂-R^{x}, C₁-C₆-Alkyl, C₂-C₆-Alkenyl, C₂-C₆-Alkinyl, C₁-C₆-Alkoxy, C₃-C₆-Cycloalkyl, fünf- oder sechsgliedrigem Heteroaryl und Aryl ausgewählt ist, wobei das Heteroaryl eins, zwei oder drei aus N, O und S ausgewählte Heteroatome enthält und wobei
R^{x} für C₁-C₄-Alkyl, C₁-C₄-Halogenalkyl, unsubstituiertes Aryl oder durch einen, zwei, drei, vier oder fünf unabhängig voneinander aus C₁-C₄-Alkyl ausgewählte Substituenten R^{x1} substituiertes Aryl steht, wobei die aliphatischen Gruppierungen von R¹ nicht weiter substituiert sind oder eine, zwei, drei oder bis zur maximal möglichen Anzahl an gleichen oder verschiedenen Gruppen R^{1a} tragen, die unabhängig voneinander ausgewählt sind aus:
R^{1a} Halogen, OH, CN, C₁-C₆-Alkoxy, C₃-C₆-Cycloalkyl, C₃-C₆-Halogencycloalkyl, C₁-C₄-Halogenalkoxy, C₁-C₆-Alkylthio und Phenoxy, wobei die Phenylgruppe unsubstituiert ist oder einen, zwei, drei, vier oder fünf aus der aus Halogen, OH, C₁-C₄-Alkyl, C₁-C₄-Halogenalkyl, C₁-C₄-Alkoxy und C₁-C₄-Halogen-alkoxy bestehend Gruppe ausgewählte Substituenten R^{11a} trägt, wobei die Cycloalkyl-, Heteroaryl- und Arylgruppierungen von R¹ nicht weiter substituiert sind oder einen, zwei, drei, vier, fünf oder bis zur maximal möglichen Anzahl an gleichen oder verschiedenen Gruppen R^{1b} tragen, die unabhängig voneinander ausgewählt sind aus:
R^{1b} Halogen, OH, CN, C₁-C₄-Alkyl, C₁-C₄-Alkoxy, C₁-C₄-Halogenalkyl, C₃-C₆-Cycloalkyl, C₃-C₆-Halogencycloalkyl, C₁-C₄-Halogenalkoxy und C₁ -C₆-Alkyl thio,
n für 0, 1 oder 2 steht,
R² jeweils unabhängig aus den für R¹ definierten Substituenten ausgewählt ist, wobei die möglichen Substituenten für R² R^{2a} bzw. R^{2b} sind, die R^{1a} bzw. R^{1b} entsprechen,
R³, R⁴ unabhängig voneinander aus Wasserstoff, Halogen, OH, CN, NO₂, SH, C₁-C₆-Alkylthio, NH₂, NH (C₁-C₄-Alkyl) , N (C₁-C₄-Alkyl) ₂, NH-SO₂-R^{x}, C₁-C₆-Alkyl, C₂-C₆-Alkenyl, C₂-C₆-Alkinyl, C₁-C₆-Alkoxy, C₁-C₆-Halogenalkoxy, einem gesättigten oder teilweise ungesättigten drei-, vier-, fünf-, sechs-, sieben-, acht-, neun- oder zehngliedrigen Carbocyclus oder Heterocyclus ausgewählt sind, wobei jeweils eine oder zwei CH₂-Gruppen des Carbo- und Heterocyclus durch eine unabhängig aus C(=O) und C(=S), fünf- oder sechsgliedrigem Heteroaryl und Aryl ausgewählte Gruppe ersetzt sein können, wobei der Heterocyclus und das Heteroaryl unabhängig voneinander ein, zwei, drei oder vier aus N, O und S ausgewählte Heteroatome enthalten,
wobei die aliphatischen Gruppierungen von R³ und R⁴ unabhängig voneinander nicht weiter substituiert sind oder eine, zwei, drei oder bis zur maximal möglichen Anzahl an gleichen oder verschiedenen Gruppen R^{3a} bzw. R^{4a} tragen, die unabhängig voneinander ausgewählt sind aus:
R^{3a}, R^{4a} Halogen, OH, CN, NO₂, SH, NH₂, NH(C₁-C₄-Alkyl), N(C₁-C₄-Alkyl)₂, NH(C(=O)-C₁-C₄-Alkyl), N(C(=O)-C₁-C₄-Alkyl)₂, C₁-C₆-Alkoxy, C₃-C₆-Cycloalkyl, C₃-C₆-Halogencycloalkyl, C₁-C₄-Halogenalkoxy, C₁-C₆-Alkylthio, Aryl und Phenoxy, wobei die Aryl- und Phenylgruppen unabhängig voneinander unsubstituiert sind oder einen, zwei, drei, vier oder fünf aus der aus Halogen, OH, CN, NO₂, SH, NH₂, NH(C₁-C₄-Alkyl), N(C₁-C₄-Alkyl)₂, NH(C(=O)-C₁-C₄-Alkyl), N(C(=O)-C₁-C₄-Alkyl)₂, NH-SO₂-R^{x}, C₁-C₆-Alkylthio, C₁-C₄-Alkyl, C₁-C₄-Halogen-alkyl, C₁-C₄-Alkoxy und C₁-C₄-Halogenalkoxy bestehenden Gruppe ausgewählte Substituenten tragen, wobei die carbocyclischen, heterocyclischen, Heteroaryl- und Arylgruppierungen von R³ und R⁴ unabhängig voneinander nicht weiter substituiert sind oder eine, zwei, drei, vier, fünf oder bis zur maximal möglichen Anzahl an gleichen oder verschiedenen Gruppen R^{3b} bzw. R^{4b} tragen, die unabhängig voneinander ausgewählt sind aus:
R^{3b}, R^{4b} Halogen, OH, CN, NO₂, SH, NH₂, NH(C₁-C₄-Alkyl), N(C₁-C₄-Alkyl)₂, NH(C(=O)-C₁-C₄-Alkyl), N(C(=O)-C₁-C₄-Alkyl)₂, NH-SO₂-R^{x}, C₁-C₄-Alkyl, C₁-C₄-Alkoxy, C₁-C₄-Halogenalkyl, C₃-C₆-Cycloalkyl, C₃-C₆-Halogencycloalkyl, C₁-C₄-Halogenalkoxy, C₁-C₆-Alkylthio, C₁-C₆-Halogenalkylthio, C₁-C₄-Alkoxy-C₁-C₄-alkyl, Phenyl und Phenoxy, wobei die Phenylgruppen unsubstituiert sind oder einen, zwei, drei, vier oder fünf aus der aus Halogen, OH, C₁-C₄-Alkyl, C₁-C₄-Halogenalkyl, C₁-C₄-Alkoxy und C₁-C₄-Halogenalkoxy bestehenden Gruppe ausgewählte Substituenten tragen,
und wobei R^{x} wie oben definiert ist,
oder R³, R⁴ zusammen mit dem Kohlenstoffatom, an das sie gebunden sind (in Formel I mit * markiert) einen gesättigten oder teilweise ungesättigten drei-, vier-, fünf-, sechs-, sieben-, acht-, neun- oder zehngliedrigen Carbocyclus oder Heterocyclus bilden, wobei der Heterocyclus ein, zwei, drei oder vier aus N, O und S ausgewählte Heteroatome enthält, wobei das Heteroatom N einen aus C₁-C₄-Alkyl, C₁-C₄-Halogenalkyl und SO₂Ph ausgewählten Substituenten R^{N} tragen kann, wobei Ph für unsubstituiertes Phenyl oder durch einen, zwei oder drei aus C₁-C₄-Alkyl ausgewählte Substituenten substituiertes Phenyl steht, und wobei das Heteroatom S in Form seines Oxids SO oder SO₂ vorliegen kann, und wobei der Carbocyclus bzw. Heterocyclus unsubstituiert ist oder einen, zwei, drei oder vier unabhängig voneinander aus Halogen, OH, CN, NO₂, SH, NH₂, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₄-Alkoxy-C₁-C₄-alkyl, Phenyl und Phenoxy ausgewählte Substituenten R³⁴ trägt, wobei die Phenylgruppen unsubstituiert sind oder einen, zwei, drei, vier oder fünf aus der aus Halogen, OH, C₁-C₄-Alkyl, C₁-C₄-Halogenalkyl, C₁-C₄-Alkoxy und C₁-C₄-Halogenalkoxy bestehenden Gruppe ausgewählte Substituenten R^{34a} trägt, und wobei jeweils eine oder zwei CH₂-Gruppen des Carbo- oder Heterocyclus durch eine unabhängig aus C(=O) und C(=S) ausgewählte Gruppe ersetzt sein können,
R⁵ für Wasserstoff, Halogen, OH, CN, NO₂, SH, NH₂, NH(C₁-C₄-Alkyl), N(C₁-C₄-Alkyl)₂, NH-SO₂-R^{x}, C₁-C₆-Alkyl, C₂-C₆-Alkenyl, C₂-C₆-Alkinyl, C₁-C₆-Alkoxy, C₃-C₆-Cycloalkyl, einen gesättigten oder teilweise ungesättigten drei-, vier-, fünf-, sechs-, sieben-, acht-, neun- oder zehngliedrigen Heterocyclus, fünf- oder sechsgliedriges Heteroaryl oder Aryl steht, wobei der Heterocyclus oder das Heteroaryl ein, zwei oder drei aus N, O und S ausgewählte Heteroatome enthält und wobei R^{x} wie oben definiert ist, und
wobei die aliphatischen Gruppierungen von R⁵ nicht weiter substituiert sind oder eine, zwei, drei oder bis zur maximal möglichen Anzahl an gleichen oder verschiedenen Gruppen R^{5a} tragen, die unabhängig voneinander ausgewählt sind aus:
R⁵ Halogen, OH, CN, C₁-C₆-Alkoxy, C₃-C₆-Cycloalkyl, C₃-C₆-Halogencycloalkyl, C₁-C₄-Halogenalkoxy, C₁-C₆-Alkylthio und Phenoxy, wobei die Phenylgruppe unsubstituiert ist oder einen, zwei, drei, vier oder fünf aus der aus Halogen, OH, C₁-C₄-Alkyl, C₁-C₄-Halogenalkyl, C₁-C₄-Alkoxy und C₁-C₄-Halogenalkoxy bestehenden Gruppe ausgewählte Substituenten R^{55a} trägt,
wobei die Cycloalkyl-, Heterocyclus-, Heteroaryl- und Arylgruppierungen von R⁵ nicht weiter substituiert sind oder eine, zwei, drei, vier, fünf oder bis zur maximal möglichen Anzahl an gleichen oder verschiedenen Gruppen R^{5b} tragen, die unabhängig voneinander ausgewählt sind aus:
R^{5b} Halogen, OH, CN, C₁-C₄-Alkyl, C₁-C₄-Alkoxy, C₁-C₄-Halogenalkyl, C₃-C₆-Cycloalkyl, C₃-C₆-Halogencycloalkyl, C₁-C₄-Halogenalkoxy und C₁-C₆-Alkyl thio,
R⁶ unabhängig aus den für R⁵ definierten Substituenten ausgewählt ist, wobei die möglichen Substituenten für R⁶ R^{6a}, R^{66a} bzw. R^{6b} sind, die R^{5a}, R^{55a} bzw. R^{5b} entsprechen,
oder R⁵ und R⁶ zusammen mit dem Kohlenstoffatom, an das sie gebunden sind (in Formel I mit C** markiert) einen gesättigten oder teilweise ungesättigten drei-, vier-, fünf-, sechs-, sieben-, acht-, neun- oder zehngliedrigen Carbo- oder Heterocyclus bilden, wobei der Heterocyclus ein, zwei, drei oder vier aus N, O und S ausgewählte Heteroatome enthält, und wobei der Carbocyclus oder Heterocyclus unsubstituiert ist oder einen, zwei, drei oder vier unabhängig voneinander aus Halogen, OH, CN, NO₂, SH, NH₂, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₄-Alkoxy-C₁-C₄-alkyl, Phenyl und Phenoxy ausgewählte Substituenten R⁵⁶ trägt, wobei die Phenylgruppen unsubstituiert sind oder einen, zwei, drei, vier oder fünf aus der aus Halogen, OH, C₁-C₄-Alkyl, C₁-C₄-Halogenalkyl, C₁-C₄-Alkoxy und C₁-C₄-Halogenalkoxy bestehenden Gruppe ausgewählte Substituenten R^{56a} tragen, und wobei jeweils eine oder zwei CH₂-Gruppen des Carbo- oder Heterocyclus durch eine unabhängig aus C(=O) und C(=S) ausgewählte Gruppe ersetzt sein können,
und
R⁷ und R⁸ zusammen mit den Kohlenstoffatomen, an die sie gebunden sind, ein fünf- oder sechsgliedriges Heteroaryl bilden, wobei das Heteroaryl ein, zwei oder drei aus N, O und S ausgewählte Heteroatome enthält und wobei das Heteroaryl null, eins, zwei, drei oder vier Substituenten (R⁷⁸)ₒ trägt, wobei
o für 0, 1, 2 oder 3 steht und
R⁷⁸ unabhängig aus Halogen, OH, CN, NO₂, SH, NH₂, NH(C₁-C₄-Alkyl), N(C₁-C₄-Alkyl)₂, NH(C(=O)-C₁-C₄-Alkyl), N(C(=O)-C₁-C₄-Alkyl)₂, NH-SO₂-R^{x}, CH(=O), C(=O)-C₁-C₆-Alkyl, C(=O)NH-(C₁-C₆-Alkyl), CR'=NOR", C₁-C₆-Alkyl, C₂-C₆-Alkenyl, C₂-C₆-Alkinyl, C₁-C₆-Alkoxy, C₁-C₆-Halogenalkoxy, C₂-C₆-Alkenyloxy, C₂-C₆-Alkinyloxy, C₃-C₆-Cycloalkyl, C₃-C₆-Cycloalkenyl, einem drei-, vier-, fünf- oder sechsgliedrigem gesättigten oder teilweise ungesättigten Heterocyclus, fünf- oder sechsgliedrigen Heteroaryl und Phenyl ausgewählt ist, wobei der Heterocyclus oder das Heteroaryl ein, zwei oder drei aus N, O und S ausgewählte Heteroatome enthält, wobei R' und R" unabhängig voneinander aus C₁-C₄-Alkyl, C₂-C₆-Alkenyl, C₂-C₆-Alkinyl, C₃-C₆-Cycloalkyl, einem gesättigten oder teilweise ungesättigten drei-, vier-, fünf-, sechs-, sieben-, acht-, neun- oder zehngliedrigen Heterocyclus, fünf- oder sechsgliedrigem Heteroaryl oder Aryl ausgewählt sind, wobei der Heterocyclus oder das Heteroaryl ein, zwei oder drei aus N, O und S ausgewählte Heteroatome enthält und wobei R' und/oder R" unabhängig voneinander unsubstituiert sind oder ein, zwei oder drei unabhängig voneinander aus Halogen, OH, CN, NO₂, SH, NH₂, NH(C₁-C₄-Alkyl), N(C₁-C₄-Alkyl)₂, NH-SO₂-R^{x}, C₁-C₆-Alkyl, C₁-C₆-Halogenalkyl, C₂-C₆-Alkenyl, C₂-C₆-Halogenalkenyl, C₂-C₆-Alkinyl, C₂-C₆-Halogenalkinyl, C₁-C₆-Alkoxy, C₁-C₆-Halogenalkoxy, C₃-C₆-Cycloalkyl, C₃-C₆-Halogencycloalkyl und Phenyl ausgewählte R'" tragen, und wobei R^{x} wie oben definiert ist,
und
wobei die aliphatischen Gruppierungen von R⁷⁸ nicht weiter substituiert sind oder eine, zwei, drei oder bis zur maximal möglichen Anzahl an gleichen oder verschiedenen Gruppen R^{78a} tragen, die unabhängig voneinander ausgewählt sind aus:
R^{78a} Halogen, OH, CN, C₁-C₆-Alkoxy, C₃-C₆-Cycloalkyl, C₃-C₆-Cycloalkenyl, C₃-C₆-Halogencycloalkyl, C₃-C₆-Halogencycloalkenyl, C₁-C₄-Halogenalkoxy, C₁-C₆-Alkylthio, fünf- oder sechsgliedrigem Heteroaryl, Phenyl und Phenoxy, wobei die Heteroaryl-, Phenyl- und Phenoxygruppe unsubstituiert ist oder einen, zwei, drei, vier oder fünf aus der aus Halogen, OH, C₁-C₄-Alkyl, C₁-C₄-Halogenalkyl, C₁-C₄-Alkoxy und C₁-C₄-Halogenalkoxy bestehenden Gruppe ausgewählte Substituenten R^{78a'} trägt,
wobei die alicyclischen, Phenyl-, heterocyclischen und Heteroarylgruppierungen von R⁷⁸ nicht weiter substituiert sind oder eine, zwei, drei, vier, fünf oder bis zur maximalen Anzahl an gleichen oder verschiedenen Gruppen R^{78b} tragen, die unabhängig voneinander ausgewählt sind aus:
R^{78b} Halogen, OH, CN, C₁-C₄-Alkyl, C₁-C₄-Alkoxy, C₁-C₄-Halogenalkyl, C₃-C₆-Cycloalkyl, C₃-C₆-Halogencycloalkyl, C₁-C₄-Halogenalkoxy und C₁-C₆-Alkylthio,
und die N-Oxide und die landwirtschaftlich unbedenklichen Salze davon.

2. Verbindungen nach Anspruch 1, wobei m für 0 steht.

3. Verbindungen nach Anspruch 1 oder 2, wobei n für 0 steht.

4. Verbindungen nach einem der Ansprüche 1 bis 3, wobei R³ und R⁴ unabhängig voneinander aus C₁-C₄-Alkyl ausgewählt sind.

5. Verbindungen nach einem der Ansprüche 1 bis 4, wobei R⁵ und R⁶ unabhängig voneinander aus Wasserstoff, F, Cl, Br und C₁-C₆-Alkyl ausgewählt sind oder R⁵ und R⁶ zusammen mit dem Kohlenstoffatom, an das sie gebunden sind, einen wie in Anspruch 1 definierten gesättigten drei-, vier-, fünf- oder sechsgliedrigen unsubstituierten oder substituierten Carbocyclus bilden.

6. Verbindungen nach einem der Ansprüche 1 bis 5, wobei o für 0 steht.

7. Verbindungen nach einem der Ansprüche 1 bis 5, wobei R⁶ und R⁷ zusammen mit den Kohlenstoffatomen, an die sie gebunden sind, ein fünfgliedriges Heteroaryl bilden, wobei das Heteroaryl ein, zwei oder drei aus N, O und S ausgewählte Heteroatome enthält und wobei das Heteroaryl null, eins, zwei, drei oder vier Substituenten (R⁷⁸)ₒ trägt.

8. Verbindungen nach einem der Ansprüche 1 bis 5, wobei R⁶ und R⁷ zusammen mit den Kohlenstoffatomen, an die sie gebunden sind, ein sechsgliedriges Heteroaryl bilden, wobei das Heteroaryl ein, zwei oder drei aus N, O und S ausgewählte Heteroatome enthält und wobei das Heteroaryl null, einen, zwei, drei oder vier Substituenten (R⁷⁸)ₒ trägt.

9. Verfahren zur Synthese von Verbindungen I nach Anspruch 1, wobei R⁵ und R⁶ für Halogen stehen, umfassend den Schritt der:
a) Umsetzung einer Verbindung IIA 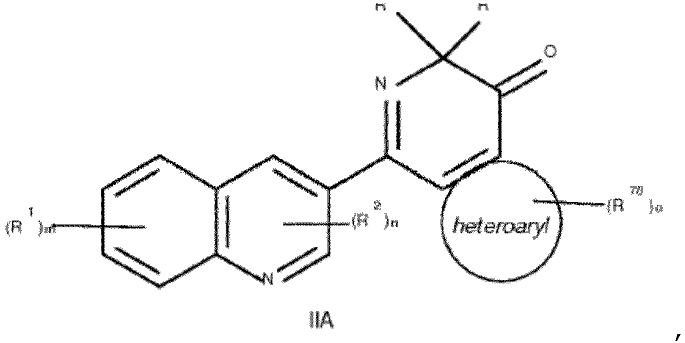 wobei das von R⁷ und R⁸ gebildete gegebenenfalls substituierte Heteroaryl durch einen als "Heteroaryl" bezeichneten Kreis wiedergegeben ist und wobei die Variablen wie für Verbindung I in einem der Ansprüche 1 bis 4 und 6 bis 8 definiert sind, mit einem Halogenierungsmittel.

10. Zwischenprodukte IIA, definiert wie in Anspruch 9.

11. Zusammensetzung, umfassend eine wie in einem der Ansprüche 1 bis 8 definierte Verbindung der Formel I, ein N-Oxid oder ein landwirtschaftlich unbedenkliches Salz davon.

12. Zusammensetzung nach Anspruch 11, die zusätzlich einen weiteren Wirkstoff umfasst.

13. Verwendung einer wie in einem der Ansprüche 1 bis 8 definierten Verbindung der Formel I und/oder eines landwirtschaftlich unbedenklichen Salzes davon oder einer der wie in einem der Ansprüche 11 oder 12 definierten Zusammensetzungen zur Bekämpfung phytopathogener Pilze, mit Ausnahme der Verwendung am menschlichen oder tierischen Körper.

14. Verfahren zur Bekämpfung phytopathogener Pilze, bei dem man die Pilze oder die gegen Pilzbefall zu schützenden Materialien, Pflanzen, den Boden oder Samen mit einer wirksamen Menge mindestens einer wie in einem der Ansprüche 1 bis 8 definierten Verbindung der Formel I oder mit einer wie in einem der Ansprüche 11 oder 12 definierten Zusammensetzung behandelt, mit Ausnahme therapeutischer Verfahren der Behandlung des menschlichen oder tierischen Körpers.

15. Saatgut, beschichtet mit mindestens einer wie in einem der Ansprüche 1 bis 8 definierten Verbindung der Formel I und/oder einem landwirtschaftlich unbedenklichen Salz davon oder mit einer wie in einem der Ansprüche 11 oder 12 definierten Zusammensetzung, in einer Menge von 0,1 bis 10 kg pro 100 kg Saatgut.

## Revendications

1. Composés de formule I 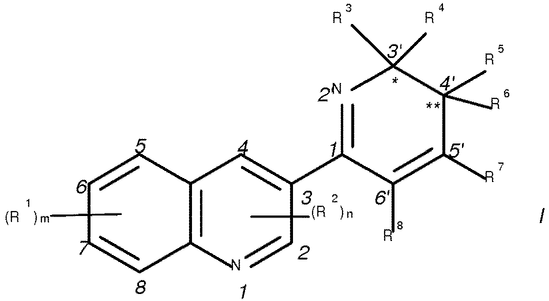 dans lesquels
m vaut 0, 1, 2, 3 ou 4 ;
R¹ est dans chaque cas choisi indépendamment parmi halogène, OH, CN, NO₂, SH, NH₂, NH(C₁-C₄-alkyl), N(C₁-C₄-alkyl)₂, NH-SO₂-R^{x}, C₁-C₆-alkyle, C₂-C₆-alcényle, C₂-C₆-alcynyle, C₁-C₆-alcoxy, C₃-C₆-cycloalkyle, hétéroaryle de cinq ou six chaînons et aryle ; où hétéroaryle contient un, deux ou trois hétéroatomes choisis parmi N, O et S ; et où
R^{x} est C₁-C₄-alkyle, C₁-C₄-halogénoalkyle, aryle non substitué ou aryle qui est substitué par un, deux, trois, quatre ou cinq substituants R^{x1} choisis indépendamment parmi C₁-C₄-alkyle ;
où les motifs aliphatiques de R¹ ne sont pas substitués davantage ou portent un, deux, trois ou jusqu'au nombre maximum possible de groupements R^{1a} identiques ou différents qui sont choisis indépendamment les uns des autres parmi :
R^{1a} : halogène, OH, CN, C₁-C₆-alcoxy, C₃-C₆-cycloalkyle, C₃-C₆-halogénocycloalkyle, C₁-C₄-halogéno-alcoxy, C₁-C₆-alkylthio et phénoxy, où le groupement phényle est non substitué ou porte un, deux, trois, quatre ou cinq substituants R^{11a} choisis dans le groupe constitué par halogène, OH, C₁-C₄-alkyle, C₁-C₄-halogénoalkyle, C₁-C₄-alcoxy et C₁-C₄-halogénoalcoxy ;
où les motifs cycloalkyle, hétéroaryle et aryle de R¹ ne sont pas substitués davantage ou portent un, deux, trois, quatre, cinq ou jusqu'au nombre maximum de groupements R^{1b} identiques ou différents qui sont choisis indépendamment les uns des autres parmi :
R^{1b} : halogène, OH, CN, C₁-C₄-alkyle, C₁-C₄-alcoxy, C₁-C₄-halogénoalkyle, C₃-C₆-cycloalkyle, C₃-C₆-halogénocycloalkyle, C₁-C₄-halogénoalcoxy et C₁-C₆-alkylthio ;
n vaut 0, 1 ou 2 ;
R² est dans chaque cas choisi indépendamment parmi les substituants tels que définis pour R¹, où les substituants possibles pour R² sont R^{2a} et R^{2b}, respectivement, qui correspondent à R^{1a} et R^{1b}, respectivement ;
R³, R⁴ sont choisis indépendamment parmi hydrogène, halogène, OH, CN, NO₂, SH, C₁-C₆-alkylthio, NH₂, NH(C₁-C₄-alkyl), N(C₁-C₄-alkyl)₂, NH-SO₂-R^{x}, C₁-C₆-alkyle, C₂-C₆-alcényle, C₂-C₆-alcynyle, C₁-C₆-alcoxy, C₁-C₆-halogénoalcoxy, un carbocycle ou hétérocycle saturé ou partiellement insaturé de trois, quatre, cinq, six, sept, huit, neuf, ou dix chaînons, où dans chaque cas un ou deux groupements CH₂ du carbo- et hétérocycle peuvent être remplacés par un groupement choisi indépendamment parmi C(=O) et C(=S), hétéroaryle de cinq ou six chaînons et aryle ; où l'hétérocycle et l'hétéroaryle contiennent indépendamment un, deux, trois ou quatre hétéroatomes choisis parmi N, O et S ;
où les motifs aliphatiques de R³ et R⁴ ne sont indépendamment pas substitués davantage ou portent un, deux, trois ou jusqu'au nombre maximum possible de groupements R^{3a} ou R^{4a} identiques ou différents, respectivement, qui sont choisis indépendamment les uns des autres parmi :
R^{3a}, R^{4a} : halogène, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, C₁-C₆-alcoxy, C₃-C₆-cycloalkyle, C₃-C₆-halogénocycloalkyle, C₁-C₄-halogénoalcoxy, C₁-C₆-alkylthio, aryle et phénoxy, où les groupements aryle et phényle sont indépendamment non substitués ou portent un, deux, trois, quatre ou cinq substituants choisis dans le groupe constitué par halogène, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, NH-SO₂-R^{x}, C₁-C₆-alkylthio, C₁-C₄-alkyle, C₁-C₄-halogénoalkyle, C₁-C₄-alcoxy et C₁-C₄-halogénoalcoxy ;
où les motifs carbocycliques, hétérocycliques, hétéroaryle et aryle de R³ et R⁴ ne sont indépendamment pas substitués davantage ou portent un, deux, trois, quatre, cinq ou jusqu'au nombre maximum de groupements R^{3b} ou R^{4b} identiques ou différents, respectivement, qui sont choisis indépendamment les uns des autres parmi:
R^{3b}, R^{4b} : halogène, OH, CN, NO₂, SH, NH₂, NH(C₁-C₄-alkyl), N(C₁-C₄-alkyl)₂, NH(C(=O)C₁-C₄-alkyl), N(C(=O)C₁-C₄-alkyl)₂, NH-SO₂-R^{x}, C₁-C₄-alkyle, C₁-C₄-alcoxy, C₁-C₄-halogénoalkyle, C₃-C₆-cycloalkyle, C₃-C₆-halogéno-cycloalkyle, C₁-C₄-halogénoalcoxy, C₁-C₆-alkylthio, C₁-C₆-halogénoalkylthio, C₁-C₄-alcoxy-C₁-C₄-alkyle, phényle et phénoxy, où les groupements phényle sont non substitués ou portent un, deux, trois, quatre ou cinq substituants choisis dans le groupe constitué par halogène, OH, C₁-C₄-alkyle, C₁-C₄-halogénoalkyle, C₁-C₄-alcoxy et C₁-C₄-halogénoalcoxy ;
et où R^{x} est tel que défini ci-dessus ;
ou R³, R⁴, conjointement avec l'atome de carbone auquel ils sont fixés (marqué par * dans la formule I), forment un carbocycle ou hétérocycle saturé ou partiellement insaturé de trois, quatre, cinq, six, sept, huit, neuf, ou dix chaînons ; où l'hétérocycle contient un, deux, trois ou quatre hétéroatomes choisis parmi N, O et S, où l'hétéroatome N peut porter un substituant R^{N} choisi parmi C₁-C₄-alkyle, C₁-C₄-halogénoalkyle et SO₂Ph, où Ph est phényle non substitué ou phényle qui est substitué par un, deux ou trois substituants choisis parmi C₁-C₄-alkyle, et où l'hétéroatome S peut se trouver sous la forme de son oxyde SO ou SO₂, et où le carbocycle ou l'hétérocycle est non substitué ou porte un, deux, trois ou quatre substituants R³⁴ choisis indépendamment parmi halogène, OH, CN, NO₂, SH, NH₂, C₁-C₆-alkyle, C₁-C₆-halogénoalkyle, C₁-C₆-alcoxy, C₁-C₆-halogénoalcoxy, C₁-C₆-alkylthio, C₁-C₆-halogénoalkylthio, C₁-C₄-alcoxy-C₁-C₄-alkyle, phényle et phénoxy, où les groupements phényle sont non substitués ou portent un, deux, trois, quatre ou cinq substituants R^{34a} choisis dans le groupe constitué par halogène, OH, C₁-C₄-alkyle, C₁-C₄-halogénoalkyle, C₁-C₄-alcoxy et C₁-C₄-halogénoalcoxy ; et où dans chaque cas un ou deux groupements CH₂ du carbo- ou hétérocycle peuvent être remplacés par un groupement choisi indépendamment parmi C(=O) et C(=S) ;
R⁵ est hydrogène, halogène, OH, CN, NO₂, SH, NH₂, NH (C₁-C₄-alkyl), N(C₁-C₄-alkyl)₂, NH-SO₂-R^{x}, C₁-C₆-alkyle, C₂-C₆-alcényle, C₂-C₆-alcynyle, C₁-C₆-alcoxy, C₃-C₆-cycloalkyle, hétérocycle saturé ou partiellement insaturé de trois, quatre, cinq, six, sept, huit, neuf, ou dix chaînons, hétéroaryle de cinq ou six chaînons ou aryle ; où l'hétérocycle ou hétéroaryle contient un, deux ou trois hétéroatomes choisis parmi N, O et S ; et où R^{x} est tel que défini ci-dessus ; et
où les motifs aliphatiques de R⁵ ne sont pas substitués davantage ou portent un, deux, trois ou jusqu'au nombre maximum possible de groupements R^{5a} identiques ou différents qui sont choisis indépendamment les uns des autres parmi :
R^{5a} : halogène, OH, CN, C₁-C₆-alcoxy, C₃-C₆-cycloalkyle, C₃-C₆-halogénocycloalkyle, C₁-C₄-halogéno-alcoxy, C₁-C₆-alkylthio et phénoxy, où le groupement phényle est non substitué ou porte un, deux, trois, quatre ou cinq substituants R^{55a} choisis dans le groupe constitué par halogène, OH, C₁-C₄-alkyle, C₁-C₄-halogénoalkyle, C₁-C₄-alcoxy et C₁-C₄-halogénoalcoxy ;
où les motifs cycloalkyle, hétérocycle, hétéroaryle et aryle de R⁵ ne sont pas substitués davantage ou portent un, deux, trois, quatre, cinq ou jusqu'au nombre maximum de groupements R^{5b} identiques ou différents qui sont choisis indépendamment les uns des autres parmi :
R^{5b} : halogène, OH, CN, C₁-C₄-alkyle, C₁-C₄-alcoxy, C₁-C₄-halogénoalkyle, C₃-C₆-cycloalkyle, C₃-C₆-halogénocycloalkyle, C₁-C₄-halogénoalcoxy et C₁-C₆-alkylthio ;
R⁶ est choisi indépendamment parmi les substituants tels que définis pour R⁵, où les substituants possibles pour R⁶ sont R^{6a}, R^{66a} et R^{6b}, respectivement, qui correspondent à R^{5a}, R^{55a} et R^{5b}, respectivement ;
ou R⁵ et R⁶, conjointement avec l'atome de carbone auquel ils sont fixés (marqué par C** dans la formule I), forment un carbo- ou hétérocycle saturé ou partiellement insaturé de trois, quatre, cinq, six, sept, huit, neuf, ou dix chaînons ; où l'hétérocycle contient un, deux, trois ou quatre hétéroatomes choisis parmi N, O et S, et où le carbocycle ou l'hétérocycle est non substitué ou porte un, deux, trois ou quatre substituants R⁵⁶ choisis indépendamment parmi halogène, OH, CN, NO₂, SH, NH₂, C₁-C₆-alkyle, C₁-C₆-halogénoalkyle, C₁-C₆-alcoxy, C₁-C₆-halogénoalcoxy, C₁-C₆-alkylthio, C₁-C₆-halogénoalkylthio, C₁-C₄-alcoxy-C₁-C₄-alkyle, phényle et phénoxy ; où les groupements phényle sont non substitués ou portent un, deux, trois, quatre ou cinq substituants R^{56a} choisis dans le groupe constitué par halogène, OH, C₁-C₄-alkyle, C₁-C₄-halogénoalkyle, C₁-C₄-alcoxy et C₁-C₄-halogénoalcoxy ; et où dans chaque cas un ou deux groupements CH₂ du carbo- ou hétérocycle peuvent être remplacés par un groupement choisi indépendamment parmi C(=O) et C(=S) ; et
R⁷ et R⁸, conjointement avec les atomes de carbone auxquels ils sont fixés, forment un hétéroaryle de cinq ou six chaînons ; où l'hétéroaryle contient un, deux ou trois hétéroatomes choisis parmi N, O et S, et où l'hétéroaryle porte zéro, un, deux, trois ou quatre substituants (R⁷⁸)ₒ, où
o vaut 0, 1, 2 ou 3 ; et
R⁷⁸ sont choisis indépendamment parmi halogène, OH, CN, NO₂, SH, NH₂, NH(C₁-C₄-alkyl), N(C₁-C₄-alkyl)₂, NH(C(=O)C₁-C₄-alkyl) , N(C(=O)C₁-C₄-alkyl)₂, NH-SO₂-R^{x}, CH(=O), C(=O)C₁-C₆-alkyle, C(=O)NH(C₁-C₆-alkyl), CR'=NOR'', C₁-C₆-alkyle, C₂-C₆-alcényle, C₂-C₆-alcynyle, C₁-C₆-alcoxy, C₁-C₆-halogénoalcoxy, C₂-C₆-alcényloxy, C₂-C₆-alcynyloxy, C₃-C₆-cycloalkyle, C₃-C₆-cycloalcényle, hétérocycle de trois, quatre, cinq ou six chaînons saturé ou partiellement insaturé, hétéroaryle de cinq ou six chaînons et phényle ; où l'hétérocycle ou l'hétéroaryle contient un, deux ou trois hétéroatomes choisis parmi N, O et S ; où R' et R" sont choisis indépendamment parmi C₁-C₄-alkyle, C₂-C₆-alcényle, C₂-C₆-alcynyle, C₃-C₆-cycloalkyle, hétérocycle saturé ou partiellement insaturé de trois, quatre, cinq, six, sept, huit, neuf, ou dix chaînons, hétéroaryle de cinq ou six chaînons ou aryle ; où l'hétérocycle ou l'hétéroaryle contient un, deux ou trois hétéroatomes choisis parmi N, O et S, et où R' et/ou R" sont indépendamment non substitués ou portent un, deux ou trois R"' choisis indépendamment parmi halogène, OH, CN, NO₂, SH, NH₂, NH(C₁-C₄-alkyl), N(C₁-C₄-alkyl)₂, NH-SO₂-R^{x}, C₁-C₆-alkyle, C₁-C₆-halogénoalkyle, C₂-C₆-alcényle, C₂-C₆-halogénoalcényle, C₂-C₆-alcynyle, C₂-C₆-halogénoalcynyle, C₁-C₆-alcoxy, C₁-C₆-halogénoalcoxy, C₃-C₆-cycloalkyle, C₃-C₆-halogénocycloalkyle et phényle ; et où R^{x} est tel que défini ci-dessus ; et
où les motifs aliphatiques de R⁷⁸ ne sont pas substitués davantage ou portent un, deux, trois ou jusqu'au nombre maximum possible de groupements R^{78a} identiques ou différents qui sont choisis indépendamment les uns des autres parmi :
R^{78a} : halogène, OH, CN, C₁-C₆-alcoxy, C₃-C₆-cycloalkyle, C₃-C₆-cycloalcényle, C₃-C₆-halogénocyclo-alkyle, C₃-C₆-halogénocycloalcényle, C₁-C₄-halogéno-alcoxy, C₁-C₆-alkylthio, hétéroaryle de cinq ou six chaînons, phényle et phénoxy, où le groupement hétéroaryle, phényle et phénoxy est non substitué ou porte un, deux, trois, quatre ou cinq substituants R^{78a'} choisis dans le groupe constitué par halogène, OH, C₁-C₄-alkyle, C₁-C₄-halogénoalkyle, C₁-C₄-alcoxy et C₁-C₄-halogénoalcoxy ;
où les motifs alicycliques, phényle, hétérocycliques et hétéroaryle de R⁷⁸ ne sont pas substitués davantage ou portent un, deux, trois, quatre, cinq ou jusqu'au nombre maximum de groupements R^{78b} identiques ou différents qui sont choisis indépendamment les uns des autres parmi :
R^{78b} : halogène, OH, CN, C₁-C₄-alkyle, C₁-C₄-alcoxy, C₁-C₄-halogénoalkyle, C₃-C₆-cycloalkyle, C₃-C₆-halogénocycloalkyle, C₁-C₄-halogénoalcoxy et C₁-C₆-alkylthio ;
ainsi que les N-oxydes et les sels acceptables sur le plan agricole de ceux-ci.

2. Composés selon la revendication 1, dans lesquels m vaut 0.

3. Composés selon la revendication 1 ou 2, dans lesquels n vaut 0.

4. Composés selon l'une quelconque des revendications 1 à 3, dans lesquels R³ et R⁴ sont choisis indépendamment parmi C₁-C₄-alkyle.

5. Composés selon l'une quelconque des revendications 1 à 4, dans lesquels R⁵ et R⁶ sont choisis indépendamment parmi hydrogène, F, Cl, Br et C₁-C₆-alkyle, ou R⁵ et R⁶, conjointement avec l'atome de carbone auquel ils sont fixés, forment un carbocycle saturé de trois, quatre, cinq ou six chaînons non substitué ou substitué tel que défini selon la revendication 1.

6. Composés selon l'une quelconque des revendications 1 à 5, dans lesquels o vaut 0.

7. Composés selon l'une quelconque des revendications 1 à 5, dans lesquels R⁶ et R⁷, conjointement avec les atomes de carbone auxquels ils sont fixés, forment un hétéroaryle de cinq chaînons ; où l'hétéroaryle contient un, deux ou trois hétéroatomes choisis parmi N, O et S, et où l'hétéroaryle porte zéro, un, deux, trois ou quatre substituants (R⁷⁸)ₒ.

8. Composés selon l'une quelconque des revendications 1 à 5, dans lesquels R⁶ et R⁷, conjointement avec les atomes de carbone auxquels ils sont fixés, forment hétéroaryle de six chaînons ; où l'hétéroaryle contient un, deux ou trois hétéroatomes choisis parmi N, O et S, et où l'hétéroaryle porte zéro, un, deux, trois ou quatre substituants (R⁷⁸)ₒ.

9. Procédé de synthèse de composés I selon la revendication 1, dans lesquels R⁵ et R⁶ sont halogène, comprenant l'étape
a) de réaction d'un composé IIA 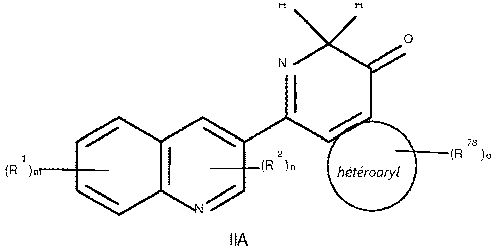 où l'hétéroaryle éventuellement substitué formé par R⁷ et R⁸ est esquissé par un cercle appelé « hétéroaryl » et où les variables sont telles que définies pour le composé I selon l'une quelconque des revendications 1 à 4 et 6 à 8, avec un agent d'halogénation.

10. Composés intermédiaires IIA, tels que définis selon la revendication 9.

11. Composition, comprenant un composé de formule I tel que défini selon l'une quelconque des revendications 1 à 8, un N-oxyde ou un sel acceptable sur le plan agricole de celui-ci.

12. Composition selon la revendication 11, comprenant en outre une autre substance active.

13. Utilisation d'un composé de formule I tel que défini selon l'une quelconque des revendications 1 à 8, et/ou d'un sel acceptable sur le plan agricole de celui-ci, ou des compositions telles que définies selon l'une quelconque des revendications 11 ou 12, pour lutter contre des champignons phytopathogènes, excepté l'utilisation dans l'organisme humain ou animal.

14. Méthode de lutte contre des champignons phytopathogènes, comprenant le traitement des champignons ou des matériaux, des plantes, du sol ou des semences devant être protégés contre une attaque fongique, par une quantité efficace d'au moins un composé de formule I tel que défini selon l'une quelconque des revendications 1 à 8, ou d'une composition telle que définie selon l'une quelconque des revendications 11 ou 12, excepté des méthodes thérapeutiques de traitement de l'organisme humain ou animal.

15. Semence, enrobée par au moins un composé de formule I tel que défini selon l'une quelconque des revendications 1 à 8, et/ou un sel acceptable sur le plan agricole de celui-ci, ou par une composition telle que définie selon l'une quelconque des revendications 11 ou 12, selon une quantité allant de 0,1 à 10 kg pour 100 kg de semences.
